# FASCICULE DE BREVET EUROPEEN

(11) **EP 2 205 593 B1**
(45) Date de publication et mention de la délivrance du brevet: **13.01.2016**
(21) Numéro de dépôt: 08862034.9
(22) Date de dépôt: 03.10.2008
(51) Int. Cl.: C07D 403/04, A61K 31/517

(54) **DÉRIVÉS DE QUINAZOLINEDIONE, LEUR PRÉPARATION ET LEURS APPLICATIONS THÉRAPEUTIQUES**
DERIVATE VON QUINAZOLINEDIONEN, VERFAHREN ZU DEREN HERSTELLUNG UND DEREN THERAPEUTISCHE ANWENDUNGEN
QUINAZOLINEDIONE DERIVATIVES, PREPARATION THEREOF AND THERAPEUTIC USES THEREOF

(30) Priorité: 03.10.2007 FR 0706931
(43) Date de publication de la demande: 14.07.2010
(73) Titulaire: SANOFI, 75008 Paris (FR)
(72) Inventeur: CLAUSS, Annie, 75013 Paris (FR); GLAESS, Christophe, F-75013 Paris (FR); MARCINIAK, Gilbert, F-75013 Paris (FR); MUZET, Nicolas, F-75013 Paris (FR); NAVE, Jean-François, F-75013 Paris (FR); SEYER, André, F-75013 Paris (FR); VIVET, Bertrand, F-75013 Paris (FR)
(74) Mandataire: Nony
(86) Numéro de dépôt international: PCT/FR2008/001384
(87) Numéro de publication internationale: WO 2009/077680

(56) Documents cités:
- WO-A-93/19068
- PERRY M J ET AL: "CHEMOTHERAPEUTIC POTENTIAL OF PHOSPHODIESTERASE INHIBITORS" CURRENT OPINION IN CHEMICAL BIOLOGY, CURRENT BIOLOGY LTD, LONDON, GB, vol. 2, no. 4, 1 janvier 1998 (1998-01-01), pages 472-481, XP000856012 ISSN: 1367-5931
- PITTS W J ET AL: "Identification of purine inhibitors of phosphodiesterase 7 (PDE7)" BIOORGANIC & MEDICINAL CHEMISTRY LETTERS, OXFORD, GB, vol. 14, no. 11, 7 juin 2004 (2004-06-07), pages 2955-2958, XP004841322 ISSN: 0960-894X
- LOWE J.A. ET AL: "Structure-Activity Relationship of Quinazolinedione Inhibitors of Calcium-Independant Phosphodiesterase" JOURNAL OF MEDICINAL CHEMISTRY, vol. 34, 1991, pages 624-628, XP002483510
- SALDOU N. ET AL: "Comparison of Recombinant Human PDE4 Isoforms: Interaction with Substrate and Inhibitors" CELLULAR SIGNALING, vol. 10, no. 6, 1998, pages 427-440, XP002483511

## Description

L'invention a pour objet des dérivés de quinazolinedione, leurs procédés d'obtention et leurs applications thérapeutiques.

L'invention concerne des dérivés de quinazolinedione, inhibiteurs de la phosphodiestérase 7 (PDE7). Certains de ces dérivés inhibent également la phosphodiestérase 8 (PDE8).

Les phosphodiestérases (PDEs) sont des enzymes intracellulaires responsables de l'hydrolyse des messagers secondaires AMPc (adénosine-3',5'-monophosphate cyclique) et GMPc (guanosine-3',5'-monophosphate cyclique) en nucléotides 5'-monophosphates inactifs. L'AMPc et le GMPc jouent un rôle essentiel dans les voies de signalisation cellulaire et interviennent dans de nombreux processus physiologiques.

L'inhibition des phosphodiestérases se traduit par une augmentation des concentrations intracellulaires d'AMPc et de GMPc, produisant l'activation spécifique de voies de phosphorylation impliquées dans des réponses fonctionnelles variées. L'augmentation des concentrations intracellulaires en AMPc ou en GMPc à l'aide d'inhibiteurs sélectifs de phosphodiestérases apparaît comme une approche prometteuse pour le traitement de diverses maladies (Bender et Beavo, Pharmacol Rev (2006) 58, 488-520). Les inhibiteurs de phosphodiestérases présentent donc un intérêt comme agents thérapeutiques et comme outils pharmacologiques.

A ce jour, onze familles de phosphodiestérases ont été identifiées. Elles se distinguent par leur structure primaire, leur spécificité de substrat et leur sensibilité vis-à-vis de divers effecteurs et inhibiteurs spécifiques de PDEs. Chaque famille est constituée d'un ou plusieurs gènes qui sont exprimés dans différents tissus sous forme de variants d'épissage (Bender et Beavo, Pharmacol Rev (2006) 58, 488-520; Lugnier, Pharmacol Therapeut (2006) 109, 366-398).

Les PDE4, 7, et 8 hydrolysent spécifiquement l'AMPc et les PDE5, 6 et 9 le GMPc.

La famille PDE7 est représentée par les isoformes PDE7A et PDE7B, provenant de deux gènes distincts.

La PDE7A humaine (Michaeli et al, J Biol Chem (1993) 268, 12925-12932; Han et al, J Biol Chem (1997) 272, 16152-16157 ; Wang et al, Biochem Biophys Res Commun (2000) 276, 1271-1277) et la PDE7B humaine (Sasaki et al, Biochem Biophys Res Commun (2000), 271, 575-583 ; Gardner et al, Biochem Biophys Res Commun (2000) 272, 186-192) hydrolysent sélectivement l'AMPc avec des constantes de Michaelis (Km) de 0.1 à 0.2 µM et de 0.13 à 0.2 µM, respectivement. La partie catalytique de la PDE7B présente environ 67% d'homologie avec celle de la PDE7A.

Trois variants d'épissage sont connus pour la PDE7A. Les PDE7A1 et PDE7A3 sont exprimées principalement dans les cellules du système immunitaire et des poumons alors que la PDE7A2 est surtout exprimée dans les muscles du squelette, le coeur et les reins. Pour la PDE7B, trois variants ont également été récemment identifiés (Giembycz et Smith, Drugs Future (2006) 31, 207-229).

Les profils de distribution tissulaire de PDE7A et PDE7B sont très différents suggérant que ces deux isoformes ont des fonctions distinctes du point de vue physiologique. Alors que la PDE7A est abondamment exprimée dans les cellules hématopoïétiques, les poumons, le placenta, les cellules de Leydig, la rate, les tubes collecteurs des reins et les surrénales, une forte expression de PDE7B est détectée dans le pancréas, le coeur, la thyroïde et les muscles du squelette (Giembycz et Smith, Drugs Future (2006) 31, 207-229). Une co-expression des RNA messagers (RNAm) de la PDE7A et de la PDE7B est cependant observée dans certains tissus. C'est le cas dans les ostéoblastes (Ahlstrom et al, Cell Mol Biol Lett (2005) 10, 305-319) et dans certaines régions du cerveau : plusieurs zones du cortex, le gyrus denté, la plupart des composants du système olfactif, le striatum, de nombreux noyaux du thalamus et les cellules pyramidales de l'hippocampe (Miro et al, Synapse (2001) 40, 201-214; Reyes-Irisarri et al, Neuroscience (2005) 132, 1173-1185). Par contre, dans certaines zones du cerveau, seulement une des deux isoformes est exprimée. Ainsi, seuls les RNAm de PDE7A sont présents dans de nombreux noyaux du tronc cérébral. De même, les RNAm de PDE7B sont présents en fortes concentrations dans le noyau accumbens et le noyau dorsal moteur du nerf vague alors que les RNAm de PDE7A n'y sont pas détectés (Miro et al, Synapse (2001) 40, 201-214; Reyes-Irisarri et al, Neuroscience (2005) 132, 1173-1185).

La protéine PDE7A1 est bien exprimée dans les lymphocytes T du sang, les lignées de cellules épithéliales des bronches, les fibroblastes du poumon et les éosinophiles (Smith et al, Am J Physiol Lung Cell Mol Physiol (2003) 284 : L279-L289). Plusieurs rapports suggèrent que la PDE7A pourrait jouer un rôle dans l'activation des lymphocytes T (Li et al, Science (1999) 283, 848-851; Glavas et al, PNAS (2001) 98, 6319-6324; Nakata et al, Clin Exp Immunol, (2002) 128, 460-466; Smith et al, Mol Pharmacol (2004) 66, 1679-1689). La protéine PDE7A1 est aussi exprimée dans des cellules qui jouent un rôle central dans la pathogenèse de l'asthme et de la broncho-pneumopathie chronique obstructive (COPD), tels que les lymphocytes T (CD4+ et CD8+), les monocytes, les neutrophiles, les macrophages alvéolaires, les cellules des muscles lisses des voies aériennes et des vaisseaux pulmonaires (Smith et al, Am J Physiol Lung Cell Mol Physiol (2003) 284 : L279-L289). La localisation de la PDE7A dans les cellules pro-inflammatoires et les cellules du système immunitaire et son rôle potentiel dans l'activation des lymphocytes T suggèrent que des inhibiteurs sélectifs de PDE7 pourraient avoir des applications dans le domaine des maladies liées aux lymphocytes T et dans celui des maladies des voies pulmonaires.

La protéine PDE7A est aussi présente dans les lymphocytes B et dans la lignée cellulaire de lymphocytes B WSU-CLL provenant d'un patient atteint d'une leucémie lymphocytaire chronique. Le traitement des cellules WSU-CLL par l'IC242, un inhibiteur spécifique de PDE7, augmente l'expression de la PDE7A (Lee et al, Cell Signal (2002) 14, 277-284). Par ailleurs, il a été montré que l'expression de la protéine PDE7B est environ 5 à 90 fois plus élevée dans les cellules mononuclées du sang périphérique (PBMC) de patients atteints de leucémie lymphocytaire chronique (CLL-PBMC) que dans les PBMC isolées du sang de sujets normaux (WO 2007/067946). Le BRL-50481, un inhibiteur sélectif de PDE7, induit de manière dose-dépendante l'apoptose des CLL-PBMC mais il est sans effet sur les PBMC de sujets normaux. Ces observations suggèrent que des inhibiteurs sélectifs de PDE7 pourraient être efficaces dans le traitement de ce type de leucémie.

Les inhibiteurs sélectifs de PDE4 augmentent la densité minérale osseuse chez le rat et la souris et leurs effets semblent liés à une diminution de l'activité des ostéoclastes et à une augmentation de celle des ostéoblastes (Miyamoto et al, Biochem Pharmacol (1997) 54, 613-617 ; Waki et al, Jpn J Pharmacol (1999) 79, 477-483 ; Kinoshita et al, Bone (2000) 27, 811-817). Une activité PDE7 a été détectée dans les ostéoblastes (Ahlström et al, Cell Mol Biol Lett (2005) 10, 305-319). En augmentant la concentration intracellulaire d'AMPc, les inhibiteurs de PDE7, comme les inhibiteurs de PDE4, pourraient s'avérer efficaces dans le traitement de l'ostéopénie et de l'ostéoporose.

Par ailleurs, des études récentes (WO2006/092691, WO2006/092692) rapportent les activités pharmacologiques de divers inhibiteurs de PDE7 dans des modèles de douleur neuropathique chez le rat, suggèrant des applications dans le traitement de divers types de douleurs et plus particulièrement dans celui de la douleur neuropathique.

La présente invention a en particulier pour objet des dérivés de quinazolinedione, puissants inhibiteurs de PDE7, ou inhibiteurs de PDE7 et de PDE8 selon les dérivés, leur préparation et leurs applications thérapeutiques.

L'invention a pour objet des composés de formule générale (I) qui suit : 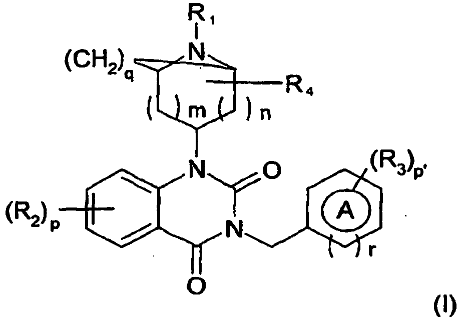 dans laquelle
- A représente un groupe aryle ou un groupe hétéroaryle;
- R₁ représente :
   ▪ un atome d'hydrogène,
   ▪ -C(O)R dans lequel R est un atome d'hydrogène, un groupe (C₁-C₆) alcoxy, un groupe aryle, un groupe (C₃-C₆) cycloalkyle, ou un groupe (C₁-C₆) alkyle, ledit alkyle étant éventuellement substitué par :
      - un ou plusieurs groupe(s) hydroxy,
      - un groupe benzyloxy,
      - un groupe (C₁-C₆) alcoxy, éventuellement substitué par un aryle, ou
      - un groupe (C₃-C₆) cycloalkyle,
   ▪ un groupe (C₁-C₆) alkyle éventuellement substitué;
- R₂ représente :
   ▪ un atome d'hydrogène,
   ▪ un atome d'halogène,
   ▪ un groupe cyano,
   ▪ un groupe nitro,
   ▪ un groupe (C₁-C₆) alkyle éventuellement substitué par un -NH₂, ou bien par un groupe -NHC(O)Rb,
   ▪ un groupe -ORa dans lequel Ra représente :
      - un atome d'hydrogène,
      - un groupe (C₁-C₆) alkyle éventuellement substitué par un ou plusieurs atome(s) d'halogène, par un ou plusieurs groupe(s) hydroxy, par un groupe aryle et/ou par un ou plusieurs groupe(s) cyano,
      - un groupe (C₂-C₆) alcynyle,
      - un groupe aryle ;
- R₃ représente :
   ▪ un atome d'hydrogène,
   ▪ un atome d'halogène,
   ▪ un groupe hydroxy,
   ▪ un groupe cyano,
   ▪ un groupe -SCF₃,
   ▪ un groupe nitro,
   ▪ un groupe oxo,
   ▪ un groupe -S(O)₀₋₂-alkyle, un groupe -S(O)₀₋₂-hétérocycloalkyle, un groupe -0-SO₂-aryle éventuellement substitué par un ou plusieurs atome(s) d'halogène ;
   ▪ un groupe -alkyl-amino-alkyle ou -cycloalkyl-arhino-alkyle, chacun éventuellement substitué sur l'alkyle terminal,
   ▪ un groupe sulfonamide éventuellement substitué,
   ▪ un groupe aryle ou un groupe hétéroaryle, ledit groupe étant monocyclique ou polycyclique et étant de plus éventuellement substitué par un groupe (C₁-C₆) alkyle, par un ou plusieurs atome(s) d'halogène, ou par un groupe (C₁-C₆) alcoxy,
   ▪ un groupe hétérocycloalkyle éventuellement substitué par un groupe (C₁-C₆) alkyle,
   ▪ un groupe (C₁-C₆) alkyle éventuellement substitué par :
      - un ou plusieurs atome(s) d'halogène,
      - un groupe aryle pouvant être substitué par un ou plusieurs atome(s) d'halogène, ou par un ou plusieurs groupe(s) hydroxy,
      - un groupe hétéroaryle,
      - un ou plusieurs groupe(s) hydroxy pouvant être substitué(s) par un groupe aryle lui-même éventuellement substitué par un ou plusieurs atome(s) d'halogène, ou
      - un groupe hétérocycloalkyle éventuellement sunstitué par un groupe CO(O)Ra, ou par un groupe (C₁-C₆) alkyle,
   ▪ un groupe -C(O)NRbRc,
   ▪ un groupe -C(O)ORc, ou un groupe -O-C(O)ORc
   ▪ un groupe (C₁-C₆) alcoxy, éventuellement substitué par
      - un groupe amino-alkyle,
      - un groupe amino-cycloalkyle,
      - un groupe cycloalkyle,
      - un groupe hétérocycloalkyle
      - un groupe hétéroaryle monocyclique ou polycyclique,
      - un ou plusieurs groupement(s) hydroxy,
      - un ou plusieurs atome(s) d'halogène,
      - un groupe (C₁-C₆) alcoxy,
      - un groupe -C(O)ORc,
      - un groupe -C(O)NRbRc,
      - un groupe oxo, et/ou
      - un groupe aryle, lui-même éventuellement substitué par un ou plusieurs atome(s) d'halogène, un groupe cyano, un groupe (C₁-C₆) alcoxy, un groupe -O-halogénoalkyle et/ou par un groupe halogénoalkyle,
   ▪ un groupe -O-cycloalkyle, un groupe -O-aryle, ou un groupe -O-hétérocycloalkyle, chacun éventuellement substitué par
      - un groupe aryle, lui-même éventuellement substitué par un ou plusieurs atome(s) d'halogène, ou par un groupe (C₁-C₆) alkyle,
      - un groupe oxo,
      - un ou plusieurs atome(s) d'halogène, et/ou
      - un groupe (C₁-C₆) alkyle, lui-même pouvant être substitué par un groupe aryle et/ou un groupe oxo,
   ▪ un groupe -NH-CO-NH-aryle, un groupe -NH-CO-NH-hétéroaryle, ou un groupe -NH-CO-NH-(C₁-C₆) alkyle, chacun étant éventuellement substitué par un ou plusieurs atome(s) d'halogène, par un groupe cyano, par un groupe nitro, par un ou plusieurs groupe(s) hydroxy, ou par un groupe (C₁-C₆) alcoxy,
   ▪ un groupe -N-(C₁-C₆) alkyle, le groupe (C₁-C₆) alkyle pouvant être substitué par
      - un ou plusieurs groupe(s) oxo, et/ou
      - un ou plusieurs groupe(s) aryle éventuellement substitué(s) par un ou plusieurs atome(s) d'halogène et/ou par un groupe SO₂,
   ▪ un groupe -NH-CO-aryle, un groupe -NH-CO-hétéroaryle, chacun étant éventuellement substitué par un ou plusieurs atome(s) d'halogène ;
      ou bien R₃ forme avec A un groupe hétéroaryle polycyclique éventuellement substitué par un groupe (C₁-C₆) alcoxy, un groupe (C₁-C₆) alkyle éventuellement substitué par un groupe aryle pouvant lui-même être substitué par un ou plusieurs atome(s) d'halogène ;
- R₄ représente un atome d'hydrogène, un groupe oxo, ou un groupe (C₁-C₆) alkyle ;
- Rb représente :
   - un atome d'hydrogène,
   - un groupe (C₁-C₆) alkyle éventuellement substitué par un ou plusieurs atome(s) d'halogène, par un ou plusieurs groupe(s) hydroxy, cyano, amino, hétérocycloalkyle, (C₁-C₆) alcoxy, ou par un groupe aryle éventuellement substitué par un ou plusieurs atome(s) d'halogène,
   - un groupe (C₃-C₆) cycloalkyle,
   - un groupe (C₂-C₆) alcynyle,
   - un groupe (C₁-C₆) alcoxy,
   - un groupe aryle éventuellement substitué par un ou plusieurs atome(s) d'halogène;
- Rc représente un atome d'hydrogène, ou un groupe (C₁-C₆) alkyle éventuellement substitué par un ou plusieurs atome(s) d'halogène ;
   ou alors Rb et Rc forment ensemble avec l'atome d'azote auquel ils sont attachés un groupe hétéroaryle polycyclique, ou un groupe hétérocycloalkyle ;
- m et n représentent indépendamment l'un de l'autre la valeur 0, 1 ou 2, étant entendu que m+n ≤ 3 ;
- p et p' représentent indépendamment l'un de l'autre la valeur 1, 2 ou 3, étant entendu que lorsque p est supérieur ou égal à 2, alors les groupes R₂ sont sur des atomes de carbone distincts et peuvent être différents les uns des autres, et lorsque p' est supérieur ou égal à 2 alors les groupes R₃ sont sur des atomes de carbone distincts et peuvent être différents les uns des autres;
- q représente la valeur 0 ou 2, étant entendu que lorsque q = 0, alors le groupe hétérocyclique azoté rattaché à l'azote situé en position 1 du noyau 2,4-dioxo-1,2,3,4 tétrahydroquinazoline n'est plus ponté et est du type : 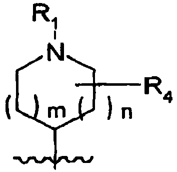
- r représente la valeur 0 ou 1.

Les composés de formule générale (I) peuvent comporter un ou plusieurs carbones asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

De par leur structure, les composés de formule générale (I) peuvent également exister sous forme d'isomères de type rotamère ou d'atropoisomère.

Les composés de formule (I) peuvent également exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou la séparation des composés de formule générale (I) font également partie de l'invention.

Les composés de formule générale (I) peuvent également se trouver sous forme cristalline, amorphe ou huileuse, ces formes faisant partie de l'invention.

Les composés de formule générale (I) peuvent, en outre, se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Selon la présente invention, les N-oxydes des composés comportant une amine font également partie de l'invention.

Les composés de formule (I) selon la présente invention comprennent également ceux dans lesquels un ou plusieurs atomes d'hydrogène, de carbone ou d'halogène, notamment de chlore ou de fluor ont été remplacés par leurs isotopes radioactifs, par exemple le tritium pour remplacer l'hydrogène ou le carbone 14 pour remplacer le carbone 12. De tels composés marqués sont utiles dans des travaux de recherche, de métabolisme ou de pharmacocinétique, dans des essais biologiques et pharmacologiques en tant qu'outils.

Dans le cadre de l'invention, on définit ce qui suit :
- dans (C₁-C₆), les indices numériques déterminent le nombre possible d'atomes de carbone présents dans une chaîne ou un cycle. Ainsi, à titre d'exemple, C₁-C₆ représente une chaîne carbonée pouvant avoir de 1 à 6 atomes de carbone. De même, à titre d'exemple, (C₁-C₅) représente une chaîne carbonée qui peut avoir de 1 à 5 atomes de carbone, ou encore (C₃-C₆) peut représenter un cycle carboné saturé pouvant avoir de 3 à 6 atomes de carbone ;
- alcoxy: un groupe -O-alkyle à chaîne aliphatique saturée, linéaire ou ramifiée;
- alcynyle : un groupe aliphatique mono- ou poly-insaturé, linéaire ou ramifié, comprenant par exemple une ou deux insaturations acétyléniques. Par exemple, un groupe (C₂-C₆) alcynyle peut représenter un ethynyle, propynyle ...;
- alkyle : un groupe aliphatique saturé, linéaire ou ramifié; par exemple, un groupe (C₁-C₆) alkyle représente une chaîne carbonée de 1 à 6 atomes de carbone, linéaire ou ramifiée, notamment un méthyle, éthyle, propyle, isopropyle, butyle, isobutyle, secbutyle, tertbutyle, pentyle ;
- aminoalkyle : un groupe -NH(C₁-C₆) alkyle, ou encore -N((C₁-C₆) alkyle)₂ ;
- aryle : un système aromatique monocyclique éventuellement substitué comprenant de 5 à 14 chaînons par cycle, de préférence de 5 à 10 chaînons par cycle. A titre d'exemples de groupes aryles monocycliques, on peut citer le phényle, ou le naphtyle ; le groupe aryle peut être substitué par un groupe pouvant être un ou plusieurs atome(s) d'halogène, un groupe hydroxy, un groupe cyano, un groupe trifluorométhylthio, un groupe nitro, un groupe alkyle, un groupe alcoxy, un groupe alkylthio, un groupe méthylsulfonyle, un groupe alkyl-amino-alkyle ou alkyl-amino-cycloalkyle éventuellement substitué, un groupe alkyl-amino-alcoxy ou cycloalkyl-amino-alcoxy ou un groupe sulfonamide, par exemple ;
- aryle polycyclique : un système aromatique polycyclique éventuellement substitué comprenant de 5 à 14 chaînons par cycle, de préférence de 5 à 10 chaînons par cycle, et comprenant de 2 à 10 cycles, dont au moins un des cycles est aromatique. A titre d'exemple de groupe aryles polycycliques, on peut citer l'acéanthrylène, l'anthracène, l'azulène, le coronène, le rubicène, le naphtalène ; le groupe aryle polycyclique peut être substitué par un groupe pouvant être un ou plusieurs atome(s) d'halogène, un groupe hydroxy, un groupe cyano, un groupe trifluorométhylthio, un groupe nitro, un groupe alkyle, un groupe alcoxy, un groupe alkylthio, un groupe méthylsulfonyle, un groupe alkyl-amino-alkyle ou alkyl-amino-cycloalkyle éventuellement substitué, un groupe alkyl-amino-alcoxy ou cycloalkyl-amino-alcoxy ou un groupe sulfonamide, par exemple ;
- un cycle ponté : une structure bicyclique, comprenant selon l'invention un atome d'azote, dans laquelle au moins 2 atomes de carbone sont reliés par une liaison simple ou une chaîne carbonée pouvant comporter 2 atomes de carbone. A titre d'exemple, le cycle précité est du type : 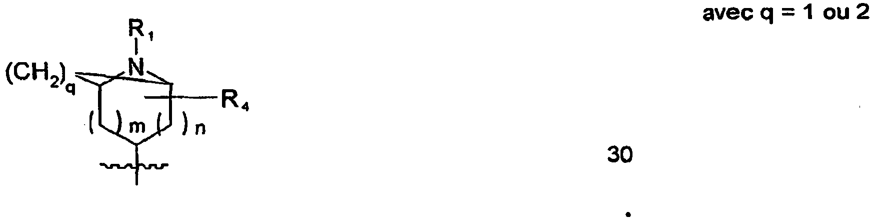
- cycloalkyle : un groupe aliphatique cyclique saturé comprenant de 3 à 8 atomes de carbone. A titre d'exemple, on peut citer les groupes cyclopropyle, méthylcyclopropyle, cyclobutyle, cyclopentyle, cyclohexyle ;
- halogène: un fluor, un chlore, un brome ou un iode ;
- halogénoalkyle : (C₁-C₆) alkyle substitué par un à trois atome(s) d'halogène;
- hétéroaryle : un système aromatique monocyclique comprenant de 5 à 14 chaînons, de préférence de 5 à 10 chaînons et comprenant un à plusieurs hétéroatomes tels que les atomes d'azote, d'oxygène ou de soufre. Les atomes d'azote peuvent être sous forme de N-oxydes. Par exemple, un hétérocycle monocyclique peut être un pyrane, une pyrazine, un pyrazole, une pyridazine, une pyridine, une pyrimidine, un pyrrole, un isothiazole, un isoxazole, un furane, un imidazole, une morpholine, un thiophène, une pipérazine, une diazétidine, une dihydropyrrolidine, une pipéridine, ou une azépine, etc ; un hétérocycle bicyclique peut être une isoquinoléine, une ptéridine, un chromane, etc ; un hétérocycle tricyclique peut être une phénanthroline, un xanthène, etc
- hétéroaryle polycyclique: un système aromatique polycyclique éventuellement substitué comprenant de 5 à 14 chaînons par cycle, de préférence de 5 à 10 chaînons par cycle, et comprenant de 2 à 10 cycles, comprenant de plus un à plusieurs hétéroatomes tels que les atomes d'azote, d'oxygène ou de soufre sur au moins l'un des cycles, et dont au moins des cycles est aromatique. A titre d'exemple de groupe hétéroaryles polycycliques, on peut citer l'indole, le benzofurane, le benzimidazole, le benzothiophène, le benzotriazole, le benzothiazole, la benzoxazole, la quinoline, l'isoquinoline, l'indazole, la quinazoline, la phthalazine, la quinoxaline, la naphtyridine, le 2,3-dihydro-1H-indole, le 2,3-dihydro-benzofurane, le 2,3-dihydro-indène, la tétrahydroquinoline, la tétrahydroisoquinoline, la tétrahydroisoquinazoline ;
- un hétérocycloalkyle: un cycle saturé, éventuellement substitué, comprenant de 3 à 8 atomes et comprenant un à plusieurs hétéroatomes tels que les atomes d'azote, d'oxygène ou de soufre sur au moins l'un des cycles, ou plusieurs hétéroatomes identiques ou différents entre eux. Par exemple, un hétérocycloalkyle peut être une pyrrolidine, une morpholine, une pipérazine, une diazétidine, une dihydropyrrolidine, une pipéridine, une pipéradine, une azépane, une imidazolidine, thiomorpholine, tétrahydropyrane, tétrahydrothiopyrane, pipérazine, diazépane, etc ;
- hydroxy : un groupe -OH ;
- nitro : un groupe -NO₂ ;
- oxo : un groupe -C(O)- ;
- sulfonamide : groupe répondant à la formule SO₂-N-alkyl ou SO₂-N-cycloalkyl, alkyl et cycloalkyl étant tels que définis ci-dessus ;
- trifluorométhylthio est défini par la formule -S-CF₃,

Il est de plus entendu que dans la présente description, lorsqu'un atome ou un groupe est substitué ou éventuellement substitué par un ou plusieurs groupe(s) ou atome(s) définis, les substituants peuvent être identiques ou différents entre eux, et être portés le cas échéant par le même atome ou des atomes différents.

Parmi les composés objet de l'invention, on peut citer un groupe de composés de formule (I) dans laquelle A représente un groupe aryle, en particulier un phényle ou hétéroaryle, en particulier un groupe pyridyle, et tous les autres substituants et indices sont tels que définis dans la formule générale (I).

Parmi les composés objet de l'invention, on peut citer un groupe de composés de formule (I) dans laquelle q = 0, m et n = 1, et tous les autres substituants et indices sont tels que définis dans la formule générale (I).

Parmi les composés objet de l'invention, on peut citer un groupe de composés de formule (I), dans laquelle R₂ représente un groupe (C₁-C₆) alkyle, en particulier un méthyle, substitué par un groupe -NHC(O)Rb dans lequel Rb, les autres groupes et indices sont tels que définis pour le composé de formule générale (I).

Parmi les composés objet de l'invention, on peut citer un groupe de composés de formule générale (I), dans laquelle R₂ représente un groupe -ORa, le groupe Ra et tous les autres groupes étant tels que définis pour le composé de formule (I).

Parmi les composés objet de l'invention, on peut citer un groupe de composés de formule générale (I), dans laquelle R₂ est un atome d'halogène, ou un cyano, ou un hydrogène, ou un hydroxyle, ou un groupe (C₁-C₆) alkyle, éventuellement substitué par un -NH₂, ou bien par un groupe -NHC(O)Rb, les autres groupes étant tels que définis pour le composé de formule (I).

Parmi les composés objet de l'invention, on peut citer un groupe de composés de formule générale (I), dans laquelle A est un phényle, R₁ est un groupe -C(O)R dans lequel R représente un atome d'hydrogène, q est égal à 0, n et m valent 1, et R₂ est - ORa, Ra étant tel que défini dans la formule générale (I).

Parmi les composés objet de l'invention, on peut citer un groupe de composés de formule générale (I), dans laquelle A est un phényle, R₁ est un groupe -C(O)R dans lequel R représente un atome d'hydrogène, q est égal à 0, n et m valent 1, p est égal à 2, l'un des R₂ est -ORa, Ra étant tel que défini dans la formule générale (I), et l'autre des R₂ est un atome d'halogène.

Parmi les composés objet de l'invention, on peut citer un groupe de composés de formule générale (I), dans laquelle A est un phényle, R₁ est un groupe -C(O)R dans lequel R représente un atome d'hydrogène, q est égal à 0, n et m valent 1, et R₂ est un méthyle substitué par un groupe -NH-CO-Rb, Rb étant tel que défini dans la formule générale (I).

Avantageusement, dans les composés de formule (I), le groupe R₂ est en position 6 du noyau 2,4-dioxo-1,2,3,4 tétrahydroquinazoline. Les composés de formule (I) peuvent également avoir un groupe R2 en position 7 du noyau 2,4-dioxo-1,2,3,4 tétrahydroquinazoline.

Avantageusement, dans les composés de formule (I), p = 1 ou 2.

Dans un mode particulier de l'invention, lorsque p' = 2, alors les deux groupes R₃ sont en position 3 et 4 du noyau A et peuvent être différents l'un de l'autre.

Les combinaisons des groupes de composés de l'invention précités font également partie de l'invention à titre de modes de réalisation selon l'invention.

A titre d'exemple de composés préférés, on peut citer les composés suivants :
n°1 : 2-{[3-(3,4-diméthoxybenzyl)-1-(1-formylpipéridin-4-yl)-2,4-dioxo-1,2,3,4-tétrahydroquinazolin-6-yl]oxy}propanenitrile
n°2 : 1-(1-acétylpipéridin-4-yl)-3-(3,4-diméthoxybenzyl)-6-hydroxyquinazoline-2,4(1H,3H)-dione
n°3 : {[1-(1-acétylpipéridin-4-yl)-3-(3,4-diméthoxybenzyl)-2,4-dioxo-1,2,3,4-tétrahydroquinazolin-6-yl]oxy}acétonitrile
n°4 : 2-{[1-(1-acétylpipéridin-4-yl)-3-(3,4-diméthoxybenzyl)-2,4-dioxo-1,2,3,4-tétrahydroquinazolin-6-yl]oxy}propanenitrile
n°6 : {[3-(3,4-diméthoxybenzyl)-1-(1-formylpipéridin-4-yl)-2,4-dioxo-1,2,3,4-tétrahydroquinazolin-6-yl]oxy}acétonitrile
n°11 : 4-[3-(3,4-diméthoxybenzyl)-6-[2-fluoro-1-(fluorométhyl)éthoxy]-2,4-dioxo-3,4-dihydroquinazolin-1(2H)-yl]pipéridine-1-carbaldéhyde
n°12 :1-(1-acétylpipéridin-4-yl)-3-(3,4-diméthoxybenzyl)-6-[2-fluoro-1-(fluorométhyl)éthoxy]quinazoline-2,4(1H,3H)-dione
n°13 : 4-[3-(3,4-diméthoxybenzyl)-2,4-dioxo-6-(2,2,2-trifluoroéthoxy)-3,4-dihydroquinazolin-1(2H)-yl]pipéridine-1-carbaldéhyde
n°14 : 1-(1-acétylpipéridin-4-yl)-6-(2,2-difluoroéthoxy)-3-(3,4-diméthoxybenzyl)quinazoline-2,4(1H,3H)-dione
n°16 : 4-[6-(2,2-difluoroéthoxy)-3-(3,4-diméthoxybenzyl)-2,4-dioxo-3,4-dihydroquinazolin-1(2H)-yl]pipéridine-1-carbaldéhyde
n°20 : N-{[3-(3,4-diméthoxybenzyl)-1-(1-formylpipéridin-4-yl)-2,4-dioxo-1,2,3,4-tétrahydroquinazolin-6-yl]méthyl}acétamide
n°22 : chlorhydrate de 1-(1-acétylpipéridin-4-yl)-6-(aminométhyl)-3-(3,4-diméthoxybenzyl)quinazoline-2,4(1H,3H)-dione
n°23 : N-{[3-(3,4-diméthoxybenzyl)-1-(1-formylpipéridin-4-yl)-2,4-dioxo-1,2,3,4-tétrahydroquinazolin-6-yl]méthyl}formamide
n°24 : N-{[1-(1-acétylpipéridin-4-yl)-3-(3,4-diméthoxybenzyl)-2,4-dioxo-1,2,3,4-tétrahydroquinazolin-6-yl]méthyl}formamide
n°25: N-{[1-(1-acétylpipéridin-4-yl)-3-(3,4-diméthoxybenzyl)-2,4-dioxo-1,2,3,4-tétrahydroquinazolin-6-yl]méthyl}acétamide
n°32 : 4-[6-(2,2-difluoroéthoxy)-2,4-dioxo-3,4-dihydroquinazolin-1 (2H)-yl]pipéridine-1-carbaldéhyde
n°33 : 4-[3-(3,4-dichlorobenzyl)-6-(2,2-difluoroéthoxy)-2,4-dioxo-3,4-dihydroquinazolin-1 (2H)-yl]pipéridine-1-carbaldéhyde
n°34 : 4-[3-(4-chlorobenzyl)-6-(2,2-difluoroéthoxy)-2,4-dioxo-3,4-dihydroquinazolin-1 (2H)-yl]pipéridine-1-carbatdéhyde
n°35 : 4-{[6-(2,2-difluoroéthoxy)-1-(1-formylpipéridin-4-yl)-2,4-dioxo-1,4-dihydroquinazolin-3(2H)-yl]méthyl}benzoate de méthyle
n°36 : acide 4-{[6-(2,2-difluoroéthoxy)-1-(1-formylpipéridin-4-yl)-2,4-dioxo-1,4-dihydroquinazolin-3(2H)-yl]méthyl}benzoïque
n°37: 4-{[6-(2,2-difluoroéthoxy)-1-(1-formylpipéridin-4-yl)-2,4-dioxo-1,4-dihydroquinazolin-3(2H)-yl]méthyl}-N-(2-méthoxyéthyl)benzamide
n°38 4-[3-(3,4-diméthoxybenzyl)-6-méthyl-2,4-dioxo-3,4-dihydroquinazolin-1 (2H)-yl]pipéridine-1-carbaldéhyde
n°39 : 4-[6-(2,2-difluoroéthoxy)-3-(3-hydroxy-4-méthoxybenzyl)-2,4-dioxo-3,4-dihydroquinazolin-1(2H)-yl]pipéridine-1-carbaldéhyde
n°40 : 4-[6-(2,2-difluoroéthoxy)-3-[3-(2-hydroxyéthoxy)-4-méthoxybenzyl]-2,4-dioxo-3,4-dihydroquinazolin-1(2H)-yl]pipéridine-1-carbaldéhyde
n°41 : 4-[6-(2,2-difluoroéthoxy)-3-(3-éthoxy-4-méthoxybenzyl)-2,4-dioxo-3,4-dihydroquinazolin-1(2H)-yl]pipéridine-1-carbaldéhyde
n°42 : 4-[6-(2,2-difluoroéthoxy)-3-[4-méthoxy-3-(2-méthoxyéthoxy)benzyl]-2,4-dioxo-3,4-dihydroquinazolin-1(2H)-yl]pipéridine-1-carbaldéhyde
n°43 : 4-[6-(2,2-difluoroéthoxy)-3-(3,4-diméthoxybenzyl)-2,4-dioxo-3,4-dihydroquinazolin-1 (2H)-yl]azépane-1-carbaldéhyde
n°47 : 4-[6-(2,2-difluoroéthoxy)-3-[3-(3-hydroxypropoxy)-4-méthoxybenzyl]-2,4-dioxo-3,4-dihydroquinazolin-1 (2H)-yl]pipéridine-1-carbaldéhyde
n°48 : 4-[5-chloro-3-(3,4-diméthoxybenzyl)-2,4-dioxo-3,4-dihydroquinazolin-1 (2H)-yl]pipéridine-1-carbaldéhyde
n°49 : 4-{3-[3-(cyclopentyloxy)-4-méthoxybenzyl]-6-(2,2-difluoroéthoxy)-2,4-dioxo-3,4-dihydroquinazolin-1(2H)-yl}pipéridine-1-carbaldéhyde
n°50 : 2-(5-{[6-(2,2-difluoroéthoxy)-1-(1-formylpipéridin-4-yl)-2,4-dioxo-1,4-dihydroquinazolin-3(2H)-yl]méthyl}-2-méthoxyphénoxy)acétamide
n°51 : 4-[6-(2,2-difluoroéthoxy)-3-(3,4-diméthoxybenzyl)-2,4-dioxo-3,4-dihydroquinazolin-1(2H)-yl]-3-méthylpipéridine-1-carbaldéhyde
n°52 : 3-[6-(2,2-difluoroéthoxy)-3-(3,4-diméthoxybenzyl)-2,4-dioxo-3,4-dihydroquinazolin-1 (2H)-yl]-8-azabicyclo[3.2.1]octane-8-carbaldéhyde
n°56 : 4-{3-[4-(cyclopentyloxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°57 : 4-[3-(3-chlorobenzyl)-6-[2-fluoro-1-(fluorométhyl)éthoxy]-2,4-dioxo-3,4-dihydroquinazolin-1(2H)-yl]pipéridine-1-carbaldéhyde
n°58 : 4-[3-(4-chlorobenzyl)-6-[2-fluoro-1-(fluorométhyl)éthoxy]-2,4-dioxo-3,4-dihydroquinazolin-1(2H)-yl]pipéridine-1-carbaldéhyde
n°59 : 4-{3-[3-(cyclopentyloxy)-4-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°72 : 4-[3-(3,4-diméthoxybenzyl)-6-(2-hydroxyéthoxy)-2,4-dioxo-3,4-dihydroquinazolin-1 (2H)-yl]pipéridine-1-carbaldéhyde
n°74 : 4-[3-(3,4-dichlorobenzyl)-6-[2-fluoro-1-(fluorométhyl)éthoxy]-2,4-dioxo-3,4-dihydroquinazolin-1 (2H)-yl]pipéridine-1-carbaldéhyde
n°76 : 4-{3-[(6-chloropyridin-3-yl)méthyl]-6-[2-fluoro-1-(fluorométhyl)éthoxy]-2,4-dioxo-3,4-dihydroquinazolin-1(2H)-yl}pipéridine-1-carbaldéhyde
n°78 : 4-[3-(3-chloro-4-méthoxybenzyl)-6-[2-fluoro-1-(fluorométhyl)éthoxy]-2,4-dioxo-3,4-dihydroquinazolin-1(2H)-yl]pipéridine-1-carbaldéhyde
n°79 : 4-[3-(3,4-diméthoxybenzyl)-6-(2-fluoroéthoxy)-2,4-dioxo-3,4-dihydroquinazolin-1 (2H)-yl]pipéridine-1-carbaldéhyde
n ° 89: 2-[5-({6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]acétamide
n°90 : 4-{6-[2-fluoro-1-(fluorométhyl)éthoxy]-3-(3-hydroxy-4-méthoxybenzyl)-2,4-dioxo-3,4-dihydroquinazolin-1(2H)-yl}pipéridine-1-carbaldéhyde
n°91:4-[3-(3,4-diméthoxybenzyl)-6-éthoxy-2,4-dioxo-3,4-dihydroquinazolin-1 (2H)-y)]pipéridine-1-carbaldéhyde
n°97 : 4-[5,7-dichloro-3-(3,4-diméthoxybenzyl)-2,4-dioxo-3,4-dihydroquinazolin-1 (2H)-yl]pipéridine-1-carbaldéhyde
n° 102: 4-[7-chloro-3-(3,4-diméthoxybenzyl)-2,4-dioxo-3,4-dihydroquinazolin-1 (2H)-yl]pipéridine-1-carbaldéhyde
n° 108 : 4-{6-[2-fluoro-1-(fluorométhyl)éthoxy]-3-(3-fluoro-4-méthoxybenzyl)-2,4-dioxo-3,4-dihydroquinazolin-1 (2H)-yl}pipéridine-1-carbaldéhyde
n°111 : 4-[6-(difluorométhoxy)-3-(3,4-diméthoxybenzyl)-2,4-dioxo-3,4-dihydroquinazolin-1(2H)-yl]pipéridine-1-carbaldéhyde
n° 112 : 4-[3-(3,4-diméthoxybenzyl)-6-(1-méthyléthoxy)-2,4-dioxo-3,4-dihydroquinazolin-1(2H)-yl]pipéridine-1-carbaldéhyde
n° 114 : 4-{6-[2-fluoro-1-(fluorométhyl)éthoxy]-3-[4-méthoxy-3-(1-méthyléthoxy)benzyl]-2,4-dioxo-3,4-dihydroquinazolin-1(2H)-yl}pipéridine-1-carbaldéhyde
n° 116 : 4-{6-[2-fluoro-1-(fluorométhyl)éthoxy]-3-(3-méthoxybenzyl)-2,4-dioxo-3,4-dihydroquinazolin-1 (2H)-yl}pipéridine-1-carbaldéhyde
n° 117 : 4-{3-[3,5-bis(trifluorométhyl)benzyl]-6-[2-fluoro-1-(fluorométhyl)éthoxy]-2,4-dioxo-3,4-dihydroquinazolin-1(2H)-yl}pipéridine-1-carbaldéhyde
n° 118 : 4-[3-(3-éthoxybenzyl)-6-[2-fluoro-1-(fluorométhyl)éthoxy]-2,4-dioxo-3,4-dihydroquinazolin-1 (2H)-yl]pipéridine-1-carbaldéhyde
n° 124 : 4-{3-[3-chloro-4-(2-méthoxyéthoxy)benzyl]-6-[2-fluoro-1-(fluorométhyl)éthoxy]-2,4-dioxo-3,4-dihydroquinazolin-1(2H)-yl}pipéridine-1-carbaldéhyde
n° 130 : 4-[3-(3,4-diéthoxybenzyl)-6-[2-fluoro-1-(fluorométhyl)éthoxy]-2,4-dioxo-3,4-dihydroquinazolin-1 (2H)-yl]pipéridine-1-carbaldéhyde
n° 131 : 4-[3-(4-éthoxy-3-méthoxybenzyl)-6-[2-fluoro-1-(fluorométhyl)éthoxy]-2,4-dioxo-3,4-dihydroquinazolin-1(2H)-yl]pipéridine-1-carbaldéhyde
n°133: 4-{6-[2-fluoro-1-(fluorométhyl)éthoxy]-3-(4-méthoxy-3-méthylbenzyl)-2,4-dioxo-3,4-dihydroquinazolin-1(2H)-yl}pipéridine-1-carbaldéhyde
n°134 : 4-{6-[2-fluoro-1-(fluorométhyl)éthoxy]-2,4-dioxo-3-[4-(trifluorométhyl)benzyl]-3,4-dihydroquinazolin-1(2H)-yl}pipéridine-1-carbaldéhyde
n°135 : 4-{6-[2-fluoro-1-(fluorométhyl)éthoxy]-2,4-dioxo-3-[4-(trifluorométhyl)benzyl]-3,4-dihydroquinazolin-1(2H)-yl}pipéridine-1-carbaldéhyde
n° 143 : 4-{3-[4-(benzyloxy)-3-méthoxybenzyl]-6-[2-fluoro-1-(fluorométhyl)éthoxy]-2,4-dioxo-3,4-dihydroquinazolin-1 (2H)-yl}pipéridine-1-carbaldéhyde
n° 145 : 4-{6-[2-fluoro-1-(fluorométhyl)éthoxy]-3-(3-méthoxy-4-nitrobenzyl)-2,4-dioxo-3,4-dihydroquinazolin-1(2H)-yl}pipéridine-1-carbaldéhyde
n°155 : 4-[3-(4-éthoxybenzyl)-6-[2-fluoro-1-(fluorométhyl)éthoxy]-2,4-dioxo-3,4-dihydroquinazolin-1(2H)-yl]pipéridine-1-carbaldéhyde
n°158 : 4-{6-[2-fluoro-1-(fluorométhyl)éthoxy]-3-[4-(morpholin-4-ylméthyl)benzyl]-2,4-dioxo-3,4-dihydroquinazolin-1(2H)-yl}pipéridine-1-carbaldéhyde
n° 160 : 4-{6-[2-fluoro-1-(fluorométhyl)éthoxy]-3-(4-morpholin-4-ylbenzyl)-2,4-dioxo-3,4-dihydroquinazolin-1 (2H)-yl}pipéridine-1-carbaldéhyde
n°165 : 4-[3-(biphényl-4-ylméthyl)-6-[2-fluoro-1-(fluorométhyl)éthoxy]-2,4-dioxo-3,4-dihydroquinazolin-1(2H)-yl]pipéridine-1-carbaldéhyde
n° 166 : 4-{6-[2-fluoro-1-(fluorométhyl)éthoxy]-3-[4-(méthylsulfanyl)benzyl]-2,4-dioxo-3,4-dihydroquinazolin-1 (2H)-yl}pipéridine-1-carbaldéhyde
n°167 : 4-{6-[2-fluoro-1-(fluorométhyl)éthoxy]-2,4-dioxo-3-(4-pyridin-3-ylbenzyl)-3,4-dihydroquinazolin-1 (2H)-yl}pipéridine-1-carbaldéhyde
n°170 : 4-{6-[2-fluoro-1-(fluorométhyl)éthoxy]-3-(3-méthoxy-4-méthylbenzyl)-2,4-dioxo-3,4-dihydroquinazolin-1(2H)-yl}pipéridine-1-carbaldéhyde
n° 175 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]acétamide
n° 178 : 4-{6-[2-fluoro-1-(fluorométhyl)éthoxy]-3-(3-méthoxy-4-propoxybenzyl)-2,4-dioxo-3,4-dihydroquinazolin-1 (2H)-yl}pipéridine-1-carbaldéhyde
n°183 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthylacétamide
n° 184 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N,N-diméthylacétamide
n° 185 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthoxy-N-méthylacétamide
n° 186 : 4-{3-[4-(cyclopentyloxy)-3-éthoxybenzyl]-6-[2-fluoro-1-(fluorométhyl)éthoxy]-2,4-dioxo-3,4-dihydroquinazolin-1(2H)-yl}pipéridine-1-carbaldéhyde
n° 188 : 4-{3-[4-(cyclopentyloxy)-3-(1-méthyléthoxy)benzyl]-6-[2-fluoro-1-(fluorométhyl)éthoxy]-2,4-dioxo-3,4-dihydroquinazolin-1(2H)-yl}pipéridine-1-carbaldéhyde
n° 189 : 4-{3-[4-(cyclopentyloxy)-3-propoxybenzyl]-6-[2-fluoro-1-(fluorométhyl)éthoxy]-2,4-dioxo-3,4-dihydroquinazolin-1(2H)-yl}pipéridine-1-carbaldéhyde
n° 190 : 4-{3-[4-(cyclopentyloxy)-3-hydroxybenzyl]-6-[2-fluoro-1-(fluorométhyl)éthoxy]-2,4-dioxo-3,4-dihydroquinazolin-1(2H)-yl}pipéridine-1-carbaldéhyde
n° 193 : 4-{3-[4-(difluorométhoxy)-3-méthoxybenzyl]-6-[2-fluoro-1-(fluorométhyl)éthoxyl-2,4-dioxo-3,4-dihydroquinazolin-1(2H)-yl)pipéridine-1-carbaldéhyde
n°194 : 4-{3-[4-(difluorométhoxy)-3-éthoxybenzyl]-6-[2 fluoro-1-(fluorométhyl)éthoxy]-2,4-dioxo-3,4-dihydroquinazolin-1(2H)-yl}pipéridine-1-carbaldéhyde
n°200 : 4-{6-[2-fluoro-1-(fluorométhyl)éthoxy]-2,4-dioxo-3-(4-thiophén-3-ylbenzyl)-3,4-dihydroquinazolin-1(2H)-yl}pipéridine-1-carbaldéhyde
n°201 : 4-{6-[2-fluoro-1-(fluorométhyl)éthoxy]-2,4-dioxo-3-(4-pyridin-4-ylbenzyl)-3,4-dihydroquinazolin-1(2H)-yl}pipéridine-1-carbaldéhyde
n°203 : 4-{6-[2-fluoro-1-(fluorométhyl)éthoxy]-3-[(1-méthyl-1H-indol-6-yl)méthyl]-2,4-dioxo-3,4-dihydroquinazolin-1(2H)-yl}pipéridine-1-carbaldéhyde
n°206 : 4-{3-[4-(cyclopropylméthoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°207 : 2-[4-({6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]-N-méthylacétamide
n°212 :4-{6-[2-fluoro-1-(fluorométhyl)éthoxy]-2,4-dioxo-3-[4-(1H-pyrazol-1-yl)benzyl]-3,4-dihydroquinazolin-1(2H)-yl}pipéridine-1-carbaldéhyde
n°213 : 4-{6-[2-fluoro-1-(fluorométhyl)éthoxy]-2,4-dioxo-3-(4-pyridin-2-ylbenzyl)-3,4-dihydroquinazolin-1(2H)-yl}pipéridine-1-carbaldéhyde
n°215 : 4-{6-[2-fluoro-1-(fluorométhyl)éthoxy]-2,4-dioxo-3-(4-thiophén-2-ylbenzyl)-3,4-dihydroquinazolin-1(2H)-yl}pipéridine-1-carbaldéhyde
n°216 : 4-{6-[2-fluoro-1-(fluorométhyl)éthoxy]-2,4-dioxo-3-(quinoléin-7-ylméthyl)-3,4-dihydroquinazolin-1 (2H)-yl}pipéridine-1-carbaldéhyde
n°218 : 4-{6-[2-fluoro-1-(fluorométhyl)éthoxy]-3-[(6-méthoxynaphtalén-2-yl)méthyl]-2,4-dioxo-3,4-dihydroquinazolin-1(2H)-yl}pipéridine-1-carbaldéhyde
n °223 : 4-{3-[4-(1H-benzimidazol-1-yl)benzyl]-6-[2-fluoro-1-(fluorométhyt)éthoxy]-2,4-dioxo-3,4-dihydroquinazolin-1(2H)-yl}pipéridine-1-carbaldéhyde
n°224 : 4-{6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde
n°226 : 4-{6-[2-fluoro-1-(fluorométhyl)éthoxy]-3-[3-méthoxy-4-(tétrahydrofuran-3-yloxy)benzyl]-2,4-dioxo-3,4-dihydroquinazolin-1(2H)-yl}pipéridine-1-carbaldéhyde
n°228 : 4-[3-{4-{(1-benzylpyrrolidin-3-yl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°230 : 4-[3-(1-benzothiophén-5-ylméthyl)-6-[2-fluoro-1-(fluorométhyl)éthoxy]-2,4-dioxo-3,4-dihydroquinazolin-1(2H)-yl]pipéridine-1-carbaldéhyde
n°232 : 4-{6-[2-fluoro-1-(fluorométhyl)éthoxy]-3-[3-méthoxy-4-(1-méthyléthoxy)benzyl]-2,4-dioxo-3,4-dihydroquinazolin-1(2H)-yl}pipéridine-1-carbaldéhyde
n°233 : 4-[3-(3,4-diméthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°234 : 4-[3-{4-[(1-acétylpyrrolidin-3-yl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°239 : 4-[3-{4-[(4-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°240 : 4-[3-{4-[(4-chlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°242 : 4-[3-{4-[(3-chlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°243 : 4-{6-[2-fluoro-1-(fluorométhyl)éthoxy]-2,4-dioxo-3-(3-thiophén-3-ylbenzyl)-3,4-dihydroquinazolin-1(2H)-yl}pipéridine-1-carbaldéhyde
n°245 : 4-[3-(4-éthoxy-3-méthoxybenzyl)-6-(2-hydroxyéthoxy)-2,4-dioxo-3,4-dihydroquinazolin-1(2H)-yl]pipéridine-1-carbaldéhyde
n°246 : 4-[3-{4-[2-(2,3-dihydro-1H-indol-1-yl)-2-oxoéthoxy]-3-méthoxybenzyl}-6-[2-fluoro-1-(fluorométhyl)éthoxy]-2,4-dioxo-3,4-dihydroquinazolin-1 (2H)-yl]pipéridine-1-carbaldéhyde
n°250 : 4-[3-{4-[(3,4-dichlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°251 : 4-{6-[2-fluoro-1-(fluorométhyl)éthoxy]-3-[3-méthoxy-4-(2-oxo-2-pipéridin-1-yléthoxy)benzyl]-2,4-dioxo-3,4-dihydroquinazolin-1(2H)-yl}pipéridine-1-carbaldéhyde
n°254 : 4-{3-[3-éthoxy-4-(thiophén-2-ylméthoxy)benzyl]-6-[2-fluoro-1-(fluorométhyl)éthoxy]-2,4-dioxo-3,4-dihydroquinazolin-1 (2H)-yl}pipéridine-1-carbaldéhyde
n°258: 4-[3-(3,4-diméthoxybenzyl)-6-[2-fluoro-1-(hydroxyméthyl)éthoxyl-2,4-dioxo-3,4-dihydroquinazolin-1(2H)-yl]pipéridine-1-carbaldéhyde
n°263 : acide (2R)-2-[2-(cyclopentyloxy)-5-({6-[2-fluoro-1-(fluorométhyl)éthoxy]-1-(1 -formylpipéridin-4-yl)-2,4-dioxo-1,4-dihydroquinazolin-3(2H)-yl}méthyl)phénoxy]propanoïque
n°264:4-{6-[2-fluoro-1-(fluorométhyl)éthoxy]-3-[(1-méthyl-3-thiophén-2-yl-1H-pyrazol-5-yl)méthyl]-2,4-dioxo-3,4-dihydroquinazolin-1(2H)-yl]pipéridine-1-carbaldéhyde
n°270 : 4-{6-[2-fluoro-1-(fluorométhyl)éthoxy]-3-[4-(5-méthyl-1,2,4-oxadiazol-3-yl)benzyl]-2,4-dioxo-3,4-dihydroquinazolin-1 (2H)-yl}pipéridine-1-carbaldéhyde
n°275 4-{6-[2-fluoro-1-(fluorométhyl)éthoxy]-2,4-dioxo-3-(4-pyrimidin-5-ylbenzyl)-3,4-dihydroquinazolin-1(2H)-yl}pipéridine-1-carbaldéhyde
n°276 : 4-{6-[2-fluoro-1-(fluorométhyl)éthoxy]-3-[(1-méthyl-3-phényl-1H-pyrazol-5-yl)méthyl]-2,4-dioxo-3,4-dihydroquinazolin-1(2H)-yl}pipéridine-1-carbaldéhyde
n°278 : 4-[6-[2-fluoro-1-(fluorométhyl)éthoxy]-2,4-dioxo-3-{[6-(1H-pyrazol-1-yl)pyridin-3-yl]méthyl}-3,4-dihydroquinazolin-1(2H)-yl}pipéridine-1-carbaldéhyde
n°279: 4-{6-[2-fluoro-1-(fluorométhyl)éthoxy]-2,4-dioxo-3-[(2-thiophén-2-ylpyrimidin-5-yl)méthyl]-3,4-dihydroquinazolin-1(2H)-yl}pipéridine-1-carbaldéhyde
n°280 : 4-{6-[2-fluoro-1-(fluorométhyl)éthoxy]-3-[4-(1-méthyl-1H-pyrazol-3-yl)benzyl]-2,4-dioxo-3,4-dihydroquinazolin-1(2H)-yl}pipéridine-1-carbaldéhyde
n°282 : 4-{6-[2-fluoro-1-(fluorométhyl)éthoxy]-3-[4-(3-méthyl-1,2,4-oxadiazol-5-yl)benzyl]-2,4-dioxo-3,4-dihydroquinazolin-1(2H)-yl}pipéridine-1-carbaldéhyde
n°283 : acide [2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]acétique
n°285 :4-{6-[2-fluoro-1-(fluorométhyl)éthoxy]-2,4-dioxo-3-(thiéno[2,3-b]pyridin-2-ylméthyl)-3,4-dihydroquinazolin-1(2H)-yl}pipéridine-1-carbaldéhyde
n°286 : 4-{6-[2-fluoro-1-(fluorométhyl)éthoxy]-2,4-dioxo-3-[(6-phénylpyridin-3-yl)méthyl]-3,4-dihydroquinazolin-1 (2H)-yl}pipéridine-1-carbaldéhyde
n°287 : 4-{6-[2-fluoro-1-(fluorométhyl)éthoxy]-3-[(6-morpholin-4-ylpyridin-3-yl)méthyl]-2,4-dioxo-3,4-dihydroquinazolin-1(2H)-yl}pipéridine-1-carbaldéhyde
n°289 : 4-{6-[2-fluoro-1-(fluorométhyl)éthoxy]-2,4-dioxo-3-[(6-thiophén-2-ylpyridin-3-yl)méthyl]-3,4-dihydroquinazolin-1(2H)-yl}pipéridine-1-carbaldéhyde
n°292 : 4-{6-[2-fluoro-1-(fluorométhyl)éthoxy]-3-[(1-méthyl-5-phényl-1H-pyrazol-3-yl)méthyl]-2,4-dioxo-3,4-dihydroquinazolin-1(2H)-yl)pipéridine-1-carbaldéhyde
n°294 : 4-({6-[2-fluoro-1-(fluorométhyl)éthoxy]-1-(1-formylpipéridin-4-yl)-2,4-dioxo-1,4-dihydroquinazolin-3(2H)-yl}méthyl)biphényl-2-carbonitrile
n°295: (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthylpropanamide
n°297 : 4-{7-fluoro-6-[2-fluoro-1-(fluorométhyl)éthoxy]-2,4-dioxo-3-(4-thiophén-2-ylbenzyl)-3,4-dihydroquinazolin-1(2H)-yl}pipéridine-1-carbaldéhyde
n°298 : 4-{6-[2-fluoro-1-(fluorométhyl)éthoxy]-3-[3-méthoxy-4-(morpholin-4-ylméthyl)benzyl]-2,4-dioxo-3,4-dihydroquinazolin-1(2H)-yl}pipéridine-1-carbaldéhyde
n°299 : 4-{6-[2-fluoro-1-(fluorométhyl)éthoxy]-3-[3-méthoxy-4-(pipéridin-1-ylméthyl)benzyl]-2,4-dioxo-3,4-dihydroquinazolin-1(2H)-yl]pipéridine-1-carbaldéhyde
n°300 : 4-[3-{4-[(3,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n° 301 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-éthylacétamide
n°302 : acide (2S)-2-[2-(cyclopentyloxy)-5-({6-[2-fluoro-1-(fluorométhyl)éthoxy-1-(1-formylpipéridin-4-yl)-2,4-dioxo-1,4-dihydroquinazolin-3(2H)-yl}méthyl)phénoxylpropanoïque
n°305 : 4-{6-[2-fluoro-1-(fluorométhyl)éthoxy]-3-(3-méthoxy-4-{[(3R)-2-oxo-1-phénylpyrrolidin-3-yl]oxy}benzyl)-2,4-dioxo-3,4-dihydroquinazolin-1(2H)-yl}pipéridine-1-carbaldéhyde
n°306 : 4-{3-[4-(cyclobutylméthoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
n°307 : 4-{3-[4-(benzyloxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°308 : 4-{7-fluoro-6-[2-fluoro-1-(fluorométhyl)éthoxy]-3-(4-hydroxy-3-méthoxybenzyl)-2,4-dioxo-3,4-dihydroquinazolin-1(2H)-yl}pipéridine-1-carbaldéhyde
n°309: 4-{3-[4-(cyclopropylméthoxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°310:4-{7-fluoro-6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde
n°311 : 4-{7-fluoro-6-[2-fluoro-1-(fluorométhyl)éthoxy]-3-[3-méthoxy-4-(1-méthyléthoxy)benzyl]-2,4-dioxo-3,4-dihydroquinazolin-1(2H)-yl}pipéridine-1-carbaldéhyde
n°312 : 4-[3-(4-éthoxy-3-méthoxybenzyl)-7-fluoro-6-[2-fluoro-1-(fluorométhyl)éthoxy]-2,4-dioxo-3,4-dihydroquinazolin-1(2H)-yl)pipéridine-1-carbaldéhyde
n°315 :4-(7-fluoro-6-[2-fluoro-1-(fluorométhyl)éthoxy]-3-{[6-(3-méthoxyphényl)pyridin-3-yl]méthyl}-2,4-dioxo-3,4-dihydroquinazolin-1(2H)-yl}pipéridine-1-carbaldéhyde
n°316 : 4-{7-fluoro-6-[2-fluoro-1-(fluorométhyl)éthoxy]-3-{[6-(2-fluorophényl)pyridin-3-yl]méthyl}-2,4-dioxo-3,4-dihydroquinazolin-1(2H)-yl}pipéridine-1-carbaldéhyde
n°317 : 4-{7-fluoro-6-[2-fluoro-1-(fluorométhyl)éthoxy]-3-{[6-(4-fluorophényl)pyridin-3-yl]méthyl}-2,4-dioxo-3,4-dihydroquinazolin-1(2H)-yl}pipéridine-1-carbaldéhyde
n°318 : 4-{7-fluoro-6-[2-fluoro-1-(fluorométhyl)éthoxy]-3-{[6-(4-méthoxyphényl)pyridin-3-yl]méthyl}-2,4-dioxo-3,4-dihydroquinazolin-1(2H)-yl}pipéridine-1-carbaldéhyde
n°319 : 4-{7-fluoro-6-[2-fluoro-1-(fluorométhyl)éthoxy]-2,4-dioxo-3-[(6-thiophén-2-ylpyridin-3-yl)méthyl]-3,4-dihydroquinazolin-1(2H)-yl}pipéridine-1-carbaldéhyde
n°320 : 4-{3-[3-éthoxy-4-(thiophén-2-ylméthoxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°321 4-{7-fluoro-6-[2-fluoro-1-(fluorométhyl)éthoxy]-3-[4-(1-méthyl-1H-pyrazol-3-yl)benzyl]-2,4-dioxo-3,4-dihydroquinazolin-1(2H)-yl}pipéridine-1-carbaldéhyde
n°322 : 4-{7-fluoro-6-[2-fluoro-1-(fluorométhyl)éthoxy]-2,4-dioxo-3-(4-pyrimidin-5-ylbenzyl)-3,4-dihydroquinazolin-1(2H)-yl}pipéridine-1-carbaldéhyde
n°323 : 4-{7-fluoro-6-[2-fluoro-1-(fluorométhyl)éthoxy]-3-[(1-méthyl-3-thiophén-2-yl-1H-pyrazol-5-yl)méthyl]-2,4-dioxo-3,4-dihydroquinazolin-1(2H)-yl}pipéridine-1-carbaldéhyde
n°324 : 4-{7-fluoro-6-[2-fluoro-1-(fluorométhyl)éthoxy]-3-[3-méthoxy-4-(2-oxo-2-pipéridin-1-yléthoxy)benzyl]-2,4-dioxo-3,4-dihydroquinazolin-1(2H)-yl}pipéridine-1-carbaldéhyde
n°325 : 4-[3-{4-[2-(2,3-dihydro-1H-indol-1-yl)-2-oxoéthoxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°326 : 4-{7-fluoro-6-[2-fluoro-1-(fluorométhyl)éthoxy]-3-[4-(5-méthyl-1,2,4-oxadiazol-3-yl)benzyl]-2,4-dioxo-3,4-dihydroquinazolin-1(2H)-yl)pipéridine-1-carbaldéhyde
n°327 : 4-[3-{4-[(3-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°328 : 4-[3-{[6-(3,5-dichlorophényl)pyridin-3-yl]méthyl}-6-[2-fluoro-1-(fluorométhyl)éthoxy]-2,4-dioxo-3,4-dihydroquinazolin-1(2H)-yl]pipéridine-1-carbaldéhyde
n°329 : 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)biphényl-2-carbonitrile
n°330:4-{7-fluoro-6-[2-fluoro-1-(fluorométhyl)éthoxy]-2,4-dioxo-3-[4-(1H-pyrazol-1-yl)benzyl]-3,4-dihydroquinazolin-1(2H)-yl}pipéridine-1-carbaldéhyde
n°331 :4-{7-fluoro-6-[2-fluoro-1-(fluorométhyl)éthoxy]-3-{[6-(3-fluorophényl)pyridin-3-yl]méthyl}-2,4-dioxo-3,4-dihydroquinazolin-1(2H)-yl}pipéridine-1-carbaldéhyde
n°332 : 3-[5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)pyridin-2-yl]benzonitrile
n°333 : :4-[3-(3,4-diéthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°334 : 4-[3-{4-[(4-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°335 : 4-{7-fluoro-6-[2-fluoro-1-(fluorométhyl)éthoxy]-3-[4-(morpholin-4-ylméthyl)benzyl]-2,4-dioxo-3,4-dihydroquinazolin-1(2H)-yl}pipéridine-1-carbaldéhyde
n°336 : 4-{7-fluoro-6-[2-fluoro-1-(fluorométhyl)éthoxy]-2,4-dioxo-3-{[6-(1H-pyrazol-1-yl)pyridin-3-yl]méthyl}-3,4-dihydroquinazolin-1(2H)-yl}pipéridine-1-carbaldéhyde
n°337 : 4-{7-fluoro-6-[2-fluoro-1-(fluorométhyl)éthoxy]-3-(4-morpholin-4-ylbenzyl)-2,4-dioxo-3,4-dihydroquinazolin-1(2H)-yl}pipéridine-1-carbaldéhyde
n°338 : 4-{7-fluoro-6-[2-fluoro-1-(fluorométhyl)éthoxy]-3-(3-méthoxy-4-propoxybenzyl)-2,4-dioxo-3,4-dihydroquinazolin-1(2H)-yl}pipéridine-1-carbaldéhyde
n°339 : 4-{3-[4-(1H-benzimidazol-1-yl)benzyl]-7-fluoro-6-[2-fluoro-1-(fluorométhyl)éthoxy]-2,4-dioxo-3,4-dihydroquinazolin-1(2H)-yl)pipéridine-1-carbaldéhyde
n°340 : 5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxybenzonitrile
n°341 : 3-(3,4-diméthoxybenzyl)-6-[2-fluoro-1-(fluorométhyl)éthoxy]-1-(1-formylpipéridin-4-yl)-2,4-dioxo-1,2,3,4-tétrahydroquinazoline-7-carbonitrile
n°342 : 4-[3-(4-bromobenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°343:4-[3-{4-[(3,4-dichlorobenzyl)oxy]-3-(2-méthoxyéthoxy)benzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°344 : 4-{3-[4-(benzyloxy)benzyl]-7-fluoro-6-{2-fluoro-1-(fluorométhyl)éthoxy]-2,4-dioxo-3,4-dihydroquinazolin-1(2H)-yl}pipéridine-1-carbaldéhyde
n°345:4-[3-{4-[(3,4-dichlorobenzyl)oxy]-3-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°349 : 4-[3-{4-[(3,4-dichlorobenzyl)oxyl-3-(2-fluoroéthoxy)benzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°350 : 4-[3-{4-[(2-chloro-4-fluorobenzyl)oxy]-3-méthoxybenzyl}-7-fluoro-6-[2-fluoro-1-(fluorométhyl)éthoxy]-2,4-dioxo-3,4-dihydroquinazolin-1 (2H)-yl]pipéridine-1-carbaldéhyde
n°351 : 4-[3-{4-[(2,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°352 : 4-[3-{4-[(2-chloro-6-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°353 : 4-[3-{4-[(2,6-dichlorobenzyl)oxy]-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
n°354 : 4-[3-{4-[(2-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°355 : 4-[7-fluoro-3-{4-[(2-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°357 : 2-[(3,4-dichlorobenzyl)oxy]-5-({7-fluoro-6-[2-fluoro-1-(fluorométhyl)éthoxy]-1-(1-formylpipéridin-4-yl)-2,4-dioxo-1,4-dihydroquinazolin-3(2H)-yl)méthyl)benzonitrile
n°358 : 4-[3-{4-[(3,4-dichlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°360 : 4-{7-fluoro-6-[2-fluoro-1-(fluorométhyl)éthoxy]-2,4-dioxo-3-[4-(2-phényléthyl)benzyl]-3,4-dihydroquinazolin-1(2H)-yl)pipéridine-1-carbaldéhyde
n°362 : 4-[3-{4-[(4,5-dichloro-2-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde n°369 : 4-[3-{4-[(4-chlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°371 4-[3-{3-chloro-4-{(4-chlorobenzyl)oxy]-5-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°373 : 4-[3-{3-chloro-4-[(2,4-dichlorobenzyl)oxy]-5-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°375 : 4-[7-fluoro-3-{4-[(4-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°376 : 4-[3-{4-[(3,5-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°377 : 4-[3-(4-{[4-chloro-3-(trifluorométhyl)benzyl]oxy}-3-méthoxybenzyl)-7-fluoro-6-[2-fluoro-1-(fluorométhyl)éthoxyl-2,4-dioxo-3,4-dihydroquinazolin-1(2H)-yl]pipéridine-1-carbaldéhyde n°379 : 4-[3-{4-[(3-chlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°380 : 4-[3-{4-[(3,5-difluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°381 : 4-{3-[4-(benzyloxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
n°382 : 4-[3-{4-[(3-chloro-5-fluorobenzyl)oxy]-3-méthoxybenzyl}-7-fluoro-[2-fluoro-1-(fluorométhyl)éthoxy]-2,4-dioxo-3,4-dihydroquinazolin-1 (2H)-yl]pipéridine-1-carbaldéhyde
n°383 : 4-{7-fluoro-6-[2-fluoro-1-(fluorométhyl)éthoxy]-3-(3-méthoxy-4-{[4-(trifluorométhyl)benzyl]oxy}benzyl)-2,4-dioxo-3,4-dihydroquinazolin-1(2H)-yl}pipéridine-1-carbaldéhyde
n°384 : 4-[3-{4-[(2,5-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°385 : 4-{[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]méthyl}benzonitrile
n°386 : 3-{[4-({7-fluoro-6-[2-fluoro-1-(fluorométhyl)éthoxyl-1-(1-formylpipéridin-4-yl)-2,4-dioxo-1,4-dihydroquinazolin-3(2H)-yl}méthyl)-2-méthoxyphénoxy]méthyl}benzonitrile
n°387 : 4-[3-{4-[(4-chloro-2-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°388 : 4-[3-{4-[1-(3,4-dichlorophényl)éthoxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°389 : 4-{7-fluoro-6-[2-fluoro-1-(fluorométhyl)éthoxy]-3-{4-[(3-hydroxybenzyl)oxy]-3-méthoxybenzyl}-2,4-dioxo-3,4-dihydroquinazolin-1 (2H)-yl}pipéridine-1-carbaldéhyde
n°390 : 4-[7-fluoro-3-{4-[(3-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
n°391 : 4-[3-{4-[(3,4-difluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
n°392 : 4-{3-[4-(5,6-dichloro-1H-benzimidazol-1-yl)-3-méthoxybenryl]-7-fluoro-6-[2-fluoro-1-(fluorométhyl)éthoxy]-2,4-dioxo-3,4-dihydroquinazolin-1(2H)-yl}pipéridine-1-carbaldéhyde
n°393 : 3,4-dichlorobenzènesulfonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)phényle
n°394 : 3,4-dichlorobenzènesulfonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényle
n°403 : 3,4-dichloro-N-[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényQbenzamide

La présente invention a également pour objet des procédés de préparation du composé de formule générale (I).

Les composés de l'invention peuvent être préparés par les méthodes illustrées dans les schémas 1 à 4 qui suivent.

Dans ce qui suit, on entend par groupe partant, un groupe pouvant être facilement substitué, avec départ d'une paire électronique, par rupture d'une liaison hétérolytique. Ce groupe peut ainsi être remplacé facilement par un autre groupe, lors d'une réaction de substitution par exemple. De tels groupes partants sont, par exemple, les halogènes, ou un groupe hydroxy activé tel qu'un mésylate, tosylate, triflate, etc. Des exemples de groupes partants ainsi que des références pour leur préparation sont donnés dans « Advanced Organic Chemistry », J. March, 3rd Edition, Wiley Interscience, p 310-316.

On entend par groupe protecteur PG, un groupe permettant d'empêcher la réactivité d'une fonction ou position, lors d'une réaction chimique pouvant l'affecter, et qui restitue la molécule après clivage selon des méthodes connues de l'homme du métier.

Par groupe protecteur temporaire des amines ou alcools, on entend les groupes protecteurs tels que ceux décrits dans Protective Groups in Organic Synthesis, Greene T.W. et Wuts P.G.M., Ed. Willey Intersciences 1999 et dans Protecting Groups, Kocienski P.J., 1994, Georg Thieme Verlag.

On peut citer par exemple des groupements protecteurs temporaires des amines : benzyles, carbamates, (tels que *tert*-butyloxycarbonyle clivable en milieu acide, benzyloxycarbonyle clivable par hydrogénolyse), des groupements protecteurs temporaires d'acides carboxyliques : esters d'alkyle (tels que méthyle ou éthyle, *tert-*butyle hydrolysables en milieu basique ou acide) et benzyliques hydrogénolysables, des groupements protecteurs temporaires d'alcools ou de phénols tels que les éthers de tétrahydropyranyle, métyloxyméthyle ou méthyléthoxyméthyle, *tert*-butyle et benzyle, des groupements protecteurs temporaires de dérivés carbonylés tels que les acétals linéaires ou cycliques comme par exemple 1,3-dioxane-2-yle ou 1,3-dioxolan-2-yle ; et se référer aux méthodes générales bien connues décrites dans Protective Groups, cité ci-dessus.

Selon les cas, l'homme de l'art sera à même de choisir les groupes protecteurs appropriés. Les composés de formule (I) peuvent comporter des groupes précurseurs d'autres fonctions qui sont générées ultérieurement en une ou plusieurs autres étapes.

Dans les schémas généraux de synthèse qui suivent, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

Les énantiomères purs des composés de l'invention peuvent être obtenus à partir de précurseurs énantiomèriquement purs ou bien par chromatographie sur des phases chirales ou bien, lorsque les composés comportent des fonctions acides ou des amines par cristallisations sélectives de sels diastéréoisomèriques obtenus par réaction des composés (I) avec, respectivement, des amines ou des acides chiraux.

Selon un mode de réalisation de l'invention, les composés de formule générale (I) peuvent être obtenus selon les Schémas 1 à 4 qui suivent. Par souci de clarté, on a choisi le groupe R4 comme étant un hydrogène, p et p' représentent 1 et 2 respectivement et on a fixé les groupes R₂ et R₃ tels qu'indiqués dans les schémas. Cependant, il s'entend que R₄ peut être tel que défini dans la formule générale de (I), que R₂ et R₃ peuvent avoir les positions indiquées dans la formule générale (I), et que p et p' peuvent être tels que définis dans la formule générale de (I).

Les voies de synthèse décrites ci-après servent uniquement à titre illustratif et ne sont en aucun cas limitatives. L'homme du métier pourra appliquer sans difficultés l'enseignement ci-dessous aux composés de formule (I) pour lesquels R, R₁, R₂, R₃, R₄, Ra, Rb, Rc, m, n, p, p' et q sont tels que définis dans la formule générale (I).

Selon le schéma 1, le composé de formule (IV) est obtenu par une réaction d'amination réductrice en faisant réagir un composé de formule (II) dans lequel R' représente un groupe (C₁-C₆) alkyle, et R₂ est tel que défini pour le composé de formule (I), avec un composé de formule (III) en milieu acide et en présence d'un agent réducteur tel que le tri-acétoxy-borohydrure de sodium. Le groupe GP du composé de formule (III) est un groupe protecteur de la fonction amine, qui peut avantageusement être du tert-butyloxycarbonyle (boc). Le composé de formule (IV) ainsi formé est ensuite acylé selon les méthodes bien connues de l'homme du métier, avec un chloroformate d'alkyle ou d'aryle pour donner le composé de formule (V) dans lequel R" représente un groupe (C₁-C₆) alkyle ou un groupe aryle substitué. Une réaction d'hydrolyse en milieu basique permet d'obtenir les composés de formule (VI) qui par une réaction de couplage avec un composé de formule (VII), dans lequel R₃ est tel que défini pour le composé de formule (I), conduit aux composés de formule (VIII). Une réaction de cyclisation intra-moléculaire en milieu basique permet d'obtenir les dérivés de quinazolinedione de formule (IX). Le groupe GP protecteur de la fonction amine est ensuite clivé, en milieu acide par exemple lorsque GP est un boc, pour donner les composés de formule (la) qui par une réaction d'acylation donne les composés de formule (Ib).

Les composés de formule (la) sont des composés de formule (I) et peuvent servir d'intermédiaire à d'autres composés de formule (I), tels que les composés de formule (Ib).

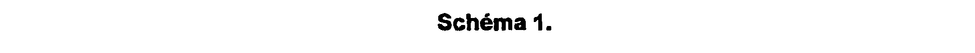

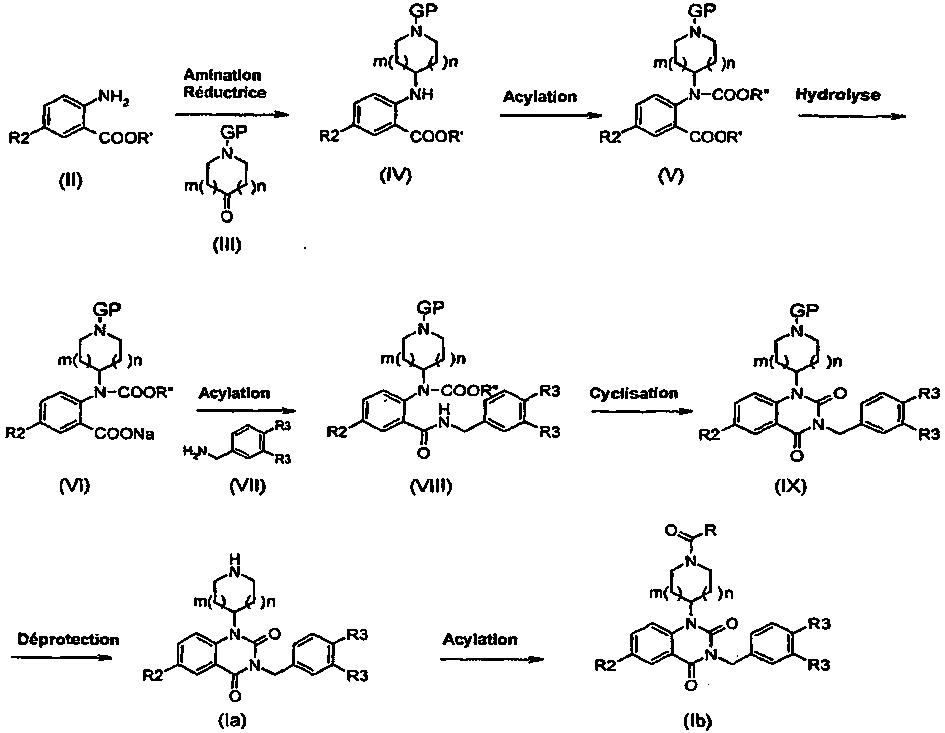

Les composés de formule (t) pour lesquels R₂ représente -ORa, Ra étant tel que défini pour le composé de formule (I), répondent à la formule (Id). Ils peuvent être obtenus à partir des composés de formule (X) selon le schéma 2 suivant. Les composés de formule (Ic), obtenus par une réaction d'hydrogénolyse des composés de formule (X), sont soumis par exemple à une réaction d'alkylation avec un agent alkylant de type Ra-X dans lequel Ra est tel que défini pour le composé de formule (I) et X représente un groupement partant (tel qu'un atome d'halogène par exemple) en présence d'une base telle que du carbonate de césium (Cs₂CO₃), ou encore à une réaction de Mitsunobu (Synthesis 1981, 1) avec un alcool de type Ra-OH, Ra étant tel que défini pour le composé de formule (I), pour donner des composés de formule (Id).

Les composés de formule (X) ainsi que les composés de formule (Ic) sont des composés de formule (I) et peuvent servir d'intermédiaire à d'autres composés de formule (I), tels que les composés de formule (Id).

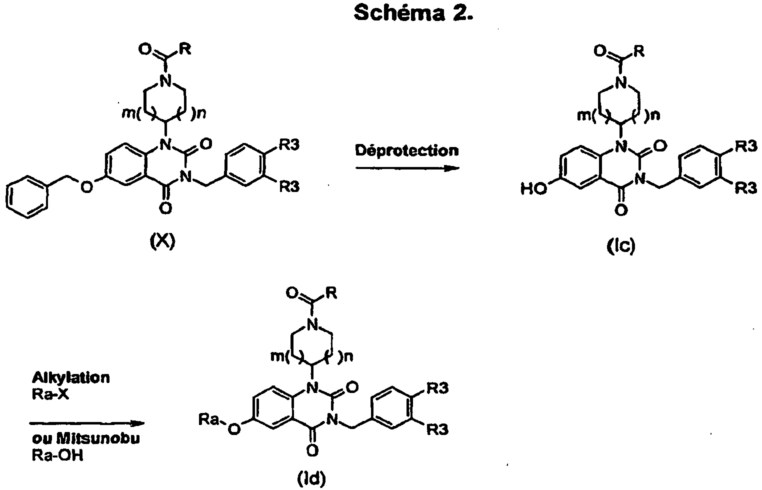

Alternativement les composés de formule (Id) peuvent être obtenus en suivant le procédé décrit dans le schéma 3.

Les composés de formule (XII) sont obtenus par une réaction de substitution nucléophile aromatique impliquant un composé de formule (XI) dans lequel R' est tel que défini précédemment et un alcool de type Ra-OH, dans lequel Ra est tel que défini pour le composé de formule (I), en présence d'une base. La réduction du groupement nitro des composés de formule (XII) conduit aux dérivés anilino correspondants (XIII). Une réaction d'amination réductrice avec un composé de formule (III), dans lequel GP est un groupe protecteur de fonctions amines, tel par exemple que le boc, conduit aux composés de formule (XIV). L'obtention des composés de formule (XV) se fait par réaction d'un composé de formule (XIV) avec de l'isocyanate de potassium (KNCO) en milieu acide. Une réaction de cyclisation intra-moléculaire en milieu basique permet d'obtenir les composés de formule (XVI). Le groupement protecteur GP est clivé par des méthodes bien connues de l'homme de l'art pour donner les composés de formule (XVII). Une réaction d'acylation conduit aux composés de formule (XVIII). Finalement, on obtient les composés de formule (Id) soit par réaction d'alkylation avec un dérivé de type (XIX) dans lequel X représente un groupement partant tel qu'un atome d'halogène en présence d'une base comme par exemple le carbonate de césium, soit encore par une réaction de Mitsunobu avec un alcool benzylique de type (XX). Dans les composés (XIX) et (XX), R₃ est tel que défini précédemment.

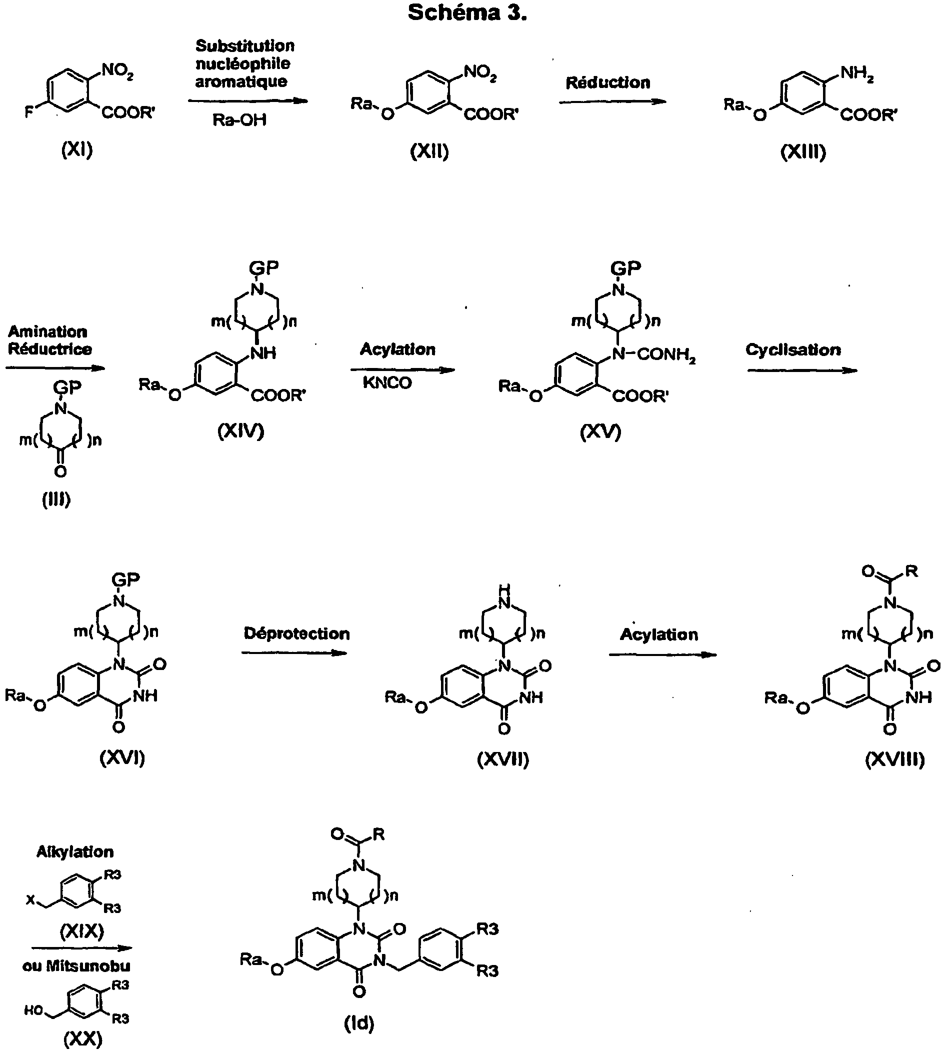

Les composés de formule (le) et (If) dans lesquels R₂ représente plus particulièrement un groupe de type -CH₂-NHC(O)Rb, Rb étant défini comme dans le composé de formule (I) peuvent être préparés selon le Schéma 4 suivant.

Il est entendu que dans le schéma 3, le groupement R2 illustré est de type -O-Ra et est en position 6 de la structure quinazoline-dione (voir par exemple composé (XVIII)), mais qu'il est également possible d'avoir un deuxième groupement R2 tel que déini dans la formule générale de (I) en position 7 du même groupement quinazoline-dione.

La réduction du groupement nitro des composés de type (XXI), dans lequel R' et GP' sont tels que définis précédemment, le groupe GP' étant avantageusement du boc, conduit aux dérivés anilino correspondants (XXII), qui par une réaction d'amination réductrice en faisant réagir en milieu acide et aventageusement en présence d'un agent réducteur tel que le tri-acétoxy-borohydrure de sodium, avec un composé de formule (III) dans lequel GP représente un groupement protecteur d'amine benzyloxycarbonyl, donnent des composés de formule (XXIII). Une réaction d'acylation avec un chloroformate d'alkyle ou d'aryle dans lequel R" représente un groupe (C₁-C₆) alkyle ou un groupe aryle substitué conduit aux composés de formule (XXIV). Les analogues de quinazolinedione de formule (XXV) peuvent être obtenus par une réaction d'hydrolyse en milieu basique puis par une réaction de couplage avec un composé de formule (VII) dans lequel R₃ est tel que défini pour le composé de formule (I), suivie par une réaction de cyclisation intra-moléculaire en milieu basique. Le groupe GP' (préférablement un boc) est ensuite clivé en milieu acide pour conduire aux composés de formule (XXVI), qui par acylation donnent des composés de formule (XXVII), dans lesquels Rb est tel que défini pour le composé de formule (I). Le groupe protecteur GP de (XXVII) est clivé par une réaction d'hydrogénolyse pour donner les composés de formule (le). Finalement les composés de formule (If) sont obtenus par une réaction d'acylation des composés de formule (le).

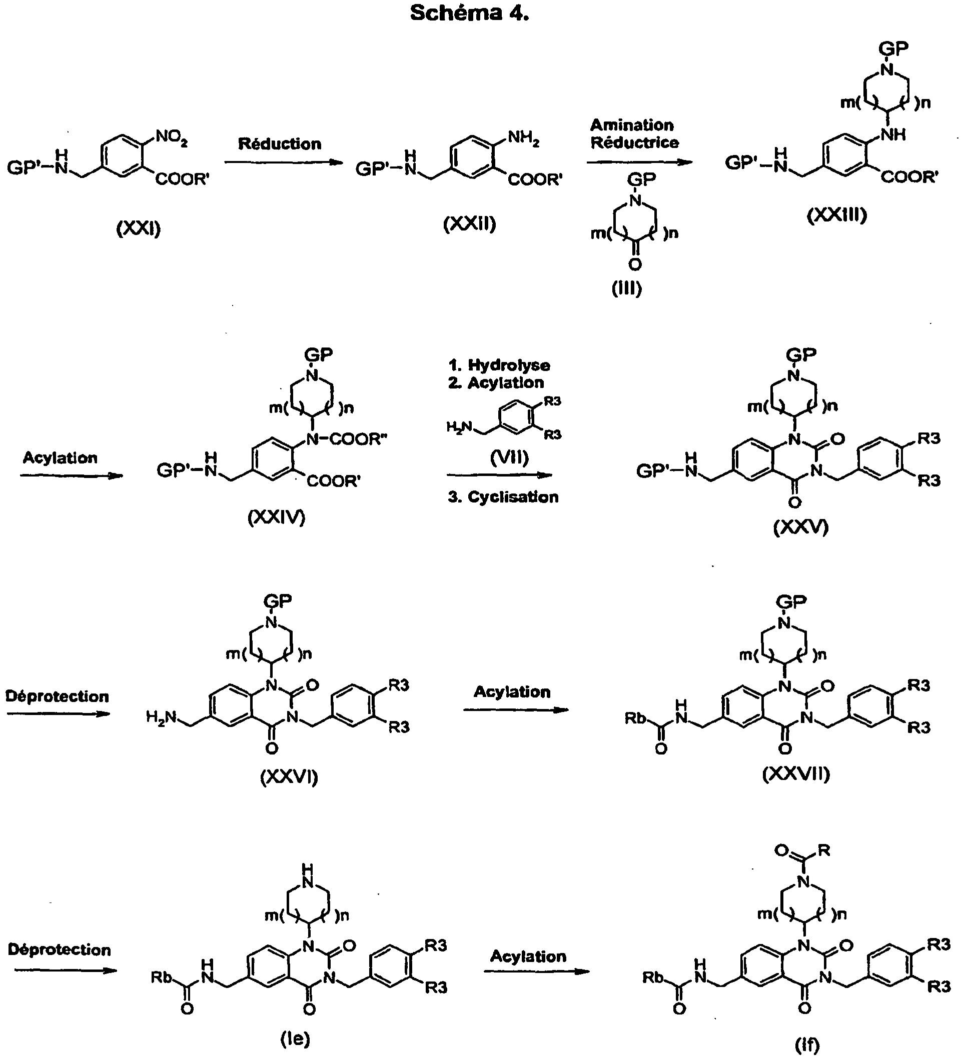

Il va de soi que l'homme du métier sera à même de choisir, à la lumière de ses connaissances et de la littérature, d'autres groupements protecteurs appropriés permettant l'introduction de tous les groupements décrits dans la formule générale (I).

Lorsque le composé de formule (I) comporte un cycle ponté, il peut être indifféremment obtenu par l'une des voies de synthèse décrites ci-dessus.

Les modes opératoires et exemples suivants décrivent la préparation de certains composés conformes à l'invention. Ces modes opératoires et exemples ne sont pas imitatifs et ne font qu'illustrer la présente invention.

Dans les modes opératoires et exemples ci-dessous :
- Les spectres de masse sont réalisés sur un spectromètre quadripolaire de type Platform LCZ (WATERS) ou de type ZQ 4000 (WATERS) en mode d'ionisation par électrospray positif ;
- Les spectres de RMN (résonnance magnétique nucléaire) sont réalisés sur un spectromètre à transformée de Fourier (BRUKER), à la température de 300°K (protons échangeables non enregistrés) ;
- s = singulet,
- d = doublet,
- m = multiplet,
- br = signal large (broad signal)
- t = triplet,
- q = quadruplet
- DMSO-d₆ = diméthylsulfoxyde deutéré
- CDCl₃ = chloroforme deutéré ;

Les mélanges de solvants sont quantifiés en rapports volumétriques ;

Les spectres RMN et spectres de masse confirment les structures des composés obtenus selon les exemples ci-dessous.

Dans les exemples qui suivent, on utilise les abréviations suivantes :
ACN : acétonitrile
AcOEt : acétate d'éthyle
AcOH : acide acétique
DBU : 1,8-diazabicyclo[5.4.0]undec-7-ène
DCM : dichlorométhane
DCE : 1,2-dichloroéthane
DIAD : diisopropyl azodicarboxylate
DIEA : di-isopropylamine
DMF : N,N-diméthylformamide
EtOH : éthanol
HBTU : O-(benzotriazol-1-yl)-N,N,N',N', tetraméthyluronium hexafluorophosphate
IBCF : isobutylchloroformate
MeOH : méthanol
NaBH(OAc)₃ : tri-acétoxy-borohydrure de sodium
TA : température ambiante
min : minute
THF : tétrahydrofurane
NEt₃ = triéthylamine
TFA : acide trifluoroacétique

### EXEMPLES

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau ci-après, qui illustre les structures chimiques et les propriétés physiques de quelques composés selon l'invention.

### EXEMPLE 1 : composé n°6

### Préparation du {[3-(3,4-diméthoxybenzyl)-1-(1-formylpipéridin-4-yl)-2,4-dioxo-1,2,3,4-tétrahydroquinazolin-6-yl]oxy}acétonitrile

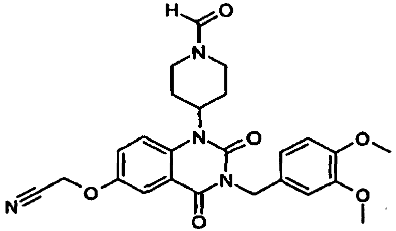

### Etape 1.1 :

### 4-{[4-(Benzyloxy)-2-(méthoxycarbonyl)phényl]amino}pipéridine-1-carboxylate de 1,1-diméthyléthyle

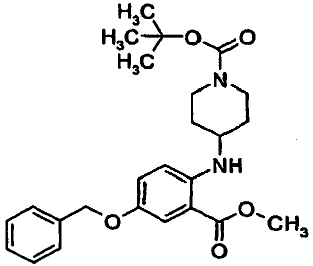

On irradie sous champ de micro-ondes (Biotage Initiator Sixty) pendant 20 min à 110°C, un mélange de 2 g de 2-amino-5-(benzyloxy)benzoate de méthyle, 3,1 g de 4-oxopipéridine-1-carboxylate de 1,1-diméthyléthyle et 3,29 g de NaBH(OAc)₃ dans 10 ml de AcOH. On répète la même réaction avec 2 autres lots de 2 g de 2-amino-5-(benzyloxy)benzoate de méthyle. On rassemble les 3 milieux réactionnels. On reprend dans l'AcOEt. On lave la phase organique à l'eau, par une solution saturée de NH₄Cl, par une solution saturé de NaHCO₃, sèche sur Na₂SO₄, filtre et évapore le solvant sous pression réduite. On chromatographie le résidu sur gel de silice en éluant par un mélange AcOEt/Heptane (5/95, v/v) jusqu'à (30/70, v/v) pour donner 10,2 g du produit attendu.

### Etape 1.2 :

### 4-{[4-(Benzyloxy)-2-(méthoxycarbonyl)phényl][(2-méthylpropoxy)carbonyl]amino}pipéridine-1-carboxylate de 1,1-diméthyléthyle

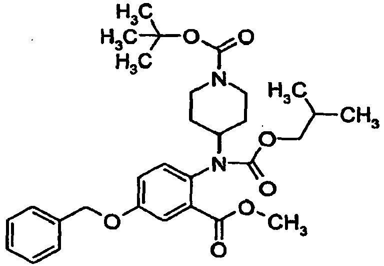

On irradie sous champ de micro-ondes pendant 30 min à 80°C, un mélange de 2 g de 4-{[4-(Benzyloxy)-2-(méthoxycarbonyl)phényl]amino}pipéridine-1-carboxylate de 1,1-diméthyléthyle obtenu à l'étape 1.1, de 0,87 ml de DIEA, de 1,78 ml d'IBCF, 1g de NaOH dans 10 ml de DCE. On répète la même réaction avec 4 autres lots de 2 g de 4-{[4-(Benzyloxy)-2-(méthoxycarbonyl)phényl]amino}pipéridine-1-carboxylate de 1,1-diméthyléthyle. On rassemble les 5 milieux réactionnels. On reprend dans l'AcOEt, filtre et évapore le filtrat sous pression réduite. On chromatographie le résidu sur gel de silice en éluant par un mélange AcOEt/Heptane (10/90, v/v) jusqu'à (50/50, v/v) pour donner 9,3 g du produit attendu.

### Etape 1.3 :

### Sel de sodium de l'acide 5-(benzyloxy)-2-({1-[(1,1-diméthyléthoxy)carbonyl] pipéridin-4-yl}[(2-méthylpropoxy)carbonyl]amino)benzoïque

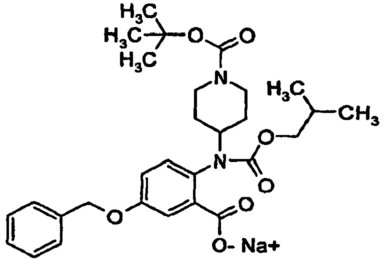

On chauffe 3h00 à 100°C un mélange de 9,3 g de 4-{[4-(Benzyloxy)-2-(méthoxycarbonyl)phényl][(2-méthylpropoxy)carbonyl]amino}pipéridine-1-carboxylate de 1,1-diméthyléthyle obtenu à l'étape 1.2, de 34,4 ml de NaOH 2N dans 57 ml de MeOH. On évapore la solution sous pression réduite et on ajoute du DCM. On sèche sur Na₂SO₄, filtre et évapore le solvant sous pression réduite pour donner 8,7 g du produit attendu.

### Etape 1.4 :

### 4-[{4-(Benzyloxy)-2-[(3,4-diméthoxybenzyl)carbamoyl]phényl} (isobutoxycarbonyl)amino]pipéridine-1-carboxylate de 1,1-diméthyléthyle

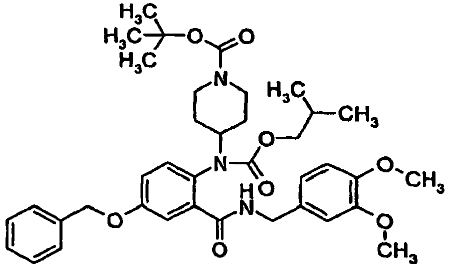

On agite 15 min à TA un mélange de 6g de sel de sodium de l'acide 5-(benzyloxy)-2-({1-[(1,1-diméthyléthoxy)carbonyl]pipéridin-4-yl}[(2-méthylpropoxy)carbonyl]amino)benzoïque obtenu à l'étape 1.3, de 4,42 g de DIEA dans 250 ml de DMF. On ajoute de 6,48 g d'HBTU et on laisse agiter pendant 30 min. On ajoute 2,48 g de vératrylamine et on agite le mélange réactionnel pendant 48h00. On évapore sous pression réduite, reprend le résidu dans de l'AcOEt, lave avec une solution saturée de NH₄Cl, par une solution saturé de NaHCO₃, sèche sur Na₂SO₄, filtre et évapore le solvant sous pression réduite. On chromatographie le résidu sur gel de silice en éluant par un mélange AcOEt/Heptane (20/80, v/v) jusqu'à (60/40, v/v) pour donner 7,5 g du produit attendu.

### Etape 1.5 :

### 4-[6-(Benzyloxy)-3-(3,4-diméthoxybenzyl)-2,4-dioxo-3,4-dihydroquinazolin-1(2H)-yl]pipéridine-1-carboxylate de 1,1-diméthyléthyle

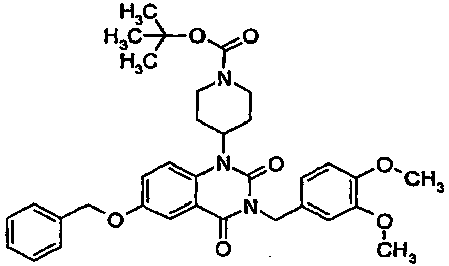

On irradie sous champ de micro-ondes pendant 30 min à 110°C, un mélange de 2.5g de 4-[{4-(benzyloxy)-2-[(3,4-diméthoxybenzyl)carbamoyl]phényl}(isobutoxycarbonyl) amino]pipéridine-1-carboxylate de 1,1-diméthyléthyle obtenu à l'étape 1.4, 7,4 g de NaOH dans 18,5 ml de DCE. On répète la même réaction avec 2 autres lots de 2,5 g de 4-[{4-(benzyloxy)-2-[(3,4-diméthoxybenzyl)carbamoyl]phényl} (isobutoxycarbonyl)amino]pipéridine-1-carboxylate de 1,1-diméthyléthyl. On rassemble les 3 milieux réactionnels. On reprend dans du DCM, lave à l'eau, sèche sur Na₂SO₄, filtre et évapore le solvant sous pression réduite pour donner 6,6 g du produit attendu.

### Etape 1.6 :

### 6-(Benzyloxy)-3-(3,4-diméthoxybenzyl)-1-pipéridin-4-ylquinazoline-2,4(1H,3H)-dione

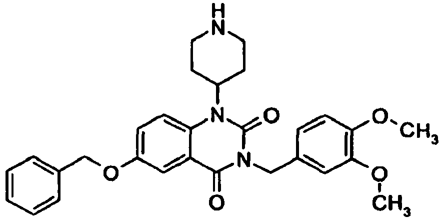

On agite pendant 2h00 à TA un mélange de 3,5 g de 4-[6-(benzyloxy)-3-(3,4-diméthoxybenzyl)-2,4-dioxo-3,4-dihydroquinazolin-1(2H)-yl]pipéridine-1-carboxylate de 1,1-diméthyléthyle obtenu à l'étape 1.5 et 25 ml de TFA dans 50 ml de DCM. On neutralise avec du K₂CO₃. On filtre, évapore le filtrat sous pression réduite. On reprend dans du DCM, lave avec une solution saturée de NaHCO₃, puis par une solution de NaOH à 8%. On sèche sur Na₂SO₄, filtre et évapore le solvant sous pression réduite pour donner 2,67 g du produit attendu.

### Etape 1.7 :

### 4-[6-(Benzyloxy)-3-(3,4-diméthoxybenzyl)-2,4-dioxo-3,4-dihydroquinazolin-1(2H)-yl]pipéridine-1-carbaldéhyde

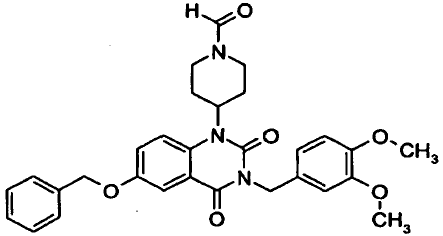

On irradie sous champ de micro-ondes pendant 1h00 à 140°C un mélange de 0,6 g de 6-(benzyloxy)-3-(3,4-diméthoxybenzyl)-1-pipéridin-4-ylquinazoline-2,4(1H,3H)-dione obtenu à l'étape 1.6. de 0,113 g de formiate d'ammonium dans 5 ml d'ACN. On filtre, évapore le filtrat sous pression réduite pour donner 0,62 g du produit attendu.

### Etape 1.8 : composé No.5 :

### 4-[3-(3,4-Diméthoxybenzyl)-6-hydroxy-2,4-dioxo-3,4-dihydroquinazolin-1(2H)-yl]pipéridine-1-carbaldéhyde

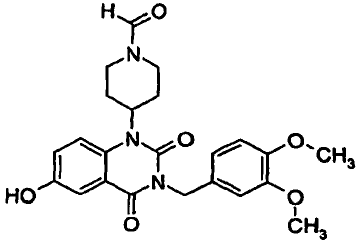

On irradie sous champ de micro-ondes pendant 2h00 à 80°C un mélange de 0,618 g de 4-[6-(benzyloxy)-3-(3,4-diméthoxybenzyl)-2,4-dioxo-3,4-dihydroquinazolin-1(2H)-yl]pipéridine-1-carbaldéhyde obtenu à l'étape 1.7, de 0,44 g de formiate d'ammonium et de 0,124 g de Pd/C (10%) dans 10ml d'EtOH préalablement purgé avec de l'azote. On filtre, évapore le filtrat sous pression réduite pour donner 0,513 g du produit attendu.

### Etape 1.9 : composé No.6

### {[3-(3,4-Diméthoxybenzyl)-1-(1-fortnylpipéridin-4-yl)-2,4-dioxo-1,2,3,4-tétrahydroquinazolin-6-yl]oxy}acétonitrile

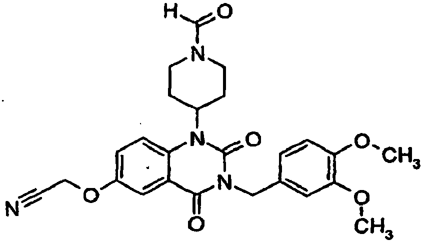

On agite à TA pendant 15 min 0,17 g de 4-[3-(3,4-diméthoxybenzyl)-6-hydroxy-2,4-dioxo-3,4-dihydroquinazolin-1(2H)-yl]pipéridine-1-carbaldéhyde obtenu à l'étape 1.8, de 0,25 g de Cs₂CO₃ dans 3ml de DMF. On ajoute 0,056 g de bromoacétonitrile et on irradie ensuite le mélange réactionnel sous champ de micro-ondes pendant 15 min à 100°C. On filtre, évapore sous pression réduite. On chromatographie le résidu sur gel de silice en éluant par un mélange MeOH/DCM (1/99, v/v) jusqu'à (4/96, v/v) pour donner 0,112 g du produit attendu.

### EXEMPLE 2 : Composé No.3

### Préparation du {[1-(1-acétylpipéridin-4-yl)-3-(3,4-diméthoxybenzyl)-2,4-dioxo-1,2,3,4-tétrahydroquinazolin-6-yl]oxy}acétonitrile

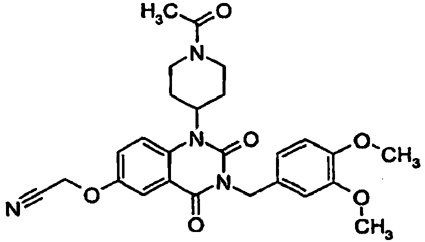

### Etape 2.1 :

### 1-(1-Acétylpipéridin-4-yl)-6-(benzyloxy)-3-(3,4-diméthoxybenzyl)quinazoline-2,4(1H,3H)-dione

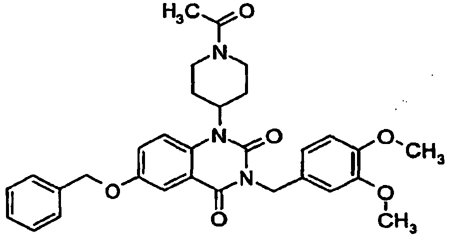

On ajoute 0,14 g de chlorure d'acétyle à un mélange de 0,6 g de 6-(benzyloxy)-3-(3,4-diméthoxybenzyl)-1-pipéridin-4-ylquinazoline-2,4(1H,3H)-dione obtenu selon l'étape 1.6, de 0,24 g de NEt₃ dans 10 ml de DCM refroidit à 0°C. On agite à TA pendant toute la nuit. On lave 2 fois avec une solution saturée de NH₄Cl, filtre, puis évapore le filtrat sous pression réduite pour donner 0,64 g du produit attendu.

### Etape 2.2 : Composé No.2

### 1-(1-Acétylpipéridin-4-yl)-3-(3,4-diméthoxybenzyl)-6-hydroxyquinazoline-2,4(1H,3H)-dione

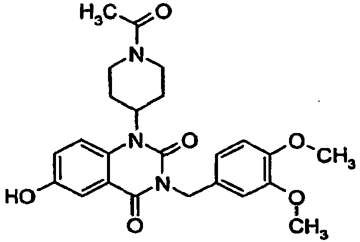

On irradie sous champ de micro-ondes pendant 2h00 à 80°C un mélange de 0,64 g de 1-(1-Acétylpipéridin-4-yl)-6-(benzyloxy)-3-(3,4-diméthoxybenzyl)quinazoline-2,4(1*H*,3*H*)-dione obtenu à l'étape 2.1, de 0,44 g de formiate d'ammonium et de 0,125 g de Pd/C (10%) dans 10 ml d'EtOH préalablement purgé avec de l'azote. On filtre, évapore le filtrat sous pression réduite pour donner 0,48 g du produit attendu.

### Etape 2.3 : Composé No.3

### {[1-(1-Acétyipipéridin-4-yl)-3-(3,4-diméthoxybenzyl)-2,4-dioxo-1,2,3,4-tétrahydroquinazolin-6-yl]oxy}acétonitrile

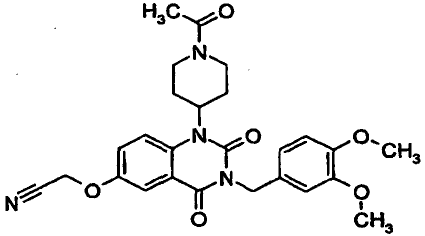

On agite à TA pendant 15min 0,12 g de 1-(1-acétylpipéridin-4-yl)-3-(3,4-diméthoxybenzyl)-6-hydroxyquinazoline-2,4(1H,3H)-dione obtenu à l'étape 2.2, de 0,172 g de Cs₂CO₃ dans 3ml de DMF. On ajoute 0,038 g de bromoacétonitrile et on irradie ensuite le mélange réactionnel sous champ de micro-ondes pendant 15 min à 100°C. On filtre, évapore le filtrat sous pression réduite. On chromatographie le résidu sur gel de silice en éluant par un mélange MeOH/DCM (1/99, v/v) jusqu'à (4/96, v/v) pour donner 0,094 g du produit attendu.

### EXEMPLE 3 : Composé n°34

### Synthèse du 4-[3-(4-chlorobenzyl)-6-(2,2-difluoroéthoxy)-2,4-dioxo-3,4-dihydroquinazolin-1(2H)-yl]pipéridine-1-carbaldéhyde

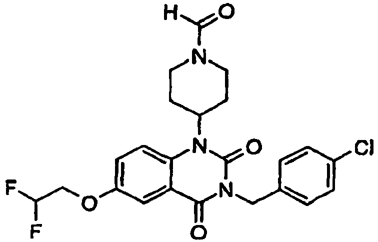

### Etape 3.1 :

### 5-(2,2-Difluoroéthoxy)-2-nitrobenzoate de méthyle

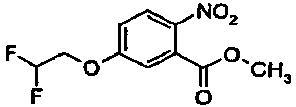

On ajoute 8,53 g de 2,2-difluoroéthanol à une solution de 17,31 g de 5-fluoro-2-nitrobenzoate de méthyle, de 9,64 g de NEt₃, de 32,71 g de 2,8,9-triisobutyl-2,5,8,9-tetraaza-1-phosphabicyclo[3.3.3]undecane dans 250 ml de THF anhydre. On agite 30 min à TA. On évapore le solvant sous pression réduite. On ajoute de l'eau et on extrait à l'AcOEt. On lave avec une solution aqueuse d'HCl 1N, à l'eau puis avec une solution saturée de NaCl. On sèche sur MgSO₄, filtre, évapore le solvant sous pression réduite pour donner 21 g du produit attendu.

### Etape 3.2 :

### 2 Amino-5-(2,2-difluoroéthoxy)benzoate de méthyle

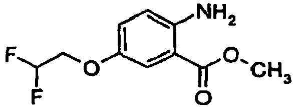

On agite sous atmosphère d'hydrogène pendant 24h00 à TA 21 g de 5-(2,2-difluoroéthoxy)-2-nitrobenzoate de méthyle obtenu à l'étape 3.1 et de 1 g de Pd/C (10%) dans un mélange de 300 ml d'AcOEt, de 50 ml d'EtOH et de 5 ml d'AcOH.

On filtre, évapore sous pression réduite pour donner 18,6 g du produit attendu.

### Etape 3.3 :

### 4-{[4-(2,2-Difluoroéthoxy)-2-(méthoxycarbonyl)phényl]amino}pipéridine-1-carboxylate de 1,1-diméthyléthyle

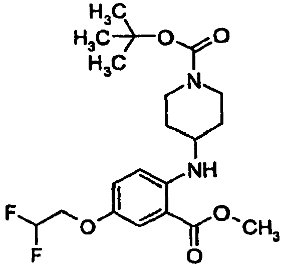

On chauffe à 90°C pendant 10 min un mélange de 4 g de 2-amino-5-(2,2-difluoroéthoxy)benzoate de méthyle, de 6,88 g de 4-oxopipéridine-1-carboxylate de 1,1-diméthyléthyle obtenu à l'étape 3.2 dans 15 ml d'AcOH. On laisse refroidir à TA et on ajoute 7,3 g de NaBH(OAc)₃. On laisse agiter 12h00 à TA. On extrait à l'AcOEt, on lave avec une solution saturée de K₂CO₃, puis à l'eau. On sèche sur MgSO₄, filtre, évapore sous pression réduite pour donner 6,63 g du produit attendu.

### Etape 3.4 :

### 4-{Carbamoyl[4-(2,2-difluoroéthoxy)-2-(méthoxycarbonyl)phényl] amino}pipéridine-1-carboxylate de 1,1-diméthyléthyle

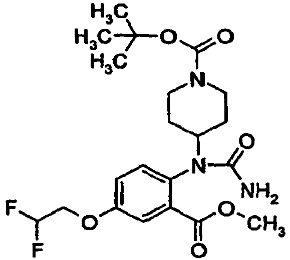

On ajoute 1,95 g d'isocyanate de potassium en solution dans 4 ml d'eau à une solution de 6,63 g de 4-{[4-(2,2-difluoroéthoxy)-2-(méthoxycarbonyl)phényl] amino}pipéridine-1-carboxylate de 1,1-diméthyléthyle obtenu à l'étape 3.3 dans 40 ml d'AcOH. On agite 12h00 à TA. On extrait à l'AcOEt, on lave avec une solution saturée de K₂CO₃, puis à l'eau. On sèche sur MgSO₄, filtre, évapore sous pression réduite pour donner 6,95 g du produit attendu.

### Etape 3.5 :

### 4-[6-{2,2-Difluoroéthoxy)-2,4-dioxo-3,4-dihydroquinazolin-1(2H)-yl]pipéridine-1-carboxylate de 1,1-diméthyléthyle

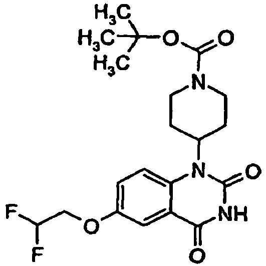

On irradie sous champ de micro-ondes pendant 30 min à 130°C, 2.5g de 4-{carbamoyl[4-(2,2-difluoroéthoxy)-2-(méthoxycarbonyl)phényl]amino}pipéridine-1-carboxylate de 1,1-diméthyléthyle obtenu à l'étape 3.4 en solution dans un mélange de 10 ml de dioxane et de 5ml d'une solution aqueuse de NaOH 1N. On extrait à l'AcOEt, on neutralise avec une solution aqueuse d'HCl 1N, lave à l'eau, sèche sur MgSO₄, filtre, évapore sous pression réduite. Le résidu obtenu est trituré dans un mélange AcOEt/pentane pour donner le produit attendu. La même réaction est reproduite avec 2 autres lots de 2,5 g de 4-{carbamoyl[4-(2,2-difluoroéthoxy)-2-(méthoxycarbonyl)phényl]amino} pipéridine-1-carboxylate de 1,1-diméthyléthyle obtenu à l'étape 3.4 pour donner au total 5,63 g du produit attendu.

### Etape 3.6 :

### 6-(2,2-Difluoroéthoxy)-1-pipéridin-4-ylquinazoline-2,4(1H,3H)-dione

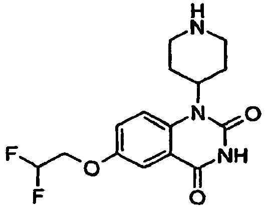

On agite 2h00 à TA une solution de 5,63 g de 4-[6-(2,2-difluoroéthoxy)-2,4-dioxo-3,4-dihydroquinazolin-1(2*H*)-yl]pipéridine-1-carboxylate de 1,1-diméthyléthyle obtenu à l'étape 3.5 dans 70 ml d'acide formique. On évapore le solvant sous pression réduite pour donner 6,13 g du produit attendu sous forme de sel d'acide formique.

### Etape 3.7 :

### 4-[6-(2,2-Difluoroéthoxy)-2,4-dioxo-3,4-dihydroquinazolin-1(2H)-yl]pipéridine-1-carbaldéhyde

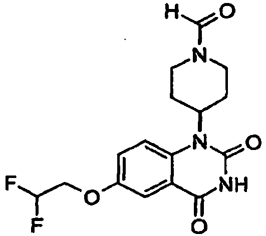

On irradie sous champ de micro-ondes pendant 1h00 à 140°C, un mélange de 6,13 g de 6-(2,2-difluoroéthoxy)-1-pipéridin-4-ylquinazoline--2,4(1*H*,3*H*)-dione obtenu à l'étape 3.6, de 3,12 g de formiate d'ammonium dans 28 ml d'ACN et 28ml de dioxane. On verse le mélange réactionnel dans de l'eau. On filtre, lave le précipité à l'eau puis à l'éther pour donner 4,47 g du produit attendu.

### Etape 3.8 : Composé n°34

### 4-[3-(4-Chlorobenzyl)-6-(2,2-difluoroéthoxy)-2,4-dioxo-3,4-dihydroquinazolin-1(2H)-yl]pipéridine-1-carbaldéhyde

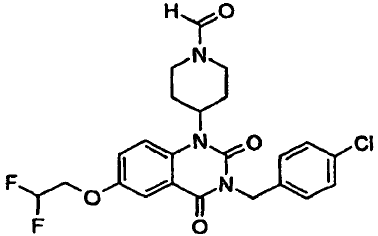

On agite 1h00 à TA un mélange de 0,15 g de 4-[6-(2,2-difluoroéthoxy)-2,4-dioxo-3,4-dihydroquinazolin-1(2*H*)-yl]pipéridine-1-carbaldéhyde obtenu à l'étape 3.7, 0,096 g de 1-(bromométhyl)-4-chlorobenzène et de 0,3 g de Cs₂CO₃ dans 3 ml de DMF. On ajoute de l'AcOEt, lave à l'eau puis avec une solution saturée de NaCl. On sèche sur MgSO₄, filtre, évapore sous pression réduite. On chromatographie le résidu sur gel de silice en éluant à l'AcOEt pour donner 0,116 g du produit attendu.

### Exemple 4 : Composé n°49

### Synthèse du 4-{3-[3-(cyclopentyloxy)-4-méthoxybenzyl]-6-(2,2-difluoroéthoxy)-2,4-dioxo-3,4-dihydroquinazolin-1(2H)-yl}pipéridine-1-carbaldéhyde

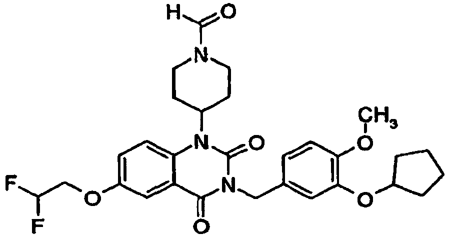

On ajoute 0,172 g de DIAD à une solution de 0,15 g de 4-[6-(2,2-difluoroéthoxy)-2,4-dioxo-3,4-dihydroquinazolin-1(2*H*)-yl]pipéridine-1-carbaldéhyde obtenu à l'étape 3.7, de 0,142 g de [4-(cyclopentyloxy)-3-méthoxyphényl]méthanol et de 0,223 g de PPh₃ dans 3 ml de THF anhydre. On agite 12h00 à TA puis 1h00 à 60°C. On évapore sous pression réduite et on purifie le résidu sur gel de silice en éluant à l'AcOEt pour donner 0,083 g du produit attendu.

### EXEMPLE 5 : Composé n°20

### Synthèse du N-{[3-(3,4-diméthoxybenzyl)-1-(1-formyipipéridin-4-yl)-2,4-dioxo-1,2,3,4-tétrahydroquinazolin-6-yl]méthyl}acétamide

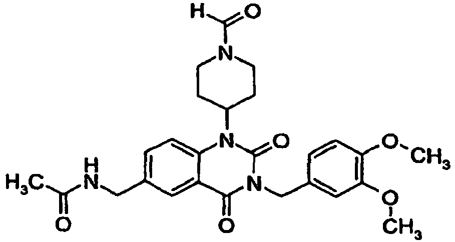

### Etape 5.1 :

### 2-Amino-5-({[(1,1-diméthyléthoxy)carbonyl]amino}méthyl)benzoate de méthyle

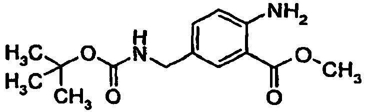

On irradie sous champ de micro-ondes pendant 5 min à 120°C un mélange de 0,273 g de 5-{[(tert-butoxycarbonyl)amino]méthyl}-2-nitrobenzoate de méthyle, 0,166 g de formiate d'ammonium et de 0,094 g de Pd/C (10%) dans 10 ml d'EtOH préalablement purgé avec de l'azote. On filtre, évapore le filtrat sous pression réduite. On chromatographie le résidu sur gel de silice en éluant par un mélange AcOEt/Heptane (5/95, v/v) jusqu'à (30/70, v/v) pour donner 0,2 g du produit attendu.

### Etape 5.2 :

### 4-{[4-({[(1,1-Diméthyléthoxy)carbonyl]amino}méthyl)-2-(méthoxycarbonyl)phényl]amino}pipéridine-1-carboxylate de benzyle

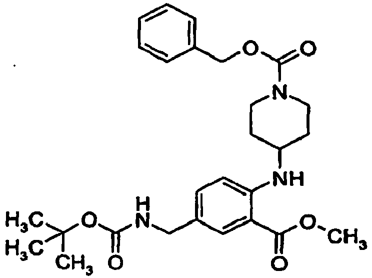

On ajoute goutte à goutte à TA une solution de 1,66 g de 4-oxopipéridine-1-carboxylate de benzyle et de 1g de 2-amino-5-({[(1,1-diméthyléthoxy) carbonyl]amino}méthyl)benzoate de méthyle obtenu à l'étape 5.1 dans 20 ml de DCM à une suspension de 2,04 g de NaBH(OAc)₃ dans un mélange de 20 ml de DCM et de 0,41 ml d'AcOH. On agite 15h00 à TA puis on rajoute 2,04 g de NaBH(OAc)₃. Après 6h00 d'agitation, on ajoute 1,66 g de 4-oxopipéridine-1-carboxylate de benzyle et on agite 48h00 à TA. On ajoute une solution saturée de NaHCO₃, on extrait au DCM. La phase organique est lavée avec une solution saturée de NaHCO₃, 2 fois avec une solution saturée de NH₄Cl. On sèche sur Na₂SO₄, filtre, évapore le filtrat sous pression réduite. On chromatographie le résidu sur gel de silice en éluant par un mélange AcOEt/Heptane (5/95, v/v) jusqu'à (40/60, v/v) pour donner 1,6 g du produit attendu.

### Etape 5.3 :

### 4-{[4-({[(1,1-Diméthyléthoxy)carbonyl]amino}méthyl)-2-(méthoxycarbonyl)phényl](éthoxycarbonyl)amino}pipéridine-1-carboxylate de benzyle

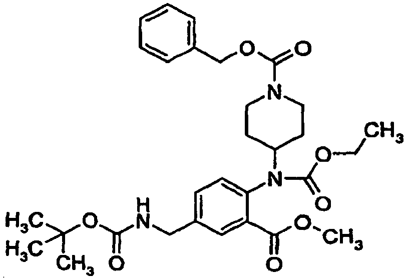

On ajoute 2,08 g de DIEA puis 1,745 g de chloroformate d'éthyle à une solution de 1,6 g de 4-{[4-({[(1,1-Diméthyléthoxy)carbonyl]amino}méthyl)-2-(méthoxycarbonyl) phényl]amino}pipéridine-1-carboxylate de benzyle obtenu à l'étape 5.2 dans 11 ml de DCM. On agite à TA pendant 4 jours. On évapore sous pression réduite. On reprend le résidu dans 10 ml de pyridine (10 ml) et on ajoute 0,7 g de chloroformate d'éthyle. On agite 4h00 à TA. On évapore sous pression réduite. On chromatographie le résidu sur gel de silice en éluant par un mélange AcOEt/Heptane (10/90, v/v) jusqu'à (30/70, v/v) pour donner 0,875 g du produit attendu.

### Etape 5.4 :

### 4-[3-(3,4-Diméthoxybenzyl)-6-({[(1,1-diméthyléthoxy)carbonyl]amino}méthyl)-2,4-dioxo-3,4-dihydroquinazolin-1(2H)-yl]pipéridine-1-carboxylate de benzyle

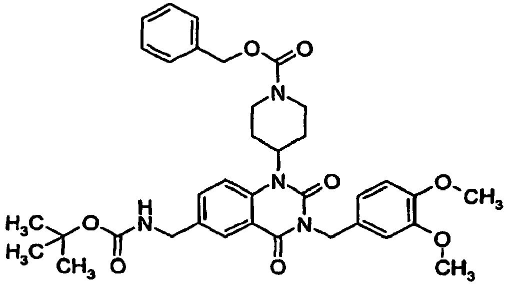

On agite 15h00 à TA un mélange de 0,165 g de 4-{[4-({[(1,1-Diméthyléthoxy) carbonyl]amino}méthyl)-2-(méthoxycarbonyl)phényl](éthoxycarbonyl) amino}pipéridine-1-carboxylate de benzyle obtenu à l'étape 5.3, de 0,028 g de LiOH dans 5 ml de THF/H20 (70/30). On irradie ensuite sous champ de micro-ondes pendant 1h00 à 100°C. On filtre et on évapore le filtrat sous pression réduite. On reprend le résidu dans 5 ml de DMF. On ajoute 0,108 g de DIEA et on agite 10min à TA. On ajoute 0,159 g de HBTU et on agite 30 min à TA. On ajoute ensuite 0,061 g de vératrylamine et on agite 1h00 à TA. On ajoute 0,5 ml de DBU et on agite 48h00 à TA. On évapore sous pression réduite, reprend le résidu dans de l'AcOEt. On lave 3 fois avec une solution saturée de NH₄Cl et 2 fois à l'eau. On sèche sur Na₂SO₄, filtre, évapore le filtrat sous pression réduite. On chromatographie le résidu sur gel de silice en éluant par un mélange AcOEt/DCM (10/90, v/v) jusqu'à (20/80, v/v) pour donner 0,104 g du produit attendu.

### Etape 5.5 :

### 4-[6-(Aminométhyl)-3-(3,4-diméthoxybenzyl)-2,4-dioxo-3,4-dihydroquinazolin-1(2H)-yl]pipéridine-1-carboxylate de benzyle

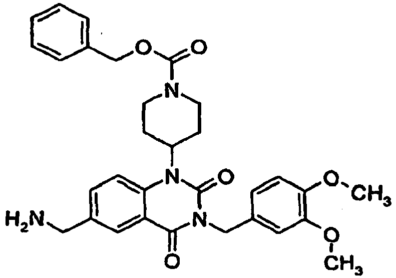

On agite 2h00 à TA une solution de 0,102 g de 4-[3-(3,4-Diméthoxybenzyl)-6-({[(1,1 -diméthyléthoxy)carbonyl]amino}méthyl)-2,4-dioxo-3,4-dihydroquinazolin-1(2*H*)-yl]pipéridine-1-carboxylate de benzyle obtenu à l'étape 5.4 et de 0,5 ml de TFA dans 9,5 ml de DCM. On ajoute une solution saturée de NaHCO₃. On sèche la phase organique sur Na₂SO₄, filtre, évapore le filtrat sous pression réduite pour donner 0,09 g du produit attendu.

### Etape 5.6 :

### 4-{6-[(Acétylamino)méthyl]-3-(3,4-diméthoxybenzyl)-2,4-dioxo-3,4-dihydroquinazolin-1(2H)-yl}pipéridine-1-carboxylate de benzyle

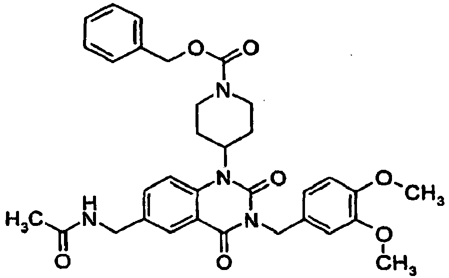

On ajoute 0,049 g d'anhydride acétique à une solution de 0,09 g de 4-[6-(aminométhyl)-3-(3,4-diméthoxybenzyl)-2,4-dioxo-3,4-dihydroquinazolin-1(2*H*)-yl]pipéridine-1-carboxylate de benzyle obtenu à l'étape 5.5, de 0,09 ml de NEt₃ dans 3 ml de DCM et on agite 1h00 à TA. On ajoute du DCM et on lave avec une solution saturée de NH₄Cl, puis par une solution HCl 1N, NaOH 2N puis à l'eau. On sèche la phase organique sur Na₂SO₄, filtre, évapore le filtrat sous pression réduite pour donner 0,104 g du produit attendu.

### Etape 5.7 :

### N-{[3-(3,4-Diméthoxybenzyl)-2,4-dioxo-1-pipéridin-4-yl-1,2,3,4-tétrahydroquinazolin-6-yl]méthyl}acétamide

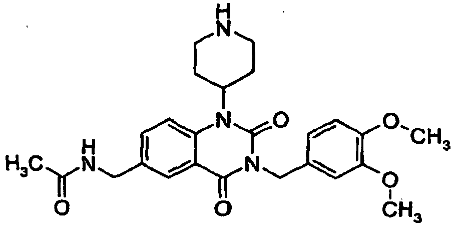

On irradie sous champ de micro-ondes pendant 30 min à 80°C un mélange de 0,1 g de 4-{6-[(acétylamino)méthyl]-3-(3,4-diméthoxybenzyl)-2,4-dioxo-3,4-dihydroquinazolin-1(2*H*)-yl}pipéridine-1-carboxylate de benzyle obtenu à l'étape 5.6, de 0,016 g de formiate d'ammonium et 0,018 g de Pd/C (10%) dans 2 ml d'EtOH préalablement purgé avec de l'azote. On filtre, évapore le filtrat sous pression réduite pour donner 0,077 g du produit attendu.

### Etape 5.8 : Composé n°20

### N-{[3-(3,4-Diméthoxybenzyl)-1-(1-formylpiperidin-4-yl)-2,4-dioxo-1,2,3,4-tétrahydroquinazolin-6-yl]méthyl}acétamide

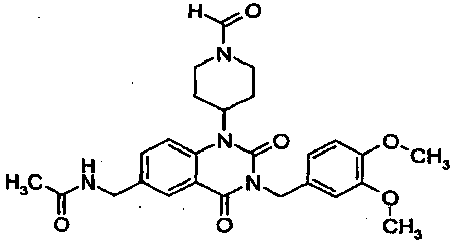

On irradie sous champ de micro-ondes pendant 1h00 à 140°C un mélange de 0,070 g de ***N*-{[3-(3,4-diméthoxybenzyl)-2,4**-**dioxo-1-pipéridin-4-yl-1,2,3,4-**tétrahydroquinazolin-6-yl]méthyl}acétamide obtenu à l'étape 5.7, de 0,028 g de formiate d'ammonium dans 2 ml d'ACN. On filtre, évapore le filtrat sous pression réduite. On chromatographie le résidu sur gel de silice en éluant par un mélange MeOH/DCM (0.5/99.5, v/v) jusqu'à (7/93, v/v) pour donner 0,035 g du produit attendu.

Le tableau suivant illustre les structures chimiques et les propriétés physiques de composés répondant à la formule générale (I) selon l'invention, ainsi que de certains de leurs intermédiaires (notamment les composés 32, 55, 120 et 257)

| ***Composé N°*** | ***STRUCTURE*** | ***NOMENCLATURE*** | ***RMN ou MASSE*** |
|---|---|---|---|
| 1 | 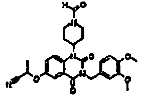 | 2-{[3-(3,4-diméthoxybenzyl)-1-(1-formylpipéridin-4-yl)-2,4-dioxo-1,2,3,4-tétrahydroquinazolin-6-yl]oxy}propanenitrile | ¹H NMR (500 MHz, DMSO-*d*₆) δ ppm 1.71(d, 3H) 1.79(brdd, 2H) 2.41(dq, 1H) 2.53(dq, 1H) 2.80(td, 1H) 3.25(t, 1H) 3.70(s, 3H) 3.71(s, 3H) 3.80(d, 1H) 4.31(d, 1H) 4.77(brs, 1H) 5.03(s, 2H) 5.60(q, 1H) 6.83(dd, 1H) 6.85 (d, 1H) 6,98(s, 1H) 7.51 (dd, 1H) 7.74(d, 1H) 7.84(d, 1H) 8.03(s, 1H) |
| 2 | 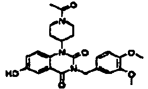 | 1-(1-acétylpipéridin-4-yl)-3-(3,4-diméthoxybenzyl)-6-hydroxyquinazoline-2,4(1H,3H)-dione | ¹H NMR (500 MHz, DMSO-*d*₆) δ ppm 1.69(d, 1H) 1.74(d, 1H) 2.05(s, 3H) 2.42(dq, 1H) 2.57(dq, 1H) 2.70(t, 1H) 3.24(t, 1H) 3.70(br s, 3H) 3.71(s, 3H) 3.92(d, 1H) 4.51(d, 1H) 4.69(brs, 1H) 5.02(s, 2H) 6.81(dd, 1H) 6.86(d, 1H) 6.97(d, 1H) 7.21 (dd, 1H) 7.44(d, 1H) 7.65(d, 1H) 9.88(brs, 1H) |
| 3 | 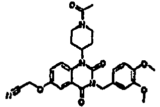 | {[1-(1-acétylpipéridin-4-yl)-3-(3,4-diméthoxybenzyl)-2,4-dioxo-1,2,3,4-tétrahydroquinazolin-6-yl]oxy}acétonitrile | ¹H NMR (500 MHz, DMSO-*d*₆) δ ppm 1.71(d, 1H) 1.76(d, 1H) 2.04(s, 3H) 2.41(dq, 1H) 2.56(dq, 1H) 2.70(t, 1H) 3.24(t, 1H) 3.70(s, 3H) 3.71(s, 3H) 3.92(d, 1H) 4.51(d, 1H) 4.73(brs,1 H) 5.04(s, 2H) 5.29(s, 2H) 6.82(dd, 1H) 6.85(d, 1H) 6.98(d, 1H) 7.50(dd, 1H) 7.70(d, 1H) 7.83(d, 1H) |
| 4 | 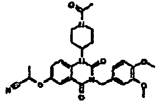 | 2-{[1-(1-acétylpipéridin-4-yl)-3-(3,4-diméthoxybenzyl)-2,4-dioxo-1.2,3,4-tétrahydroquinazolin-6-yl]oxy}propanenitrile | ¹H NMR (500 MHz, DMSO-*d*₆) δ ppm 1.71(d, 3H) 1.72(d, 2H) 1.77(d, 1H) 2.04(s, 3H) 2.41 (qd, 1H) 2.56(dq, 1H) 2.70(d, 1H) 3.24(t, 1H) 3.70(s, 3H) 3.71(s, 3H) 3.92(d, 1H) 4.51(d, 1H) 4.73(brs, 1H) 5.03(s, 2H) 5.60(q, 1H) 6.83(d, 1 H) 6.85(m, 1H) 6.98(d, 1H) 7.51(dd, 1H) 7.74(d, 1H) 7.83(d, 1H) |
| 5 | 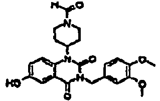 | 4-[3-(3,4-diméthoxybenzyl)-6-hydroxy-2,4-dioxo-3,4-dihydroquinazolln-1(2H)-yl]pipéridine-1-carbaldéhyde | ¹H NMR (500 MHz, DMSO-*d*₆) δ ppm 1.75(d, 1H) 1.78(d, 1H) 2.41(qd, 1H) 2.53(qd, 1H) 2.79(td, 1H) 3.25(td, 1H) 3.70(s, 3H) 3.71(s, 3H) 3.80(d, 1H) 4.31(d, 1H)4.72 (brs, 1H) 5.02(s, 2H) 6.82(dd, 1H) 6.86(d, 1H) 6.97(d, 1H) 7.21(dd, 1H) 7.44(d, 1H) 7.66(d, 1H) 8.03(s, 1H) 9.85(brs, 1H) |
| 6 | 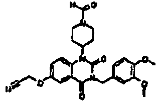 | {[3-(3,4-diméthoxybenzyl)-1-(1-formylpipéridin-4-yl)-2,4-dioxo-1,2,3,4-tétrahydroquinazolin-6-yl]oxy}acétonitrile | ¹H NMR (500 MHz, DMSO-*d*₆) δ ppm 1.78(d, 1H) 1.81(d, 1H) 2.42(qd, 1H) 2.54(dt, 1H) 2.81 (td, 4H) 3.26(dt, 1 H) 3.71(s, 3H) 3.72(s, 3H) 3.81(d, 1 H) 4.32(d, 1H) 4.77(brs, 1H) 5.05(s, 2H) 5.30(s, 2H) 6.84(dd, 1H) 6.87(d, 1H) 6.99(d, 1H) 7.51(dd, 1H) 7.71 (d, 1H) 7.85(d, 1H) 8.04(s, 1H) |
| 7 | 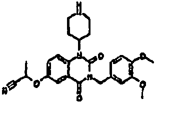 | 2-{[3-(3,4-diméthoxybenzyl)-2,4-dioxo-1-pipéridin-4-yl-1,2,3,4-tétrahydroquinazolin-6-yl]oxy}propanenitrile | ¹H NMR (500 MHz, DMSO-*d*₆) δ ppm1.69(d, 3H) 1.75(d, 2H) 2.65(td, 2H) 2.87(t, 2H) 3.21(d, 2H) 3.69(s, 3H) 3.70(s, 3H) 4.68(brs, 1H) 5.04(s, 2H) 5.59(q, 1H) 6.84(m, 2H) 6.98(d, 1H) 7.50(dd, 1H) 7.73(d, 1H) 7.80(d, 1H) |
| 8 | 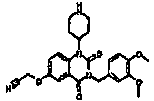 | {[3-(3,4-diméthoxybenzyl)-2,4-dioxo-1-piperidin-yl-1,2,3,4-tétrahydroquinazolin-6-yl]oxy}acétonitrile | ¹H NMR (600 MHz, DMSO-*d*₆) δ ppm 1.77(d, 2H) 2.67(dit, 2H) 2.89(t, 2H) 3.23(d, 2H) 3.69(s, 3H) 3.70(s, 3H) 4.69(brs, 1H) 5.05(s, 2H) 5.28(s, 2H) 6.84(m, 2H) 6.99(s, 1H) 7.49(dd, 1H) 7.70(d, 1H) 7.80(d, 1H) |
| 9 | 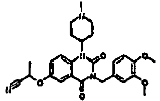 | 2-{[3-(3,4-diméthoxybenzyl)-1-(1-méthylpipéridin-4-yl)-2,4-dioxo-1,2,3,4-tétrahydroquinazolin-6-yl]oxy}propanenitrile | ¹H NMR (500 MHz, DMSO-*d*₆) δ ppm 1.59(d, 2H) 1.66(d, 3H) 2.04(t, 2H) 2.16(s, 3H) 2.62(dt, 2H) 2.82(d, 2H) 3.66(s, 3H) 3.67(s, 3H) 4.43(brs, 1H) 5.01(s, 2H) 5.54(q, 1H) 6.79(m, 1H) 6.82(d, 1H) 6.95(d, 1H) 7.45(dd, 1H) 7.88(d, 1H) 7.70(d, 1H) |
| 10 | 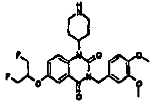 | 3-(3,4-diméthoxybenzyl)-6-[2-fluoro-1-(fluorométhyl)éthoxy]-1-pipéridin-4-ylqulnazoline-2,4(1H,3H)-dione | ¹H NMR (500 MHz, DMSO-*d*₆) δ ppm 1.85(d, 2H) 2.78(td, 2H) 3.04(t, 2H) 3.33(d, 2H) 3.71(s, 6H) 4.61-4.82(m, 4H) 5.04(brt, 1H) 5.05(s, 2H) 6.86(m, 2H) 6.99(s, 1H) 7.49(dd, 1H) 7.68(d, 1H) 7.76(d, 1H) 8.05(brs, 1H) |
| 11 | 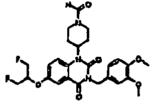 | 4-[3-(3,4-diméthoxybenzyl-6-[2-fluoro-1-(fluorométhyl)éthoxy]-2,4-dioxo-3,4-dihydroquinazolin-1(2H)-yl]pipéridine-1-carbaldéhyde | ¹H NMR (500 MHz, DMSO-*d*₆) δ ppm 1.76(d, 1H) 1.80(d, 1H) 2.41 (qd, 1H) 2.53(qd, 1H) 2.80(td, 1H) 3.25(dt, 1H) 3.70(s, 6H) 3.80(d, 1H) 4.31 (d, 1H) 4.81-4.83(m, 5H) 5.02(m, 1H) 5.03(s, 2H) 6.83(dd, 1H) 6.85(d, 1H) 6.97(d, 1H) 7.47(dd, 1H) 7.67(d, 1H) 7.77(d, 1H) 8.03(s, 1H) |
| 12 | 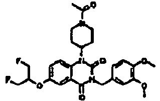 | 1-(1-acétylpipérldln-4-yl)-3-(3,4-diméthoxybenzyl)-6-[2-fluoro-1-(fluorométhyl)éthoxy]quinazoline -2,4(1H,3H)-dione | ¹H NMR (500 MHz, DMSO-*d*₆) δ ppm 1.70(d, 1H) 1.76(d, 1H) 2.04(s, 3H) 2.41(dq, 1H) 256(dq, 1H) 2.70(t, 1H) 3.25(t, 1H) 3.70(s, 3H) 3.71(s, 3H) 3.92(d, 1H) 4.51(d, 1H) 4.61-4.83(m, 5H) 5.02(m, 1H) 5.03(s, 2H) 6.82(dd, 1H) 6.85(m, 1H) 6.97(d, 1H) 7.47(dd, 1H) 7.67(d, 1H) 7.77(d, 1H) |
| 13 | 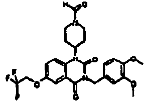 | 4-[3-(3,4-diméthoxybenzyl)-2,4-dioxo-6-(2,2,2-trifluoroéthoxy)-3,4-dihydroquinazolin-1(2H)-yl]pipéridine-1-carbaldéhyde | ¹H NMR (600 MHz, DMSO-*d*₆) δ ppm 1.75(d, 1H) 1.79(d, 1H) 2.40(dq, 1H)) 2.52(dq, 1H) 2.79(td, 1H) 3.25(td, 1H) 3.69(s, 6H) 3.79(d. 1H) 4.30(d, 1H) 4.75(brs, 1H) 4.88(q, 2H) 5.02(s, 2H) 6.82(d, 1H) 6.85(d, 1H) 6.97(s, 1H) 7.49(dd,1H) 7.66(d, 1H) 7.79(d, 1H) 8.02(s, 1H) |
| 14 | 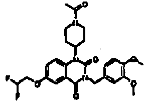 | 1-(1-acétylpipéridin-4-yl)-6-(2,2-difluoroéthoxy)-3-(3,4-diméthoxybenzyl)quinazoline-2,4(1H,3H)-dione | ¹H NMR (500 MHz, DMSO-*d*₆) δ ppm 1.70(d, 1H) 1.74(d, 1H) 2.04(s, 3H) 2.41(qd, 1H) 2.58(qd, 1H) 2.70(t, 1H) 3.24(t, 1H) 3.70(t, 3H) 3.70(s, 3H) 3.91(d, 1H) 4.43(td, 2H) 4.50(d, 1H) 4.73(brs,1H) 5.03 (s, 2H) 6.41(tt, 1H) 6.82(dd, 1H) 6.85(d, 1H) 6.97(d, 1H) 7.46(dd, 1H) 7.60(d, 1H) 7.79(d, 1H) |
| 15 | 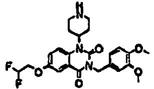 | 6-(2,2-difluoroéthoxy)-3-(3,4-diméthoxybenzyl)-1-pipéridin-4-ylquinazoline-2,4(1H,3H)-dione | ¹H NMR (500 MHz, DMSO-*d*₆) δ ppm 1.83(d, 2H) 2.75(td, 2H) 3.00(t, 2H) 3.30(d, 2H) 3.71(s, 6H) 4.44(td, 2H) 4.75(b. s, 1H) 5.06(s, 2H) 6.42(tt, 1H) 6.83-6.89(m, 2H) 7.00(s, 1H) 7.48(dd, 1H) 7.62(d, 1H) 7.78(d, 1H) |
| 16 | 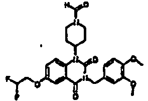 | 4-[6(2,2-difluoroéthoxy)-3-(3,4-diméthoxybenryl)-2,4-dioxo-3,4-dihydroquinazolin-1(2H)-yl]pipéridine-1-carbaldéhyde | ¹H NMR (500 MHz, DMSO-*d*₆) δ ppm 1.76(d, 1H) 1.80(d, 1H) 2.41(qd, 1H) 2.53(qd, 1H) 2.81(td, 1H) 3.26(td, 1H) 3.71(s, 6H) 3.80(d, 1H) 4.31(d, 1H) 4.44(td, 2H) 4.77(brs, 1H) 5.04(s, 2H) 6.42(tt, 1H) 6.83(m, 1H) 6.86(d, 1H) 6.86(d, 1H) 6.98(d, 1H) 1.47(dd, 1H) 7.61 (d, 1H) 7.80(d, 1H) 8.03(s, 1H) |
| 17 | 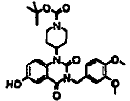 | 4-[3-(3,4-diméthoxybenzyl)-6-hydroxy-2,4-dioxo-3,4-dihydroquinazolin-1 (2H)-yl]pipéridine-1-carboxylate de 1,1-diméthyléthyle | ¹H NMR (500 MHz, DMSO-*d*₆) δ ppm 1.43(s, 9H) 1.67(d, 2H) 2.48(m, 2H) 2.92(brs, 2H) 3.70(s, 3 H) 3.71 (s, 3H) 4.05(br s, 2H) 4.61 (brs, 1H) 5.02(s, 2H) 6.80(dd, 1H) 6.86(d, 1H) 6.97(d, 1H) 7.20(dd, 1H) 7.43(d, 1H) 7.82(d, 1H) 9.95(brs, 1H) |
| 18 | 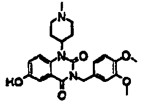 | 3-(3,4-diméthoxybenzyl)-6-hydroxy-1-(1-méthylpipéridin-4-yl)quinazoline-2,4(1H,3H)-dione | ¹H NMR (500 MHz, DMSO-*d*₆) δ ppm 1.60(d, 2H) 2.06(td, 2H) 2.19(s, 3H) 2.65(qd, 2H) 2.85(d, 2H) 3.70(s, 3 H) 3.71(s, 3H) 4.43(br.s, 1H) 5.02(s, 2H) 6.81(m, 1H) 8.85(d, 1H) 6.98(d, 1H) 7.19(dd, 1H) 7.41(d, 1H) 7.55(d, 1H) 9.82(s, 1H) |
| 19 | 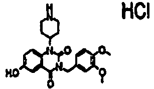 | chlorhydrate de 3-(3,4-diméthoxybenzyl)-6-hydroxy-1-pipéridin-4-ylquinazoline-2,4(1H,3H)-dione | ¹H NMR (500 MHz, DMSO-*d*₆) δ ppm 1.86(d, 2H) 2.83(m, 2H) 3.12(t, 2H) 3.37(m, 2H) 3.70(s, 6H) 4.78(brs, 1H) 5.03(s, 2H) 6.83(dd,1H) 8.85(d,1H) 6.98(d, 1H) 7.21(dd, 1H) 7.44(d, 1H) 7.70(d, 1H) 8.70(brs, 2H) 9.91(s, 1H) |
| 20 | 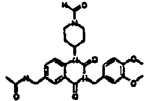 | N-{[3-(3,4-diméthoxybenzyl)-1-(1-formylpipéridin-4-yl)-2,4-dioxo-1,2,3,4-tétrahydroqulnazolin-6-yl]méthyl}acétamide | ¹H NMR (500 MHz, DMSO-*d₆*) δ pm1.75(d, 1H) 1.78(d, 1H) 1.86(s, 3H) 2.40(qd, 1H) 2.53(qd, 1H) 2.79(t, 1H) 3.25(t, 1H) 3.69(s, 3H) 3.70(s, 3H) 3.80(d, 1H) 4.29(d, 2H) 4.31 (d., 1 H) 4.77(brs, 1H) 5.02(s, 2H) 6.80(dd, 1H) 6.85(d, 1H) 6.96(d, 1H) 7.65(dd, 1H) 7.76(d, 1H) 7.96(d, 1H) 8.02(s, 1H) 8.46(t, 1H) |
| 21 | 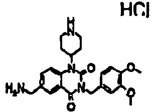 | chlorhydrate de 6-(aminométhyl)-3-(3,4-diméthoxybenzyl)-1-pipéridin-4-ylquinazoline-2,4(1H,3H)-dione | 1H NMR (500 MHz, DMSO-*d*₆) δ ppm 1.89(d, 2H) 2.83-2.92(m, 2H) 3.16(brs, 2H) 3.38-3.42(m, 2H) 3.71(s, 6H) 4.13(brs, 2H) 4.82-4.97(m, 1H) 5.07(s, 2H) 6.83-6.88(m, 2H) 7.00(s, 1H) 7.91-7.99(m, 2H) 8.24(s, 1H) 8.42(br s, 3H) 9.16(brs, 1H) |
| 22 | 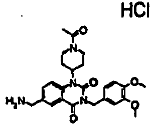 | chlorhydrate de 1-(1-acétylpipéridin-4-yl)-6-(aminométhyl)-3-(3,4-diméthoxybenzyl)quinazoline-2.4(1H,3H)-dione | ¹H NMR (500 MHz, DMSO-*d*₆) δ ppm 1.67-1.79(m, 2H) 2.05(s, 3H) 2.36-2.47(m, 1H) 2.51-2.61 (m, 1H) 2.73(t, 1H) 3.27(t, 1H) 3.71 (s, 6H) 3.93(d, 1H) 4.11-4.15(m, 2H) 4.52(d, 1H) 4.78(brs, 1H) 5.05(s, 2H) 6.82(d, 1H) 6.86(d, 1H) 6.98(s, 1H) 7.87(s, 2H) 8.24(s, 1H) 8.32(brs, 3H) |
| 23 | 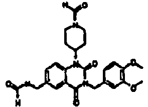 | N-{[-(3,4-diméthoxybenzyl)-1-(1-formylpipéridin-4-yl)-2,4-dioxo-1,2,3,4-tétrahydroquinazolin-6-yl]méthyl}formamide | ¹H NMR (500 MHz, DMSO-*d*₆) δ ppm 1.73-1.83(m, 2H) 2.36-2.45(m., 1H) 2.48-2.57(m, 1H) 2.77-2.84(m, 1H) 3.23-3.29(m, 1H) 3.70(s, 1H) 3.71(s, 3H) 3.81(d, 1H) 4.31(d, 1H) 4.37(d, 2H) 4.77(brs, 1H) 5.04(s, 2H) 6.82(dd, 1H) 6.86(d, 1H) 6.98(d, 1H) 7.67(dd, 1H) 7.78(d, 1H) 7.99(d, 1H) 8.03(s, 1H) 8.16(s, 1H) 8.82(t, 1H) |
| 24 | 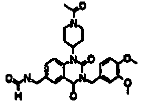 | N-{(1-(1-acétylpipéridin-4-yl)-3-(3,4-diméthoxybenzyl)-2,4-dioxo-1,2,3,4-tétrahydroquinazolin-6-yl]méthyl}formamide | ¹H NMR (500 MHz, DMSO-*d₆*) δ ppm 1.67-1.78(m, 2H) 2.05(s, 3H) 2.35-2.48(m, 1H) 2.53-2.82(m, 1H) 2.72(t, 1H) 3.25(t, 1H) 3.70(s, 3H) 3.71(s, 3H) 3.93(d,1H) 4.37(d, 2H) 4.52(d, 1H) 4.75(brs, 1H) 5.04(s, 2H) 6.82(dd, 1H) 6.86(d, 1H) 6.98(d, 1H) 7.67(dd, 1H) 7.78(d,1H) 7.99(d, 1H) 8.16(s, 1H) 8.63(t, 1H) |
| 25 | 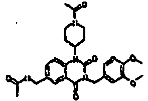 | N-{[1-(1-acétylpipéridin-4-yl)-3-(3,4-diméthoxybenzyl)-2,4-dioxo-(3,4-diméthoxybenzyl)-2,4-dioxo-1,2,3,4-tétrahydroquinazolin-6-yl]méthyl}acétamide | ¹H NMR (500 MHz, OMSO-*d*₆) δ ppm 1.63-1.81(m, 2H) 1.87(s, 3H) 2.05(s, 3H) 2.32-2.42(m, 1H) 2.54-2.63(m, 1H) 2.71 (dt, 1 H) 3.25(dt, 1H) 3.70(s, 3H) 3.71(s, 3H) 3.93(d, 1H) 4.30(d, 2H) 4.52(d, 1H) 4.76(brs, 1H) 5.04(s, 2H) 6.81 (dd 1H) 6.86(d, 1H) 6.97(d, 1H) 7.65(dd, 1H) 7.76(d, 1H) 7.97(d, 1 H) 8.47(t, 1H) |
| 26 | 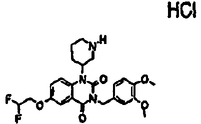 | chlorhydrate de 6-(2,2-difluoroéthoxy)-3-(3,4-diméthoxybenzyl)-1-pipéridin-3-ylquinazoline-2,4(1H,3H)-dione | ¹H NMR (600 MHz, DMSO-*d*₆) δ ppm 1.86-1.97(m, 3H) 2.54-2.62(m, 1H) 2.85-2.92(m, 1H) 3.31-3.35(m, 1H) 3.37-3.42(m, 1H) 3.71(s, 3H) 3.72(s, 3H) 3.73-3.78(m,1H) 3.78(t, 1H) 4.45(td, 2H) 4.87(br s, 1H) 5.05(s, 2H) 6.41(tt, 1H) 6.83-6.88(m, 2H) 6.99(s, 1H) 7.52(dd, 1H) 7.63(d, 1H) 7.72(d, 1H) 8.95(brs, 1H) |
| 27 | 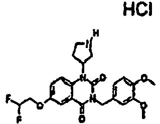 | chlorhydrate de 6-(2,2-difluoroéthoxy)-3-(3,4-diméthoxybenzyl)-1-pyrrolidin-3-ylquinazoline-2,4(1H,3H)-dione | ¹H NMR (500 MHz, DMSO-*d*₆) δ ppm 2.25-2.32(m, 1H) 2.42-2.48(m, 1H) 3.11-3.19(m, 1H) 3.38-3.45(m, 1H) 3.58-3.64(m, 1H) 3.65-3.72(m, 1H) 3.73(s, 3H) 3.71(s, 3H) 4.45(td, 2H) 5.05-5.12(m, 2H) 5.47-5.54(m, 1H) 6.42(tt, 1H) 6.82-6.88(m, 2H) 7.01(s, 1H) 7.55(dd, 1H) 7.62(d, 1H) 7.71(d, 1H) 8.87(brs, 1H) |
| 28 | 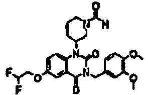 | 3-[6-(2,2-difluoroéthoxy)-3-(3,4-diméthoxybenzyl)-2,4-dioxo-3,4-dihydroquinazolin-1(2H)-yl]pipéridine-1-carbaldéhyde | ¹H NMR (600 MHz, DMSO-*d*₆) mélange de deux isomères δ ppm 1.52-1.62(m, 0.5H) 1.64-1.73(m, 0.5H) 1.75-1.84(m, 1H) 1.84-1.90(m, 1H) 2.54-2.67(m, 1.5H) 3.05(t, 0.5H) 3.59(t, 0.5H) 3.70(s, 3.5H) 3.70(s, 3H) 3.77(dd, 0.5H) 4.06(t, 0.5H) 4.17-4.27(m, 1H) 4.31 (brs, 0.5H) 4.42(td, 2H) 4.44(brs, 0.5H) 5.04(s, 2H) 6.38(tt, 1H) 6.80-6.84(m, 1H) 6.84-6.88(m, 1H) 6.98(brs, 1H) 7.43(td, 1H) 7.60(brs, 1H) 7.62(d, 0.5H) 7.79(d, 0.5H) 7.99(s, 0.5H) 8.09(s, 0.5H) |
| 29 | 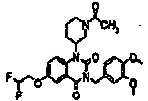 | 1-(1-acétylpipéridin-3-yl)-6-(2,2-difluoroéthoxy)-3-(3,4-diméthoxybenzyl)quinazoline-2,4(1H,3H)-dione | 1H NMR (500 MHz, DMSO-*d*₆) mélange de deux isomères δ ppm 1.55-1.65(m. 0.5H) 1.67-1.89(m, 2.5H) 1.99(s, 1.5H) 2.06(s, 1.5H) 2.52-2.65(m, 1H) 3.06(t, 0.5H) 3.30-3.37(m, 0.5H) 3.52(t, 0.5H) 3.70(s, 3H) 3.71(s, 3H) 3.52(t, 0.5H) 3.84(d, 0.5H) 3.91 (d, 0.5H) 4.09(t, 0.5H) 4.28(brs, 0.5H) 4.39-4.48(m, 3H) 4.49(brs, 0.5H) 5.05(d, 2H) 6.41(tt, 1H) 6.81-6.89(m, 2H) 6.99(d, 1H) 7.44-7.48(m, 1H) 7.59-7.62(m, 1H) 7.64(d, 0.5H) 7.82(d, 0.5H) |
| 30 | 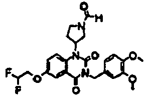 | 3-[6-(2,2-difluoroéthoxy)-3-(3,4-diméthoxybenzyl)-2,4-dioxo-3,4-dihydroquinazolin-1(2H)-yl]pyrrolidine-1-carbaldéhyde | 1H NMR (500 MHz, DMSO-*d*₆) mélange de deux isomères δ ppm 2.18-2.27(m, 1H) 2.36-2.46(m, 1H) 3.41-3.48(m, 0.5H) 3.53-3.60(m, 0.5H) 3.63-3.73(m, 1.5H) 3.85-3.95(m, 1.5H) 4.43(td, 2H) 5.06(s, 2H) 5.38-5.45(m, 0.5H) 5.46-5.54(m, 0.5H) 6.41(tt, 1H) 6.82-6.88(m, 2H) 7.00(s, 1H) 7.46 - 7.51(m, 1H) 7.61(d, 1H) 7.66(d, 0.5H) 7.72(d, 0.5H) 8.16(s, 0.5H) 8.20(s, 0.5H) |
| 31 | 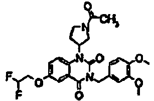 | 1-(1-acétylpyrrolidin-3-yl)-6-(2,2-difluoroéthoxy)-3-(3,4-diméthoxybenzyl)quinazoline-2,4(1H,3H)-dione | ¹H NMR (500 MHz, DMSO-*d*₆) mélange de deux isomères δ ppm1.98(s, 1.5H) 2.04(s, 1.5H) 2.17-2.25(m, 0.5H) 2.27-2.35(m, 0.5H) 2.57-2.66(m, 1H) 3.40-3.45(m, 0.5H) 3 56-3.61(m, 0.5H) 3.70-3.78(m, 7.5H) 3.82-3.95(m, 1.5H) 4.48(td, 2H) 5.10(d, 2H) 5.45-5.58(m, 1H) 6.46(tt, 1H) 6.87-6.92(m, 2H) 7.05(dd, 1H) 7.53(td, 1H) 7.66(t, 1H) 7.72 (dd, 1H) |
| 32 | 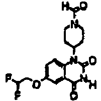 | 4-[6-(2,2-difluoroéthoxy)-2,4-dioxo-3,4-dlhydroquinazolin-1(2H)-yl]pipéridine-1-carbaldéhyde | ¹H NMR (600 MHz, DMSO-*d*₆) δ ppm 1.72-1.82(m, 2H) 2.35-2.44(m, 1H) 2.47-2.55(m, 1H) 2.79(td, 1H) 3.25(td, 1H) 3.80(d, 1H) 4.32(d, 1H) 4.42(td, 2H) 4.71 (brs, 1H) 6.40(tt, 1H) 7.43(dd, 1H) 7.55(d, 1H) 7.74(d, 1H) 8.03(s, 1H) |
| 33 | 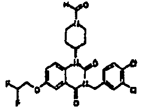 | 4-[3-(3,4-dichlorobenzyl)-6-(2,2-difluoroéthoxy)-2,4-dioxo-3,4-dihydroquinazolin-1(2H)-yl]pipéridine-1-carbaldéhyde | ¹H NMR (500 MHz, DMSO-*d*₆) δ ppm 1.80(dd, 2H) 2.32-2.43(m, 1H) 2.44-2.52(m, 1H) 2.80(td, 1H) 3.26(dt, 1H) 3.80(brd, 1H) 4.31(br d, 1H) 4.44(td, 2H) 4.78(brs, 1H) 5.09(s, 2H) 6.41(tt, 1H) 7.30(dd, 1H) 7.49(dd, 1H) 7.58(d, 1H) 7.60(d, 1H) 7.62(d, 1H) 7.81(d, 1H) 8.03(s, 1H) |
| 34 | 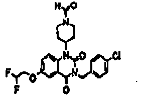 | 4-[3-(4-chlorobenzyl)-6-(2,2-difluoroéthoxy)-2,4-dioxo-3,4-dihydroquinazolin-1 (2H)-yl]pipéridine-1-carbaldéhyde | ¹H NMR (500 MHz, DMSO-*d*₆) δ ppm 1.72-1.83(m, 2H) 2.32-2.44(m, 1H) 2.45-2.53(m, 1H) 2.80(dt, 1H) 3.25-3.30(dt, 1H) 3.80 (d, 1H) 4.30 (d, 1H) 4.44 (td, 2H) 4.78(brs, 1H) 5.09(s, 2H) 6.42 (tt, 1H) 7.32-7.40(m, 4H) 7.48(dd, 1H) 7.61(d, 1H) 7.81(d, 1H) 8.03(s, 1H) |
| 35 | 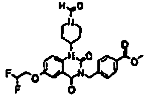 | 4-{[6-(2,2-difluoroéthoxy)-1-(1-formylpipéridin-4-yl)-2,4-dioxo-1,4-dihydroquinazolin-3(2H)-yl]méthyl}benzoate de méthyle | ¹H NMR (500 MHz, DMSO-*d*₆) δ ppm 1.78(dd, 1H) 2.33-2.42(m, 1H) 2.44-2.52(m, 1H) 2.80(dt, 1H) 3.26(dt, 1H) 3.79(brd, 1H) 3.84(s, 3H) 4.30(brd, 1H) 4.45(td, 2H) 4.79(brs, 1H) 5.18(s, 2 H) 6.42(tt, 1H) 7.43(d, 2H) 7.49(dd, 1H) 7.62(d, 1H) 7.83(d, 1H) 7.91(d, 2H) 8.02(s, 1H) |
| 36 | 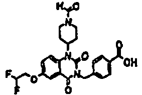 | acide 4-{[6-(2,2-difluoroéthoxy)-1-(1-formylpipéridin-4-yl)-2,4-dioxo-1,4-dihydroquinazolin-3(2H)-yl]méthyl}benzoïque | ¹H NMR (500 MHz, DMSO-*d*₆) δ ppm 1.79(dd, 2H) 2.38(qd, 1H) 2.49(qd, 1H) 2.80(dt, 1H) 3.26(dt, 1H) 3.79(d, 1H) 4.31 (d, 1H) 4.45(td, 2H) 4.79(brs, 1H) 5.17(s, 2H) 6.42(tt, 1 H) 7.40(d, 2H) 7.49(dd, 1H) 7.62(d, 1H) 7.83(d, 1H) 7.88(d, 2H) 8.02(s, 1H) 12.94(brs, 1H) |
| 37 | 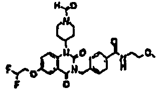 | 4-{[6-(2,2-difluoroéthoxy)-1-(1-formylpipéridin-4-yl)-2,4-dioxo-1,4-dihydroquinazolin-3(2H)-yl]méthyl}-N-(2-méthoxyéthyl)benzamide | 1H NMR (500 MHz, DMSO-*d*₆) δ ppm 1.72-1.82(m, 2H) 2.32-2.42(m, 1H) 2.44-2.54(m, 1H) 2.75-2.83(m, 1H) 3.21-3.29(m, 4H) 3.37-3.45(m, 4H) 3.77(br.d., 1H) 4.29(br.d., 1H) 4.44(td, 2H) 4.78(br.s., 1H) 5.14(s, 2H) 6.41(tt, 1H) 7.35(m, 2H) 7.48(dd, 1H) 7.61(d, 1H) 7.77(m, 2H) 7.81(d, 1H) |
| 38 | 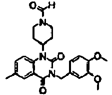 | 4-[3-(3,4-diméthoxybenzyl)-6-méthyl-2,4-dioxo-3,4-dihydroquinazolin-1 (2H)-yl]pipéridine-1-carbaldéhyde | ¹H NMR (500 MHz, DMSO-*d*₆) δ ppm 1.76(dd, 2H) 2.37(s, 3H) 2.39-2.42(m, 1H) 2.50-2.54(m, 1H) 2.77-2.82(m, 1H) 3.19-3.30(m, 1H) 3.70(s, 6H) 3.79(d, 1H) 4.30(d, 1H) 4.76(brs, 1H) 5.02(s, 2H) 6.82(dd, 1H) 6.85(d, 1H) 6.97(d, 1H) 7.60(dd, 1H) 7.71(d, 1H) 7.89(s, 1H) 8.03(s, 1H) |
| 39 | 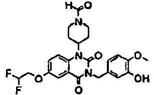 | 4-[6-(2,2-difluoroéthoxy)-3-(3-hydroxy-4-méthoxybenzyl)-2,4-dioxo-3,4-dihydroquinazolin-1(2H)-yl]pipéridine-1-carbaldéhyde | ¹H NMR (500 MHz, DMSO-*d*₆) δ ppm 1.70-1,80(m, 2H) 2.35-1,45(m, 1H) 2.47-2.56(m, 1H) 2.80(dt, 1H) 3.25(dt, 1H) 3.70(s, 3H) 3.79(brd, 1H) 4.30(brd, 1H) 4.43(td, 2H) 4.77(brs, 1H) 4.96(s, 2H) 6.41 (tt, 1H) 6.73(d, 1H) 6.78-6.82(m, 2H) 7.46(dd, 1H) 7.60(d, 1H) 7.79(d, 1H) 8.03(s, 1H) 8.95 (s, 1H) |
| 40 | 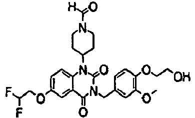 | 4-[6-(2,2-difluoroéthoxy)-3-[3-(2-hydroxyéthoxy)-4-méthoxybenzyl]-2,4-dioxo-3,4-dihydroquinazolin-1 (2H)-yl]pipéridine-1-carbaldéhyde | 1H NMR (600 MHz, DMSO-*d*₆) δ ppm 1.76(dd, 2H) 2.35-2.45(m, 1H) 2.50-2.54(m, 1H) 2.79(dt, 1H) 3.25(dt, 1H) 3.68(dt, 2H) 3.70(s, 3H) 3.79(brd, 1H) 3.90(t, 2H) 4.30(brd, 1H) 4.42(td, 2H) 4.75(brs, 1H) 4.77(t, 1H) 5.01(s, 2H) 6.39(tt, 1H) 6.85(m, 2H) 6.97(s, 1H) 7.45(dd, 1H) 7.60(d, 1H) 7.77(d, 1H) 8.02(s, 1H) |
| 41 | 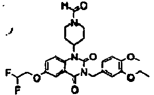 | 4-[6-(2,2-difiuoroéthaxy)-3-(3-éthoxy-4-méthoxybenzyl)-2,4-dioxo-3,4-dihydroquinazolin-1(2H)-yl]pipéridine-1-carbaldéhyde | ¹H NMR (600 MHz, DMSO-*d*₆) δ ppm 1.29(t, 3H) 1.76(dd, 2H) 2.33-2.45(m, 1H) 2.50-2.56(m, 1H) 2.76-2.82(m, 1H) 3.21-3.31(m, 1H) 3.70(s, 3H) 3.79(brd, 1H) 3.94(q, 2H) 4.30(brd, 1H) 4.41 (dt, 2H) 4.75(brs, 1H) 5.01(s, 2H) 6.39(tt, 1H) 6.81-6.86(m, 2H) 6.96(s, 1H) 7.45(dd, 1H) 7.60(d, 1H) 7.77(d, 1H) 8.02(s, 1H) |
| 42 | 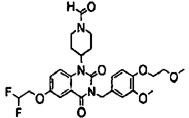 | 4-[6-(2,2-difluoroéthoxy)-3-[4-méthoxy-3-(2-méthoxyéthoxy)benzyl]-2,4-dioxo-3,4-dihydroquinazolin-1 (2H)-yl]pipéridine-1-carbaldéhyde | ¹H NMR (600 MHz, DMSO-*d*₆) δ ppm 1.69-1.82(m, 2H) 2.35-2.45(m, 1H) 2.45-2.55(m, 1H) 2.78(t, 1H) 3.21-3.28(m, 1H) 3.61(brs, 2H) 3.70(s, 3H) 3.78(d, 1H) 4.00(brs. 2H) 4.30(d, 1H) 4.42(t, 2H) 4.75(brs, 1H) 5.01(s, 2H) 6.39(t, 1H) 6.82- 6.88(m, 2H) 6.97(s, 1H) 7.45(d, 1H) 7.59(s, 1H) 7.77(d, 1H) 8.02(s, 1H) |
| 43 | 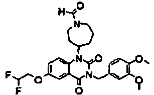 | 4-[6-(2,2-difluoroéthoxy)-3-(3,4-diméthoxybenzyl)-2,4-dioxo-3,4-dihydroquinazolin-1(2H)-yl]azépane-1-carbaldéhyde | ¹H NMR (500 MHz, DMSO-*d*₆) mélange de deux isomères δ ppm 1.72-1.81 (m, 1H) 1.85(brd, 1H) 1.86-2.03(m, 2H) 2.47(brs, 2H) 3.30-3.40(m, 1 H) 3.42-3.52(m, 1H) 3.58-3.64(m, 2H) 3.70(s, 3H) 3.71(s, 3H) 4.42(td, 2H) 4.45(brs, 0.5H) 5.04(brs, 2H) 5.21(brs, 0.5H) 6.40(tt, 1H) 6.78-6.83(m, 1H) 6.86(d, 1H) 6.98(s, 1H) 7.38-7.48(m, 1H) 7.59(brs, 1H) 7.64-7.74(m, 1H) 8.08(brs, 0.5H) 8.12(s, 0.5H) |
| 44 | 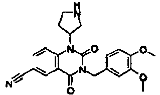 | 3-(3,4-diméthoxybenzyl)-2,4-dioxo-1-pyrrolidin-3-yl-1,2,3,4-tétrahydroquinazoline-6-carbonitrile | ¹H NMR (600 MHz, DMSO-*d*₆) δ ppm 1.96-2.09(m, 2H) 2.78-2.84(m, 1H) 3.05-3.12(m, 2H) 3.18-3.27(m, 1H) 3.70(s, 3H) 3.71(s, 3H) 5.04(s, 2H) 5.47-5.53(m, 1H) 6.83(brs, 2H) 6.99(s, 1H) 8.15(dd, 1H) 8.20(d, 1H) 8.47(d, 1H) |
| 45 | 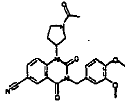 | 1-(1-acétylpyrrolidin-3-yl)-3-(3,4-diméthoxybenzyl)-2,4-dioxo-1,2,3,4-tétrahydroquinazoline-6-carbonitrile | ¹H NMR (600 MHz, DMSO-*d*₆) mélange de deux isomères δ ppm 1.91 (s, 1.5H) 1.98(s, 1.5H) 2.14-2.22(m, 0.5H) 2.25-2.32(m, 0.5H) 2.43-2.50(m, 0.5H) 2.52-2.58(m, 0.5H) 3.31-3.39(m, 0.5H) 3.50-3.55(m, 0.5H) 3.63-3.73(m, 7.5H) 3.79-3.87(m, 1.5H) 5.00-5.06(m, 2H) 5.43-5.55(m, 1H) 6.82-6.90(m, 2H) 6.98(d, 1H) 7.84(dd, 1H) 8.17(dt, 1H) 8.43(dd, 1H) |
| 46 | 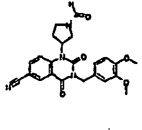 | 3-(3,4-diméthoxybenzyl)-1-(1-formylpyrrolidin-3-yl)-2,4-dioxo-1,2,3,4-tétrahydroquinazoline-6-carbonitrile | ¹H NMR (500 MHz, DMSO-*d*₆) mélange de deux isomères δ ppm 2.19-2.27(m, 1H) 2.36-2.43(m, 1H) 3.43-3.47(m, 0.4H) 3.53-3.59(m, 0.6) 3.62-3.73(m, 1.6H) 3.70(s, 3H) 3.71 (s, 3H) 3.84-3.94(m, 1.4H) 5.04(s, 2H) 5.42-5.49(m, 0.4H) 5.50-5.56(m, 0.6H) 6.86(s, 2H) 6.99(s, 1H) 7.85(d, 0.6H) 7.90(d, 0.4H) 8.15-8.22(m, 2H) 8.45(s, 1H) |
| 47 | 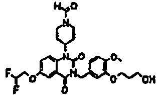 | 4-[8-(2,2-difluoroéthoxy)-3-[3-(3-hydroxypropoxy)-4-méthoxybenzyl]-2,4-dioxo-3,4-dihydroquinazolin-1 (2H)-yl]pipéridine-1-arbaldéhyde | ¹H NMR (600 MHz, DMSO-*d*₆) δ ppm 1.72-1.79(m, 2H) 1.82(q, 2H) 2.36-2.44(m, 1H) 2.49-2.55(m, 1H) 2.78(td, 1H) 3.25(td, 1H) 3.51-3.54(m, 2H) 3.70(s, 3H) 3.76-3.80(m, 1H) 3.95(t, 2H) 4.28-4.32(m, 1H) 4.41(td, 2H) 4.47(t, 1H) 4.75(s, 1H) 5.01(s, 2H) 6.39(tt, 1H) 6.81-6.86(m, 2H) 6.96-6.97(m, 1H) 7.45(dd, 1H) 7.59(d, 1H) 7.77(d, 1H) 8.02(s, 1H) |
| 48 | 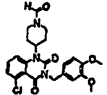 | 4-[5-chloro-3-(3,4-diméthoxybenzy) 2,4-dioxo-3,4-dihydroquinazolin-1(2H)-yl]pipéridine-1-carbaldéhyde | ¹H NMR (500 MHz, DMSO-*d*₆) δ ppm 1.78-1.88(m, 2H) 2.34-2.44(m, 1H) 2.46-2.56(m, 1H) 2.80(t, 1H) 3.25(t, 1H) 3.71(s, 6H) 3.80(d, 1H) 4.31(d, 1H) 4.71 (brs, 1H) 5.00(s, 2H) 6.83(d, 1H) 6.87(d, 1H) 6.97(s, 1H) 7.37(d, 1H) 7.69(dd, 1H) 7.77(d, 1H) 8.03(s, 1H) |
| 49 | 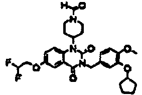 | 4-{3-[3-(cyclopentyloxy)-4-méthoxybenzyl]-6-(2,2-difluoroéthoxy)-2,4-dioxo-3,4-dihydroquinazolin-1 (2H)-yl}pipérid 1-carbaldéhyde | 1H NMR (500 MHz, DMSO-*d*₆) δ ppm 1.5-1.6(m, 2H) 1.63-1.71(m, 3H) 1.68-1.78(m, 1H) 1.78-1.98(m, 3H) 2.35-2.46(m, 1H) 2.47-2.57(m, 1H) 2.79(dt, 1H) 326(dt, 1H) 3.69(s, 3H) 3.79(br.d., 1H) 4.30(br.d., 1H) 4.43(td, 2H) 4.66-4.70(m, 1H) 4.76(br.s., 1H) 5.01(s, 2H) 6.41(tt, 1H) 6.82 - 6.86(m, 2H) 6.96(s, 1H) 7.46(dd, 1H) 7.6(d, 1H) 7.80(d, 1H) 8.03(s, 1H) |
| 50 | 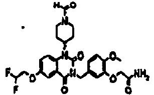 | 2-(5-{[6-(2,2-difluoroéthoxy)-1-(1-formylpipéridin-4-yl)-2,4-dioxo-1,4-dihydroquinazolin-3(2H)-yl]méthyl} méthoxyphénoxy)acétamide | ¹H NMR (500 MHz, DMSO-*d*₆) δ ppm 1.72-1.82(m, 2H) 2.35-2.45(m, 1H) 2.46.2.56(m, 1H) 2.79(td, 1H) 3.25(td, 1H) 3.74(s, 3H) 3.79(d, 1H) 4.31(d, 1H) 4.35(s, 2H) 4.43(td, 2H) 4.76(brs, 1H) 5.00(s, 2H) 6.41(tt, 1H) 6.88-6.95(m, 3H) 7.31(brs, 1H) 7.37(brs, 1H) 7.46(dd, 1H) 7.59(d, 1H) 7.79(d, 1H) 8.02(s, 1H) |
| 51 | 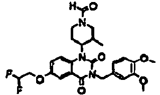 | 4-[6-(2,2-difluoroéthoxy)-3-(3,4-diméthoxybenzyl)-2,4-dioxo-3,4-dihydroquinazolin-1(2H)-yl]-3-méthylpipéridine-1-carbaldéhyde | ¹H NMR (500 MHz, DMSO-*d*₆) mélange de deux isomères δ ppm 0.92(t, 3H) 1.72-1.82(m, 1H) 2.23(brs, 1H) 2.70-2.77(m, 0.5H) 3.00-3.10(m, 0.5H) 3.12-3.20(m, 1.5H) 3.44(d, 0.5H) 3.63(dd, 0.5H) 3.69(s, 6H) 3.80(d, 0.5H) 4.04(d, 0.5H) 4.29(d, 0.5H) 4.43(td, 2H) 4.62-4.70(m, 1H) 5.03(s, 2H) 6.41(tt, 1H) 6.80(dd, 1H) 6.85(d, 1H) 6.97(d, 1H) 7.46(dd, 1H) 7.60(d, 1H) 7.64(dd, 1H) 7.97(s, 0.5H) 8.11(s, 0.5H) |
| 52 | 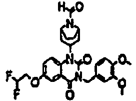 | 3-[6-(2,2-difluoroéthoxy)-3-(3,4-diméthoxybenzyl)-2,4-dioxo-3,4-dihydroquinazolin-1(2H)-yl]-8-azabicyclo[3.2.1]octane-8-carbaldéhyde | 1H NMR (600 MHz, DMSO-*d*₆) δ ppm 1.85-1.95(m, 4H) 2.18(t, 2H) 2.27-2.33(m, 1H) 2.34-2.41(m, 1H) 3.70(s, 1H) 3.71(s, 1H) 4.25-4.29(m, 1H) 4.40(td, 2H) 4.50-4.54(m, 1H) 4.55(br.s., 1H) 5.05(s, 2H) 6.38(tt, H) 6.80(d, 1H) 6.85(d, 1H) 6.98(s, 1H) 7.45(dd, 1H) 7.56(d, 1H) 7.58(d, 1H) 8.12(s, 1H) |
| 53 | 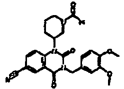 | 3-(3,4-diméthoxybenzyl)-1-(1-formylpipéridin-3-yl)-2,4-dioxo-1,2,3,4-tétrahydroquinazoline-6-carbonitrile | ¹H NMR (500 MHz, DMSO-*d*₆) mélange de deux isomères δ ppm 1.52-1.63(m, 0.5H) 1.85-1.73(m, 0.5H) 1.74-1.83(m, 1H) 1.87-1.93(m, 1H) 2.54-2.64(m, 1.5H) 3.06(dt, 0.5H) 3.57(t, 0.5H) 3.70(s, 3H) 3.71(s, 3H) 3.79(dd, 0.5H) 4.03(t, 0.5H) 4.20(d, 0.5H) 4.30(d, 0.5H) 4.38(brs, 0.5H) 4.53(brs, 0.5H) 4.99-5.07(m, 2H) 6.84-6.88(m, 2H) 6.98(s, 1H) 7.86(d, 0.5H) 8.01(d, 0.5H) 7.98(s, 0.5H) 8.08(s, 0.5H) 8.14-8.18(m, 1H) 8.44(s, 1H) |
| 54 | 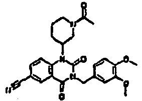 | 1-(1-acétylpipéridin-3-yl)-3-(3,4-diméthoxybenzyl)-2,4-dioxo-1,2,3,4-tétrahydroquinazoline-6-carbonitrile | ¹H NMR (500 MHz, DMSO-*d*₆) mélange de deux isomères δ ppm 1.56-1.66(m, 0.5H) 1.70-1.82(m, 1.5H) 1.87(brd, 1H) 1.97(s, 1.5H) 2.05(s, 1.5H) 2.54(dd, 1H) 3.05(brt, 0.5H) 3.48(t, 0.5H) 3.70-3.72(m, 6H) 3.83(d, 0.5H) 3.94(brd, 0.5H) 4.04(t, 0.5H) 4.33(brs, 0.5H) 4.45(dd, 1H) 4.53(brs, 0.5H) 4.99-5.07(m, 2H) 6.83-6.87(m, 2H) 6.98(d, 1H) 7.84(d, 0.5H) 8.02(d, 0.5H) 8.16(dt, 1H) 8.44(dd, 1H) |
| 55 | 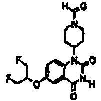 | 4-{6-[2-fluoro-1-(fluoreméthyl)éthoxy]-2,4-dioxo-3,4-dihydroquinazolin-1(2H)-yl}pipéridine-1-carbaldéhyde | 1H NMR (600 MHz, DMSO-*d*₆) δ ppm 1.73-1.82(m, 2H) 2.36-2.44(m, 1H) 2.44-2.55(m, 1H) 2.79(td, 1H) 3.25(td, 1H) 3.80(brd, 1H) 4.32(brd, 1H) 4.62-4.67(m, 1H) 4.68-4.76(m, 3H) 4.76-4.82(m, 1H) 4.98-5.06(m, 1H) 7.44(dd, 1H) 7.81(d, 1H) 7.73(d, 1H) 8.03(s, 1H) 11.46(brs, 1H) |
| 56 | 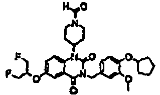 | 4-{3-[4-(cyclopentyloxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde | LCMS m/z [MH]+ 572 |
| 57 | 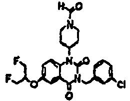 | 4-[3-(3-chlorobenzyl)-6-[2-fluoro-1-(fluorométhyl)éthoxy]-2,4-dioxo-3,4-dihydroquinazolin-1(2H)-yl]pipéridine-1-carbaldéhyde | LCMS m/z [MH]+ 492 |
| 58 | 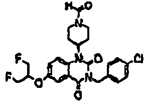 | 4-[3-(4-chlorobenzyl)-6-[2-fluoro-1-(fluorométhyl)éthoxy]-2,4-dioxo-3,4-dihydroquinazolin-1(2H)-yl]pipéridine-1-carbaldéhyde | LCMS m/z [MH]+ 492 |
| 59 | 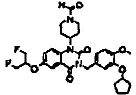 | 4-{3-[3-(cyclopentyloxy)-4-méthoxybenzyl]-6-[2-fluoro-1-(fluorométhyl)éthoxy]-2,4-dioxo-3,4-dihydroquinazolin-1(2H)-yl}pipéridine-1-carbaldéhyde | LCMS m/z [MH]+ 572 |
| 60 | 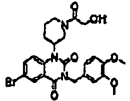 | 6-bromo-3-(3,4-diméthoxybenzyl)-1-[1-(hydroxyacétyl)pipéridin-3-yl]quinazoline-2,4(1H,3H)-dione | LCMS m/z [MH]+ 532 |
| 61 | 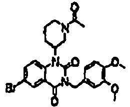 | 1-(1-acétylpipéridin-3-yl)-6-bromo-3-(3,4-diméthoxybenzyl)quinazoline-2,4(1H,3H)-dione | LCMS m/z [MH]+ 516 |
| 62 | 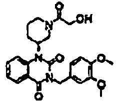 | 3-(3,4-diméthoxybenzyl)-1-[1-(hydroxyacétyl)pipéridin-3-yl]quinazoline-2,4(1H,3H)-dione | LCMS m/z [MH]+ 454 |
| 63 | 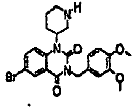 | 6-bromo-3-(3,4-diméthoxybenzyl)-1-[pipéridin-3-yl]quinazoline-2,4(1H,3H)-dione | LCMS m/z [MH]+ 474 |
| 64 | 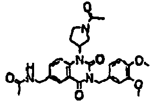 | N-{[1-(1-acétylpyrrolidin-3-yl)-3-(3,4-diméthoxybenzyl)-2,4-dioxo-1,2,3,4-tétrahydroquinazolin-6-yl]méthyl}acétamide | LCMS m/z [MH]+ 495 |
| 65 | 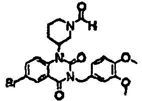 | 3-[6-bromo-3-(3,4-diméthoxybenzyl)-2,4-dioxo-3,4-dihydroquinazolin-1(2H)-yl]pipéridine-1-carbaldéhyde | LCMS m/z [MH]+ 502 |
| 66 | 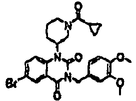 | 6-bromo-1-[1-(cyclopropylcarbonyl)plpéridi n-3-yl]-3-(3,4-diméthoxybenzyl)quinazoline -2,4(1H,3H)-dione | LCMS m/z [MH]+ 542 |
| 67 | 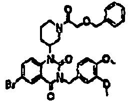 | 1-{1-[(benzyloxy)acétyl]pipéridin-3-yl}-6-bromo-3-(3,4-diméthoxybenzyl)quinazoline-2,4(1H,3H)-dione | LCMS m/z [MH]+ 622 |
| 68 | 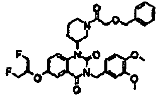 | 1-{1-[(benzyloxy)acétyl]pipéridin-3-yl}-3-(3,4-diméthoxybenzyl)-6-[2-fluoro-1-(fluorométhyl)éthoxy]quinazoline-2,4(1H,3H)-dione | LCMS m/z [MH]+ 638 |
| 69 | 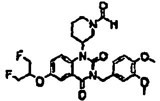 | 3-[3-(3,4-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | LCMS m/z [MH]+ 518 |
| 70 | 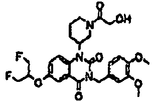 | 3-(3,4-diméthoxybenzyl)-6-[2-fluoro-1-(fluorométhyl)éthoxy]-1-[1-(hydroxyacétyl)pipéridin-3-yl]quinazoline-2,4(1H,3H)-dione | LCMS m/z [MH]+ 548 |
| 71 | 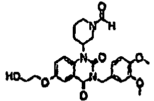 | 3-[3-(3,4-diméthoxybenzyl)-6-(2-hydroxyéthoxy)-2,4-dioxo-3,4-dihydroquinazolin-1 (2H)-yl]pipéridine-1-carbaldéhyde | LCMS m/z [MH]+ 484 |
| 72 | 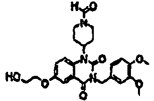 | 4-[3-(3,4-diméthoxybenzyl)-6-(2-hydroxyéthoxy)-2,4-dioxo-3,4-dihydroquinazolin-1(2H)-yl]pipéridine-1-carbaldéhyde | LCMS m/z [MH]+ 484 |
| 73 | 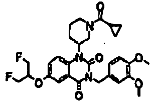 | 1-[1-(cyclopropylcarbonyl)pipéridin-3-yl]-3-(3,4-diméthoxybenzyl)-6-[2-fluoro-1-(fluorométhyl)éthoxy]quinazolin e-2,4(1H,3H)-dione | LCMS m/z [MH]+ 558 |
| 74 | 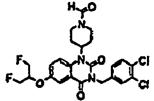 | 4-[3-(3,4-dichlorobenzyl)-6-[2-fluoro-1-(fluorométhyl)éthoxy]-2,4-dioxo-3,4-dihydroquinazolin-1 (2H)-yl]pipéridine-1-carbaldéhyde | LCMS m/z [MH]+ 526 |
| 75 | 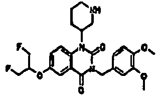 | 3-(3,4-diméthoxybenzyl)-6-[2-fluoro-1-(fluorométhyl)éthoxy]-1-pipéridin-3-ylquinazoline-2,4(1H,3H)-dione | LCMS m/z [MH]+ 490 |
| 76 | 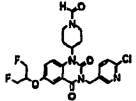 | 4-{3-[(6-chloropyridin-3-yl)méthyl]-6-[2-fluoro-1-(fluorométhyl)éthoxy]-2,4-dioxo-3,4-dihydroquinazolin-1(2H)-yl}pipéridine-1-carbaldéhyde | LCMS m/z [MH]+ 493 |
| 77 | 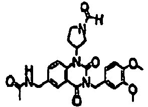 | N-{[3-(3,4-diméthoxybenzyl)-1-(1-formylpyrrolidin-3-yl)-2,4-dioxo-1,2,3,4-tétrahydroquinazolin-6-yl]méthyl}acétamide | LCMS m/z [MN]+ 481 |
| 78 | 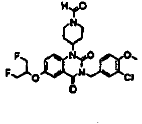 | 4-[3-(3-chloro-4-méthoxybenzyl)-6-[2-fluoro-1-(fluorométhyl)éthoxy]-2,4-dioxo-3,4-dihydroquinazolin-1(2H)-yl]pipéridine-1-carbaldéhyde | LCMS m/z [MH] + 522 |
| 79 | 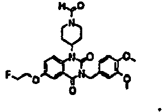 | 4-[3-(3,4-diméthoxybenzyl)-6-(2-fluoroéthoxy)-2,4-dioxo-3,4-dihydroquinazolin-1(2H)-yl]pipéridine-1-carbaldéhyde | LCMS m/z [MH]+ 486 |
| 80 | 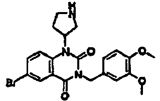 | 6-brome-3-(3,4-diméthoxybenzyl)-1-[pyrrolidin-3-yl]quinazoline-2,4(1H,3H)dione | LCMS m/z [MH]+ 460 |
| 81 | 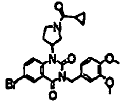 | 6-bromo-1-[1-(cyclopropylcarbonyl)pyrrolidin-3-yl]-3-(3,4-diméthoxybenzyl)quinazoline-2,4(1H,3H)-dione | LCMS m/z [MR]+ 528 |
| 82 | 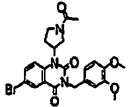 | 1-(1-acétylpyrrolidin-3-yl)-6-bromo-3-(3,4-diméthoxybenzyl)quinazoline-2,4(1H,3H)-dione | LCMS m/z [MH]+ 502 |
| 83 | 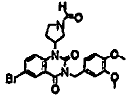 | 3-[6-bromo-3-(3,4-diméthoxybenzyl)-2,4-dioxo-3,4-dihydroquinazolin-1 (2H)-yl]pyrrolidine-1-carbaldéhyde | LCMS m/z [MH]+ 488 |
| 84 | 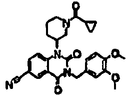 | 1-[1-(cyclopropylcarbonyl)pipéridin-3-yl]-3-(3,4-diméthoxybenzyl)-2,4-dioxo-1,2,3,4-tétrahydroquinazoline-6-carbonitrile | LCMS M/z [MH]+ 489 |
| 85 | 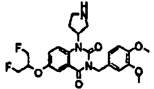 | 3-(3,4-diméthoxybenzyl)-6-[2-fluoro-1-(fluorométhyl)éthoxy]-1-pyrrolidin-3-ylquinazoline-2,4(1H,3H)-dlone | LCMS m/z [MH]+ 476 |
| 86 | 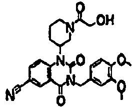 | 3-(3,4-diméthoxybenzyl)-1-[1-(hydroxyacétyl)pipéridin-3-yl]-2,4-dioxo-1,2,3,4-tétrahydroquinazoline-6-carbonitrile | LCMS m/z [MH]+ 479 |
| 87 | 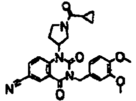 | 1-[1-(cyclopropylcarbonyl)pyrrolidin-3-yl]-3-(3,4-diméthoxybenzyl)-2,4-dioxo-1,2,3,4-tétrahydroquinazoline-6-carbonitrile | LCMS m/z [MH]+ 475 |
| 88 | 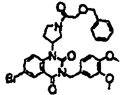 | 1-{1-[(benzyloxy)acétyl]pyrrolidin-3-yl)-6-bromo-3-(3,4-diméthoxybenzyl)quinazoline-2,4(1H,3H)-dione | LCMS m/z [MH]+ 608 |
| 89 | 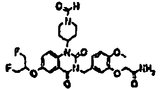 | 2-[5-({6-[2-fluoro-1-(fluorométhyl)éthoxy]-1-(1-formylpipéridin-4-yl-2,4-dioxo-1,4-dihydroquinazolin-3(2H)-yl}méthyl)-2-méthoxyphénoxy]acétamide | LCMS m/z [MH]+ 561 |
| 90 | 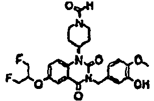 | 4-{6-[2-fluoro-1-(fluoromethyl)éthoxy]-3-(3-hydroxy-4-méthoxybenzyl)-2,4-dioxo-3,4-dihydroquinazolin-1(2H)-yl}pipéridine-1-carbaldéhyde | LCMS m/z [MH]+ 504 |
| 91 | 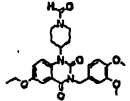 | 4-[3-(3,4-diméthoxybenzyl)-6-éthoxy-2,4-dioxo-3,4-dihydroquinazolin-1(2H)-yl]pipéridine-1-carbaldéhyde | LCMS m/z [MH]+ 468 |
| 92 | 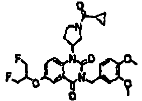 | 1-[1-(cyclopropylcarbonyl)pyrrolidin-3-yl]-3-(3,4-diméthoxybenzyl)-6-[2-fluor-1-(fluorométhyl)éthoxy]quinazoline-2,4(1H,3H)-dione | LCMS m/z [MH]+ 544 |
| 93 | 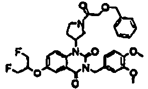 | 1-{1-[(benzyloxy)acétyl]pyrrolidin-3-yl}-3-(3,4-diméthoxybenzyl)-6-[2-fluoro-1-(fuorométhyl)éthoxy]quinazolin e-2,4(1H,3H)-dione | LCMS m/z [MH]+ 624 |
| 94 | 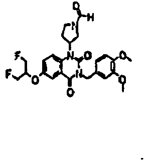 | 3-[3-(3,4-diméthoxybenzyl)-6-[2-fluoro-1-(fluorométhyl)éthoxy]-2,4-dioxo-3,4-hydroquinazolin-1(2H)-yl]pyrrolidine-1-carbaldéhyde | LCMS m/z [MH]+ 504 |
| 95 | 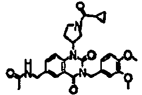 | N-({1-[1-(cyclopropylcarbonyl)pyrrolidin-3-yl]-3-(3,4-diméthoxybenzyl)-2,4-dioxo-1,2,3,4-tétrahydroquinazolin-6-yl}méthyl)acétamide | LCMS m/z [MH]+ 521 |
| 96 | 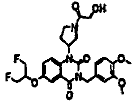 | 3-(3,4-diméthoxybenzyl)-6-[2-fluoro-1-(fluorométhyl)éthoxy]-1-[1-(hydroxyacétyl)pyrrolidin-3-yl]quinazoline-2,4(1H,3H)-dione | LCMS m/z [M+Na]+ 534 |
| 97 | 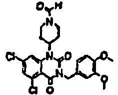 | 4-[5,7-dichloro-3-(3,4-diméthoxybenzyl)-2,4-dioxo-3,4-dihydroquinazolin-1 (2H)-yl]pipéridine-1-carbaldéhyde | LCMS m/z [MH]+ 492 |
| 98 | 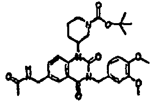 | 3-{6-[(acétylamino)méthyl]-3-(3,4-diméthoxybenzyl)-2,4-dioxo-3,4-dihydroquinazolin-1(2H)-yl}pipéridine-1-carboxylate de 1,1-diméthyléthyle | LCMS m/z [M+Na]+ 589 |
| 99 | 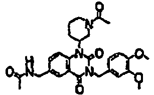 | N-{[3-(3,4-diméthoxybenzyl)-1-(1-formylpipéridin-3-yl-2,4-dioxo-1,2,3,4-tétrahydroquinazolin-6-yl]méthyl}acétamide | LCMS m/z [MH]+ 495 |
| 100 | 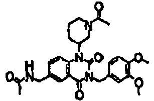 | N-{[1-(1-acétylpipéridin-yl)-3-(3,4-diméthoxybenzyl-2,4-dioxo-1,2,3,4-tétrahydroquinazolin-6-yl]méthyl}acétamide | LCMS m/z [MH]+ 509 |
| 101 | 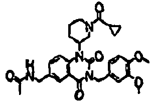 | N-({1-[1-(cyclopropylcarbonyl)pipéridin-3-yl]-3-(3,4-diméthoxybenzyl)-2,4-dioxo-1,2,3,4-tétrahydroquinazolin-6-yl}méthyl)acétamide | LCMS m/z [MH]+ 535 |
| 102 | 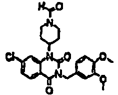 | 4-[7-chloro-3-(3,4-diméthoxybenzyl)-2,4-dioxo-3,4-dihydroquinazolln-1(2H)-yl]pipéridine-1-carbaldéhyde | LCMS m/z [MH]+ 458 |
| 103 | 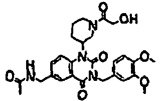 | N-({3-(3,4-diméthoxybenzyl)-1-[1-(hydroxyacétyl)pipéridin-3-yl]-2,4-dioxo-1,2,3,4-tétrahydroquinazolin-8-yl}méthyl)acétamide | LCMS m/z [MH]+ 525 |
| 104 | 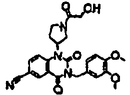 | 3-(3,4-diméthoxybenzyl)-1-[1-(hydroxyacétyl)pyrrolidin-3-yl]-2,4-dioxo-1,2,3,4-tétrahydroquinazoline-6-carbonitrile | LCMS m/z [MH]+ 465 |
| 105 | 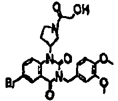 | 6-bromo-3-(3,4-diméthoxybenzyl)-1-[1-(hydroxyacétyl)pyrrolidin-3-yl]quinazoline-2,4(1H,3H)-dione | LCMS m/z [MH]+ 518 |
| 106 | 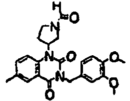 | 3-[3-(3,4-diméthoxybenzyl)-6-méthyl-2,4-dioxo-3,4-dihydroquinazolin-1(2H)-yl]pyrrolidine-1-carbaldéhyde | LCMS m/z [MH]+ 424 |
| 107 | 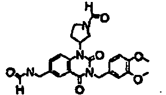 | N-{[3-(3,4-diméthoxybenzyl)-1-(1-formylpyrrolidin-3-yl)-2,4-dioxo-1n2,3,4-tétrahydroquinazolin-6-yl]méthyl}formamide | LCMS m/z [MH]+ 467 |
| 108 | 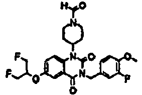 | 4-{6-[2-fluoro-1-(fluorométhyl)éthoxy]-3-(3-fluoro-4-méthoxybenzyl)-2,4-dioxo-3,4-dihydroquinazolin-1(2H)-yl}pipéridine-1-carbaldéhyde | LCMS m/z [MHJ+ 506 |
| 109 | 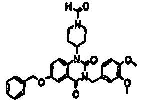 | 4-[6(benzyloxy)-3-(3,4-diméthoxybenzyl)-2,4-dioxo-3,4-dihydroquinazolin-1(2H)-yl]pipéridine-1-carbaldéhyde | LCMS m/z [MH]+ 530 |
| 110 | 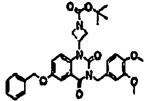 | 3-[6-(benzyloxy)-3-(3,4-diméthoxybenzyl)-2,4-dioxo-3,4-dihydroquinazolin-1(2H)-yl]azétidine-1-carboxylate de 1,1-diméthyléthyle | LCMS m/z [MH]+ 574 |
| 111 | 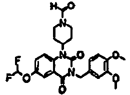 | 4-[6-(difluorométhoxy)-3-(3,4-diméthoxybenzyl)-2,4-dioxo-3,4-dihydroqulnazolin-1(2H)-yl]pipéridine-1-carbaldéhyde | LCMS m/z [MH]+ 490 |
| 112 | 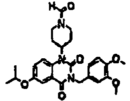 | 4-[3-(3,4-diméthoxybenzyl)-6-(1-méthyléthoxy)-2,4-dioxo-3,4-dihydroquinazolin-1(2H)-yl]pipéridine-1-carbaldéhyde | LCMS m/z [MH]+ 482 |
| 113 | 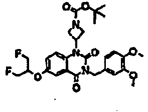 | 3-[3-(3,4-diméthoxybenzyl)-6-[2-fluoro-1-(fluorométhyl)éthoxy]-2,4-dioxo-3,4-dihydroquinazolin-1(2H)-yl]azétidine-1-carboxylate de 1,1-diméthyléthyle | LCMS m/z [MH]+ 562 |
| 114 | 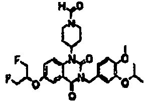 | 4-{6-[2-fluoro-1-(fluorométhyl)éthoxy]-3-[4-méthoxy-3-(1-méthyléthoxy)benzyl]-2,4-dioxo-3,4-dihydroquinazolin-(2H)-yl}pipéridine-1-carbaldéhyde | LCMS m/z [MH]+ 546 |
| 115 | 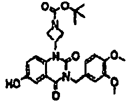 | 3-[3-(3,4-diméthoxybenzyl)-6-hydroxy-2,4-dioxo-3,4-dihydroquinazolin-1(2H)-yl]azétidine-1-carboxylate de 1,1-diméthyléthyle | LCMS m/z [MH]+ 484 |
| 116 | 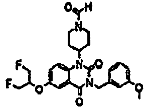 | 4-{6-[2-fluoro-1-(fluorométhyl)éthoxy]-3-(3-méthoxybenzyl)-2,4-dioxo-3,4-dihydroquinazolin-1(2H)-yl}pipéridine-1-carbaldéhyde | LCMS m/z [MH]+ 488 |
| 117 | 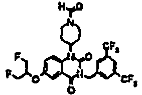 | 4-{3-[3,5-bis(trifluorométhyl)benzyl]-6-[2-fluoro-1-(fluorométhyl)éthoxy]-2,4-dioxo-3,4-dihydroquinazolin-1(2H)-yl)pipéridine-1-carbaldéhyde | LCMS m/z [MH]+ 564 |
| 118 | 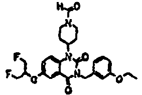 | 4-[3-(3-éthoxybenzyl)-6-[2-fluoro-1-(fluorométhyl)éthoxyl-2,4-dioxo-3,4-dihydroquinazolin-1(2H)-yl]pipéridine-1-carbaldéhyde | LCMS m/z [MH]+ 502 |
| 119 | 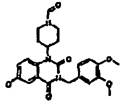 | 4-[3-(3,4-diméthoxybenzyl)-6-hydroxy-2,4-dioxo-3,4-dihydraquinazolin-1(2H)-yl]pipéridine-1-carbaldéhyde | 440 |
| 120 | 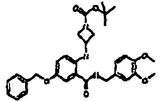 | 3-({4-(benzyloxy)-2-[(3,4-diméthoxybenzyl)carbamoyl]phényl}amino)azétdine-1-carboxylate de 1,1-diméthyléthyle | 548 |
| 121 | 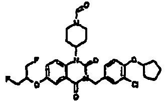 | 4-{3-[3-chloro-4-(cyclopentyloxy)benzyl]-6-[2-fluoro-1-(fluorométhyl)éthoxy]-2,4-dioxo-3,4-dihydroquinazolin-1(2H)-yl}pipéridine-1-carbaldéhyde | 576 |
| 122 | 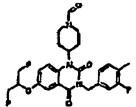 | 4-(6-(2-fluoro-1-(fluorométhyl)éthoxy]-3-(3-fluoro-4-méthylbenzyl)-2,4-dioxo-3,4-dihydroquinazolin-1(2H)-yl}pipéridine-1-carbaldéhyde | 490 |
| 123 | 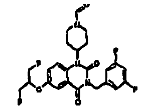 | 4-(3-(3,5-difluorobenzyl)-6-[2-fluoro-1-(fluorométhyl)éthoxy]-2,4-dioxo-3,4-dihydroquinazolin-1(2H)-yl]pipéridine-1-carbaldéhyde | 494 |
| 124 | 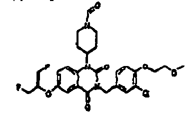 | 4-{3-[3-chloro-4-(2-méthoxyéthoxy)benzyl]-6-[2-fluoro-1-(fluorométhyl)éthoxy]-2,4-dioxo-3,4-dihydroquinazolin-1(2H)-yl}pipéridine-1-carbaldéhyde | 566 |
| 125 | 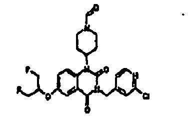 | 4-{3-[(2-chloropyridin-4-yl)méthyl]-6-(2-fluoro-1-(fluorométhyl)éthoxy]-2,4-dioxo-3,4-dihydroquinazolin-1(2H)-yl}pipéridine-1-carbaldéhyde | 493 |
| 126 | 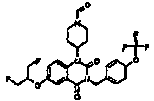 | 4-{6-[2-fluoro-1-(fluorométhyl)éthoxy]-2,4-dioxo-3-[4-(trifluorométhoxy)benzyl)-3,4-dihydroquinazolin-1(2H)-yl}pipéridine-1-carbaldéhyde | 542 |
| 127 | 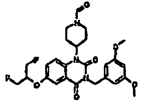 | 4-[3-(3,5-diméthoxybenzyl)-6-[2-fluoro-1-(fluorométhyl)éthoxyl-2,4-dioxo-3,4-dihydroquinazolin-1(2H)-yl]pipéridine-1-carbaldéhyde | 518 |
| 128 | 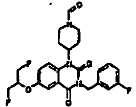 | 4-[3-(3-fluorobenzyl)-6-[2-fluoro-1-(fluorométhyl)éthoxy]-2,4-dioxo-3,4-dihydroquinazolin-1(2H)-yl]pipéridine-1-carbaldéhyde | 476 |
| 129 | 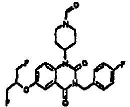 | 4-[3-(4-fluorobenzyl)-6-[2-fluoro-1-(fluorométhyl)éthoxy]-2,4-dioxo-3,4-dihydroquinazolin-1(2H)-yl]pipéridine-1-carbaldéhyde | 476 |
| 130 | 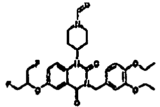 | 4-[3-(3,4-diéthoxybenzyl)-6-[2-fluoro-1-(fluorométhyl)éthoxy]-2,4-dioxo-3,4-dihydroquinazolin-1(2H)-yl]pipéridine-1-carbaldéhyde | 546 |
| 131 | 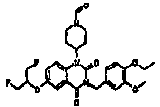 | 4-[3-(4-éthoxy-3-méthoxybenzyl)-6-[2-fluoro-1-(fluorométhyl)éthoxy]-2,4-dioxo-3,4-dihydroquinazolin-1(2H)-yl]pipéridine-1-carbaldéhyde | 532 |
| 132 | 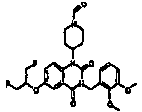 | 4-[3-(2,3-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 518 |
| 133 | 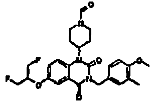 | 4-{6-[2-fluoro-1-(fluorométhyl)éthoxy]-3-(4-méthoxy-3-méthylbenzyl)-2,4-dioxo-3,4-dihydroquinazolin-1(2H)-yl}pipéridine-1-carbaldéhyde | 502 |
| 134 | 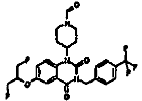 | 4-{6-[2-fluoro-1-(fluorométhyl)éthoxy]-2,4-dioxo-3-[4-(trifluorométhyl)benzyl]-3,4-dihydroquinazolin-1(2H)-yl}pipéridine-1-carbaldéhyde | 526 |
| 135 | 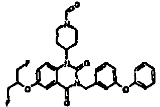 | 4-{6[2-fluoro-1-(fluorométhyl)éthoxy}-2,4-dioxo-3-(3-phénoxybenzyl)-3,4-dihydroquinazolin-1(2H)-yl}pipéridine-1-carbaldéhyde | 550 |
| 136 | 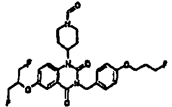 | 4-{6-[2-fluoro-1-(fluorométhyl)éthoxy]-3-[4-(3-fluoropropoxy)benzyl]-2,4-dioxo-3,4-dihydroquinazolin-1(2H)-yl}pipéridine-1-carbaldéhyde | 534 |
| 137 | 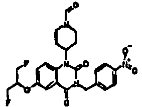 | 4-{6-(2-fluoro-1-(fluorométhyl)éthoxy]-3-(4-nitrobenzyl)-2,4-dioxo-3,4-dihydroquinazolin-1(2H)-yl}pipéridine-1-carbaldéhyde | 503 |
| 138 | 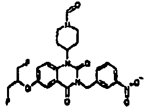 | 4-{6-[2-fluoro-1-(fluorométhyl)éthoxy]-3-(3-nitrobenzyl)-2,4-dioxo-3,4-dihydroquinazolin-1(2H)-yl}pipéridine-1-carbaldéhyde | 503 |
| 139 | 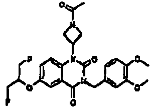 | 1-(1-acétylazétidin-3-yl)-3-(3,4-diméthoxybenzyl)-6-[2-fluoro-1-(fluorométhyl)éthoxy]quinazoline-2,4(1H,3H)-dione | 504 |
| 140 | 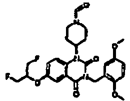 | 4-[3-(2,5-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 518 |
| 141 | 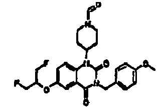 | 4-{6-[2-fluoro-1-(fluorométhyl)éthoxy]-3-(4-méthoxybenzyl)-2,4-dioxo-3,4-dihydroquinazolin-1(2H)-yl}pipéridine-1-carbaldéhyde | 488 |
| 142 | 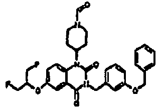 | 4-{3-[3-(benzyloxy)benzyl]-6-[2-fluoro-1-(fluorométhyl)éthoxy]2,4-dioxo-3,4-dihydroquinazolin-1(2H)-yl}pipéridine-1-carbaldéhyde | 564 |
| 143 | 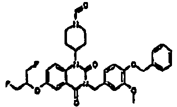 | 4-{3-[4-(benzyloxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde | 594 |
| 144 | 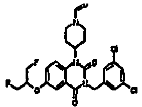 | 4-[3-(3,5-dichlorobenzyl)-6-[2-fluoro-1-(fluorométhyl)éthoxy]-2,4-dioxo-3,4-dihydroquinazolin-1(2H)-yl]pipéridine-1-carbaldéhyde | 526 |
| 145 | 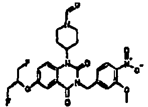 | 4-{6-[2-fluoro-1-(fluorométhyl)éthoxy]-3-(3-méthoxy-4-nitrobenzyl)-2,4-dioxo-3,4-dihydroquinazolin-1(2H)-yl}pipéridine-1-carbaldéhyde | 533 |
| 146 | 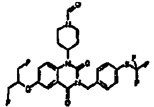 | 4-(6-[2-fluoro-1-(fluorométhyl)éthoxy]-2,4-dioxo-3-{4-dihydroquinazolin-1(2H)-yl}pipéridine-1-carbaldéhyde | 558 |
| 147 | 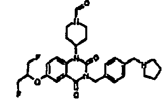 | 4-{6-[2-fluoro-1-(fluorométhyl)éthoxy]-2,4-dioxo-3-[4-(pyrrolidin-1-ylméthyl)benzyl]-3,4-dihydroquinazolin-1(2H)-yl}pipéridine-1-carbaldéhyde | 541 |
| 148 | 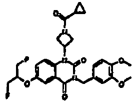 | 1-[1-(cyclopropylcarbonyl)azétidin-3-yl]-3-(3,4-diméthoxybenzyl)-6-[2-fluoro-1-(fluorométhyl)éthoxy]quinazoline-2,4(1H,3H)-dione | 530 |
| 149 | 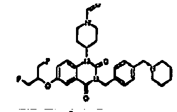 | 4-{6-[2-fluoro-1-(fluorométhyl)éthoxy]-2,4-dioxo-3-[4-(pipéridin-1-ylméthyl)benzyl]-3,4-dihydroquinazolin-1(2H)-yl}pipéridine-1-carbaldéhyde | 555 |
| 150 | 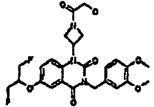 | 3-(3,4-diméthoxybenzyl)-6-[2-fluoro-1-(fluorométhyl)éthoxy]-1-[1-(hydroxyacétyl)azétidin-3-yl]quinazoline-2,4(1H,3H)-dione | 520 |
| 151 | 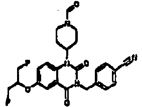 | 4-({6-[2-fluoro-1-(fluorométhyl)éthoxy]-1-(1-formylpipéridin-4-yl)-2,4-dioxo-1,4-dihydroquinazolin-3(2H)-yl}méthyl)benzonitrile | 483 |
| 152 | 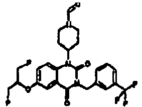 | 4-{6-[2-fluoro-1-(fluorométhyl)éthoxy]-2,4-dioxo-3-[3-(trifluorométhyl)benzyl]-3,4-dihydroquinazolin-1(2H)-yl}pipéridine-1-carbaldéhyde | 526 |
| 153 | 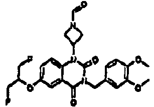 | 3-(3-(3,4-diméthoxybenzyl)-6-[2-fluoro-1-(fluorométhyl)éthoxy]-2,4-dioxo-3,4-dihydroquinazolin-1(2H)-yl]azétidine-1-carbaldéhyde | 490 |
| 154 | 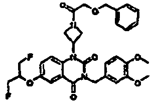 | 1-{1-[(benzyloxy)acétyl]azétidin-3-yl}-3-(3,4-diméthoxybenzyl)-6-[2-fluoro-1-(fluorométhyl)éthoxy]quinazoline-2,4(1H,3H)-dione | 610 |
| 155 | 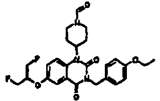 | 4-[3-(4-éthoxybenzyl)-6-[2-fluoro-1-(fluorométhyl)éthoxy]2,4-dioxo-3,4-dihydroquinazolin-1(2H)-yl]pipéridine-1-carbaldéhyde | 502 |
| 156 | 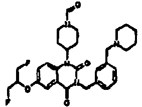 | 4-{6-[2-fluoro-1-(fluorométhyl)éthoxy]-2,4-dioxo-3-(3-(pipéridin-1-ylméthyl)benzyl]-3,4-dihydroquinazolin-1(2H)-yl}pipéridine-1-carbaldéhyde | 555 |
| 157 | 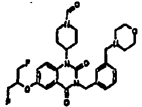 | 4-{6-[2-fluoro-1-(fluorométhyl)éthoxy]-3-[3-(morpholin-4-ylméthyl)benzyl]-2,4-dioxo-3,4-dihydroquinazolin-1(2H)-yl}pipéridine-1-carbaldéhyde | 557 |
| 158 | 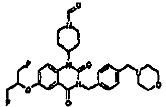 | 4-{6-[2-fluoro-1-(fluorométhyl)éthoxy]-3-[4-(morpholin-4-ylméthyl)benzyl]-2,4-dioxo-3,4-dihydroquinazolin-1(2H)-yl}pipéridine-1-carbaldéhyde | 557 |
| 159 | 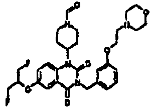 | 4-{6-[2-fluoro-1-(fluorométhyl)éthoxy]-3-[3-(2-morpholin-4-yléthoxy)benzyl]-2,4-dioxo-3,4-dihydroquinazolin-1(2H)-yl}pipéridine-1-carbaldéhyde | 587 |
| 160 | 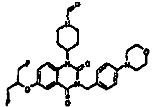 | 4-{6-[2-fluoro-1-(fluorométhyl)éthoxy]-3-(4-morpholin-4-ylbenzyl)-2,4-dioxo-3,4-dihydroquinazolin-1(2H)-yl}pipéridine-1-carbaldéhyde | 543 |
| 161 | 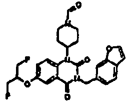 | 4-[3-(1-benzofur-6-ylméthyl)-6-[2-fluoro-1-(fluorométhyl)éthoxy]-2,4-dioxo-3,4-dihydroquinazolin-1(2H)-yl]pipéridine-1-carbaldéhyde | 498 |
| 162 | 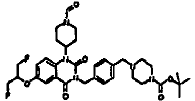 | 4-[4-({6-[2-fluoro-1-(fluorométhyl)éthoxy]-1-(1-formylpipéridin-4-yl)-2,4-dioxo-1,4-dihydroquinazolin-3(2H)-yl)méthyl)benzyl]pipérazine-1-carboxylate de 1,1-diméthyléthyle | 656 |
| 163 | 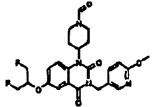 | 4-{6-[2-fluoro-1-(fluorométhyl)éthoxy]-3-[(6-méthoxypyridin-3-yl)méthyl]-2,4-dioxo-3,4-dihydroquinazolin-1(2H)-yl}pipéridine-1-carbaldéhyde | 489 |
| 164 | 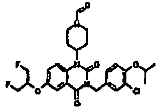 | 4-{3-[3-chloro-4-(1-méthyléthoxy)benzyl]-6-[2-fluoro-1-(fluorométhyl)éthoxy]-2,4-dioxo-3,4-dihydroquinazolin-1(2H)-yl}pipéridine-1-carbaldéhyde | 550 |
| 165 | 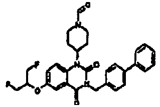 | 4-[3-(biphényl-4-ylméthyl)-6-[2-fluoro-1-(fluorométhyl)éthoxy]-2,4-dioxo-3,4-dihydroquinazolin-1(2H)-yl]pipéridine-1-carbaldéhyde | 534 |
| 166 | 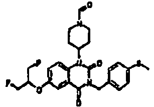 | 4-{6-[2-fluoro-1-(fluorométhyl)éthoxy]-3-[4-(méthylsulfanyl)benzyl]-2,4-dioxo-3,4-dihydroquinazolin-1(2H)-yl}pipéridine-1-carbaldéhyde | 504 |
| 167 | 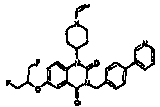 | 4-{6-[2-fluoro-1-(fluorométhyl)éthoxy]-2,4-dioxo-3-(4-pyridin-3-ylbenzyl)-3,4-dihydroquinazolin-1(2H)-yl}pipéridine-1-carbaldéhyde | 535 |
| 168 | 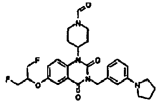 | 4-{6-[2-fluoro-1-(fluorométhyl)éthoxy]-2,4-dioxo-3-(3-pyrrolidin-1-ylbenzyl)-3,4-dihydroquinazolin-1(2H)-yl}pipéridine-1-carbaldéhyde | 527 |
| 169 | 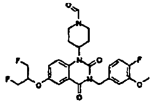 | 4-{6-[2-fluoro-1-(fluorométhyl)éthoxy]-3-(4-fluoro-3-méthoxybenzyl)-2,4-dioxo-3,4-dihydroquinazolin-1(2H)-yl}pipéridine-1-carbaldéhyde | 506 |
| 170 | 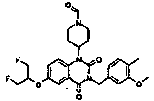 | 4-{6-[2-fluoro-1-(fluorométhyl)éthoxy]-3-(3-méthoxy-4-méthylbenzyl)-2,4-dioxo-3,4-dihydroquinazolin-1(2H)-yl}pipéridine-1-carbaldéhyde | 502 |
| 171 | 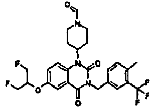 | 4-{6-[2-fluoro-1-(fluorométhyl)éthoxy]-3-[4-méthyl-3-(trifluorométhyl)benzyl]-2,4-dioxo-3,4-dihydroquinazolin-1(2H)-yl}pipéridine-1-carbaldéhyde | 540 |
| 172 | 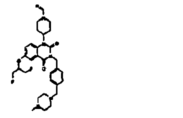 | 4-{6-[2-fluoro-1-(fluorométhyl)éthoxy]-3-{4-[(4-méthylpipérazin-1-yl)méthyl]benryl}-2,4-dioxo-3,4-dihydroquinazolin-1(2H)-yl}pipéridine-1-carbaldéhyde | 570 |
| 173 | 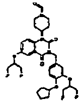 | 4-[3-{4-(cyclopentyloxy)-3-[2-fluoro-1-(fluorométhyl)éthoxy]benzyl}-6-[2-fluoro-1-(fluorométhyl)éthoxy]-2,4-dioxo-3,4-dihydroquinazolin-1(2H)-yl]pipéridine-1-carbaldéhyde | 636 |
| 174 | 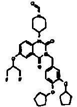 | 3-[3,4-bis(cyclopentyloxy)benzyl]-1-(1-éthénylpipéridin-4-yl)-6-[2-fluoro-1-(fluorométhyl)éthoxy]quinazoline-2,4(1H,3H)-dione | 626 |
| 175 | 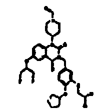 | 2-(2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]acétamide | 615 |
| 176 | 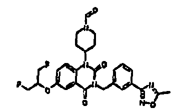 | 4-{6-[2-fluoro-1-(fluorométhyl)éthoxy]-3-[3-(5-méthyl-1,2,4-oxadiazol-3-yl)benzyl)-2,4-dioxo-3,4-dihydroquinazolin-1(2H)-yl}pipéridine-1-carbaldéhyde | 540 |
| 177 | 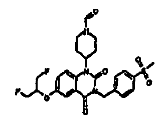 | 4-(6-[2-fluoro-1-(fluorométhyl)éthoxy]-3-[4-(méthylsulfonyl)benzyl]-2,4-dioxo-3,4-dihydroquinazolin-1(2H)-yl}pipéridine-1-carbaldéhyde | 536 |
| 178 | 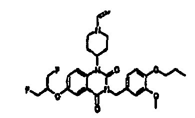 | 4-{6-[2-fluoro-1-(fluorométhyl)éthoxy]-3-(3-méthoxy-4-propoxybenzyl)-2,4-dioxo-3,4-dihydroquinazolin-1(2H)-yl}pipéridine-1-carbaldéhyde | 546 |
| 179 | 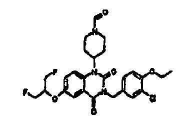 | 4-[3-(3-chloro-4-éthoxybenzyl)-6-[2-fluoro-1-(fluorométhyl)éthoxy]-2,4-dioxo-3,4-dihydroquinazolin-1(2H)-yl]pipéridine-1-carbaldéhyde | 536 |
| 180 | 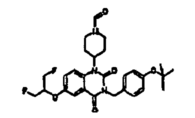 | 4-{3-[4-(1,1-diméthyléthoxy)benzyl]-6-[2-fluoro-1-(fluorométhyl)éthoxy]-2,4-dioxo-3,4-dihydroquinazolin-1(2H)-yl}pipéridine-1-carbaldéhyde | 530 |
| 181 | 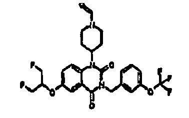 | 4-{6-[2-fluoro-1-(fluorométhyl)éthoxy]-2,4-dioxo-3-[3-(trifluorométhoxy)benzyl]-3,4-dihydroquinazolin-1(2H)-yl}pipéridine-1-carbaldéhyde | 542 |
| 182 | 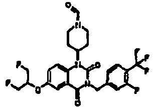 | 4-{6-[2-fluoro-1-(fluorométhyl)éthoxy]-3-[3-fluoro-4-(trifluorométhyl)benzyl)-2,4-dioxo-3,4-dihydroquinazolin-1(2H)-yl}pipéridine-1-carbaldéhyde | 544 |
| 183 | 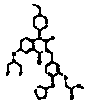 | 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthylacétamide | 629 |
| 184 | 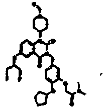 | 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N,N-diméthylacétamide | 643 |
| 185 | 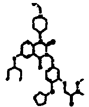 | 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthoxy-N-méthylacétamide | 659 |
| 186 | 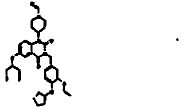 | 4-{3-[4-(cyclopentyloxy)-3-éthoxybenzyl]-6-[2-fluoro-1-(fluoromethyl)éthoxy]-2,4-dioxo-3,4-dihydroquinazolin-1(2H)-yl}pipéridine-1-carbaldéhyde | 586 |
| 187 | 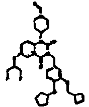 | 4-{3-[3-(cyclobutylméthoxy)-4-(cyclopentyloxy)benzyl]-6-[2-fluoro-1-(fluorométhyl)éthoxy]-2,4-dioxo-3,4-dihydroquinazolin-1(2H)-yl)pipéridine-1-carbaldéhyde | 626 |
| 188 | 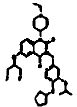 | 4-{3-[4-(cyclopentyloxy)-3-(1-méthyléthoxy)benzyl]-6-[2-fluoro-1-(fluorométhyl)éthoxy]-2,4-dioxo-3,4-dihydroquinazolin-1(2H)-yl}pipéridine-1-carbaldéhyde | 600 |
| 189 | 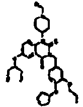 | 4-{3-[4-(cyclopentyloxy)-3-propoxybenzyl]-6-[2-fluoro-1-(fluorométhyl)éthoxy]-2,4-dioxo-3,4-dihydroquinazolin-1(2H)-yl}pipéridine-1-carbaldéhyde | 600 |
| 190 | 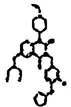 | 4-{3-[4-(cyclopentyloxy)-3-hydroxybenzyl]-6-[2-fluoro-1-(fluorométhyl)éthoxy]-2,4-dioxo-3,4-dihydroquinazolin-1(2H)-yl}pipéridine-1-carbaldéhyde | 558 |
| 191 | 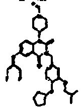 | 4-{3-[4-(cyclopentyloxy)-3-(2-méthylpropoxy)benzyl]-6-[2-fluoro-1-(fluorométhyl)éthoxy]-2,4-dioxo-3,4-dihydroquinazolin-1(2H)-yl}pipéridine-1-carbaldéhyde | 614 |
| 192 | 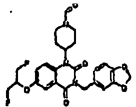 | 4-[3-(1,3-benzodioxol-5-ylméthyl)-6-[2-fluoro-1-(fluorométhyl)éthoxy]-2,4-dioxo-3,4-dihydroquinazolin-1(2H)-yl]pipéridine-1-carbaldéhyde | 502 |
| 193 | 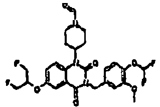 | 4-{3-[4-(diflorométhoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde | 554 |
| 194 | 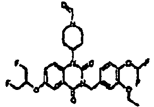 | 4-{3-[4-(difluorométhoxy)-3-éthoxybenzyl]-6-[2-fluoro-1-(fluorométhyl)éthoxy]-2,4-dioxo-3,4-dihydroquinazolin-1(2H)yl}pipéridine-1-carbaldéhyde | 568 |
| 195 | 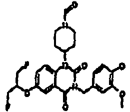 | 4-{6-[2-fluoro-1-(fluoraméthyl)éthoxy]-3-(4-hydroxy-3-méthoxybenzyl)-2,4-dioxo-3,4-dihydroquinazolin-1(2H)-yl}pipéridine-1-carbaldéhyde | 504 |
| 196 | 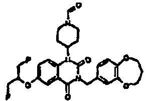 | 4-[3-(3,4-dihydro-2H-1,5-benzodioxépin-7-ylméthyl)-6-[2-fluoro-1-(fluorométhyl)éthoxy]-2,4-dioxo-3,4-dihydroqulnazolin-1(2H)-yl]pipéridine-1-carbaldéhyde | 530 |
| 197 | 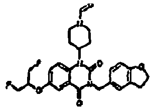 | 4-[3-(2,3-dihydro-1-benzofur-5-ylméthyl)-6-[2-fluoro-1-(fluorométhyl)éthoxy]-2,4-dioxo-3,4-dihydroquinazolin-1(2H)-yl]pipéridine-1-carbaldéhyde | 500 |
| 198 | 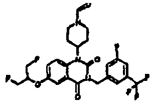 | 4-{6-[2-fluoro-1-(fluorométhyl)éthoxy]-3-[3-fluoro-5-(trifluorométhyl)benzyl]-2,4-dioxo-3,4-dihydroquinazolin-1(2H)-yl}pipéridine-1-carbaldéhyde | 544 |
| 199 | 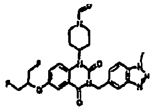 | 4-{6-[2-fluoro-1-(fluorométhyl)éthoxy]-3-[(1-méthyl-1H-benzotriazol-5-yl)méthyl]-2,4-dioxo-3,4-dihydroquinazolin-1(2H)-yl}pipéridine-1-carbaldéhyde | 513 |
| 200 | 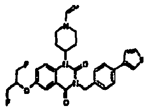 | 4-{6-[2-fluoro-1-(fluorométhyl)éthoxy]-2,4-dioxo-3-(4-thiophén-3-ylbenzyl)-3,4-dihydroquinazolin-1(2H)-yl}pipéridine-1-carbaldéhyde | 540 |
| 201 | 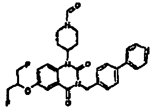 | 4-{6-[2-fluoro-1-(fluorométhyl)éthoxy]-2,4-dioxo-3-(4-pyridin-4-ylbenzyl)-3,4-dihydroquinazolin-1(2H)-yl}pipéridine-1-carbaldéhyde | 535 |
| 202 | 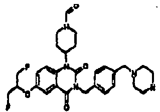 | 4-{6-[2-fluoro-1-(fluorométhyl)éthoxy)]-2,4-dioxo-3-[4-(pipérazin-1-ylméthyl)benzyl]-3,4-dihydroquinazolin-1(2H)-yl}pipéridine-1-carbaldéhyde | 555 |
| 203 | 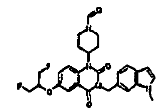 | 4-{6-[2-fluoro-1-(fluorométhyl)éthoxy]-3-[(1-méthyl-1H-indol-6-yl)méthyl]-2,4-dioxo-3,4-dihydroquinazolin-1(2H)-yl}pipéridine-1-carbaldéhyde | 511 |
| 204 | 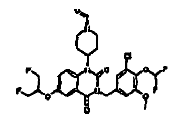 | 4-{3-[3-chloro-4-(difluorométhoxy)-5-méthoxybenzyl]-6-[2-fluoro-1-(fluorométhyl)éthoxy]-2,4-dioxo-3,4-dihydroquinazolin-1(2H)-yl)pipéridine-1-carbaldéhyde | 588 |
| 205 | 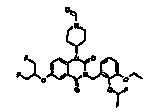 | 4-{3-[2-(difluorométhoxy)-3-éthoxybenzyl]-6-[2-fluoro-1-(fluorométhyl)éthoxyl-2,4-dioxo-3,4-dihydroquinazolin-1(2H)-yl}pipéridine-1-carbaldéhyde | 568 |
| 206 | 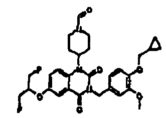 | 4-{3-[4-(cyclopropylméthoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde | 558 |
| 207 | 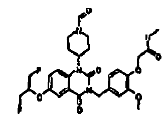 | 2-[4-({6-[2-fluoro-1-(fluorométhyl)éthoxy]-1-(1-formylpipéridin-4-yl)-2,4-dioxo-1,4-dihydroquinazolin-3(2H)-yl)méthyl)-2-méthoxyphénoxy}-N-méthylacétamide | 575 |
| 208 | 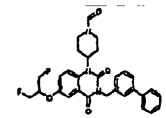 | 4-[3-(biphényl-3-ylméthyl)-6-[2-fluoro-1-(fluorométhyl)éthoxy]-2,4-dioxo-3,4-dihydroquinazolin-1(2H)-yl]pipéridine-1-carbaldéhyde | 534 |
| 209 | 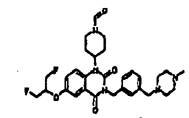 | 4-{6-[2-fluoro-1-(fluorométhyl)éthoxy]-3-{3-[(4-méthylpipérazin-1-yl)méthyl]benzyl}-2,4-dioxo-3,4-dihydroquinazolin-1(2H)-yl}pipéridine-1-carbaldéhyde | 570 |
| 210 | 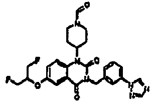 | 4-{6-[2-fluoro-1-(fluorométhyl)éthoxy]-2,4-dioxo-3-[3-(1H-1,2,4-triazol-1-yl)benzyl]-3,4-dihydroquinazolin-1(2H)-yl}pipéridine-1-carbaldéhyde | 525 |
| 211 | 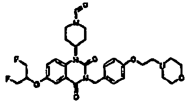 | 4-{6-[2-fluoro-1-(fluorométhyl)éthoxy]-3-[4-(2-morpholin-4-yléthoxy)benzyl]-2,4-dioxo-3,4-dihydroquinazolin-1(2H)-yl}pipéridine-1-carbaldéhyde | 587 |
| 212 | 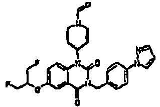 | 4-{6-[2-fluoro-1-(fluorométhyl)éthoxy]-2,4-dioxo-3-[4-(1H-pyrazol-1-yl)benzyl]-3,4-dihydroquinazolin-1(2H)-yl}pipéridine-1-carbaldéhyde | 524 |
| 213 | 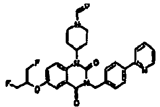 | 4-{6-[2-fluoro-1-(fluorométhyl)éthoxy]-2,4-dioxo-3-(4-pyridin-2-ylbenzyl)-3,4-dihydroquinazolin-1(2H)-yl}pipéridine-1-carbaldéhyde | 535 |
| 214 | 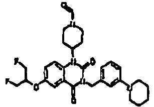 | 4-{6-[2-fluoro-1-(fluorométhyl)éthoxy]-2,4-dioxo-3-(3-pipéridin-1-ylbenzyl)-3,4-dihydroquinazolin-1(2H)-yl}pipéridine-1-carbaldéhyde | 541 |
| 215 | 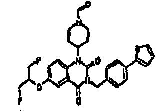 | 4-{6-[2-fluoro-1-(fluorométhyl)éthoxy]-2,4-dioxo-3-(4-thiophén-2-ylbenzyl)-3,4-dihydroquinazolin-1(2H)-yl}pipéridine-1-carbaldéhyde | 540 |
| 216 | 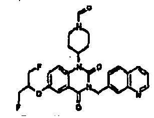 | 4-{6-[2-fluoro-1-(fluorométhyl)éthoxy]-2,4-dioxo-3-(quinoléin-7-ylméthyl)-3,4-dihydroquinazolin-1(2H)-yl}pipéridine-1-carbaldéhyde | 509 |

| ***Composé No*** | ***STRUCTURE*** | ***NOMENCLATURE*** | ***MASSE LCUVMS MH+*** |
|---|---|---|---|
| 217 | 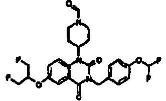 | 4-{3-[4-(difluorométhoxy)benzyl]-6-[2-fluoro-1-(fluorométhyl)éthoxy]-2,4-dioxo-3,4-dihydroquinazolin-1(2H)-yl}pipéridine-1-carbaldéhyde | 524 |
| 218 | 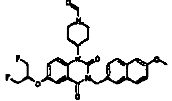 | 4-{6-[2-fluoro-1-(fluorométhyl)éthoxy]-3-[(6-méthoxynaphtalén-2-yl)méthyl]-2,4-dioxo-3,4-dihydroquinazolin-1(2H)-yl}pipéridine-1-carbaldéhyde | 538 |
| 219 | 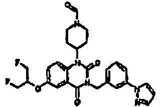 | 4-{6-[2-fluoro-1-(fluorométhyl)éthoxy]-2,4-dioxo-3-[3-(1H-pyrazol-1-yl)benzyl]-3,4-dihydroquinazolin-1(2H)-yl}pipéridine-1-carbaldéhyde | 524 |
| 220 | 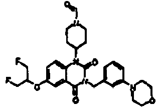 | 4-{6-[2-fluoro-1-(fluorométhyl)éthoxy]-3-(3-morpholin-4-ylbenzyl)-2,4-dioxo-3,4-dihydroquinazolin-1(2H)-yl}pipéridine-1-carbaldéhyde | 543 |
| 221 | 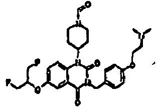 | 4-[3-{4-[2-(diméthylamino)éthoxy]benzyl}-6-[2-fluoro-1-(fluorométhyl)éthoxy]-2,4-dioxo-3,4-dihydroquinazolin-1(2H)-yl]pipéridine-1-carbaldéhyde | 545 |
| 222 | 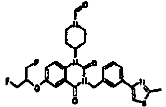 | 4-{6-[2-fluoro-1-(fluorométhyl)éthoxy]-3-[3-(2-méthyl-1,3-thiazol-4-yl)benzyl]-2,4-dioxo-3,4-dihydroquinazolin-1(2H)-yl}pipéridine-1-carbaldéhyde | 555 |
| 223 | 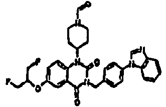 | 4-{3-[4-(1H-benzimidazol-1-yl)benzyl]-6-[2-fluoro-1-(fluorométhyl)éthoxy]-2,4-dioxo-3,4-dihydroquinazolin-1(2H)-yl}pipéridine-1-carbaldéhyde | 574 |
| 224 | 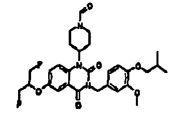 | 4-{6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde | 560 |
| 225 | 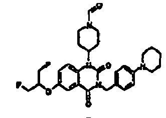 | 4-{6-[2-fluoro-1-(fluorométhyl)éthoxy]-2,4-dioxo-3-(4-pipéridin-1-ylbenzyl)-3,4-dihydroquinazolin-1(2H)-yl}pipéridine-1-carbaldéhyde | 541 |
| 226 | 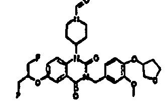 | 4-{6-[2-fluoro-1-(fluorométhyl)éthoxy]-3-[3-méthoxy-4-(tétrahydrofuran-3-yloxy)benzyl]-2,4-dioxo-3,4-dihydroquinazolin-1(2H)-yl}pipéridine-1-carbaldéhyde | 574 |
| 227 | 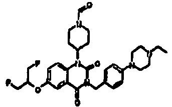 | 4-{3-[4-(4-éthylpipérazin-1-yl)benzyl]-6-[2-fluoro-1-(fluorométhyl)éthoxy]-2,4-dioxo-3,4-dihydroquinazolin-1(2H)-yl}pipéridine-1-carbaldéhyde | 570 |
| 228 | 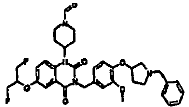 | 4-[3-{4-[(1-benzylpyrrolidin-3-yl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 663 |
| 229 | 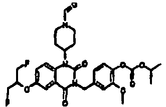 | carbonate de 4-({6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényle et de 1-méthyléthyle | 590 |
| 230 | 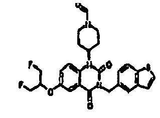 | 4-[3-(1-benzothiophén-5-ylméthyl)-6-[2-fluoro-1-(fluorométhyl)éthoxy]-2,4-dioxo-3,4-dihydroquinazolin-1(2H)-yl]pipéridine-1-carbaldéhyde | 514 |
| 231 | 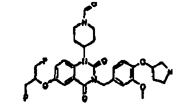 | 4{6-[2-fluoro-1-(fluorométhyl)éthoxy]-3-[3-méthoxy-4-(pyrrolidin-3-yloxy)benzyl]-2,4-dioxo-3,4-dihydroquinazolin-1(2H)-yl}pipéridine-1-carbaldéhyde | 573 |
| 232 | 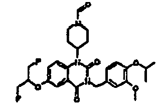 | 4-{6-[2-fluoro-1-(fluorométhyl)éthoxy]-3-[3-méthoxy-4-(1-méthyléthoxy)benzyl]-2,4-dioxo-3,4-dihydroquinazolin-1(2H)-yl}pipéridine-1-carbaldéhyde | 546 |
| 233 | 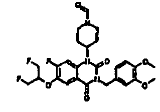 | 4-[3-(3,4-diméthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 536 |
| 234 | 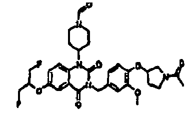 | 4-[3-{4-[(1-acétylpyrrolidin-3-yl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 615 |
| 235 | 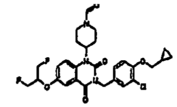 | 4-{3-[3-chloro-4-(cyclopropylméthoxy)benzyl]-6-[2-fluoro-1-(fluorométhyl)éthoxy]-2,4-dioxo-3,4-dihydroquinazolin-1(2H)-yl}pipéridine-1-carbaldéhyde | 562 |
| 236 | 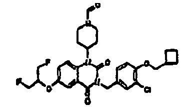 | 4-{3-[3-chloro-4-(cyclobutylméthoxy)benzyl]-6-[2-fluoro-1-(fluorométhyl)éthoxy]-2,4-dioxo-3,4-dihydroquinazolin-1(2H)-yl}pipéridine-1-carbaldéhyde | 576 |
| 237 | 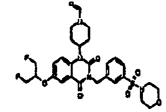 | 4-{6-[2-fluoro-1-(fluorométhyl)éthoxy]-3-[3-(morpholin-4-ylsulfonyl)benzyl]-2,4-dioxo-3,4-dihydroquinazolin-1(2H)-yl}pipéridine-1-carbaldéhyde | 607 |
| 238 | 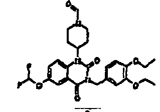 | 4-[3-(3,4-diéthoxybenzyl)-6-(difluorométhoxy)-2,4-dioxo-3,4-dihydroquinazolin-1(2H)-yl]pipéridine-9-carbaldéhyde | 518 |
| 239 | 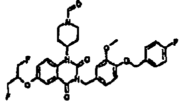 | 4-[3-{4-[(4-fluorobenzyl)oxy]-3-méthoxybenzyl-6-[2-fluoro-1-(fluorométhyl)éthoxyl-2,4-dioxo-3,4-dihydroquinazolin-1(2H)-yl]pipéridine-1-carbaldéhyde | 612 |
| 240 | 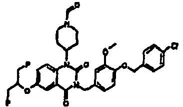 | 4-[3-{4-[(4-chlorobenzyl)oxy]-3-méthoxybenzyl)-6-[2-fluoro-1-(fluorométhyl)éthoxy]-2,4-dioxo-3,4-dihydroquinazolin-1(2H)-yl]pipéridine-1-carbaldéhyde | 628 |
| 241 | 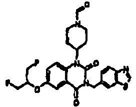 | 4-[3-(1,3-benzothiazol-6-ylméthyl)-6-[2-fluoro-1-(fluorométhyl)éthoxy]-2,4-dioxo-3,4-dihydroquinazolin-1(2H)-yl]pipéridine-1-carbaldéhyde | 515 |
| 242 | 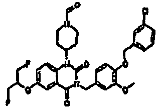 | 4-[3-{4-[(3-chlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 628 |
| 243 | 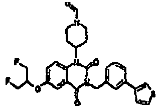 | 4-{6-[2-fluoro-1-(fluorométhyl)éthoxy]-2,4-dioxo-3-(3-thiophén-3-ylbenxyl]-3,4-dihydroquinazolin-1(2H)-yl}pipéridine-1-carbaldéhyde | 540 |
| 244 | 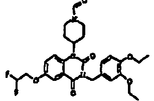 | 4-[3-(3,4-diéthoxybenzyl)-6-(2,2-difluoroéthoxy)-2,4-dioxo-3,4-dihydroquinazolin-1(2H)-yl]pipéridine-1-carbaldéhyde | 532 |
| 245 | 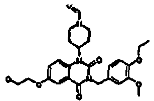 | 4-[3-(4-éthoxy-3-méthoxybenzyl)-6-(2-hydroxyéthoxy)-2,4-dioxo-3,4-dihydroquinazolin-1(2H)-yl]pipéridine-1-carbaldéhyde | 498 |
| 246 | 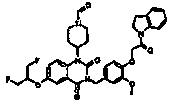 | 4-[3-{4-[2-(2,3-dihydro-1H-indol-1-yl)-2-oxoéthoxy]-3-méthoxybenzyl}-6-[2-fluoro-1-(fluorométhyl)éthoxy]-2,4-dioxo-3,4dihydroquinazolin-1(2H)-yl]pipéridine-1-carbaldéhyde | 663 |
| 247 | 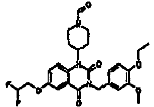 | 4-[6-2,2-difluoroéthoxy)-3-(4-éthoxy-3-méthoxybenzyl)-2,4-dioxo-3,4-dihydroquinazolin-1(2H)-yl]pipéridine-1-carbaldéhyde | 518 |
| 248 | 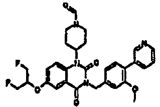 | 4-{6-[2-fluoro-1-(fluorométhyl)éthoxy]-3-(3-méthoxy-4-pyridin-3-ylbenzyl)-2,4-dioxo-3,4-dihydroquinazolin-1(2H)-yl}pipéridine-1-carbaldéhyde | 565 |
| 249 | 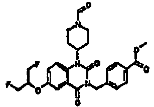 | 4-({6-[2-fluoro-1-(fluorométhyl)éthoxy]-1-(1-formylplpéridin-4-yl)-2,4-dioxo-1,4-dihydroquinazolin-3(2H)-yl}méthyl)benzoate de méthyle | 516 |
| 250 | 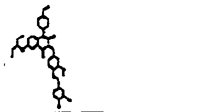 | 4-[3-{4-[(3,4-dichlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 662 |
| 251 | 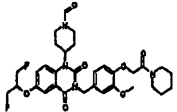 | 4-{6-[2-fluoro-1-(fluorométhyl)éthoxy]-3-[3-méthoxy-4-(2-oxo-2-pipéridin-1-yléthoxy)benzyl]-2,4-dioxo-3,4-dihydroquinazolin-1(2H)-yl}pipéridine-1-carbaldéhyde | 629 |
| 252 | 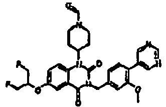 | 4-{6-[2-fluoro-1-(fluorométhyl)éthoxy]-3-(3-méthoxy-4-pyrimidin-5-ylbenzyl)-2,4-dioxo-3,4-dihydroquinazolin-1(2H)-yl}pipéridine-1-carbaldéhyde | 566 |
| 253 | 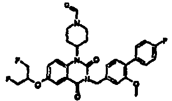 | 4-{6-[2-fluoro-1-(fluorométhyl)éthoxy]-3-[(4'-fluoro-2-méthoxybiphényl-4-yl)méthyl]-2,4-dioxo-3,4-dihydroquinazolin-1(2H)-yl}pipéridine-1-carbaldéhyde | 582 |
| 254 | 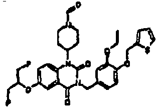 | 4-{3-[3-éthoxy-4-(thiophén-2-ylméthoxy)benzyl]-6-[2-fluoro-1-(fluorométhyl)éthoxy]-2,4-dioxo-3,4-dihydroquinazolin-1(2H)-yl}pipéridine-1-carbaldéhyde | 614 |
| 255 | 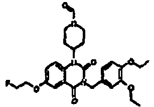 | 4-[3-(3,4-diéthoxybenzyl)-6-(2-fluoroéthoxy)-2,4-dioxo-3,4-dihydroquinazolin-1(2H)-yl]pipéridine-1-carbaldéhyde | 514 |
| 256 | 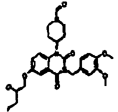 | 4-[3-(3,4-diméthoxybenzyl)-6-(3-fluoro-2-hydroxypropoxy)-2,4-dioxo-3,4-dihydroquinazolin-1(2H)-yl]pipéridine-1-carbaldéhyde | 516 |
| 257 | 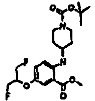 | 4-({4-[2-fluoro-1-(fluorométhyl)éthoxy)-2-(méthoxycarbonyl)phényl}amino)pipéridine-1-carboxylate de 1,1-diméthyléthyle | 429 |
| 258 | 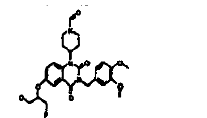 | 4-[3-(3,4-diméthoxybenzyl)-6-[2-fluoro-1-(hydroxyméthyl)éthoxy]-2,4-dioxo-3,4-dihydroquinazolin-1(2H)-yl]pipéridine-1-carbaldéhyde | 516 |
| 259 | 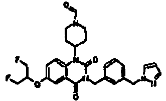 | 4-{6-[2-fluoro-1-(fluorométhyl)éthoxy]-2,4-dioxo-3-[3-(1H-pyrazol-1-ylméthyl)benzyl]-3,4-dihydroquinazolin-1(2H)-yl}pipéridine-1-carbaldéhyde | 538 |
| 260 | 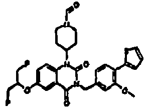 | 4-{6-[2-fluoro-1-(fluorométhyl)éthoxy]-3-(3-méthoxy-4-thiophén-2-ylbenzyl)-2,4-dioxo-3,4-dihydroquinazolin-1(2H)-yl}pipéridine-1-carbaldéhyde | 570 |
| 261 | 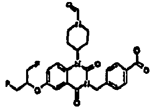 | acide 4-({6-[2-fluoro-1-(fluorométhyl)éthoxy]-1-(1-formylpipéridin-4-yl)-2,4-dioxo-1,4-dihydroquinazolin-3(2H)-yl}méthyl)benzoïque | 502 |
| 262 | 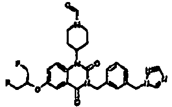 | 4-{6-[2-fluoro-1-(fluorométhyl)éthoxy]-2,4-dioxo-3-[3-(1H-1,2,4-triazol-1-ylméthyl)benzyl]-3,4-dihydroquinazolin-1(2H)-yl}pipéridine-1-carbaldéhyde | 539 |
| 263 | 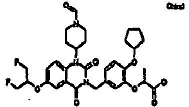 | acide (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]propanoïque | 630 |
| 264 | 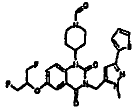 | 4-{6-[2-fluoro-1-(fluorométhyl)éthoxy]-3-[(1-méthyl-3-thiophén-2-yl-1H-pyrazol-5-yl)méthyl]-2,4-dioxo-3,4-dihydroquinazolin-1(2H)-yl}pipéridine-1-carbaldéhyde | 544 |
| 265 | 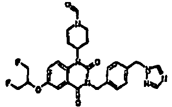 | 4-{6-[2-fluoro-1-(fluorométhyl)éthoxy]-2,4-dioxo-3-[4-(1H-1,2,4-triazol-1-ylméthyl)benzyl]-3,4-dihydroquinazolin-1(2H)-yl}pipéridine-1-carbaldéhyde | 539 |
| 266 | 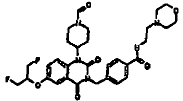 | 4-({6-[2-fluoro-1-(fluorométhyl)éthoxy]-1-(1-formylpipéridin-4-yl)-2,4-dioxo-1,4-dihydroquinazolin-3(2H)-yl}méthyl)-N-(2-morpholin-4-yléthyl)benzamide | 614 |
| 267 | 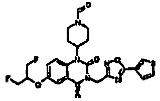 | 4-{6-[2-fluoro-1-(fluorométhyl)éthoxy]-2,4-dioxo-3-[(5-thiophén-3-yl-1,2,4-oxadiazol-3-yl)méthyl]-3,4-dihydroquinazolin-1(2H)-yl}pipéridine-1-carbaldéhyde | 532 |
| 268 | 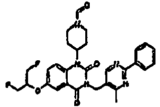 | 4-{6-[2-fluoro-1-(fluorométhyl)éthoxy]-3-[(4-méthyl-2-phénylpyrimidin-5-yl)méthyl]-2,4-dioxo-3,4-dihydroquinazolin-1(2H)-yl}pipéridine-1-carbaldéhyde | 550 |
| 269 | 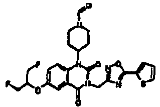 | 4-{6-[2-fluoro-1-(fluorométhyl)éthoxy]-2,4-dioxo-3-[(5-thiophén-2-yl-1,2,4-oxadiazol-3-yl)méthyl]-3,4-dihydroquinazolin-1(2H)-yl}pipéridine-1-carbaldéhyde | 532 |
| 270 | 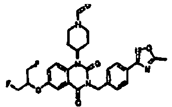 | 4-{6-[2-fluoro-1-(fluorométhyl)éthoxy]-3-[4-(5-méthyl-1,2,4-oxadiazol-3-yl)benzyl]-2,4-dioxo-3,4-dihydroquinazolin-1(2H)-yl}pipéridine-1-carbaldéhyde | 540 |
| 271 | 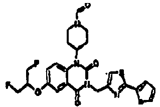 | 4-{6-[2-fluoro-1-(fluorométhyl)éthoxy]-2,4-dioxo-3-[(2-thiophén-2-yl-1,3-thiazol-4-yl)méthyl]-3,4-dihydroquinazolin-1(2H)-yl}pipéridine-1-carbaldéhyde | 547 |
| 272 | 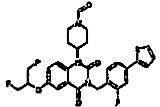 | 4-{6-[2-fluoro-1-(fluorométhyl)éthoxy]-3-(2-fluoro-4-thiophén-2-ylbenzyl)-2,4-dioxo-3,4-dihydroquinazolin-1(2H)-yl}pipéridine-1-carbaldéhyde | 558 |
| 273 | 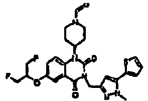 | 4-{6-[2-fluoro-1-(fluorométhyl)éthoxy]-3-[(1-méthyl-5-thiophén-2-yl-1H-pyrazol-3-yl)méthyl]-2,4-dioxo-3,4-dihydroquinazolin-1(2H)-yl}pipéridine-1-carbaldéhyde | 544 |
| 274 | 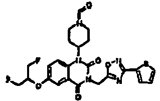 | 4-{6-[2-fluoro-1-(fluorométhyl)éthoxy]-2,4-dioxo-3-[(3-thiophén-2-yl-1,2,4-oxadiazol-5-yl)méthyl]-3,4-dihydroquinazolin-1(2H)-yl}pipéridine-1-carbaldéhyde | 532 |
| 275 | 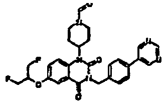 | 4-{6-[2-fluoro-1-(fluorométhyl)éthoxy]-2,4-dioxo-3-(4-pyrimidin-5-ylbenzyl)-3,4-dihydroquinazolin-1(2H)-yl}pipéridine-1-carbaldéhyde | 536 |
| 276 | 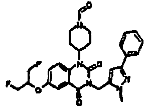 | 4-{6-[2-fluoro-1-(fluorométhyl)éthoxy]-3-[(1-méthyl-3-phényl-1H-pyrazol-5-yl)méthyl]-2,4-dioxo-3,4-dihydroquinazolin-1(2H)-yl}pipéridine-1-carbaldéhyde | 538 |
| 277 | 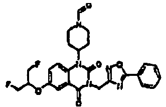 | 4-{6-[2 fluoro-1-(fluorométhyl)éthoxy]-2,4-dioxo-3-[(5-phényl-1,2,4-oxadiazol-3-yl)méthyl]-3,4-dihydroquinazolin-1(2H)-yl}pipéridine-1-carbaldéhyde | 526 |
| 278 | 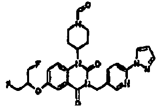 | 4-{6-[2-fluoro-1-(fluorométhyl)éthoxy]-2,4-dioxo-3-{[6-(1H-pyrazol-1-yl)pyridin-3-yl]méthyl}-3,4-dihydroquinazolin-1(2H)-yl}pipéridine-1-carbaldéhyde | 525 |
| 279 | 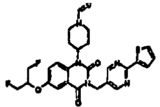 | 4-{6-[2-fluoro-1-(fluorométhyl)éthoxy]-2,4-dioxo-3-[(2-thiophén-2-ylpyrimidin-5-yl)méthyl]-3,4-dihydroquinazolin-1(2H)-yl}pipérldine-1-carbaldéhyde | 542 |
| 280 | 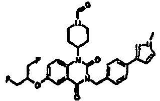 | 4-{6-[2-fluoro-1-(fluorométhyl)éthoxy]-3-[4-(1-méthyl-1H-pyrazol-3-yl)benzyl]-2,4-dioxo-3,4-dihydroquinazolin-1(2H)-yl}pipéridine-1-carbaldéhyde | 538 |
| 281 | 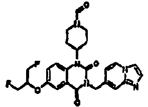 | 4-{6-[2-fluoro-1-(fluorométhyl)éthoxy]-3-(imidazo[1,2-a]pyridin-7-ylméthyl)-2,4-dioxo-3,4-dihydroquinazolin-1(2H)-yl}pipéridine-1-carbaldéhyde | 598 |
| 282 | 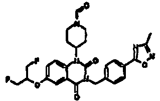 | 4-{6-[2-fluoro-1-(fluorométhyl)éthoxy]-3-[4-(3-méthyl-1,2,4-oxadiazol-5-yl)benzyl]-2,4-dioxo-3,4-dihydroquinazolin-1(2H)-yl}pipéridine-1-carbaldéhyde | 540 |
| 283 | 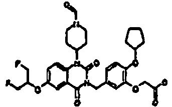 | acide [2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]acétique | 616 |
| 284 | 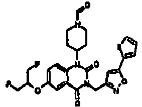 | 4-{6-[2-fluoro-1-(fluorométhyl)éthoxy]-2,4-dioxo-3-[(5-thiophén-2-ylisoxazol-3-yl)méthyl]-3,4-dihydroquinazolin-1(2H)-yl}pipéridine-1-carbaldéhyde | 531 |
| 285 | 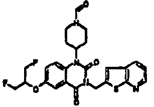 | 4-{6-[2-fluoro-1-(fluorométhyl)éthoxy]-2,4-dioxo-3-(thiéno[2,3-b]pyridin-2-ylméthyl)-3,4-dihydroquinazolin-1(2H)-yl}pipéridine-1-carbaldéhyde | 514 |
| 286 | 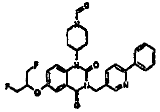 | 4-{6-[2-fluoro-1-(fluorométhyl)éthoxy]-2,4-dioxo-3-[(6-phénylpyridin-3-yl)méthyl]-3,4-dihydroquinazolin-1(2H)-yl}pipéddine-1-carbaldéhyde | 535 |
| 287 | 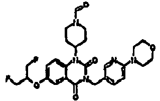 | 4-{6-[2-fluoro-1-(fluorométhyl)éthoxy]-3-[(6-morpholin-4-ylpyridin-3-yl)méthyl]-2,4-dioxo-3,4-dihydroquinazolin-1(2H)yl}pipéridine-1-carbaldéhyde | 544 |
| 288 | 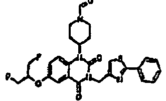 | 4-{6-[2-fluoro-1-(fluorométhyl)éthoxy]-2,4-dioxo-3-[(2-phényl-1,3-thiazol-4-yl)méthyl]-3,4-dihydroquinazolin-1(2H)-yl}pipéridine-1-carbaldéhyde | 541 |
| 289 | 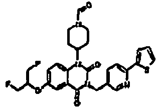 | 4-{6-[2-fluoro-1-(fluorométhyl)éthoxy]-2,4-dioxo-3-[(6-thiophén-2-ylpyridin-3-yl)méthyl]-3,4-dihydroquinazolin-1(2H)-yl}pipéridine-1-carbaldéhyde | 541 |
| 290 | 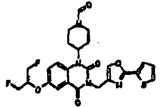 | 4-{6-[2-fluoro-1-(fluorométhyl)éthoxy]-2,4-dioxo-3-[(2-thiophén-2-yl-1,3-oxazol-4-yl)méthyl]-3,4-dihydroquinazolin-1(2H)-yl}pipéridine-1-carbaldéhyde | 531 |
| 291 | 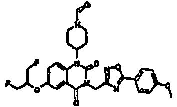 | 4-{6-[2-fluoro-1-(fluorométhyl)éthoxy]-3-{[5-(4-méthoxyphényl)-1,2,4-oxadiazol-3-yl]méthyl}-2,4-dioxo-3,4-dihydroquinazolin-1(2H)-yl}pipéridine-1-carbaldéhyde | 556 |
| 292 | 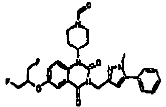 | 4-{6-[2-fluoro-1-(fluorométhyl)éthoxy]-3-[(1-méthyl-5-phényl-1H-pyrazol-3-yl)méthyl]-2,4-dioxo-3,4-dihydroquinazolin-1(2H)-yl}pipéridine-1-carbaldéhyde | 538 |
| 293 | 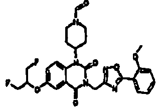 | 4-{6-[2-fluoro-1-(fluorométhyl)éthoxy]-3-{[5-(2-méthoxyphényl)-1,2,4-oxadiazol-3-yl]méthy}-2,4-dioxo-3,4-dihydroquinazolin-1(2H)-yl}pipéridine-1-carbaldéhyde | 556 |
| 294 | 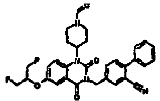 | 4-({6-[2-fluoro-1-(fluorométhyl)éthoxy)-1-(1-formylpipéridin-4-yl)-2,4-dioxo-1,4-dihydroquinazolin-3(2H)-yl}méthyl)biphényl-2-carbonitrile | 559 |
| 295 | 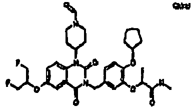 | (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthylpropanamide | 643 |
| 296 | 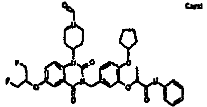 | (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-phénylpropanamide | 705 |
| 297 | 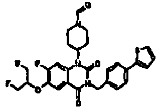 | 4-{7-fluoro-6-[2-fluoro-1-(fluorométhyl)éthoxy]-2,4-dioxo-3-(4-thiophén-2-ylbenzyl)-3,4-dihydroquinazolin-1(2H)-yl}pipéridine-1-carbaldéhyde | 558 |
| 298 | 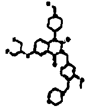 | 4-{6-[2-fluoro-1-(fluorométhyl)éthoxy]-3-[3-méthoxy-4-(morpholin-4-ylméthyl)benzyl]-2,4-dioxo-3,4-dihydroquinazolin-1(2H)-yl}pipéridine-1-carbaldéhyde | 587 |
| 299 | 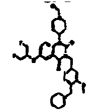 | 4-{6-[2-fluoro-1-(fluorométhyl)éthoxy]-3-(3-méthoxy-4-(pipéridin-1-ylméthyl)benzyl]-2,4-dioxo-3,4-dihydroquinazolin-1(2H)-yl}pipéridine-1-carbaldéhyde | 585 |
| 300 | 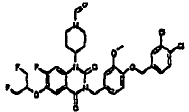 | 4-[3-{4-[(3,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde . | 680 |
| 301 | 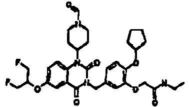 | 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-éthylacétamide | 643 |
| 302 | 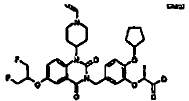 | acide (2S)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]propanoïque | 630 |
| 303 | 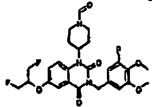 | 4-[3-(3-fluoro-4,5-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 536 |
| 304 | 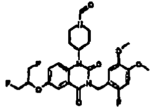 | 4-[3-(2-fluoro-4,5-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 536 |
| 305 | 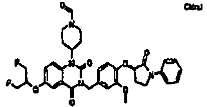 | 4-{6-[2-fluoro-1-(fluorométhyl)éthoxy]-3-(3-méthoxy-4-{[(3R)-2-oxo-1-phénylpyrrolidin-3-yl]oxy}benzyl)-2,4-dioxo-3,4-dihydroquinazolin-1(2H)-yl}pipéridine-1-carbaldéhyde | 663 |
| 306 | 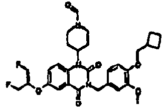 | 4-{3-[4-(cyclobutylméthoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde | 572 |
| 307 | 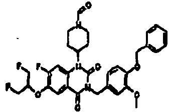 | 4-{3-[4-(benzyloxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 612 |
| 308 | 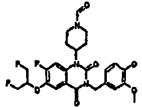 | 4-{7-fluoro-6-[2-fluoro-1-(fluorométhyl)éthoxy]-3-(4-hydroxy-3-méthoxybenzyl)-2,4-dioxo-3,4-dihydroquinazolin-1(2H)-yl}pipéridine-1-carbaldéhyde | 522 |
| 309 | 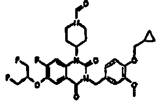 | 4-{3-[4-(cyclopropylméthoxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 576 |
| 310 | 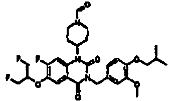 | 4-{7-fluoro-6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde | 578 |
| 311 | 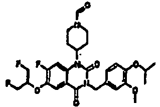 | 4-{7-fluoro-6-[2-fluoro-1-(fluorométhyl)éthoxy]-3-[3-méthoxy-4-(1-méthyléthoxy)benzyl]-2,4-dioxo-3,4-dihydroquinazolin-1(2H)-yl}pipéridine-1-carbaldéhyde | 564 |
| 312 | 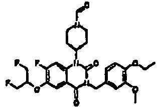 | 4-[3-(4-éthoxy-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 550 |
| 313 | 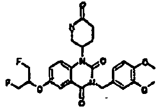 | 3-(3,4-diméthoxybenzyl)-6-[2-fluoro-1-(fluorométhyl)éthoxy]-1-(6-oxopipéridin-3-yl)quinazoline-2,4(1H,3H)-dione | 504 |
| 314 | 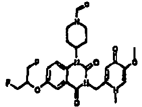 | 4-{6-[2-fluoro-1-(fluorométhyl)éthoxy]-3-[(5-méthoxy-1-méthyl-4-oxo-1,4-dihydropyridin-2-yl)méthyl]-2,4-dioxo-3,4-dihydroquinazolin-1(2H)-yl}pipéridine-1-carbaldéhyde | 519 |
| 315 | 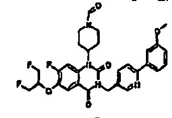 | 4-{7-fluoro-6-[2-fluoro-1-(fluorométhyl)éthoxy]-3-{[6-(3-méthoxyphényl)pyridin-3-yl]méthyl}-2,4-dioxo-3,4-dihydroquinazolin-1(2H)-yl}pipéridine-1-carbaldéhyde | 583 |
| 316 | 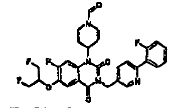 | 4-(7-fluoro-6-[2-fluoro-1-(fluorométhyl)éthoxy]-3-{[6-(2-fluorophényl)pyridin-3-yl]méthyl}-2,4-dioxo-3,4-dihydroquinazolin-1(2H)-yl}pipéridine-1-carbaldéhyde | 571 |
| 317 | 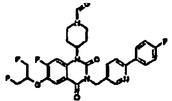 | 4-{7-fluoro-6-[2-fluoro-1-(fluorométhyl)éthoxy]-3-{[6-(4-fluorophényl)pyridin-3-yl]méthyl}-2,4-dioxo-3,4-dihydroquinazolin-1(2H)-yl}pipéridine-1-carbaldéhyde | 571 |
| 318 | 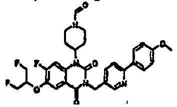 | 4-{7-fluoro-6-[2-fluoro-1-(fluorométhyl)éthoxy]-3-{[6-(4-méthoxyphényl)pyridin-3-yl]méthyl}-2,4-dioxo-3,4-dihydroquinazolin-1(2H)-yl}pipéridine-1-carbaldéhyde | 583 |
| 319 | 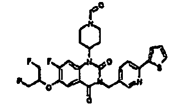 | 4-{7-fluoro-6-[2-fluoro-1-(fluorométhyl)éthoxy]-2,4-dioxo-3-[(6-thiophén-2-ylpyridin-3-yl)méthyl]-3,4-dihydroquinazolin-1(2H)-yl}pipéridine-1-carbaldéhyde | 559 |
| 320 | 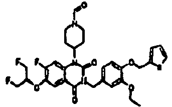 | 4-{3-[3-éthoxy-4-(thiophén-2-ylméthoxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 632 |
| 321 | 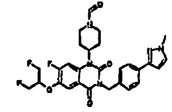 | 4-{7-fluoro-6-[2-fluoro-1-(fluorométhyl)éthoxy]-3-[4-(1-méthyl-1H-pyrazol-3-yl)benzyl]-2,4-dioxo-3,4-dihydroquinazolin-1(2H)-yl}pipéridine-1-carbaldéhyde | 556 |
| 322 | 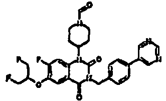 | 4-{7-fluoro-6-[2-fluoro-1-(fluorométhyl)éthoxy]-2,4-dioxo-3-(4-pyrimidin-5-ylbenzyl)-3,4-dihydroquinazolin-1(2H)-yl}pipéridine-1-carbaldéhyde | 554 |
| 323 | 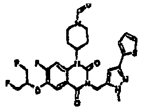 | 4-{7-fluoro-6-[2-fluoro-1-(fluorométhyl)éthoxy]-3[(1-méthyl-3-thiophén-2-yl-1H-pyrazol-5-yl)méthyl]-2,4-dioxo-3,4-dihydroquinazolin-1(2H)-yl}pipéridine-1-carbaldéhyde | 562 |
| 324 | 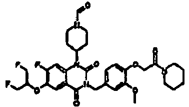 | 4-{7-fluoro-6-[2-fluoro-1-(fluorométhyl)éthoxy]-3-[3-méthoxy-4-(2-oxo-2-pipéridin-1-yléthoxy)benzyl]-2,4-dioxo-3,4-dihydroquinazolin-1(2H)-yl}pipéridine-1-carbaldéhyde | 647 |
| 325 | 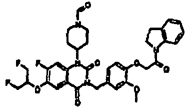 | 4-[3-{4-[2-(2,3-dihydro-1H-indol-1-yl)-2-oxoéthoxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 681 |
| 326 | 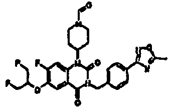 | 4-{7-fluoro-6-[2-fluoro-1-(fluorométhyl)éthoxy]-3-[4-(5-méthyl-1,2,4-oxadiazol-3-yl)benzyl]-2,4-dioxo-3,4-dihydroquinazolin-1(2H)-yl}pipéridine-1-carbaldéhyde | 558 |
| 327 | 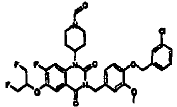 | 4-[3-{4-[(3-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 646 |
| 328 | 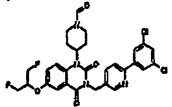 | 4-[3-{[6-(3,5-dichlorophényl)pyridin-3-yl]méthyl}-6-[2-fluoro-1-(fluorométhyl)éthoxy]-2,4-dioxo-3,4-dihydroquinazolin-1(2H)-yl]pipéridine-1-carbaldéhyde | 603 |
| 329 | 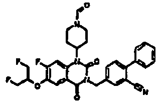 | 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)biphényl-2-carbonitiile | 577 |
| 330 | 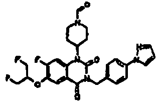 | 4-{7-fluoro-6-[2-fluoro-1-(fluorométhyl)éthoxy]-2,4-dioxo-3-[4-(1H-pyrazol-1-yl)benzyl]-3,4-dihydroquinazolin-1(2H)-yl}pipéridine-1-carbaldéhyde | 542 |
| 331 | 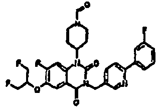 | 4-{7-fluoro-6-[2-fluoro-1-(fluorométhyl)éthoxy]-3-{[6-(3-fluorophényl)pyridin-3-yl]méthyl}-2,4-dioxo-3,4-dihydroquinazolin-1(2H)-yl}pipéridine-1-carbaldéhyde | 571 |
| 332 | 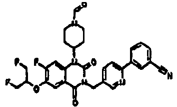 | 3-[5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)pyridin-2-yl]benzonitrile | 578 |
| 333 | 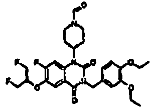 | 4-[3-(3,4-diéthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 564 |
| 334 | 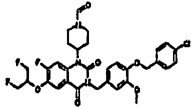 | 4-[3-{4-[(4-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 646 |
| 335 | 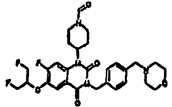 | 4-{7-fluoro-6-[2-fluoro-1-(fluorométhyl)éthoxy]-3-[4morpholin-4-ylméthyl)benzyl]-2,4-dioxo-3,4-dihydroquinazolin-1(2H)-yl}pipéridine-1-carbaldéhyde | 575 |
| 336 | 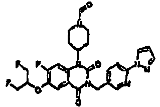 | 4-{7-fluoro-6-[2-fluoro-1-(fluorométhyl)éthoxy]-2,4-dioxo-3-{[6-(1H-pyrazol-1-yl}pyridin-3-yl]méthyl}-3,4-dihydroquinazolin-1(2H)-yl}pipéridine-1-carbaldéhyde | 543 |
| 337 | 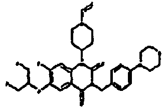 | 4-{7-fluoro-6-[2-fluoro-1-(fluorométhyl)éthoxy]-3-(4-morpholin-4-ylbenzyl)-2,4-dioxo-3,4-dihydroquinazolin-1(2H)-yl}pipéridine-1-carbaldéhyde | 561 |
| 338 | 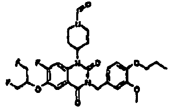 | 4-{7-fluoro-6-[2-fluoro-1-(fluorométhyl)éthoxy]-3-(3-méthoxy-4-propoxybenzyl)-2,4-dioxo-3,4-dihydroquinazolin-1(2H)-yl}pipéridine-1-carbaldéhyde | 564 |
| 339 | 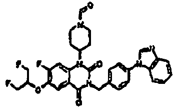 | 4-{3-[4-(1H-benzimidazol-1-yl)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 592 |
| 340 | 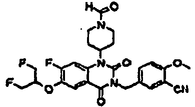 | 5-({7-fluoro-6-[2-fluoro-1-(fluorométhyl)éthoxy]-1-(1-formylpipéridin-4-yl)-2,4-dioxo-1,4-dihydroquinazolin-3(2H)-yl]méthyl)-2-méthoxybenzonitrile | 531 |
| 341 | 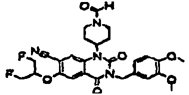 | 3-(3,4-diméthoxybenzyl)-6-[2-fluoro-1-(fluorométhyl)éthoxy]-1-(1-formylpipéridin-4-yl)-2,4-dioxo-1,2,3,4-tétrahydroquinazoline-7-carbonitrile | 543 |
| 342 | 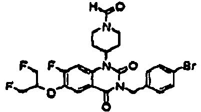 | 4-[3-(4-bromobenzyl)-7-fluoro-1-[2-fluoro-1-(fluorométhyl)éthoxy]-2,4-dioxo-3,4-dihydroquinazolin-1(2H)-yl]pipéridine-1-carbaldéhyde | 554 |
| 343 | 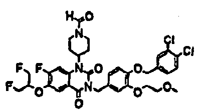 | 4-[3-{4-[(3,4-dichlorobenzyl)oxy]-3-(2-méthoxyéthoxy)benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 724 |
| 344 | 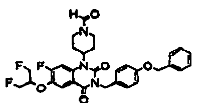 | 4-{3-[4-(benzyloxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 582 |
| 345 | 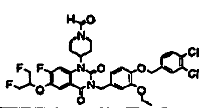 | 4-[3-{4-[(3,4-dichlorobenzyl)oxy]-3-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 694 |
| 346 | 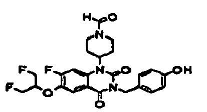 | 4-{7-fluoro-6-[2-fluoro-1-(fluorométhyl)éthoxy]-3-(4-hydroxybenzyl)-2,4-dioxo-3,4-dihydroquinazolin-1(2H)-yl}pipéridine-1-carbaldéhyde | 492 |
| 347 | 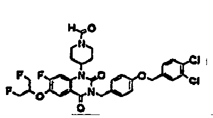 | 4-[3-{4-[(3,4-dichlorobenzyl)oxy]benzyl}-7-fluoro-6-(2-fluoro-1-(fluorométhyl)éthoxy]-2,4-dioxo-3,4-dihydroquinazolin-1(2H)-yl]pipéridine-1-carbaldéhyde | 650 |
| 348 | 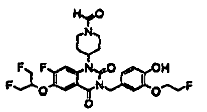 | 4-{7-fluoro-3-[3-(2-fluoroéthoxy)-4-hydroxybenzyl]-6-[2-fluoro-1-(fluorométhyl)éthoxy]-2,4-dioxo-3,4-dihydroquinazolin-1(2H)-yl}pipéridine-1-carbaldéhyde | 554 |
| 349 | 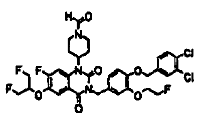 | 4-[3-{4-[(3,4-dichlorobenzyl)oxy]-3-(2-fluoroéthoxy)benzyl}-7-fluoro-6-[2-fluoro-1-(fuorométhyl)éthoxy]-2,4-dioxo-3,4-dihydroquinazolin-1(2H)-yl]pipéridine-1-carbaldéhyde | 712 |
| 350 | 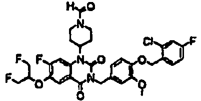 | 4-[3-{4-[{2-chloro-4-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 664 |
| 351 | 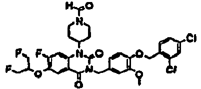 | 4-[3-{4-[(2,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 352 | 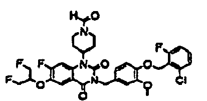 | 4-[3-{4-[(2-chloro-6-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 664 |
| 353 | 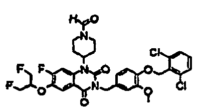 | 4-[3-{4-[(2,6-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 354 | 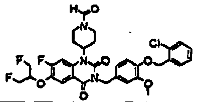 | 4-[3-{4-[(2-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 646 |
| 355 | 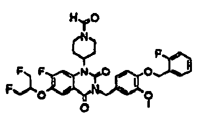 | 4-[7-fluoro-3-{4-[(2-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 630 |
| 356 | 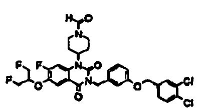 | 4-[3-{3-[(3,4-dichlorobenzyl)oxy]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 650 |
| 357 | 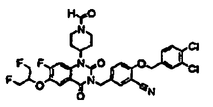 | 2-[(3,4-dichlorobenzyl)oxy]-5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)benzonitrile | 675 |
| 358 | 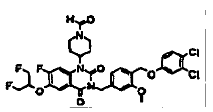 | 4-[3-{4-[(3,4-dichlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 359 | 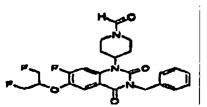 | 4-{3-benzyl-7-fluoro-6-[2-fluoro-1-(fluorométhyl)éthoxy]-2,4-dioxo-3,4-dihydroquinazolin-1(2H)-yl}pipéridine-1-carbaldéhyde | 476 |
| 360 | 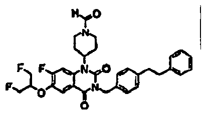 | 4-{7-fluoro-6-[2-fluoro-1-(fluorométhyl)éthoxy]-2,4-dioxo-3-[4-(2-phényléthyl)benzyl]-3,4-dihydroquinazolin-1(2H)-yl}pipéridine-1-carbaldéhyde | 580 |
| 361 | 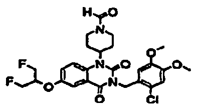 | 4-[3-(2-chloro-4,5-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 552 |
| 362 | 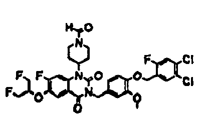 | 4-[3-{4-[(4,5-dichloro-2-fluorobenzyl)oxy]-3-méthoxybenzyl}-7-fluoro-6-[2-fluoro-1-(fluorométhyl)éthoxy]-2,4-dioxo-3,4-dihydroquinazolin-1-(2H)-yl]pipéridine-1-carbaldéhyde | 698 |
| 363 | 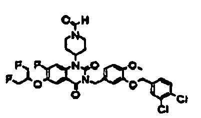 | 4-[3-{3-[(3,4-dichlorobenzyl)oxy]-4-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 364 | 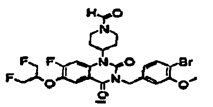 | 4-[3-(4-bromo-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 584 |
| 365 | 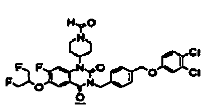 | 4-[3-{4-[(3,4-dichlorophenoxy)méthyl]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 650 |
| 366 | 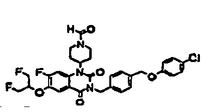 | 4-[3-{4-[(4-chlorophénoxy)méthyl]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 616 |
| 367 | 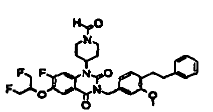 | 4-{7-fluoro-6-[2-fluoro-1-(fluorométhyl)éthoxy]-3-[3-méthoxy-4-(2-phényléthyl)benryl]-2,4-dioxo-3,4-dihydroquinazolin-1(2H)-yl}pipéridine-1-carbaldéhyde | 610 |
| 368 | 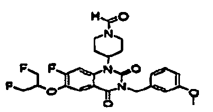 | 4-{7-fluoro-6-[2-fluoro-1-(fluorométhyl)éthoxy]-3-(3-méthoxybenzyl)-2,4-dioxo-3,4-dihydroquinazolin-1(2H)-yl}pipéridine-1-carbaldéhyde | 506 |
| 369 | 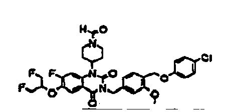 | 4-[3-{4-[(4-chlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 646 |
| 370 | 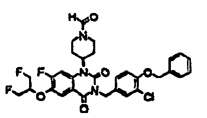 | 4-{3-[4-(benzyloxy)-3-chlorobenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 616 |
| 371 | 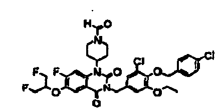 | 4-[3-{3-chloro-4-[(4-chlorobenzyl)oxy]-5-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 694 |
| 372 | 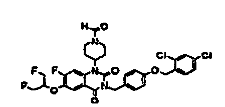 | 4-[3-{4-[(2,4-dichlorobenzyl)oxy]benzyl}-7-fluoro-6-[2-fluoro-1-(fluorométhyl)éthoxy]2,4-dioxo-3,4-dihydroquinazolin-1(2H)-yl]pipéridine-1-carbaldéhyde | 650 |
| 373 | 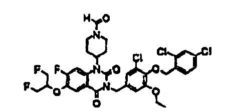 | 4-[3-{3-chloro-4-[(2,4-dichlorobenzyl)oxy]-5-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 728 |
| 374 | 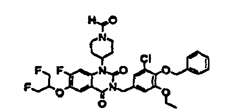 | 4-{3-[4-(benzyloxy)-3-chloro-5-éthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 660 |
| 375 | 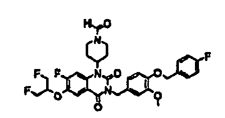 | 4-[7-fluoro-3-{4-[(4-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 630 |
| 376 | 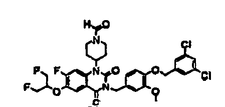 | 4-[3-{4-[(3,5-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 377 | 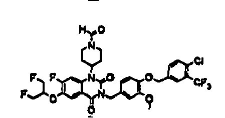 | 4-[3-(4-{[4-chloro-3-(trifluorométhyl)benzyl]oxy}-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde | 714 |
| 378 | 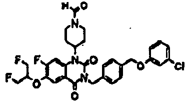 | 4-[3-{4-[(3-chlorophénoxy)méthyl]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 616 |
| 379 | 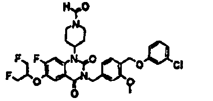 | 4-[3-{4-[(3-chlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 646 |
| 380 | 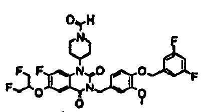 | 4-[3-{4-[(3,5-difluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 648 |
| 381 | 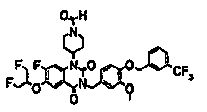 | 4-{3-[4-(benzyloxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 680 |
| 382 | 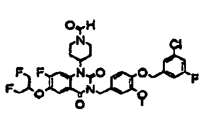 | 4-[3-{4-[(3-chloro-5-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 664 |
| 383 | 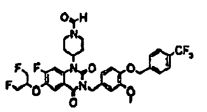 | 4-{7-fluoro-6-[2-fluoro-1-(fluorométhyl)éthoxy]-3-(3-méthoxy-4-{[4-(trifluorométhyl)benzyl]oxy}benzyl)-2,4-dioxo-3,4-dihydroquinazolin-1(2H)-yl}pipéridine-1-carbaldéhyde | 680 |
| 384 | 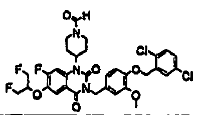 | 4-[3-{4-[(2,5-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 680 |
| 385 | 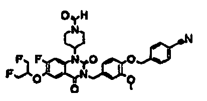 | 4-{[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxyl]méthyl}benzonitrile | 637 |
| 386 | 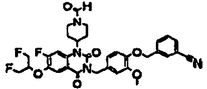 | 3-{[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]méthyl}benzonitrile | 637 |
| 387 | 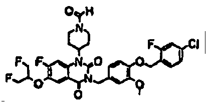 | 4-[3-{4-[(4-chloro-2-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 664 |
| 388 | 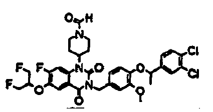 | 4-[3-{4-[1-(3,4-dichlorophényl)éthoxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 694 |
| 389 | 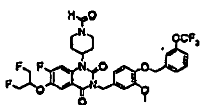 | 4-{7-fluoro-6-[2-fluoro-1-(fluorométhyl)éthoxy]-3-{4-[(3-hydroxybenzyl)oxy]-3-méthoxybenzyl}-2,4-dioxo-3,4-dihydroquinazolin-1(2H)-yl}pipéridine-1-carbaldéhyde | 696 |
| 390 | 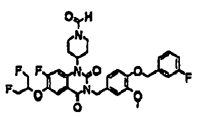 | 4-[7-fluoro-3-{4-[(3-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde | 630 |
| 391 | 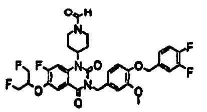 | 4-[3-{4-[(3,4-difluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 648 |
| 392 | 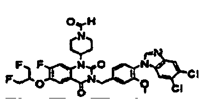 | 4-{3-[4-(5,6-dichloro-1H-benzimidazol-1-yl)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 660 |
| 393 | 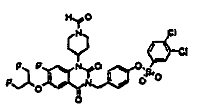 | 3,4-dichlorobenzènesulfonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)phényle | 700 |
| 394 | 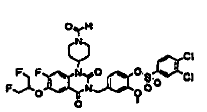 | 3,4-dichlorobenzènesulfonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényle | 730 |
| 395 | 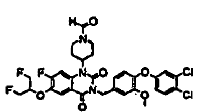 | 4-{3-[4-(3,4-dichlorophénoxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 666 |
| 396 | 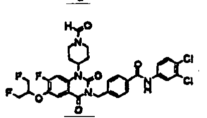 | N-(3,4-dichlorophényl)-4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)benzamide | 645 |
| 397 | 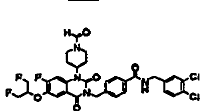 | N-(3,4-dichlorobenzyl)-4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)benzamide | 659 |
| 398 | 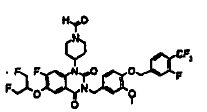 | 4-{7-fluoro-6-[2-fluoro-1-(fluorométhyl)éthoxy]-3-(4-{[3-fluoro-4-(trifluorométhyl)benzyl]oxy}-3-méthoxybenzyl)-2,4-dioxo-3,4-dihydroquinazolin-1(2H)-yl}pipéridine-1-carbaldéhyde | 698 |
| 399 | 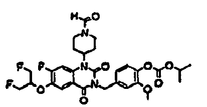 | carbonate de 4-({7-fluoro-6-[2-fluoro-1-(fluorométhyl)éthoxy]-1-(1-formylpipéridin-4-yl)-2,4-dioxo-1,4-dihydroquinazolin-3(2H)yl}méthyl)-2-méthoxyphényle et de 1-méthyléthyle | 608 |
| 400 | 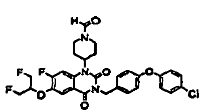 | 4-{3-[4-(4-chlorophénoxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 602 |
| 401 | 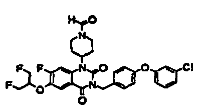 | 4-{3-[4-(3-chlorophénoxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 602 |
| 402 | 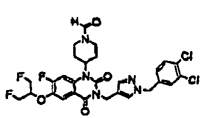 | 4-[3-{[1-(3,4-dichlorobenzyl)-1H-pyrazol-4-yl]méthyl}-7-fluoro-6-[2-fluoro-1-(fluorométhyl)éthoxy]-2,4-dioxo-3,4-dihydroquinazolin-1(2H)-yl]pipéridine-1-carbaldéhyde | 624 |
| 403 | 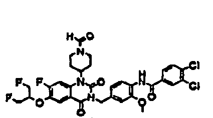 | 3,4-dichloro-N-[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényl]benzamide | 693 |
| 404 | 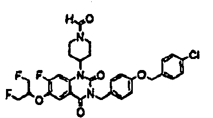 | 4-[3-{4-[(4-chlorobenzyl)oxy]benzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 616 |
| 405 | 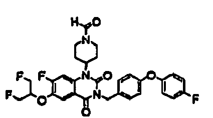 | 4-{7-fluoro-6-[2-fluoro-1-(fluorométhyl)éthoxy]-3-[4-(4-fluorophénoxy)benzyl]-2,4-dioxo-3,4-dihydroquinazolin-1(2H)-yl}pipéridine-1-carbaldéhyde | 586 |
| 406 | 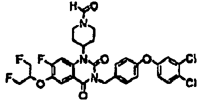 | 4-{3-[4-(3,4-dichlorophénoxy)benzyl]-7-fluoro-6-[2-fluoro-1-(fluorométhyl)éthoxyl]-2,4-dioxo-3,4-dihydroquinazolin-1(2H)-yl}pipéridine-1-carbaldéhyde | 636 |
| 407 | 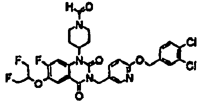 | 4-[3-({6-[(3,4-dichlorobenzyl)oxy]pyridin-3-yl}méthyl)-7-fluoro-6-[2-fluoro-1-(fluorométhyl)éthoxyl]-2,4-dioxo-3,4-dihydroquinazolin-1(2H)-yl]pipéridine-1-carbaldéhyde | 651 |
| 408 | 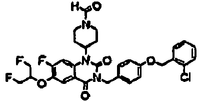 | 4-[3-{4-[(2-chlorobenzyl)oxy]benzyl}-7-fluoro-6-(2-fluoro-1-(fluorométhyl)éthoxy]-2,4-dioxo-3,4-dihydroquinazolin-1(2H)-yl]pipéridine-1-carbaldéhyde | 616 |
| 409 | 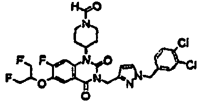 | 4-[3-{[1-(3,4-dichlorobenzyl)-1H-pyrazol-3-yl]méthyl}-7-fluoro-6-[2-fluoro-1-(fluorométhyl)éthoxy]-2,4-dioxo-3,4-dihydroquinazolin-1(2H)-yl]pipéridine-1-carbaldéhyde | 624 |
| 410 | 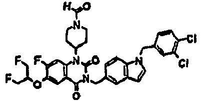 | 4-[3-{[1-(3,4-dichlorobenzyl)-1H-indol-5-yl]méthyl}-7-fluoro-6-[2-fluoro-1-(fluorométhyl)éthoxy]-2,4-dioxo-3,4-dihydroquinazolin-1(2H)-yl]pipéridine-1-carbaldéhyde | 673 |
| 411 | 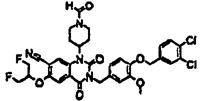 | 3-{4-[(3,4-dichlorobenzyl)oxy]-3-méthoxybenzyl}-6-[2-fluoro-1-(fluorométhyl)éthoxy]-1-(1-formylpipéridin-4-yl)-2,4-dioxo-1,2,3,4-tétrahydroquinazoline-7-carbonitrile | 687 |
| 412 | 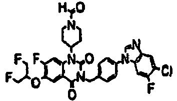 | 4-{3-[4-(5-chloro-6-fluoro-1H-benzimidazol-1-yl)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 644 |
| 413 | 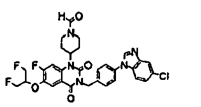 | 4-{3-[4-(5-chloro-1H-benzimidazol-1-yl)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde | 626 |
| 414 | 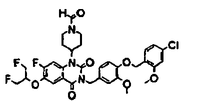 | 4-[3-{4-[(4-chloro-2-méthoxybenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 676 |
| 415 | 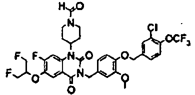 | 4-[3-{4-[(3-chloro-4-hydroxybenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde | 730 |

Les composés de l'invention ont fait l'objet d'essais pharmacologiques qui ont montré leur intérêt comme substances thérapeutiquement actives.

### 1) Mesure de l'activité inhibitrice des composés selon l'invention vis-à-vis de la PDE7

La capacité des composés de formule (I) à inhiber la PDE7 est mesurée à l'aide d'un essai enzymatique basé sur la séparation de l'AMPc radioactif (substrat de la PDE7) du 5'-AMP radioactif (produit de la réaction enzymatique) par chromatographie en couche mince sur polyéthylèneimine (PEI) cellulose, après arrêt de la réaction enzymatique. Le 5'-AMP est extrait quantitativement de la PEI cellulose et sa radioactivité mesurée à l'aide d'un compteur à scintillation liquide.

L'activité inhibitrice des composés de formule (I) vis-à-vis de la PDE7 est représentée par la constante d'inhibition CI₅₀, définie comme la concentration du composé (inhibiteur) testé dans l'essai qui permet de réduire de 50% l'activité enzymatique de la PDE7. Plus les valeurs de CI₅₀ sont faibles, plus les composés sont de puissants inhibiteurs.

### Matériel

Le [³H]-AMPc (NET 275; 25 à 40 Cl/mmole) a été acheté chez Perkin Elmer (NEN Life Sciences, Boston, Etats-Unis), le rolipram chez Sigma (St Louis, MO, Etats-Unis), les feuilles de polyéthylèneimine cellulose F en plastique pour chromatographie sur couche mince chez Merck (Darmstadt, Allemagne). Tous les autres produits utilisés sont d'origine commerciale.

### Enzyme

La PDE7 humaine a été partiellement purifiée à partir de la lignée cellulaire HUT-78 en suivant une méthode analogue à celle décrite par Bloom et Beavo (Proc Natl Acad Sci USA, (1996) 93, 14188-14192). La préparation d'enzyme obtenue est conservée à - 80°C dans un tampon contenant 20 mM de Tris-HCl (pH 7,0), 5 mM de MgCl₂, 4 mM d'EDTA, 1 mM de dithiothréitol et 20% de glycérol. La PDE7 partiellement purifiée étant contaminée par de la PDE4, il est nécessaire d'ajouter 10 µM de rolipram (inhibiteur sélectif de PDE4) dans l'essai enzymatique pour inhiber totalement l'activité PDE4. La constante de Michaelis (Km) de la PDE7 pour l'AMPc, mesurée à l'aide de l'essai radiochimique décrit ci-après, est de 21 nM.

### Solutions de composés selon l'invention

Les composés de formule (I) à tester comme inhibiteurs de PDE7 sont mis en solution dans le DMSO à une concentration de 10 mM. Ces solutions sont ensuite diluées en cascade dans le DMSO pour obtenir des solutions de concentrations désirées. Ces dernières sont ensuite diluées au vingtième dans le tampon d'essai pour donner des solutions à 5% de DMSO. Ces dernières sont finalement diluées au cinquième dans l'essai enzymatique.

La solution de rolipram (ajouté dans l'essai pour inhiber totalement l'activité PDE4 contaminante) est préparée de manière identique et apporte 1% de DMSO dans l'essai enzymatique.

### Essai enzymatique PDE7

L'essai est réalisé dans des tubes Eppendorf de 1,5 ml contenant 40 mM de Tris-HCl (pH 7,5), 15 mM de MgCl₂, 1 mM d'EGTA, 0,5 mg/ml d'albumine de sérum de boeuf, 0,063 µCi de [³H]-AMPc (correspondant à une concentration d'AMPc comprise entre 15 et 25 nM), 10 µM de rolipram et la PDE7 dans un volume final de 100 µl. L'essai est réalisé en absence (échantillon témoin) ou en présence (échantillon traité) des composés testés comme inhibiteurs de PDE7. La concentration finale de DMSO dans l'essai est de 2%. La réaction est initiée par addition d'enzyme et les échantillons sont maintenus à température ambiante pendant 30 minutes. La dilution enzymatique est ajustée de façon à obtenir un taux de conversion de 10 à 15%. La réaction enzymatique est arrêtée par immersion des tubes Eppendorf bouchés dans un bain-marie à 100°C pendant 3 minutes. Des blancs (réaction arrêtée immédiatement après addition de l'enzyme) sont inclus dans chaque expérience. Les échantillons sont ensuite centrifugés à 10,000xg pendant 1 minute et une part aliquote de 10 µl de surnageant est déposée à 2 cm du bord inférieur d'une feuille de PEI cellulose sur laquelle 10 µg d'AMPc et 10 µg de 5'-AMP ont été préalablement déposés. Pour faciliter la migration et le découpage ultérieur des bandes de PEI cellulose contenant le 5'-AMP, 18 voies de migration d'1 cm de largeur sont délimitées par plaque en grattant la cellulose avec une spatule sur 1 mm de largeur. Les plaques sont développées sur toute leur longueur avec une solution 0,30 M de LiCl dans l'eau par chromatographie ascendante. Le 5'-AMP (Rf = 0,20) et l'AMPc (Rf = 0,47) sont visualisés sous lumière U.V. à 254 nm. Les bandes de PEI cellulose contenant le 5'-AMP sont découpées et le nucléotide est extrait quantitativement dans des fioles de comptage avec 2 ml d'une solution contenant 16 M d'acide formique et 2 M de formate d'ammonium dans l'eau (agitateur rotatif pendant 15 min). Après ajout de 10 ml de liquide à scintillation (OptiPhase HiSafe 3 de Perkin Elmer / Wallac), la radioactivité est comptée à l'aide d'un compteur à scintillation liquide (modèle 1414, Perkin Elmer / Wallac). Chaque essai est réalisé en double. La radioactivité spécifiquement associée au 5'-AMP formé dans la réaction enzymatique est obtenue en soustrayant la valeur moyenne des blancs de la valeur moyenne des témoins (ou des traités).

Le pourcentage d'inhibition de la PDE7 à une concentration donnée du composé testé (inhibiteur) est calculé à l'aide de l'équation : 1% = [valeur moyenne des témoins - valeur moyenne des traités] x 100 / [valeur moyenne des témoins - valeur moyenne des blancs].

La CI₅₀ est la concentration du composé (inhibiteur) testé dans l'essai qui permet de réduire de 50% l'activité enzymatique de la PDE7.

### Résultats

A titre d'exemples illustratifs et non limitatifs, les quinazolinediones suivantes inhibent la PDE7 avec les valeurs de CI₅₀ indiquées ci-après:

| Composé | CI₅₀ (µM) |
|---|---|
| n°16 : 4-[6-(2,2-difluoroéthoxy)-3-(3,4-diméthoxybenzyl)-2,4-dioxo-3,4-dihydroquinazolin-1 (2H)-yl]pipéridine-1-carbaldéhyde | 0.015 |
| n°11 : 4-[3-(3,4-diméthoxybenzyl)-6-[2-fluoro-1-(fluorométhyl)éthoxy]-2,4-dioxo-3,4-dihydroquinazolin-1(2H)-yl]pipéridine-1-carbaldéhyde | 0.039 |
| n°72 : 4-[3-(3,4-diméthoxybenzyl)-6-(2-hydroxyéthoxy)-2,4-dioxo-3,4-dihydroquinazolin-1(2H)-yl]pipéridine-1-carbaldéhyde | 0.089 |
| n°77: N-{[3-(3,4-diméthoxybenzyl)-1-(1-formylpyrrolidin-3-yl)-2,4-dioxo-1,2,3,4-tétrahydroquinazolin-6-yl]méthyl}acétamide | 0.82 |
| n°97 : 4-[5,7-dichloro-3-(3,4-diméthoxybenzyl)-2,4-dioxo-3,4-dihydroquinazolin-1(2H)-yl]pipéridine-1-carbaldéhyde | 0.061 |
| n°111 : 4-[6-(difluorométhoxy)-3-(3,4-diméthoxybenzyl)-2,4-dioxo-3,4-dihydroquinazolin-1(2H)-yl]pipéridine-1-carbaldéhyde | 0.0067 |

### 2) Mesure de l'activité inhibitrice des composés selon l'invention vis-à-vis de la PDE8

En utilisant pour PDE8 un essai enzymatique équivalent à celui décrit pour PDE7, on obtient les valeurs suivantes de CI₅₀ :

| Composé | CI₅₀ PDE7 (µM) | CI₅₀ PDE8 (µM) |
|---|---|---|
| N° 11 | 0.039 | 0.14 |
| N° 251 | 0.046 | 0.39 |
| N° 294 | 0.037 | 0.015 |

### 3) Inhibition de la prolifération des cellules T

### Isolement des cellules mononucléaires du sang périphérique (=PBMNCs).

Les cellules mononucléaires du sang périphérique humain (human peripheral blood mononuclear cells, abréviation PBMNCs) ont été séparées du sang de donneurs sains à l'aide d'un gradient de centrifugation sur Ficoll-PaqueTM Plus (GE Healthcare Bio-Sciences AB code 17-1440-03, Uppsala). Un mélange de 5,0 ml de sang et de 5,0ml de tampon phosphate salin (PBS Gibco 14190) a été déposé sur 10,0ml de Ficoll-Paque dans un tube de 50 ml Blue max (Becton Dickinson 352070), et centrifugé à 900g pendant 30 min. L'anneau de PBMNCs a été prélevé, lavé dans du PBS, puis centrifugé à 900g pendant 10 minutes. Finalement les PBMNCs ont été suspendus (2,5x10⁵ cellules/ ml) dans un milieu complet RPMI 1640+Glutamax (Gibco 61870) additionné de 10% de FCS, dont le complément à été inactivé à la chaleur, et de Pénicilline-Streptomycine (Gibco 15140).

### Mesure de la prolifération par incorporation de [³H]- thymidine.

Les PBMNCs ont été cultivés dans des plaques à 96 puits à fond plat, à raison de 0,5 x 10⁵ cellules/ puits dans 200µl de milieu complet RPMI1640. Les cellules ont été activées par deux anticorps monoclonaux, l'un de spécificité anti-CD3, 20 ng/ml (Pharmingen 555329) et l'autre de spécificité anti-CD28, 200 ng/ml (Pharmingen 555725), en présence d'inhibiteurs de PDE7 à cinq concentrations différentes, 10µM, 3,3µM, 1,1µM, 0,36µM et 0,12 µM, ou de cyclosporine A servant de témoin positif de l'arrêt de la prolifération. Les deux anticorps monoclonaux employés simultanément induisent une intensité accrue de la réponse transcriptionnelle des cellules T activées via le CD3 seul (Diehn M. et al 2002. Genomic expression programs and the intégration of the CD28 costimulatory signal in T cell activation. Proc. Natl. Acad. Sci. USA 99, 11796-11801). De plus, les deux anticorps employés miment la condition optimale de l'activation in vivo qui met en oeuvre le TCR et le corécepteur CD28. Après 72 heures à 37°C dans 5% de CO₂ et 90% d'air humidifié, les cultures ont été marquées par 1 µCi [méthyl-³H] thymidine (Amersham TRK120). Après 8 heures d'incubation supplémentaire, les PBMNCs ont été recueillies sur un collecteur de cellules Tomtec (Wallac) en utilisant un filtre en fibres de verre (Wallac 1450-421), et l'incorporation de la [³H]-thymidine dans le noyau des cellules a été mesurée à l'aide d'un Microbeta 1450-Trilux (Wallac). L'inhibition de la prolifération, exprimée en pourcentage, a été calculée en utilisant l'équation suivante: 100 - (valeur moyenne cpm des cellules en présence du composé / valeur moyenne cpm des cellules en l'absence du composé) X 100.

### Résultats

Les activités inhibitrices des composés de formule (I) selon l'invention pour la prolifération correspondent à des valeurs de CI₅₀ (concentration de l'inhibiteur provoquant 50% de réduction de l'incorporation de la [³H]-thymidine dans les noyaux des cellules). Plus les valeurs de CI₅₀ sont faibles, plus les composés sont des inhibiteurs puissants de la prolifération des lymphocytes T.

Les quinazolinediones suivantes inhibent la prolifération des PBMNCs avec des valeurs de CI₅₀ indiquées ci-après (moyenne des trois expériences) :

| Composé | CI₅₀ (µM) |
|---|---|
| n°1 : (2-{[3-(3,4-diméthoxybenzyl)-1-(1-formylpipéridin-4-yl)-2,4-dioxo-1,2,3,4-tétrahydroquinazolin-6-yl]oxy}propanenitrile) | 0.4µM |
| n°11 : (4-[3-(3,4-diméthoxybenzyl)-6-[2-fluoro-1-(fluorométhyl)éthoxy]-2,4-dioxo-3,4-dihydroquinazolin-1(2H)-yl]pipéridine-1-carbaldéhyde) | 0.9µM |
| n°16 : (4-[6-(2,2-difluoroéthoxy)-3-(3,4-diméthoxybenzyl)-2,4-dioxo-3,4-dihydroquinazolin-1(2H)-yl]pipéridine-1-carbaldéhyde) | 1.3µM |

Les composés selon l'invention présentent de bonnes propriétés pharmacologiques et sont particulièrement aptes à être utilisés pour la préparation de médicaments, en particulier de médicaments inhibiteurs de PDE7, ou pour certains des composés de l'invention, de médicaments inhibiteurs de PDE7 et de PDE8.

Selon un autre de ses aspects, l'invention a donc pour objet des médicaments qui comprennent au moins un composé de formule (I).

Les composés selon l'invention peuvent être utiles notamment dans le traitement et/ou la prévention des maladies inflammatoires ou immuno-inflammatoires incluant par exemple l'asthme, la broncho-pneumopathie chronique obstructive (COPD), la rhinite allergique, les allergies, la maladie de Crohn, la colite ulcéreuse, la myasthénie grave, la dermatite atopique, le psoriasis, le lupus érythémateux disséminé, la polyarthrite rhumatoïde, le diabètes, la sclérose en plaques, dans le traitement des transplantations d'organes, dans le traitement et/ou la prévention de certains types de cancers tels que l'ostéosarcome ou de l'adénocarcinome, dans le traitement et/ou la prévention des maladies de l'os telles que l'ostéopénie ou l'ostéoporose, dans le traitement et/ou la prévention de la insuffisance aïgue, ou dans le traitement et/ou la prévention de la douleur, en particulier la douleur neuropathique et la douleur viscérale.

L'utilisation des composés de formule générale (I), pour la préparation d'un médicament destiné à traiter les pathologies mentionnées ci-dessus fait partie intégrante de l'invention, et en particulier les composés de formule (I) choisis parmi les composés n° 1 à 6, 11 à 14, 16, 20, 22 à 25, 32 à 43, 47 à 52, 55 à 59, 72, 74, 76, 78, 79, 89 à 91, 97, 102, 108, 111, 112, 114, 116 à 118, 124, 130, 131, 133 à 135, 143, 145, 155, 158, 160, 165 à 167, 170, 175, 178, 183 à 186, 188, 189, 190, 193, 194, 200, 201, 203, 206, 207, 212, 213, 215, 216, 218, 223, 224, 226, 228, 230, 232 à 234, 239, 240, 242, 243, 245, 246, 250, 251, 254, 258, 263, 264, 270, 275, 276, 278 à 280, 282, 283, 285 à 287, 289, 292, 294, 295, 287 à 302, 305 à 312, 315 à 345, 349 à 355, 357, 358, 360, 362, 369, 371, 373, 375 à 377, 379 à 394, et 403.

Selon un autre de ses aspects, la présente invention concerne des compositions pharmaceutiques comprenant, en tant que principe actif, au moins un composé selon l'invention. Ces compositions pharmaceutiques contiennent une dose efficace d'au moins un composé de formule (I) selon l'invention, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé salon l'invention sous forme de comprimé peut comprendre les composants suivants :

| | |
|---|---|
| Composé n°1 (2-{[3-(3,4-diméthoxybenzyl)-1-(1-formylpipéridin-4-yl)-2,4-dioxo-1,2,3,4-tétrahydroquinazolin-6-yl]oxy}propanenitrile) | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,5 mg à 800 mg de principe actif par individu, plus particulièrement de 0,5 mg à 200 mg, selon la forme galénique.

Il peut y avoir des cas où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. Composé répondant à la formule générale (I) 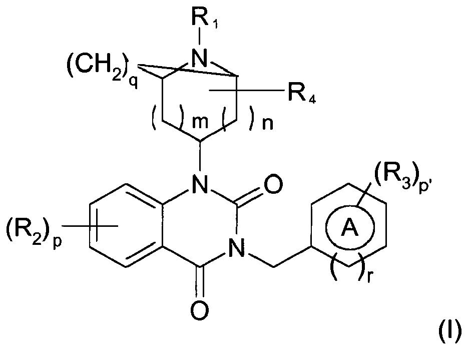 dans laquelle
- A représente un groupe aryle ou un groupe hétéroaryle;
- R₁ représente :
▪ un atome d'hydrogène,
▪ -C(O)R dans lequel R est un atome d'hydrogène, un groupe (C₁-C₆) alcoxy, un groupe aryle, un groupe (C₃-C₆) cycloalkyle, ou un groupe (C₁-C₆) alkyle, ledit alkyle étant éventuellement substitué par :
. un ou plusieurs groupe(s) hydroxy,
. un groupe benzyloxy,
. un groupe (C₁-C₆) alcoxy, éventuellement substitué par un aryle, ou
. un groupe (C₃-C₆) cycloalkyle,
▪ un groupe (C₁-C₆) alkyle éventuellement substitué;
- R₂ représente :
▪ un atome d'hydrogène,
▪ un atome d'halogène,
▪ un groupe cyano,
▪ un groupe nitro,
▪ un groupe (C₁-C₆) alkyle éventuellement substitué par un -NH₂, ou bien par un groupe -NHC(O)Rb,
▪ un groupe -ORa dans lequel Ra représente :
. un atome d'hydrogène,
. un groupe (C₁-C₆) alkyle éventuellement substitué par un ou plusieurs atome(s) d'halogène, par un ou plusieurs groupe(s) hydroxy, par un groupe aryle et/ou par un ou plusieurs groupe(s) cyano,
. un groupe (C₂C₆) alcynyle,
. un groupe aryle ;
- R₃ représente :
▪ un atome d'hydrogène,
▪ un atome d'halogène,
▪ un groupe hydroxy,
▪ un groupe cyano,
▪ un groupe -SCF₃,
▪ un groupe nitro,
▪ un groupe oxo,
▪ un groupe -S(O)₀₋₂-alkyle, un groupe -S(O)₀₋₂-hétérocycloalkyle, un groupe -0-SO₂-aryle éventuellement substitué par un ou plusieurs atome(s) d'halogène ;
▪ un groupe -alkyl-amino-alkyle ou -cycloalkyl-amino-alkyle, chacun éventuellement substitué sur l'alkyle terminal,
▪ un groupe sulfonamide éventuellement substitué,
▪ un groupe aryle ou un groupe hétéroaryle, ledit groupe étant monocyclique pu polycyclique et étant de plus éventuellement substitué par un groupe (C₁-C₈) alkyle, par un ou plusieurs atome(s) d'halogène, ou par un groupe (C₁-C₆) alcoxy,
▪ un groupe hétérocycloalkyle éventuellement substitué par un groupe (C₁-C₆) alkyle,
▪ un groupe (C₁-C₆) alkyle éventuellement substitué par :
- un ou plusieurs atome(s) d'halogène,
- un groupe aryle pouvant être substitué par un ou plusieurs atome(s) d'halogène, ou par un ou plusieurs groupe(s) hydroxy,
- un groupe hétéroaryle,
- un ou plusieurs groupe(s) hydroxy pouvant être substitué(s) par un groupe aryle lui-même éventuellement substitué par un ou plusieurs atome(s) d'halogène, ou
- un groupe hétérocycloalkyle éventuellement sunstitué par un groupe CO(O)Ra, ou par un groupe (C₁-C₆) alkyle,
▪ un groupe -C(O)NRbRc,
▪ un groupe -C(O)ORc, ou un groupe -O-C(O)ORc
▪ un groupe (C₁-C₆) alcoxy, éventuellement substitué par
- un groupe amino-alkyle,
- un groupe amino-cycloalkyle,
- un groupe cycloalkyle,
- un groupe hétérocycloalkyle
- un groupe hétéroaryle monocyclique ou polycyclique,
- un ou plusieurs groupement(s) hydroxy,
- un ou plusieurs atome(s) d'halogène,
- un groupe (C₁-C₆) alcoxy,
- un groupe -C(O)ORc,
- un groupe -C(O)NRbRc,
- un groupe oxo, et/ou
- un groupe aryle, lui-même éventuellement substitué par un ou plusieurs atome(s) d'halogène, un groupe cyano, un groupe (C₁-C₆) alcoxy, un groupe -O-halogénoalkyle et/ou par un groupe halogénoalkyle,
▪ un groupe -O-cycloalkyle, un groupe -O-aryle, ou un groupe -O-hétérocycloalkyle, chacun éventuellement substitué par
- un groupe aryle, lui-même éventuellement substitué par un ou plusieurs atome(s) d'halogène, ou par un groupe (C₁-C₆) alkyle,
- un groupe oxo,
- un ou plusieurs atome(s) d'halogène, et/ou
- un groupe (C₁-C₆) alkyle, lui-même pouvant être substitué par un groupe aryle et/ou un groupe oxo,
▪ un groupe -NH-CO-NH-aryle, un groupe -NH-CO-NH-hétéroaryle, ou un groupe-NH-CO-NH-(C₁-C₆) alkyle, chacun étant éventuellement substitué par un ou plusieurs atome(s) d'halogène, par un groupe cyano, par un groupe nitro, par un ou plusieurs groupe(s) hydroxy, ou par un groupe (C₁-C₆) alcoxy,
▪ un groupe -N-(C₁-C₆) alkyle, le groupe (C₁-C₆) alkyle pouvant être substitué par
- un ou plusieurs groupe(s) oxo, et/ou
- un ou plusieurs groupe(s) aryle éventuellement substitué(s) par un ou plusieurs atome(s) d'halogène et/ou par un groupe SO₂.
▪ un groupe -NH-CO-aryle, un groupe -NH-CO-hétéroaryle, chacun étant éventuellement substitué par un ou plusieurs atome(s) d'halogène ;
ou bien R₃ forme avec A un groupe hétéroaryle polycyclique éventuellement substitué par un groupe (C₁-C₆) alcoxy, un groupe (C₁-C₆) alkyle éventuellement substitué par un groupe aryle pouvant lui-même être substitué par un ou plusieurs atome(s) d'halogène;
- R₄ représente un atome d'hydrogène, un groupe oxo, ou un groupe (C₁-C₆) alkyle ;
- Rb représente :
. un atome d'hydrogène,
. un groupe (C₁-C₆) alkyle éventuellement substitué par un ou plusieurs atome(s) d'halogène, par un ou plusieurs groupe(s) hydroxy, cyano, amino, hétérocycloalkyle, (C₁-C₆) alcoxy, ou par un groupe aryle éventuellement substitué par un ou plusieurs atome(s) d'halogène,
. un groupe (C₃-C₆) cycloalkyle,
. un groupe (C₂-C₆) alcynyle,
. un groupe (C₁-C₆) alcoxy,
. un groupe aryle éventuellement substitué par un ou plusieurs atome(s) d'halogène;
- Rc représente un atome d'hydrogène, ou un groupe (C₁-C₆) alkyle éventuellement substitué par un ou plusieurs atome(s) d'halogène ;
ou alors Rb et Rc forment ensemble avec l'atome d'azote auquel ils sont attachés un groupe hétéroaryle polycyclique, ou un groupe hétérocycloalkyle ;
- m et n représentent indépendamment l'un de l'autre la valeur 0, 1 ou 2, étant entendu que m+n ≤ 3 ;
- p et p' représentent indépendamment l'un de l'autre la valeur 1, 2 ou 3, étant entendu que lorsque p est supérieur ou égal à 2, alors les groupes R₂ sont sur des atomes de carbone distincts et peuvent être différents les uns des autres, et lorsque p' est supérieur ou égal à 2 alors les groupes R₃ sont sur des atomes de carbone distincts et peuvent être différents les uns des autres;
- q représente la valeur 0 ou 2, étant entendu que lorsque q = 0, alors le groupe hétérocyclique azoté rattaché à l'azote situé en position 1 du noyau 2,4-dioxo-1,2,3,4 tétrahydroquinazoline n'est plus ponté et est du type : 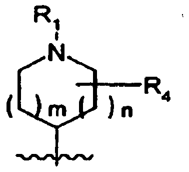
- r représente la valeur 0 ou 1.

2. Composé de formule générale (I) selon la revendication 1, **caractérisé en ce que** A représente un groupe phényle ou un groupe pyridyle.

3. Composé de formule générale (I) selon la revendication 1 ou 2, **caractérisé en ce que** q = 0, m et n = 1.

4. Composé de formule générale (I) selon l'une quelconque des revendications 1 à 3, **caractérisé en ce que** R₂ représente un groupe (C₁-C₆) alkyle, en particulier un méthyle substitué par un groupe -NH-CO-Rb, Rb étant tel que défini dans la revendication 1.

5. Composé de formule générale (I) selon l'une quelconque des revendications 1 à 3, **caractérisé en ce que** R₂ représente un groupe -ORa, Ra étant tel que défini dans la revendication 1.

6. Composé de formule générale (I) selon l'une quelconque des revendications 1 à 3, **caractérisé en ce que** R₂ est un atome d'halogène, ou un cyano, ou un hydrogène, ou un hydroxyle, ou un (C₁-C₆) alkyle éventuellement substitué par un -NH₂, ou bien par un groupe -NHC(O)Rb

7. Composé de formule générale (I) selon la revendication 1, **caractérisé en ce que** A est un phényle, R₁ est un groupe -C(O)R dans lequel R représente un atome d'hydrogène, q est égal à 0, n et m valent 1, et R₂ est -ORa.

8. Composé de formule générale (I) selon la revendication 1, **caractérisé en ce que** A est un phényle, R₁ est un groupe -C(O)R dans lequel R représente un atome d'hydrogène, q est égal à 0, n et m valent 1, et R₂ est un méthyle substitué par le groupe -NH-CO-Rb, Rb étant tel que défini dans la revendication 1.

9. Composé de formule générale (I) selon la revendication 1, **caractérisé en ce que** A est un phényle, R₁ est un groupe -C(O)R dans lequel R représente un atome d'hydrogène, q est égal à 0, n et m valent 1, p est égal à 2, l'un des R₂ est -ORa, Ra étant tel que défini dans la formule générale (I), et l'autre des R₂ est un atome d'halogène.

10. Composé de formule générale (I) selon l'une quelconque des revendications 1 à 9 **caractérisé en ce que** le groupe R₂ est en position 6 du noyau 2,4-dioxo-1,2,3,4 tétrahydroquinazoline, et **en ce qu'**il peut de plus y avoir un groupe R2, identique ou différent, en position 7 du noyau 2,4-dioxo-1,2,3,4 tétrahydroquinazoline; à l'état de base, d'hydrate ou de solvat, d'isomères ou de leurs mélanges.

11. Composé de formule générale (I) selon la revendication 1 choisi parmi les composés suivants :
- composé n°1 : 2-{[3-(3,4-diméthoxybenzyl)-1-(1-formylpipéridin-4-yl)-2,4-dioxo-1,2,3,4-tétrahydroquinazolin-6-yl]oxy}propanenitrile
- composé n°2 : 1-(1-acétylpipéridin-4-yl)-3-(3,4-diméthoxybenzyl)-6-hydroxyquinazoline-2,4(1 H,3H)-dione
- composé n°3 : {[1-(1-acétylpipéridin-4-yl)-3-(3,4-diméthoxybenzyl)-2,4-dioxo-1,2,3,4-tétrahydroquinazolin-6-yl]oxy}acétonitrile
- composé n°4 : 2-{[1-(1-acétylpipéridin-4-yl)-3-(3,4-diméthoxybenzyl)-2,4-dioxo-1,2,3,4-tétrahydroquinazolin-6-yl]oxy}propanenitrile
- composé n°6: {[3-(3,4-diméthoxybenzyl)-1-(1-formylpipéridin-4-yl)-2,4-dioxo-1,2,3,4-tétrahydroquinazolin-6-yl]oxy}acétonitrile
- composé n°11 : 4-[3-(3,4-diméthoxybenzyl)-6-[2-fluoro-1-(fluorométhyl)éthoxy]-2,4-dioxo-3,4-dihydroquinazolin-1(2H)-yl]pipéridine-1-carbaldéhyde
- composé n°12 :1-(1-acétylpipéridin-4-yl)-3-(3,4-diméthoxybenzyl)-6-[2-fluoro-1-(fluorométhyl)éthoxy]quinazoline-2,4(1H,3H)-dione
- composé n°13 : 4-[3-(3,4-diméthoxybenzyl)-2,4-dioxo-6-(2,2,2-trifluoroéthoxy)-3,4-dihydroquinazolin-1(2H)-yl]pipéridine-1-carbaldéhyde
- composé n°14 : 1-(1-acétylpipéridin-4-yl)-6-(2,2-difluoroéthoxy)-3-(3,4-diméthoxybenzyl)quinazoline-2,4(1H,3H)-dione
- composé n°16 :4-[6-(2,2-difluoroéthoxy)-3-(3,4-diméthoxybenzyl)-2,4-dioxo-3,4-dihydroquinazolin-1(2H)-yl]pipéridine-1-carbaldéhyde
- composé n°20 : N-{[3-(3,4-diméthoxybenzyl)-1-(1-formylpipéridin-4-yl)-2,4-dioxo-1,2,3,4-tétrahydroquinazolin-6-yl]méthyl}acétamide
- composé n°22 : chlorhydrate de 1-(1-acétylpipéridin-4-yl)-6-(aminométhyl)-3-(3,4-diméthoxybenzyl)quinazoline-2,4(1H,3H)-dione
- composé n°23 : N-{[3-(3,4-diméthoxybenzyl)-1-(1-formylpipéridin-4-yl)-2,4-dioxo-1,2,3,4-tétrahydroquinazolin-6-yl]méthyl}formamide
- composé n°24 : N-{[1-(1-acétylpipéridin-4-yl)-3-(3,4-diméthoxybenzyl)-2,4-dioxo-1,2,3,4-tétrahydroquinazolin-6-yl]méthyl}formamide
- composé n°25 : N-{[1-(1-acétylpipéridin-4-yl)-3-(3,4-diméthoxybenzyl)-2,4-dioxo-1,2,3,4-tétrahydroquinazolin-6-yl]méthyl}acétamide
- composé n°32 : 4-[6-(2,2-difluoroéthoxy)-2,4-dioxo-3,4-dihydroquinazolin-1(2H)-yl]pipéridine-1-carbaldéhyde
- composé n°33 : 4-[3-(3,4-dichlorobenzyl)-6-(2,2-difluoroéthoxy)-2,4-dioxo-3,4-dihydroquinazolin-1(2H)-yl]pipéridine-1-carbaldéhyde
- composé n°34 : 4-[3-(4-chlorobenzyl)-6-(2,2-difluoroéthoxy)-2,4-dioxo-3,4-dihydroquinazolin-1(2H)-yl]pipéridine-1-carbaldéhyde
- composé n°35 : 4-{[6-(2,2-difluoroéthoxy)-1-(1-formylpipéridin-4-yl)-2,4-dioxo-1,4-dihydroquinazolin-3(2H)-yl]méthyl}benzoate de méthyle
- composé n°36 : acide 4-{[6-(2,2-difluoroéthoxy)-1-(1-formylpipéridin-4-yl)-2,4-dioxo-1,4-dihydroquinazolin-3(2H)-yl]méthyl}benzoïque
- composé n°37 : 4-{[6-(2,2-difluoroéthoxy)-1-(1-formylpipéridin-4-yl)-2,4-dioxo1,4-dihydroquinazolin-3(2H)-yl]méthyl}-N-(2-méthoxyéthyl)benzamide
- composé n°38 : 4-[3-(3,4-diméthoxybenzyl)-6-méthyl-2,4-dioxo-3,4-dihydroquinazolin-1(2H)-yl]pipéridine-1-carbaldéhyde
- composé n°39 : 4-[6-(2,2-difluoroéthoxy)-3-(3-hydroxy-4-méthoxybenzyl)-2,4-dioxo-3,4-dihydroquinazolin-1 (2H)-yl]pipéridine-1-carbaldéhyde
- composé n°40 : 4-[6-(2,2-difluoroéthoxy)-3-[3-(2-hydroxyéthoxy)-4-méthoxybenzyl]-2,4-dioxo-3,4-dihydroquinazolin-1(2H)-yl]pipéridine-1-carbaldéhyde
- composé n°41 : 4-[6-(2,2-difluoroéthoxy)-3-(3-éthoxy-4-méthoxybenzyl)-2,4-dioxo-3,4-dihydroquinazolin-1(2H)-yl]pipéridine-1-carbaldéhyde
- composé n°42 : 4-[6-(2,2-difluoroéthoxy)-3-[4-méthoxy-3-(2-méthoxyéthoxy)benzyl]-2,4-dioxo-3,4-dihydroquinazolin-1(2H)-yl]pipéridine-1-carbaldéhyde
- composé n°43 : 4-[6-(2,2-difluoroéthoxy)-3-(3,4-diméthoxybenzyl)-2,4-dioxo-3,4-dihydroquinazolin-1(2H)-yl]azépane-1-carbaldéhyde
- composé n°47 : 4-[6-(2,2-difluoroéthoxy)-3-[3-(3-hydroxypropoxy)-4-méthoxybenzyl]-2,4-dioxo-3,4-dihydroquinazolin-1(2H)-yl]pipéridine-1-carbaldéhyde
- composé n°48 : 4-[5-chloro-3-(3,4-diméthoxybenzyl)-2,4-dioxo-3,4-dihydroquinazolin-1(2H)-yl]pipéridine-1-carbaldéhyde
- composé n°49 : 4-{3-[3-(cyclopentyloxy)-4-méthoxybenzyl]-6-(2,2-difluoroéthoxy)-2,4-dioxo-3,4-dihydroquinazolin-1(2H)-yl}pipéridine-1-carbaldéhyde
- composé n°50 : 2-(5-{[6-(2,2-difluoroéthoxy)-1-(1-formylpipéridin-4-yl)-2,4-dioxo-1,4-dihydroquinazolin-3(2H)-yl]méthyl}-2-méthoxyphénoxy)acétamide
- composé n°51 : 4-[6-(2,2-difluoroéthoxy)-3-(3,4-diméthoxybenzyl)-2,4-dioxo-3,4-dihydroquinazolin-1(2H)-yl]-3-méthylpipéridine-1-carbaldéhyde
- composé n°52 : 3-[6-(2,2-difluoroéthoxy)-3-(3,4-diméthoxybenzyl)-2,4-dioxo-3,4-dihydroquinazolin-1(2H)-yl]-8-azabicyclo[3.2.1]octane-8-carbaldéhyde
- composé n°56 : 4-{3-[4-(cyclopentyloxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
- composé n°57 : 4-[3-(3-chlorobenzyl)-6-[2-fluoro-1-(fluorométhyl)éthoxy]-2,4-dioxo-3,4-dihydroquinazolin-1(2H)-yl]pipéridine-1-carbaldéhyde
- composé n°58: 4-[3-(4-chlorobenzyl)-6-[2-fluoro-1-(fluorométhyl)éthoxy]-2,4-dioxo-3,4-dihydroquinazolin-1(2H)-yl]pipéridine-1-carbaldéhyde
- composé n°59 : 4-{3-[3-(cyclopentyloxy)-4-méthoxybenzyl]-6-[2-fluoro-1-(fluorométhyl)éthoxy]-2,4-dioxo-3,4-dihydroquinazolin-1(2H)-yl}pipéridine-1-carbaldéhyde
- composé n°72 : 4-[3-(3,4-diméthoxybenzyl)-6-(2-hydroxyéthoxy)-2,4-dioxo-3,4-dihydroquinazolin-1(2H)-yl]pipéridine-1-carbaldéhyde
- composé n°74 : 4-[3-(3,4-dichlorobenzyl)-6-[2-fluoro-1-(fluorométhyl)éthoxy]-2,4-dioxo-3,4-dihydroquinazolin-1(2H)-yl]pipéridine-1-carbaldéhyde
- composé n°76 : 4-{3-[(6-chloropyridin-3-yl)méthyl]-6-[2-fluoro-1-(fluorométhyl)éthoxy]-2,4-dioxo-3,4-dihydroquinazolin-1(2H)-yl}pipéridine-1-carbaldéhyde
- composé n°78 : 4-[3-(3-chloro-4-méthoxybenzyl)-6-[2-fluoro-1-(fluorométhyl)éthoxy]-2,4-dioxo-3,4-dihydroquinazolin-1(2H)-yl]pipéridine-1-carbaldéhyde
- composé n°79 : 4-[3-(3,4-diméthoxybenzyl)-6-(2-fluoroéthoxy)-2,4-dioxo-3,4-dihydroquinazolin-1 (2H)-yl]pipéridine-1-carbaldéhyde
- composé n°89 : 2-[5-({6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]acétamide
- composé n°90 : 4-{6-[2-fluoro-1-(fluorométhyl)éthoxy]-3-(3-hydroxy-4-méthoxybenzyl)-2,4-dioxo-3,4-dihydroquinazolin-1(2H)-yl}pipéridine-1-carbaldéhyde
- composé n°91 : 4-[3-(3,4-diméthoxybenzyl)-6-éthoxy-2,4-dioxo-3,4-dihydroquinazolin-1(2H)-yl]pipéridine-1-carbaldéhyde
- composé n°97 : 4-[5,7-dichloro-3-(3,4-diméthoxybenzyl)-2,4-dioxo-3,4-dihydroquinazolin-1(2H)-yl]pipéridine-1-carbaldéhyde
- composé n°102: 4-[7-chloro-3-(3,4-diméthoxybenzyl)-2,4-dioxo-3,4-dihydroquinazolin-1(2H)-yl]pipéridine-1-carbaldéhyde
- composé n°108 : 4-{6-[2-fluoro-1-(fluorométhyl)éthoxy]-3-(3-fluoro-4-méthoxybenzyl)-2,4-dioxo-3,4-dihydroquinazolin-1(2H)-yl}pipéridine-1-carbaldéhyde
- composé n°111 : 4-[6-(difluorométhoxy)-3-(3,4-diméthoxybenzyl)-2,4-dioxo-3,4-dihydroquinazolin-1(2H)-yl]pipéridine-1-carbaldéhyde
- composé n°112 : 4-[3-(3,4-diméthoxybenzyl)-6-(1-méthyléthoxy)-2,4-dioxo-3,4-dihydroquinazolin-1(2H)-yl]pipéridine-1-carbaldéhyde
- composé n°114 : 4-{6-[2-fluoro-1-(fluorométhyl)éthoxy]-3-[4-méthoxy-3-(1-méthyléthoxy)benzyl]-2,4-dioxo-3,4-dihydroquinazolin-1(2H)-yl}pipéridine-1-carbaldéhyde
- composé n°116 : 4-{6-[2-fluoro-1-(fluorométhyl)éthoxy]-3-(3-méthoxybenzyl)-2,4-dioxo-3,4-dihydroquinazolin-1(2H)-yl}pipéridine-1-carbaldéhyde
- composé n°117 : 4-{3-[3,5-bis(trifluorométhyl)benzyl]-6-[2-fluoro-1-(fluorométhyl)éthoxy]-2,4-dioxo-3,4-dihydroquinazolin-1(2H)-yl}pipéridine-1-carbaldéhyde
- composé n°118 : 4-[3-(3-éthoxybenzyl)-6-[2-fluoro-1-(fluorométhyl)éthoxy]-2,4-dioxo-3,4-dihydroquinazolin-1(2H)-yl]pipéridine-1-carbaldéhyde
- composé n°124 : 4-{3-[3-chloro-4-(2-méthoxyéthoxy)benzyl]-6-[2-fluoro-1-(fluorométhyl)éthoxy]-2,4-dioxo-3,4-dihydroquinazolin-1(2H)-yl}pipéridine-1-carbaldéhyde
n°130 : 4-[3-(3,4-diéthoxybenzyl)-6-[2-fluoro-1-(fluorométhyl)éthoxy]-2,4-dioxo-3,4-dihydroquinazolin-1(2H)-yl]pipéridine-1-carbaldéhyde
n°131 : 4-[3-(4-éthoxy-3-méthoxybenzyl)-6-[2-fluoro-1-(fluorométhyl)éthoxy]-2,4-dioxo-3,4-dihydroquinazolin-1(2H)-yl]pipéridine-1-carbaldéhyde
- composé n°133 : 4-{6-[2-fluoro-1-(fluorométhyl)éthoxy]-3-(4-méthoxy-3-méthylbenzyl)-2,4-dioxo-3,4-dihydroquinazolin-1(2H)-yl}pipéridine-1-carbaldéhyde
- composé n°134 : 4-{6-[2-fluoro-1-(fluorométhyl)éthoxy]-2,4-dioxo-3-[4-(trifluorométhyl)benzyl]-3,4-dihydroquinazolin-1(2H)-yl}pipéridine-1-carbaldéhyde
- composé n°135 : 4-{6-[2-fluoro-1-(fluorométhyl)éthoxy]-2,4-dioxo-3-[4-(trifluorométhyl)benzyl]-3,4-dihydroquinazolin-1(2H)-yl}pipéridine-1-carbaldéhyde
- composé n°143 : 4-{3-[4-(benzyloxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
- composé n°145 : 4-{6-[2-fluoro-1-(fluorométhyl)éthoxy]-3-(3-méthoxy-4-nitrobenzyl)-2,4-dioxo-3,4-dihydroquinazolin-1(2H)-yl}pipéridine-1-carbaldéhyde
- composé n°155: 4-[3-(4-éthoxybenzyl)-6-[2-fluoro-1-(fluorométhyl)éthoxy]-2,4-dioxo-3,4-dihydroquinazolin-1(2H)-yl]pipéridine-1-carbaldéhyde
- composé n°158 : 4-{6-[2-fluoro-1-(fluorométhyl)éthoxy]-3-[4-(morpholin-4-ylméthyl)benzyl]-2,4-dioxo-3,4-dihydroquinazolin-1(2H)-yl}pipéridine-1-carbaldéhyde
- composé n°160 : 4-{6-[2-fluoro-1-(fluorométhyl)éthoxy]-3-(4-morpholin-4-ylbenzyl)-2,4-dioxo-3,4-dihydroquinazolin-1(2H)-yl}pipéridine-1-carbaldéhyde
- composé n°165 : 4-[3-(biphényl-4-ylméthyl)-6-[2-fluoro-1-(fluorométhyl)éthoxy]-2,4-dioxo-3,4-dihydroquinazolin-1(2H)-yl]pipéridine-1-carbaldéhyde
- composé n°166 : 4-{6-[2-fluoro-1-(fluorométhyl)éthoxy]-3-[4-(méthylsulfanyl)benzyl]-2,4-dioxo-3,4-dihydroquinazolin-1(2H)-yl}pipéridine-1-carbaldéhyde
- composé n°167 : 4-{6-[2-fluoro-1-(fluorométhyl)éthoxy]-2,4-dioxo-3-(4-pyridin-3-ylbenzyl)-3,4-dihydroquinazolin-1(2H)-yl}pipéridine-1-carbaldéhyde
- composé n°170 : 4-{6-[2-fluoro-1-(fluorométhyl)éthoxy]-3-(3-méthoxy-4-méthylbenzyl)-2,4-dioxo-3,4-dihydroquinazolin-1(2H)-yl}pipéridine-1-carbaldéhyde
- composé n°175 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]acétamide
- composé n°178 : 4-{6-[2-fluoro-1-(fluorométhyl)éthoxy]-3-(3-méthoxy-4-propoxybenzyl)-2,4-dioxo-3,4-dihydroquinazolin-1(2H)-yl}pipéridine-1-carbaldéhyde
- composé n°183 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthylacétamide
- composé n°184 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N,N-diméthylacétamide
- composé n°185 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthoxy-N-méthylacétamide
- composé n°186 : 4-{3-[4-(cyclopentyloxy)-3-éthoxybenzyl]-6-[2-fluoro-1-(fluorométhyl)éthoxy]-2,4-dioxo-3,4-dihydroquinazolin-1(2H)-yl}pipéridine-1-carbaldéhyde
- composé n°188 : 4-{3-[4-(cyclopentyloxy)-3-(1-méthyléthoxy)benzyl]-6-[2-fluoro-1-(fluorométhyl)éthoxy]-2,4-dioxo-3,4-dihydroquinazolin-1(2H)-yl}pipéridine-1-carbaldéhyde
- composé n°189 : 4-{3-[4-(cyclopentyloxy)-3-propoxybenzyl]-6-[2-fluoro-1-(fluorométhyl)éthoxy]-2,4-dioxo-3,4-dihydroquinazolin-1(2H)-yl}pipéridine-1-carbaldéhyde
- composé n°190 : 4-{3-[4-(cyclopentyloxy)-3-hydroxybenzyl]-6-[2-fluoro-1-(fluorométhyl)éthoxy]-2,4-dioxo-3,4-dihydroquinazolin-1(2H)-yl}pipéridine-1-carbaldéhyde
- composé n°193 : 4-{3-[4-(difluorométhoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
- composé n°194 : 4-{3-[4-(difluorométhoxy)-3-éthoxybenzyl]-6-[2-fluoro-1-(fluorométhyl)éthoxy]-2,4-dioxo-3,4-dihydroquinazolin-1(2H)-yl}pipéridine-1-carbaldéhyde
- composé n°200 : 4-{6-[2-fluoro-1-(fluorométhyl)éthoxy]-2,4-dioxo-3-(4-thiophén-3-ylbenzyl)-3,4-dihydroquinazolin-1(2H)-yl}pipéridine-1-carbaldéhyde
- composé n°201 : 4-{6-[2-fluoro-1-(fluorométhyl)éthoxy]-2,4-dioxo-3-(4-pyridin-4-ylbenzyl)-3,4-dihydroquinazolin-1(2H)-yl}pipéridine-1-carbaldéhyde
- composé n°203 : 4-{6-[2-fluoro-1-(fluorométhyl)éthoxy]-3-[(1-méthyl-1H-indol-6-yl)méthyl]-2,4-dioxo-3,4-dihydroquinazolin-1(2H)-yl}pipéridine-1-carbaldéhyde
- composé n°206 : 4-{3-[4-(cyclopropylméthoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
-composé n°207 : 2-[4-({6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]-N-méthylacétamide
- composé n°212 : 4-{6-[2-fluoro-1-(fluorométhyl)éthoxy]-2,4-dioxo-3-[4-(1H-pyrazol-1-yl)benzyl]-3,4-dihydroquinazolin-1(2H)-yl}pipéridine-1-carbaldéhyde
- composé n°213 : 4-{6-[2-fluoro-1-(fluorométhyl)éthoxy]-2,4-dioxo-3-(4-pyridin-2-ylbenzyl)-3,4-dihydroquinazolin-1(2H)-yl}pipéridine-1-carbaldéhyde
- composé n°215 : 4-{6-[2-fluoro-1-(fluorométhyl)éthoxy]-2,4-dioxo-3-(4-thiophén-2-ylbenzyl)-3,4-dihydroquinazolin-1(2H)-yl}pipéridine-1-carbaldéhyde
- composé n°216 : 4-{6-[2-fluoro-1-(fluorométhyl)éthoxy]-2,4-dioxo-3-(quinoléin-7-ylméthyl)-3,4-dihydroquinazolin-1(2H)-yl}pipéridine-1-carbaldéhyde
- composé n°218 : 4-{6-[2-fluoro-1-(fluorométhyl)éthoxy]-3-[(6-méthoxynaphtalén-2-yl)méthyl]-2,4-dioxo-3,4-dihydroquinazolin-1(2H)-yl}pipéridine-1-carbaldéhyde
- composé n°223 : 4-{3-[4-(1H-benzimidazol-1-yl)benzyl]-6-[2-fluoro-1-(fluorométhyl)éthoxy]-2,4-dioxo-3,4-dihydroquinazolin-1(2H)-yl}pipéridine-1-carbaldéhyde
- composé n°224 : 4-{6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde
- composé n°226 : 4-{6-[2-fluoro-1-(fluorométhyl)éthoxy]-3-[3-méthoxy-4-(tétrahydrofuran-3-yloxy)benzyl]-2,4-dioxo-3,4-dihydroquinazolin-1(2H)-yl}pipéridine-1-carbaldéhyde
- composé n°228 : 4-[3-{4-[(1-benzylpyrrolidin-3-yl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
- composé n°230 : 4-[3-(1-benzothiophén-5-ylméthyl)-6-[2-fluoro-1-(fluorométhyl)éthoxy]-2,4-dioxo-3,4-dihydroquinazolin-1(2H)-yl]pipéridine-1-carbaldéhyde
- composé n°232 : 4-{6-[2-fluoro-1-(fluorométhyl)éthoxy]-3-[3-méthoxy-4-(1-méthyléthoxy)benzyl]-2,4-dioxo-3,4-dihydroquinazolin-1(2H)-yl}pipéridine-1-carbaldéhyde
- composé n°233 : 4-[3-(3,4-diméthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°234 : 4-[3-{4-[(1-acétylpyrrolidin-3-yl)oxy]-3-méthoxybenzyl}-6-[2-fluoro-1-(fluorométhyl)éthoxyl]-2,4-dioxo-3,4-dihydroquinazolin-1(2H)-yl]pipéridine-1-carbaldéhyde
- composé n°239 : 4-[3-{4-[(4-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
- composé n°240 : 4-[3-{4-[(4-chlorobenzyl)oxy]-3-méthoxybenzyl}-6-[2-fluoro-1-(fluorométhyl)éthoxyl-2,4-dioxo-3,4-dihydroquinazolin-1(2H)-yl]pipéridine-1-carbaldéhyde
- composé n°242 : 4-(3-{4-[(3-chlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
- composé n°243 : 4-{6-[2-fluoro-1-(fluorométhyl)éthoxy]-2,4-dioxo-3-(3-thiophén-3-ylbenzyl)-3,4-dihydroquinazolin-1(2H)-yl}pipéridine-1-carbaldéhyde
- composé n°245 : 4-[3-(4-éthoxy-3-méthoxybenzyl)-6-(2-hydroxyéthoxy)-2,4-dioxo-3,4-dihydroquinazolin-1(2H)-yl]pipéridine-1-carbaldéhyde
- composé n°246 : 4-[3-{4-[2-(2,3-dihydro-1H-indol-1-yl)-2-oxoéthoxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
- composé n°250 : 4-[3-{4-[(3,4-dichlorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
- composé n°251 : 4-{6-[2-fluoro-1-(fluorométhyl)éthoxy]-3-[3-méthoxy-4-(2-oxo-2-pipéridin-1-yléthoxy)benzyl]-2,4-dioxo-3,4-dihydroquinazolin-1(2H)-yl}pipéridine-1-carbaldéhyde
- composé n°254 : 4-{3-[3-éthoxy-4-(thiophén-2-ylméthoxy)benzyl]-6-[2-fluoro-1-(fluorométhyl)éthoxy]-2,4-dioxo-3,4-dihydroquinazolin-1(2H)-yl}pipéridine-1-carbaldéhyde
- composé n°258 : 4-[3-(3,4-diméthoxybenzyl)-6-{2-fluoro-1-(hydroxyméthyl)éthoxy]-2,4-dioxo-3,4-dihydroquinazolin-1(2H)-yl]pipéridine-1-carbaldéhyde
- composé n°263 : acide (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]propanoïque
- composé n°264 : 4-{6-[2-fluoro-1-(fluorométhyl)éthoxy]-3-[(1-méthyl-3-thiophén-2-yl-1H-pyrazol-5-yl)méthyl]-2,4-dioxo-3,4-dihydroquinazolin-1(2H)-yl}pipéridine-1-carbaldéhyde
- composé n°270 : 4-{6-[2-fluoro-1-(fluorométhyl)éthoxy]-3-[4-(5-méthyl-1,2,4-oxadiazol-3-yl)benzyl]-2,4-dioxo-3,4-dihydroquinazolin-1(2H)-yl}pipéridine-1-carbaldéhyde
- composé n°275 : 4-{6-[2-fluoro-1-(fluorométhyl)éthoxy]-2,4-dioxo-3-(4-pyrimidin-5-ylbenzyl)-3,4-dihydroquinazolin-1(2H)-yl}pipéridine-1-carbaldéhyde
- composé n°276 : 4-{6-{2-fluoro-1-(fluorométhyl)éthoxy]-3-[(1-méthyl-3-phényl-1H-pyrazol-5-yl)méthyl]-2,4-dioxo-3,4-dihydroquinazolin-1(2H)-yl}pipéridine-1-carbaldéhyde
- composé n°278 : 4-{6-[2-fluoro-1-(fluorométhyl)éthoxy]-2,4-dioxo-3-{[6-(1H-pyrazol-1-yl)pyridin-3-yl]méthyl}-3,4-dihydroquinazolin-1(2H)-yl}pipéridine-1-carbaldéhyde
- composé n°279 : 4-{6-[2-fluoro-1-(fluorométhyl)éthoxyl]-2,4-dioxo-3-[(2-thiophén-2-ylpyrimidin-5-yl)méthyl]-3,4-dihydroquinazolin-1(2H)-yl}pipéridine-1-carbaldéhyde
- composé n°280 : 4-{6-[2-fluoro-1-(fluorométhyl)éthoxy]-3-[4-(1-méthyl-1H-pyrazol-3-yl)benzyl]-2,4-dioxo-3,4-dihydroquinazolin-1(2H)-yl}pipéridine-1-carbaldéhyde
- composé n°282 : 4-{6-[2-fluoro-1-(fluorométhyl)éthoxy]-3-[4-(3-méthyl-1,2,4-oxadiazol-5-yl)benzyl]-2,4-dioxo-3,4-dihydroquinazolin-1(2H)-yl}pipéridine-1-carbaldéhyde
- composé n°283 : acide [2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]acétique
- composé n°285 : 4-{6-[2-fluoro-1-(fluorométhyl)éthoxy]-2,4-dioxo-3-(thiéno[2,3-b]pyridin-2-ylméthyl)-3,4-dihydroquinazolin-1(2H)-yl}pipéridine-1-carbaldéhyde
- composé n°286 : 4-{6-[2-fluoro-1-(fluorométhyl)éthoxy]-2,4-dioxo-3-[(6-phénylpyridin-3-yl)méthyl]-3,4-dihydroquinazolin-1(2H)-yl}pipéridine-1-carbaldéhyde
- composé n°287 : 4-{6-[2-fluoro-1-(fluorométhyl)éthoxy]-3-[(6-morpholin-4-ylpyridin-3-yl)méthyl]-2,4-dioxo-3,4-dihydroquinazolin-1(2H)-yl}pipéridine-1-carbaldéhyde
- composé n°289 : 4-{6-[2-fluoro-1-(fluorométhyl)éthoxy]-2,4-dioxo-3-[(6-thiophén-2-ylpyridin-3-yl)méthyl]-3,4-dihydroquinazolin-1(2H)-yl}pipéridine-1-carbaldéhyde
- composé n°292 : 4-{6-[2-fluoro-1-(fluorométhyl)éthoxy]-3-[(1-méthyl-5-phé nyl-1H-pyrazol-3-yl)méthyl]-2,4-dioxo-3,4-dihydroquinazolin-1(2H)-yl}pipéridine-1-carbaldéhyde
- composé n°294 : 4-({6-[2-fluoro-1-(fluorométhyl)éthoxy]-1-(1-formylpipéridin-4-yl)-2,4-dioxo-1,4-dihydroquinazolin-3(2H)-yl}méthyl)biphényl-2-carbonitrile
- composé n°295 : (2R)-2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-méthylpropanamide
- composé n°297 : 4-{7-fluoro-6-[2-fluoro-1-(fluorométhyl)éthoxy]-2,4-dioxo-3-(4-thiophén-2-ylbenzyl)-3,4-dihydroquinazolin-1(2H)-yl}pipéridine-1-carbaldéhyde
- composé n°298 : 4-{6-[2-fluoro-1-(fluorométhyl)éthoxy]-3-[3-méthoxy-4-(morpholin-4-ylméthyl)benzyl]-2,4-dioxo-3,4-dihydroquinazolin-1(2H)-yl}pipéridine-1-carbaldéhyde
- composé n°299 : 4-{6-[2-fluoro-1-(fluorométhyl)éthoxy]-3-[3-méthoxy-4-(pipéridin-1-ylméthyl)benzyl]-2,4-dioxo-3,4-dihydroquinazolin-1(2H)-yl}pipéridine-1-carbaldéhyde
- composé n°300 :4-[3-{4-[(3,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yllpipéridine-1-carbaldéhyde
- composé n°301 : 2-[2-(cyclopentyloxy)-5-({6-[2-fluoro-1-(fluorométhyl)éthoxy]-1-(1-formylpipéridin-4-yl)-2,4-dioxo-1,4-dihydroquinazolin-3(2H)-yl}méthyl)phénoxy]-N-éthylacétamide
- composé n°302 : acide (2S)-2-[2-(cyclopentyloxy)-5-({6-[2-fluoro-1-(fluorométhyl)éthoxy]-1-(1-formylpipéridin-4-yi)-2,4-dioxo-1,4-dihydroquinazolin-3(2H)-yl}méthyl)phénoxy]propanoïque
- composé n°305 : 4-{6-[2-fluoro-1-(fluorométhyl)éthoxy]-3-(3-méthoxy-4-{[(3R)-2-oxo-1-phénylpyrrolidin-3-yl]oxy}benzyl)-2.4-dioxo-3,4-dihydroquinazolin-1(2H)-yl}pipéridine-1-carbaldéhyde
- composé n°306 : 4-{3-[4-(cyclobutylméthoxy)-3-méthoxybenzyl]-6-[2-fluoro-1-(fluorométhyl)éthoxy]-2,4-dioxo-3,4-dihydroquinazolin-1(2H)-yl}pipéridine-1-carbaldéhyde
-composé n°307 : 4-{3-[4-(benzyloxy)-3-méthoxybenzyl]-7-fluoro-6-[2-f1uoro-1-(fluorométhyl)éthoxy]-2,4-dioxo-3,4-dihydroquinazolin-1(2H)-yl}pipéridine-1-carbaldéhyde
- composé n°308 : 4-{7-fluoro-6-[2-fluoro-1-(fluorométhyl)éthoxy]-3-(4-hydroxy-3-méthoxybenzyl)-2,4-dioxo-3,4-dihydroquinazolin-1(2H)-yl}pipéridine-1-carbaldéhyde
- composé n°309 : 4-{3-[4-(cyclopropylméthoxy)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde n°310:4-{7-fluoro-6-[2-fluoro-1-(fluorométhyl)éthoxy]-3-[3-méthoxy-4-(2-méthylpropoxy)benzyl]-2,4-dioxo-3,4-dihydroquinazolin-1(2H)-yl}pipéridine-1-carbaldéhyde
- composé n°311 : 4-{7-fluoro-6-[2-fluoro-1-(fluorométhyl)éthoxy]-3-[3-méthoxy-4-(1-méthyléthoxy)benzyl]-2,4-dioxo-3,4-dihydroquinazolin-1(2H)-yl}pipéridine-1-carbaldéhyde
- composé n°312 : 4-[3-(4-éthoxy-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°315 : 4-{7-fluoro-6-[2-fluoro-1-(fluorométhyl)éthoxy]-3-{[6-(3-méthoxyphényl)pyridin-3-yl]méthyl}-2,4-dioxo-3,4-dihydroquinazolin-1(2H)-yl}pipéridine-1-carbaldéhyde
- composé n°316 : 4-{7-fluoro-6-[2-fluoro-1-(fluorométhyl)éthoxyl-3-{[6-(2-fluorophényl)pyridin-3-yl]méthyl}-2,4-dioxo-3,4-dihydroquinazolin-1(2H)-yl}pipéridine-1-carbaldéhyde
- composé n°317 : 4-{7-fluoro-6-[2-fluoro-1-(fluorométhyl)éthoxy]-3-{[6-(4-fluorophényl)pyridin-3-yl]méthyl}-2.4-dioxo-3,4-dihydroquinazolin-1(2H)-yl}pipéridine-1-carbaldéhyde
- composé n°318 : 4-{7-fluoro-6-[2-fluoro-1-(fluorométhyl)éthoxy]-3-{[6-(4-méthoxyphényl)pyridin-3-yl]méthyl}-2,4-dioxo-3,4-dihydroquinazolin-1(2H)-yl}pipéridine-1-carbaldéhyde
- composé n°319 : 4-{7-fluoro-6-[2-fluoro-1-(fluorométhyl)éthoxy]-2,4-dioxo-3-[(6-thiophén-2-ylpyridin-3-yl)méthyl]-3,4-dihydroquinazolin-1(2H)-yl}pipéridine-1-carbaldéhyde
- composé n°320 : 4-{3-[3-éthoxy-4-(thiophén-2-ylméthoxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde
- composé n°321 : 4-{7-fluoro-6-[2-fluoro-1-(fluorométhyl)éthoxy]-3-[4-(1-méthyl-1H-pyrazol-3-yl)benzyl]-2,4-dioxo-3,4-dihydroquinazolin-1(2H)-yl}pipéridine-1-carbaldéhyde
- composé n°322 : 4-{7-fluoro-6-[2-fluoro-1-(fluorométhyl)éthoxy]-2,4-dioxo-3-(4-pyrimidin-5-ylbenzyl)-3,4-dihydroquinazolin-1(2H)-yl}pipéridine-1-carbaldéhyde
- composé n°323 : 4-{7-fluoro-6-[2-fluoro-1-(fluorométhyl)éthoxy]-3-[(1-méthyl-3-thiophén-2-yl-1H-pyrazol-5-yl)méthyl]-2,4-dioxo-3,4-dihydroquinazolin-1(2H)-yl}pipéridine-1-carbaldéhyde
- composé n°324 : 4-{7-fluoro-6-[2-fluoro-1-(fluorométhyl)éthoxy]-3-[3-méthoxy-4-(2-oxo-2-pipéridin-1-yléthoxy)benzyl]-2,4-dioxo-3,4-dihydroquinazolin-1(2H)-yl}pipéridine-1-carbaldéhyde
- composé n°325 : 4-[3-{4-[2-(2,3-dihydro-1H-indol-1-yl)-2-oxoéthoxyl-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°326 : 4-{7-fluoro-6-[2-fluoro-1-(fluorométhyl)éthoxy]-3-[4-(5-méthyl-1,2,4-oxadiazol-3-yl)benzyl]-2,4-dioxo-3,4-dihydroquinazolin-1(2H)-yl}pipéridine-1-carbaldéhyde
- composé n°327 : 4-[3-{4-[(3-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°328 : 4-[3-{[6-(3,5-dichlorophényl)pyridin-3-yl]méthyl}-6-[2-fluoro-1-(fluorométhyl)éthoxy]-2,4-dioxo-3,4-dihydroquinazolin-1(2H)-yl]pipéridine-1-carbaldéhyde
- composé n°329 : 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)biphényl-2-carbonitrile
- composé n°330 : 4-{7-fluoro-6-[2-fluoro-1-(fluorométhyl)éthoxy]-2,4-dioxo-3-[4-(1H-pyrazol-1-yl)benzyl]-3,4-dihydroquinazolin-1 (2H)-yl}pipéridine-1-carbaldéhyde
- composé n°331 :4-(7-fluoro-6-[2-fluoro-1-(fluorométhyl)éthoxy]-3-{[6-(3-fluorophényl)pyridin-3-yl]méthyl}-2,4-dioxo-3,4-dihydroquinazolin-1(2H)-yl)pipéridine-1-carbaldéhyde
- composé n°332 : 3-[5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)pyridin-2-yl]benzonitrile
- composé n°333 : 4-[3-(3,4-diéthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°334 : 4-[3-{4-[(4-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°335: 4-{7-fluoro-6-[2-fluoro-1-(fluorométhyl)éthoxy]-3-[4-(morpholin-4-ylméthyl)benzyl]-2,4-dioxo-3,4-dihydroquinazolin-1(2H)-yl}pipéridine-1-carbaldéhyde
- composé n°336 : 4-(7-fluoro-6-[2-fluoro-1-(fluorométhyl)éthoxy]-2,4-dioxo-3-{[6-(1H-pyrazol-1-yl)pyridin-3-yl]méthyl}-3,4-dihydroquinazolin-1(2H)-yl}pipéridine-1-carbaldéhyde
- composé n°337: 4-{7-fluoro-6-[2-fluoro-1-(fluorométhyl)éthoxy]-3-(4-morpholin-4-ylbenzyl)-2,4-dioxo-3,4-dihydroquinazolin-1(2H)-yl}pipéridine-1-carbaldéhyde
- composé n°338 : 4-{7-fluoro-6-[2-fluoro-1-(fluorométhyl)éthoxy]-3-(3-méthoxy-4-propoxybenzyl)-2,4-dioxo-3,4-dihydroquinazolin-1(2H)-yl}pipéridine-1-carbaldéhyde
- composé n°339 : 4-{3-[4-(1H-benzimidazol-1-yl)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde
- composé n°340 : 5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxybenzonitrile
- composé n°341 : 3-(3,4-diméthoxybenzyl)-6-[2-fluoro-1-(fluorométhyl)éthoxy]-1-(1-formylpipéridin-4-yl)-2,4-dioxo-1,2,3,4-tétrahydroquinazoline-7-carbonitrile
- composé n°342 : 4-[3-(4-bromobenzyl)-7-fluoro-6-[2-fluoro-1-(fluorométhyl)éthoxyl-2,4-dioxo-3,4-dihydroquinazolin-1(2H)-yl]pipéridine-1-carbaldéhyde
- composé n°343 : 4-[3-(4-[(3,4-dichlorabenzyl)oxy]-3-(2-méthoxyéthoxy)benzyl)-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°344 : 4-{3-[4-(benzyloxy)benzyl]-7-fluoro-6-[2-fluoro-1-(fluorométhyl)éthoxy]-2,4-dioxo-3,4-dihydroquinazolin-1(2H)-yl}pipéridine-1-carbaldéhyde
- composé n°345 : 4-[3-{4-[(3,4-dichlorobenzyl)oxy]-3-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°349 : 4-[3-{4-[(3,4-dichlorobenzyl)oxy]-3-(2-fluoroéthoxy)benzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°350 : 4-[3-{4-[(2-chloro-4-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°351 : 4-[3-{4-[(2,4-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°352 : 4-[3-{4-[(2-chloro-6-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°353 : 4-[3-{4-[(2,6-dichlorobenzyl)oxy]-3-méthoxybenzyl}-7-fluoro-6-[2-fluoro-1-(fluorométhyl)éthoxyl-2,4-dioxo-3,4-dihydroquinazolin- (2H)-yl]pipéridine-1-carbaldéhyde
- composé n°354 : 4-[3-{4-[(2-chlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°355 : 4-[7-fluoro-3-{4-[(2-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
- composé n°357 : 2-[(3,4-dichlorobenzyl)oxy]-5-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)benzonitrile
- composé n°358 : 4-[3-{4-[(3,4-dichlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°360 : 4-{7-fluoro-6-[2-fluoro-1-(fluorométhyl)éthoxy]-2,4-dioxo-3-[4-(2-phényléthyl)benzyl]-3,4-dihydroquinazolin-1(2H)-yl}pipéridine-1-carbaldéhyde
- composé n°362 : 4-[3-{4-[(4,5-dichloro-2-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°369 : 4-[3-(4-[(4-chlorophénoxy)méthyl]-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°371 : 4-[3-{3-chloro-4-[(4-chlorobenzyl)oxy]-5-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°373 : 4-[3-{3-chloro-4-[(2,4-dichlorobenzyl)oxy]-5-éthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°375 : 4-[7-fluoro-3-{4-[(4-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
- composé n°376 : 4-[3-{4-[(3,5-dichlorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°377 : 4-[3-(4-{[4-chloro-3-(trifluorométhyl)benzyl]oxy}-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°379 : 4-[3-{4-[(3-chlorophénoxy)méthyl]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°380 4-[3-{4-[(3,5-difluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°381 : 4-{3-[4-(benzyloxy)-3-méthoxybenzyl]-7-fluoro-6-[2-fluoro-1-(fluorométhyl)éthoxy]-2,4-dioxo-3,4-dihydroquinazolin-1 (2H)-yl}pipéridine-1-carbaldéhyde
- composé n°382 : 4-[3-{4-[(3-chloro-5-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°383 : 4-{7-fluoro-6-[2-fluoro-1-(fluorométhyl)éthoxy]-3-(3-méthoxy-4-{[4-(trifluorométhyl)benzyl]oxy}benzyl)-2,4-dioxo-3,4-dihydroquinazolin-1(2H)-yl}pipéridine-1-carbaldéhyde
- composé n°384 : 4-[3-(4-[(2,5-dichlorobenzyl)oxy]-3-méthoxybenzyl)-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°385 : 4-{[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]méthyl}benzonitrile
-composé n°386 : 3-{[4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphénoxy]méthyl}benzonitrile
- composé n°387 : 4-[3-{4-[(4-chloro-2-fluorobenzyl)oxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°388 : 4-[3-{4-[1-(3,4-dichlorophényl)éthoxy]-3-méthoxybenzyl}-7-fluoro-6-[2-fluoro-1-(fluorométhyl)éthoxy]-2,4-dioxo-3,4-dihydroquinazolin-1(2H)-yl]pipéridine-1-carbaldéhyde
- composé n°389 : 4-{7-fluoro-6-[2-fluoro-1-(fluorométhyl)éthoxy]-3-{4-[(3-hydroxybenzyl)oxy]-3-méthoxybenzyl}-2,4-dioxo-3,4-dihydroquinazolin-1(2H)-yl}pipéridine-1-carbaldéhyde
- composé n°390 : 4-[7-fluoro-3-{4-[(3-fluorobenzyl)oxy]-3-méthoxybenzyl}-6-[2-fluoro-1-(fluorométhyl)éthoxy]-2,4-dioxo-3,4-dihydroquinazolin-1(2H)-yl]pipéridine-1-carbaldéhyde
- composé n°391 : 4-[3-{4-[(3,4-difluorobenzyl)oxy]-3-méthoxybenzyl}-7-fluoro-6-[2-fluoro-1-(fluorométhyl)éthoxy]-2,4-dioxo-3.4-dihydroquinazolin-1(2H)-yl]pipéridine-1-carbaldéhyde
- composé n°392 : 4-{3-[4-(5,6-dichloro-1H-benzimidazol-1-yl)-3-méthoxybenzyl]-7-fluoro-6-[2-fluoro-1-(fluorométhyl)éthoxy]-2,4-dioxo-3,4-dihydroquinazolin-1(2H)-yl}pipéridine-1-carbaldéhyde
- composé n°393 : 3,4-dichlorobenzènesulfonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)phényle
- composé n°394 : 3,4-dichlorobenzènesulfonate de 4-({7-fluoro-6-[2-fluoro-1-(fluorométhyl)éthoxy]-1-(1-formylpipéridin-4-yl)-2,4-dioxo-1,4-dihydroquinazolin-3(2H)-yl}méthyl)-2-méthoxyphényle
- composé n°403 : 3,4-dichloro-N-[4-({7-fluoro-6-[2-fluoro-1-(fluorométhyl)éthoxy]-1-(1-formylpipéridin-4-y1)-2,4-dioxo-1,4-dihydroquinazolin-3(2H)-yl}méthyl)-2-méthoxyphényl]benzamide

12. Procédé de préparation d'un composé selon l'une quelconque des revendications 1 à 11, **caractérisé en ce que** l'on fait réagir un composé de formule 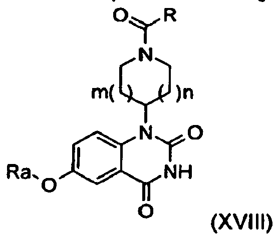 dans laquelle R, Ra, m et n sont tels que définis dans la revendication 1, et dans laquelle il peut de plus y avoir un groupement R2, tel que défini pour les composés de formule (I), en position 7 de la structure quinazoline-dione, avec un composé de formule 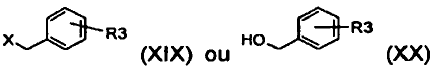 par une réaction d'alkylation ou de Mitsunobu, respectivement.

13. Composé de formule générale (XVIII) 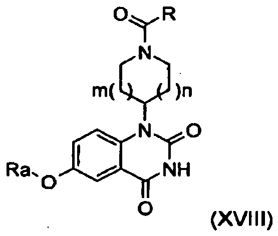 dans laquelle R, Ra, m et n sont tels que définis dans la revendication 1, et dans laquelle il peut de plus y avoir un groupement R2, tel que défini pour les composés de formule (I), en position 7 de la structure quinazoline-dione.

14. Composé de formule générale (XVIII) selon la revendication 12 choisi parmi le composé n° **32** 4-[6-(2,2-difluoroéthoxy)-2,4-dioxo-3,4-dihydroquinazolin-1(2H)-yl]pipéridine-1-carbaldéhyde et le composé n**° 55** 4-{6-[2-fluoro-1-(fluorométhyl)éthoxy]-2,4-dioxo-3.4-dihydroquinazolin-1(2H)-yl}pipéridine-1-carbaldéhyde ; à l'état de base, d'hydrate, de solvat, ou de leurs mélanges.

15. Médicament, **caractérisé en ce qu'**il comprend au moins un composé de formule (I) selon l'une quelconque des revendications 1 à 11.

16. Composition pharmaceutique, **caractérisée en ce qu'**elle comprend au moins un composé de formule (I) selon l'une quelconque des revendications 1 à 11, ainsi qu'au moins un excipient pharmaceutiquement acceptable.

17. Utilisation d'un composé de formule (I) selon l'une quelconque des revendications 1 à 11 pour la préparation d'un médicament destiné au traitement et/ou à la prévention des maladies inflammatoires ou immuno-inflammatoires incluant l'asthme, la broncho-pneumopathie chronique obstructive (COPD), la rhinite allergique, les allergies, la maladie de Crohn, la colite ulcéreuse, la myasthénie grave, la dermatite atopique, le psoriasis, le lupus érythémateux disséminé, la polyarthrite rhumatoïde, le diabète, la sclérose en plaques, dans le traitement des transplantations d'organes, dans le traitement et/ou à la prévention du cancer, en particulier de l'ostéosarcome ou de l'adénocarcinome, dans le traitement et/ou à la prévention des maladies de l'os, y compris l'ostéopénie et l'ostéoporose, dans le traitement et/ou à la prévention de la insuffisance aïgue, dans le traitement et/ou à la prévention de la douleur, la douleur neuropathique et la douleur viscérale.

18. Utilisation d'un composé de formule (I) selon la revendication 17, pour la préparation d'un médicament destiné au traitement et/ou à la prévention de la douleur neuropathique.

## Patentansprüche

1. Verbindung der allgemeinen Formel (I) 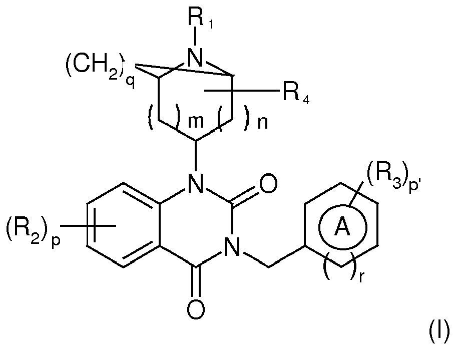 worin
- A für eine Arylgruppe oder eine Heteroarylgruppe steht;
- R₁ für:
▪ ein Wasserstoffatom,
▪ -C(0)R, worin R für ein Wasserstoffatom, eine (C₁-C₆)-Alkoxygruppe, eine Arylgruppe, eine (C₃-C₆)-Cycloalkylgruppe oder eine (C₁-C₆) -Alkylgruppe steht, wobei das Alkyl gegebenenfalls durch
. eine oder mehrere Hydroxygruppen,
. eine Benzyloxygruppe,
. eine (C₁-C₆)-Alkoxygruppe, die gegebenenfalls durch ein Aryl substituiert ist, oder
. eine (C₃-C₆)-Cycloalkylgruppe substituiert ist;
▪ eine gegebenenfalls substituierte (C₁-C₆)-Alkylgruppe
steht;
- R₂ für:
▪ ein Wasserstoffatom,
▪ ein Halogenatom,
▪ eine Cyanogruppe,
▪ eine Nitrogruppe,
▪ eine (C₁-C₆)-Alkylgruppe, die gegebenenfalls durch ein -NH₂ oder durch eine -NHC(O)Rb-Gruppe substituiert ist,
▪ eine -ORa-Gruppe, worin Ra für
. ein Wasserstoffatom,
. eine (C₁-C₆) -Alkylgruppe, die gegebenenfalls durch ein oder mehrere Halogenatome, durch eine oder mehrere Hydroxygruppen, durch eine Arylgruppe und/oder durch eine oder mehrere Cyanogruppen substituiert ist,
. eine (C₂-C₆)-Alkinylgruppe,
. eine Arylgruppe
steht, steht;
- R₃ für:
▪ ein Wasserstoffatom,
▪ ein Halogenatom,
▪ eine Hydroxygruppe,
▪ eine Cyanogruppe,
▪ eine -SCF₃-Gruppe,
▪ eine Nitrogruppe,
▪ eine Oxogruppe,
▪ eine -S(O)₀₋₂-Alkylgruppe, eine -S(O)₀₋₂-Heterocycloalkylgruppe, eine -O-SO₂-Arylgruppe, die gegebenenfalls durch ein oder mehrere Halogenatome substituiert ist,
▪ eine -Alkylaminoalkyl- oder -Cycloalkylaminoalkylgruppe, die jeweils am endständigen Alkyl gegebenenfalls substituiert ist,
▪ eine gegebenenfalls substituierte Sulfonamidgruppe,
▪ eine Arylgruppe oder eine Heteroarylgruppe, wobei die Gruppe monocyclisch oder polycyclisch ist und außerdem gegebenenfalls durch eine (C₁-C₆)-Alkylgruppe, durch ein oder mehrere Halogenatome oder durch eine (C₁-C₆)-Alkoxygruppe substituiert ist,
▪ eine Heterocycloalkylgruppe, die gegebenenfalls durch eine (C₁-C₆)-Alkylgruppe substituiert ist,
▪ eine (C₁-C₆)-Alkylgruppe, die gegebenenfalls durch
- ein oder mehrere Halogenatome,
- eine Arylgruppe, die durch ein oder mehrere Halogenatome oder eine oder mehrere Hydroxygruppen substituiert sein kann,
- eine Heteroarylgruppe,
- eine oder mehrere Hydroxygruppen, die durch eine Arylgruppe substituiert sein können, die selbst gegebenenfalls durch ein oder mehrere Halogenatome substituiert sein kann, oder
- eine Heterocycloalkylgruppe, die gegebenenfalls durch eine CO(O)Ra-Gruppe oder durch eine (C₁-C₆)-Alkylgruppe substituiert ist, substituiert ist,
▪ eine -C(O)NRbRc-Gruppe,
▪ eine -C(O)ORc-Gruppe oder eine -O-C(O)ORc-Gruppe,
▪ eine (C₁-C₆)-Alkoxygruppe, die gegebenenfalls durch
- eine Aminoalkylgruppe,
- eine Aminocycloalkylgruppe,
- eine Cycloalkylgruppe,
- eine Heterocycloalkylgruppe,
- eine monocyclische oder polycyclische Heteroarylgruppe,
- eine oder mehrere Hydroxygruppen,
- ein oder mehrere Halogenatome,
- eine (C₁-C₆)-Alkoxygruppe,
- eine -C(O)ORc-Gruppe,
- eine -C(O)NRbRc-Gruppe,
- eine Oxogruppe und/oder
- eine Arylgruppe, die selbst gegebenenfalls durch ein oder mehrere Halogenatome, eine Cyanogruppe, eine (C₁-C₆)-Alkoxygruppe, eine -O-Halogenalkylgruppe und/oder eine Halogenalkylgruppe substituiert ist,
substituiert ist,
▪ eine -O-Cycloalkylgruppe, eine -0-Arylgruppe oder eine -0-Heterocycloalkylgruppe, jeweils gegebenenfalls substituiert durch
- eine Arylgruppe, die selbst gegebenenfalls durch ein oder mehrere Halogenatome oder eine (C₁-C₆)-Alkylgruppe substituiert ist,
- eine Oxogruppe,
- ein oder mehrere Halogenatome und/oder
- eine (C₁-C₆)-Alkylgruppe, die selbst durch eine Arylgruppe und/oder eine Oxogruppe substituiert sein kann,
▪ eine -NH-CO-NH-Arylgruppe, eine -NH-CO-NH-Heteroarylgruppe oder eine -NH-CO-NH-(C₁-C₆)-Alkylgruppe, jeweils gegebenenfalls substituiert durch ein oder mehrere Halogenatome, durch eine Cyanogruppe, durch eine Nitrogruppe, durch eine oder mehrere Hydroxygruppen oder durch eine (C₁-C₆)-Alkoxygruppe,
▪ eine -N-(C₁-C₆)-Alkylgruppe, wobei die (C₁-C₆)-Alkylgruppe durch
- eine oder mehrere Oxogruppen und/oder
- eine oder mehrere Arylgruppen, gegebenenfalls substituiert durch ein oder mehrere Halogenatome und/oder durch eine SO₂-Gruppe, substituiert sein kann,
▪ eine -NH-CO-Arylgruppe, eine -NH-CO-Heteroarylgruppe, jeweils gegebenenfalls substituiert durch ein oder mehrere Halogenatome,
steht;
oder R₃ mit A eine polycyclische Heteroarylgruppe bildet, die gegebenenfalls durch eine (C₁-C₆)-Alkoxygruppe, eine (C₁-C₆)-Alkylgruppe, die gegebenenfalls durch eine Arylgruppe, die selbst durch ein oder mehrere Halogenatome substituiert ist, substituiert ist;
- R₄ für ein Wasserstoffatom, eine Oxogruppe oder eine (C₁-C₆)-Alkylgruppe steht;
- Rb für:
. ein Wasserstoffatom;
. eine (C₁-C₆)-Alkylgruppe, die gegebenenfalls durch ein oder mehrere Halogenatome, durch eine oder mehrere Hydroxy-, Cyano-, Amino-Heterocycloalkyl- oder (C₁-C₆)-Alkoxygruppen oder durch eine Arylgruppe, die gegebenenfalls durch ein oder mehrere Halogenatome substituiert ist, substituiert ist,
. eine (C₃-C₆)-Cycloalkylgruppe,
. eine (C₂-C₆)-Alkinylgruppe,
. eine (C₁-C₆)-Alkoxygruppe,
. eine Arylgruppe, die gegebenenfalls durch ein
oder mehrere Halogenatome substituiert ist, steht;
- Rc für ein Wasserstoffatom oder eine (C₁-C₆)-Alkylgruppe, die gegebenenfalls durch ein oder mehrere Halogenatome substituiert ist, steht;
oder aber Rb und Rc zusammen mit dem Stickstoffatom, an das sie gebunden sind, eine polycyclische Heteroarylgruppe oder eine Heterocycloalkylgruppe bilden;
- m und n unabhängig voneinander für den Wert 0, 1 oder 2 stehen, mit der Maßgabe, dass m+n ≤ 3;
- p und p' unabhängig voneinander für den Wert 1, 2 oder 3 stehen, mit der Maßgabe, dass dann, wenn p größer gleich 2 ist, die R₂-Gruppen an verschiedenen Kohlenstoffatomen stehen und gleich oder voneinander verschieden sein können, und dann, wenn p' größer gleich 2 ist, die R₃-Gruppen an verschiedenen Kohlenstoffatomen stehen und gleich oder voneinander verschieden sein können;
- q für den Wert 0 oder 2 steht, mit der Maßgabe, dass dann, wenn q = 0, die an den Stickstoff in 1-Position des 2,4-Dioxo-1,2,3,4-tetrahydrochinazolinkerns gebundene stickstoffhaltige heterocyclische Gruppe nicht mehr verbrückt ist und vom Typ: 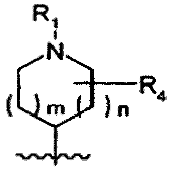 ist;
- r für den Wert 0 oder 1 steht.

2. Verbindung der allgemeinen Formel (I) nach Anspruch 1, **dadurch gekennzeichnet, dass** A für eine Phenylgruppe oder Pyridylgruppe steht.

3. Verbindung der allgemeinen Formel (I) nach Anspruch 1 oder 2, **dadurch gekennzeichnet, dass** q = 0 und m und n = 1.

4. Verbindung der allgemeinen Formel (I) nach einem der Ansprüche 1 bis 3, **dadurch gekennzeichnet, dass** R₂ für eine (C₁-C₆)-Alkylgruppe, insbesondere eine Methylgruppe, die durch eine -NH-(CO)-Rb-Gruppe substituiert ist, steht, wobei Rb die in Anspruch 1 angegebene Bedeutung besitzt.

5. Verbindung der allgemeinen Formel (I) nach einem der Ansprüche 1 bis 3, **dadurch gekennzeichnet, dass** R₂ für eine -ORa-Gruppe steht, wobei Ra die in Anspruch 1 angegebene Bedeutung besitzt.

6. Verbindung der allgemeinen Formel (I) nach einem der Ansprüche 1 bis 3, **dadurch gekennzeichnet, dass** R₂ für ein Halogenatom oder ein Cyano oder einen Wasserstoff oder ein Hydroxyl oder ein (C₁-C₆)-Alkyl, das gegebenenfalls durch ein -NH₂ oder durch eine NH-(CO)-Rb-Gruppe substituiert ist, steht.

7. Verbindung der allgemeinen Formel (I) nach Anspruch 1, **dadurch gekennzeichnet, dass** A für ein Phenyl steht, R₁ für eine -C(0)R-Gruppe, worin R für ein Wasserstoffatom steht, steht, q gleich 0 ist, n und m jeweils einen Wert von 1 haben und R₂ für -ORa steht.

8. Verbindung der allgemeinen Formel (I) nach Anspruch 1, **dadurch gekennzeichnet, dass** A für ein Phenyl steht, R₁ für eine -C(0)R-Gruppe, worin R für ein Wasserstoffatom steht, steht, q gleich 0 ist, n und m jeweils einen Wert von 1 haben und R₂ für ein Methyl, das durch eine NH-(CO)-Rb-Gruppe substituiert ist, steht, wobei Rb die in Anspruch 1 angegebene Bedeutung besitzt.

9. Verbindung der allgemeinen Formel (I) nach Anspruch 1, **dadurch gekennzeichnet, dass** A für ein Phenyl steht, R₁ für eine -C(0)R-Gruppe, worin R für ein Wasserstoffatom steht, steht, q gleich 0 ist, n und m jeweils einen Wert von 1 haben, p gleich 2 ist, einer der Reste R₂ für -ORa steht, wobei Ra die in der allgemeinen Formel (I) angegebene Bedeutung besitzt, und der andere Rest R₂ für ein Wasserstoffatom steht.

10. Verbindung der allgemeinen Formel (I) nach einem der Ansprüche 1 bis 9, **dadurch gekennzeichnet, dass** die R₂-Gruppe in 6-Position des 2,4-Dioxo-1,2,3,4-tetrahydrochinazolinkerns steht und außerdem eine gleiche oder verschiedene R₂-Gruppe in 7-Position des 2,4-Dioxo-1,2,3,4-tetrahydrochinazolinkerns vorliegen kann; in Basen-, Hydrat- oder Solvatform, in Form von Isomeren oder in Form von Mischungen davon.

11. Verbindung der allgemeinen Formel (I) nach Anspruch 1, die aus den folgenden Verbindungen ausgewählt ist:
Nr. 1: 2-{[3-(3,4-Dimethoxybenzyl)-1-(1-formylpiperidin-4-yl)-2,4-dioxo-1,2,3,4-tetrahydrochinazolin-6-yl]oxy}propannitril
Nr. 2: 1-(1-Acetylpiperidin-4-yl)-3-(3,4-dimethoxybenzyl)-6-hydroxychinazolin-2,4(1H,3H)-dion
Nr. 3: {[1-(1-Acetylpiperidin-4-yl)-3-(3,4-dimethoxybenzyl)-2,4-dioxo-1,2,3,4-tetrahydrochinazolin-6-yl]oxy}acetonitril
Nr. 4: 2-{[1-(1-Acetylpiperidin-4-yl)-3-(3,4-dimethoxybenzyl)-2,4-dioxo-1,2,3,4-tetrahydrochinazolin-6-yl]oxy}propannitril
Nr. 6: {[3-(3,4-Dimethoxybenzyl)-1-(1-formylpiperidin-4-yl)-2,4-dioxo-1,2,3,4-tetrahydrochinazolin-6-yl]oxy}acetonitril
Nr. 11: 4-[3-(3,4-Dimethoxybenzyl)-6-[2-fluor-1-(fluormethyl)ethoxy]-2,4-dioxo-3,4-dihydrochinazolin-1(2H)-yl]piperidin-1-carbaldehyd
Nr. 12: 1-(1-Acetylpiperidin-4-yl)-3-(3,4-dimethoxybenzyl)-6-[2-fluor-1-(fluormethyl)ethoxy]chinazolin-2,4(1H,3H)-dion
Nr. 13: 4-[3-(3,4-Dimethoxybenzyl)-2,4-dioxo-6-(2,2,2-trifluorethoxy)-3,4-dihydrochinazolin-1(2H)-yl]piperidin-1-carbaldehyd
Nr. 14: 1-(1-Acetylpiperidin-4-yl)-6-(2,2-difluorethoxy)-3-(3,4-dimethoxybenzyl)chinazolin-2,4(1H, 3H)-dion
Nr. 16: 4-[6-(2,2-Difluorethoxy)-3-(3,4-dimethoxybenzyl)-2,4-dioxo-3,4-dihydrochinazolin-1(2H)-yl]piperidin-1-carbaldehyd
Nr. 20: N-{[3-(3,4-Dimethoxybenzyl)-1-(1-formylpiperidin-4-yl)-2,4-dioxo-1,2,3,4-tetrahydrochinazolin-6-yl]methyl}acetamid
Nr. 22: 1-(1-Acetylpiperidin-4-yl)-6-(aminomethyl)-3-(3,4-dimethoxybenzyl)chinazolin-2,4(1H,3H)-dion-hydrochlorid
Nr. 23: N-{[3-(3,4-Dimethoxybenzyl)-1-(1-formylpiperidin-4-yl)-2,4-dioxo-1,2,3,4-tetrahydrochinazolin-6-yl]methyl}formamid
Nr. 24: N-{[1-(1-Acetylpiperidin-4-yl)-3-(3,4-dimethoxybenzyl)-2,4-dioxo-1,2,3,4-tetrahydrochinazolin-6-yl]methyl}formamid
Nr. 25: N-{[1-(1-Acetylpiperidin-4-yl)-3-(3,4-dimethoxybenzyl)-2,4-dioxo-1,2,3,4-tetrahydrochinazolin-6-yl]methyl}acetamid
Nr. 32: 4-[6-(2,2-Difluorethoxy)-2,4-dioxo-3,4-dihydrochinazolin-1(2H)-yl]piperidin-1-carbaldehyd
Nr. 33: 4-[3-(3,4-Dichlorbenzyl)-6-(2,2-difluorethoxy)-2,4-dioxo-3,4-dihydrochinazolin-1(2H)-yl]piperidin-1-carbaldehyd
Nr. 34: 4-[3-(4-Chlorbenzyl)-6-(2,2-difluorethoxy)-2,4-dioxo-3,4-dihydrochinazolin-1(2H)-yl]piperidin-1-carbaldehyd
Nr. 35: 4-{[6-(2,2-Difluorethoxy)-1-(1-formylpiperidin-4-yl)-2,4-dioxo-1,4-dihydrochinazolin-3(2H)-yl]methyl}benzoesäuremethylester
Nr. 36: 4-{[6-(2,2-Difluorethoxy)-1-(1-formylpiperidin-4-yl)-2,4-dioxo-1,4-dihydrochinazolin-3(2H)-yl]methyl}benzoesäure
Nr. 37: 4-{[6-(2,2-Difluorethoxy)-1-(1-formylpiperidin-4-yl)-2,4-dioxo-1,4-dihydrochinazolin-3(2H)-yl]methyl}-N-(2-methoxyethyl)benzamid
Nr. 38: 4-[3-(3,4-Dimethoxybenzyl)-6-methyl-2,4-dioxo-3,4-dihydrochinazolin-1(2H)-yl]piperidin-1-carbaldehyd
Nr. 39: 4-[6-(2,2-Difluorethoxy)-3-(3-hydroxy-4-methoxybenzyl)-2,4-dioxo-3,4-dihydrochinazolin-1(2H)-yl]piperidin-1-carbaldehyd
Nr. 40: 4-[6-(2,2-Difluorethoxy)-3-[3-(2-hydroxyethoxy)-4-methoxybenzyl]-2,4-dioxo-3,4-dihydrochinazolin-1(2H)-yl]piperidin-1-carbaldehyd
Nr. 41: 4-[6-(2,2-Difluorethoxy)-3-(3-ethoxy-4-methoxybenzyl)-2,4-dioxo-3,4-dihydrochinazolin-1(2H)-yl]piperidin-1-carbaldehyd
Nr. 42: 4-[6-(2,2-Difluorethoxy)-3-[4-methoxy-3-(2-methoxyethoxy)benzyl]-2,4-dioxo-3,4-dihydrochinazolin-1(2H)-yl]piperidin-1-carbaldehyd
Nr. 43: 4-[6-(2,2-Difluorethoxy)-3-(3,4-dimethoxybenzyl)-2,4-dioxo-3,4-dihydrochinazolin-1(2H)-yl]azepan-1-carbaldehyd
Nr. 47: 4-[6-(2,2-Difluorethoxy)-3-[3-(3-hydroxypropoxy)-4-methoxybenzyl]-2,4-dioxo-3,4-dihydrochinazolin-1(2H)-yl]piperidin-1-carbaldehyd
Nr. 48: 4-[5-Chlor-3-(3,4-dimethoxybenzyl)-2,4-dioxo-3,4-dihydrochinazolin-1(2H)-yl]piperidin-1-carbaldehyd
Nr. 49: 4-{3-[3-(Cyclopentyloxy)-4-methoxybenzyl]-6-(2,2-difluorethoxy)-2,4-dioxo-3,4-dihydrochinazolin-1(2H)-yl}-piperidin-1-carbaldehyd
Nr. 50: 2-(5-{[6-(2,2-Difluorethoxy)-1-(1-formylpiperidin-4-yl)-2,4-dioxo-1,4-dihydrochinazolin-3(2H)-yl]-methyl}-2-methoxyphenoxy)-acetamid
Nr. 51: 4-[6-(2,2-Difluorethoxy)-3-(3,4-dimethoxybenzyl)-2,4-dioxo-3,4-dihydrochinazolin-1(2H)-yl]-3-methylpiperidin-1-carbaldehyd
Nr. 52: 3-[6-(2,2-Difluorethoxy)3-(3,4-dimethoxybenzyl)-2,4-dioxo-3,4-dihydrochinazolin-1(2H)-yl]-8-azabicyclo[3.2.1]octan-8-carbaldehyd
Nr. 56: 4-{3-[4-(Cyclopentyloxy)-3-methoxybenzyl]-6-[2-fluor-1-(fluormethyl)ethoxy]-2,4-dioxo-3,4-dihydrochinazolin-1(2H)-yl}piperidin-1-carbaldehyd
Nr. 57: 4-[3-(3-Chlorbenzyl)-6-[2-fluor-1-(fluormethyl)ethoxy]-2,4-dioxo-3,4-dihydrochinazolin-1(2H)-yl]piperidin-1-carbaldehyd
Nr. 58: 4-[3-(4-Chlorbenzyl)-6-[2-fluor-1-(fluormethyl)ethoxy]-2,4-dioxo-3,4-dihydrochinazolin-1(2H)-yl]piperidin-1-carbaldehyd
Nr. 59: 4-{3-[3-(Cyclopentyloxy)-4-methoxybenzyl]-6-[2-fluor-1-(fluormethyl)ethoxy]-2,4-dioxo-3,4-dihydrochinazolin-1(2H)-yl}piperidin-1-carbaldehyd
Nr. 72: 4-[3-(3,4-Dimethoxybenzyl)-6-(2-hydroxyethoxy)-2,4-dioxo-3,4-dihydrochinazolin-1(2H)-yl]piperidin-1-carbaldehyd
Nr. 74: 4-[3-(3,4-Dichlorbenzyl)-6-[2-fluor-1-(fluormethyl)ethoxy]-2,4-dioxo-3,4-dihydrochinazolin-1(2H)-yl]piperidin-1-carbaldehyd
Nr. 76: 4-{3-[(6-Chlorpyridin-3-yl)methyl]-6-[2-fluor-1-(fluormethyl)ethoxy]-2,4-dioxo-3,4-dihydrochinazolin-1(2H)-yl}piperidin-1-carbaldehyd
Nr. 78: 4-[3-(3-Chlor-4-methoxybenzyl)-6-[2-fluor-1-(fluormethyl)-ethoxy]-2,4-dioxo-3,4-dihydrochinazolin-1(2H)-yl]piperidin-1-carbaldehyd
Nr. 79: 4-[3-(3,4-Dimethoxybenzyl)-6-(2-fluorethoxy)-2,4-dioxo-3,4-dihydrochinazolin-1(2H)-yl]piperidin-1-carbaldehyd
Nr. 89: 2-[5-({6-[2-Fluor-1-(fluormethyl)ethoxy]-1-(1-formylpiperidin-4-yl)-2,4-dioxo-1,4-dihydrochinazolin-3(2H)-yl}methyl)-2-methoxyphenoxy]acetamid
Nr. 90: 4-{6-[2-Fluor-1-(fluormethyl)ethoxy]-3-(3-hydroxy-4-methoxybenzyl)-2,4-dioxo-3,4-dihydrochinazolin-1(2H)-yl}piperidin-1-carbaldehyd
Nr. 91: 4-[3-(3,4-Dimethoxybenzyl)-6-ethoxy-2,4-dioxo-3,4-dihydrochinazolin-1(2H)-yl]piperidin-1-carbaldehyd
Nr. 97: 4-[5,7-Dichlor-3-(3,4-dimethoxybenzyl)-2,4-dioxo-3,4-dihydrochinazolin-1(2H)-yl]piperidin-1-carbaldehyd
Nr. 102: 4-[7-Chlor-3-(3,4-dimethoxybenzyl)-2,4-dioxo-3,4-dihydrochinazolin-1(2H)-yl]piperidin-1-carbaldehyd
Nr. 108: 4-{6-[2-Fluor-1-(fluormethyl)-ethoxy]-3-(3-fluor-4-methoxybenzyl)-2,4-dioxo-3,4-dihydrochinazolin-1(2H)-yl}piperidin-1-carbaldehyd
Nr. 111: 4-[6-(Difluormethoxy)-3-(3,4-dimethoxybenzyl)-2,4-dioxo-3,4-dihydrochinazolin-1(2H)-yl]piperidin-1-carbaldehyd
Nr. 112: 4-[3-(3,4-Dimethoxybenzyl)-6-(1-methylethoxy)-2,4-dioxo-3,4-dihydrochinazolin-1(2H)-yl]piperidin-1-carbaldehyd
Nr. 114: 4-{6-[2-Fluor-1-(fluormethyl)ethoxy]-3-[4-methoxy-3-(1-methylethoxy)benzyl]-2,4-dioxo-3,4-dihydrochinazolin-1(2H)-yl}piperidin-1-carbaldehyd
Nr. 116: 4-{6-[2-Fluor-1-(fluormethyl)-ethoxy]-3-(3-methoxybenzyl)-2,4-dioxo-3,4-dihydrochinazolin-1(2H)-yl}piperidin-1-carbaldehyd
Nr. 117: 4-{3-[3,5-Bis(trifluormethyl)benzyl]-6-[2-fluor-1-(fluormethyl)ethoxy]-2,4-dioxo-3,4-dihydrochinazolin-1(2H)-yl}piperidin-1-carbaldehyd
Nr. 118: 4-[3-(3-Ethoxybenzyl)-6-[2-fluor-1-(fluormethyl)ethoxy]-2,4-dioxo-3,4-dihydrochinazolin-1(2H)-yl]piperidin-1-carbaldehyd
Nr. 124: 4-{3-[3-Chlor-4-(2-methoxyethoxy)benzyl]-6-[2-fluor-1-(fluormethyl)ethoxy]-2,4-dioxo-3,4-dihydrochinazolin-1(2H)-yl}piperidin-1-carbaldehyd
Nr. 130: 4-[3-(3,4-Diethoxybenzyl)-6-[2-fluor-1-(fluormethyl)ethoxy]-2,4-dioxo-3,4-dihydrochinazolin-1(2H)-yl]piperidin-1-carbaldehyd
Nr. 131: 4-[3-(4-Ethoxy-3-methoxybenzyl)-6-[2-fluor-1-(fluormethyl)ethoxy]-2,4-dioxo-3,4-dihydrochinazolin-1(2H)-yl]piperidin-1-carbaldehyd
Nr. 133: 4-{6-[2-Fluor-1-(fluormethyl)ethoxy]-3-(4-methoxy-3-methylbenzyl)-2,4-dioxo-3,4-dihydrochinazolin-1(2H)-yl}piperidin-1-carbaldehyd
Nr. 134: 4-{6-[2-Fluor-1-(fluormethyl)ethoxy]-2,4-dioxo-3-[4-(trifluormethyl)benzyl]-3,4-dihydrochinazolin-1(2H)-yl}piperidin-1-carbaldehyd
Nr. 135: 4-{6-[2-Fluor-1-(fluormethyl)ethoxy]-2,4-dioxo-3-[4-(trifluormethyl)benzyl]-3,4-dihydrochinazolin-1(2H)-yl}piperidin-1-carbaldehyd
Nr. 143: 4-{3-[4-(Benzyloxy)-3-methoxybenzyl]-6-[2-fluor-1-(fluormethyl)ethoxy]-2,4-dioxo-3,4-dihydrochinazolin-1(2H)-yl}piperidin-1-carbaldehyd
Nr. 145: 4-{6-[2-Fluor-1-(fluormethyl)ethoxy]-3-(3-methoxy-4-nitrobenzyl)-2,4-dioxo-3,4-dihydrochinazolin-1(2H)-yl}piperidin-1-carbaldehyd
Nr. 155: 4-[3-(4-Ethoxybenzyl)-6-[2-fluor-1-(fluormethyl)ethoxy]-2,4-dioxo-3,4-dihydrochinazolin-1(2H)-yl]piperidin-1-carbaldehyd
Nr. 158: 4-{6-[2-Fluor-1-(fluormethyl)ethoxy]-3-[4-(morpholin-4-ylmethyl)benzyl]-2,4-dioxo-3,4-dihydrochinazolin-1(2H)-yl}piperidin-1-carbaldehyd
Nr. 160: 4-{6-[2-Fluor-1-(fluormethyl)ethoxy]-3-(4-(morpholin-4-yl)benzyl)-2,4-dioxo-3,4-dihydrochinazolin-1(2H)-yl}piperidin-1-carbaldehyd
Nr. 165: 4-[3-(Biphenyl-4-ylmethyl)-6-[2-fluor-1-(fluormethyl)ethoxy]-2,4-dioxo-3,4-dihydrochinazolin-1(2H)-yl]piperidin-1-carbaldehyd
Nr. 166: 4-{6-[2-Fluor-1-(fluormethyl)ethoxy]-3-[4-(methylsulfanyl)benzyl]-2,4-dioxo-3,4-dihydrochinazolin-1(2H)-yl}piperidin-1-carbaldehyd
Nr. 167: 4-{6-[2-Fluor-1-(fluormethyl)ethoxy]-2,4-dioxo-3-(4-(pyridin-3-yl)benzyl)-3,4-dihydrochinazolin-1(2H)-yl}piperidin-1-carbaldehyd
Nr. 170: 4-{6-[2-Fluor-1-(fluormethyl)ethoxy]-3-(3-methoxy-4-methylbenzyl)-2,4-dioxo-3,4-dihydrochinazolin-1(2H)-yl}piperidin-1-carbaldehyd
Nr. 175: 2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]acetamid
Nr. 178: 4-{6-[2-Fluor-1-(fluormethyl)ethoxy]-3-(3-methoxy-4-propoxybenzyl)-2,4-dioxo-3,4-dihydrochinazolin-1(2H)-yl}piperidin-1-carbaldehyd
Nr. 183: 2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]-N-methylacetamid
Nr. 184: 2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]-N,N-dimethylacetamid
Nr. 185: 2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]-N-methoxy-N-methylacetamid
Nr. 186: 4-{3-[4-(Cyclopentyloxy)-3-ethoxybenzyl]-6-[2-fluor-1-(fluormethyl)ethoxy]-2,4-dioxo-3,4-dihydrochinazolin-1(2H)-yl}piperidin-1-carbaldehyd
Nr. 188: 4-{3-[4-(Cyclopentyloxy)-3-(1-methylethoxy)benzyl]-6-[2-fluor-1-(fluormethyl)ethoxy]-2,4-dioxo-3,4-dihydrochinazolin-1(2H)-yl}piperidin-1-carbaldehyd
Nr. 189: 4-{3-[4-(Cyclopentyloxy)-3-propoxybenzyl]-6-[2-fluor-1-(fluormethyl)ethoxy]-2,4-dioxo-3,4-dihydrochinazolin-1(2H)-yl}piperidin-1-carbaldehyd
Nr. 190: 4-{3-[4-(Cyclopentyloxy)-3-hydroxybenzyl]-6-[2-fluor-1-(fluormethyl)ethoxy]-2,4-dioxo-3,4-dihydrochinazolin-1(2H)-yl}piperidin-1-carbaldehyd
Nr. 193: 4-{3-[4-(Difluormethoxy)-3-methoxybenzyl]-6-[2-fluor-1-(fluormethyl)ethoxy]-2,4-dioxo-3,4-dihydrochinazolin-1(2H)-yl}piperidin-1-carbaldehyd
Nr. 194: 4-{3-[4-(Difluormethoxy)-3-ethoxybenzyl]-6-[2-fluor-1-(fluormethyl)ethoxy]-2,4-dioxo-3,4-dihydrochinazolin-1(2H)-yl}piperidin-1-carbaldehyd
Nr. 200: 4-{6-[2-Fluor-1-(fluormethyl)ethoxy]-2,4-dioxo-3-(4-(thiophen-3-ylbenzyl)-3,4-dihydrochinazolin-1(2H)-yl}piperidin-1-carbaldehyd
Nr. 201: 4-{6-[2-Fluor-1-(fluormethyl)ethoxy]-2,4-dioxo-3-(4-(pyridin-4-ylbenzyl)-3,4-dihydrochinazolin-1(2H)-yl}piperidin-1-carbaldehyd
Nr. 203: 4-{6-[2-Fluor-1-(fluormethyl)ethoxy]-3-[(1-methyl-1H-indol-6-yl)methyl]-2,4-dioxo-3,4-dihydrochinazolin-1(2H)-yl}piperidin-1-carbaldehyd
Nr. 206: 4-{3-[4-(Cyclopropylmethoxy)-3-methoxybenzyl]-6-[2-fluor-1-(fluormethyl)ethoxy]-2,4-dioxo-3,4-dihydrochinazolin-1(2H)-yl}piperidin-1-carbaldehyd
Nr. 207: 2-[4-({6-[2-Fluor-1-(fluormethyl)ethoxy]-1-(1-formylpiperidin-4-yl)-2,4-dioxo-1,4-dihydrochinazolin-3(2H)-yl}methyl)-2-methoxyphenoxy]-N-methylacetamid
Nr. 212: 4-{6-[2-Fluor-1-(fluormethyl)ethoxy]-2,4-dioxo-3-[4-(1H-pyrazol-1-yl)benzyl]-3,4-dihydrochinazolin-1(2H)-yl}piperidin-1-carbaldehyd
Nr. 213: 4-{6-[2-Fluor-1-(fluormethyl)ethoxy]-2,4-dioxo-3-(4-(pyridin-2-ylbenzyl)-3,4-dihydrochinazolin-1(2H)-yl}piperidin-1-carbaldehyd
Nr. 215: 4-{6-[2-Fluor-1-(fluormethyl)ethoxy]-2,4-dioxo-3-(4-(thiophen-2-ylbenzyl)-3,4-dihydrochinazolin-1(2H)-yl}piperidin-1-carbaldehyd
Nr. 216: 4-{6-[2-Fluor-1-(fluormethyl)ethoxy]-2,4-dioxo-3-(chinolin-7-ylmethyl)-3,4-dihydrochinazolin-1(2H)-yl}piperidin-1-carbaldehyd
Nr. 218: 4-{6-[2-Fluor-1-(fluormethyl)ethoxy]-3-[(6-methoxynaphthalin-2-yl)methyl]-2,4-dioxo-3,4-dihydrochinazolin-1(2H)-yl}piperidin-1-carbaldehyd
Nr. 223: 4-{3-[4-(1H-Benzimidazol-1-yl)benzyl]-6-[2-fluor-1-(fluormethyl)ethoxy]-2,4-dioxo-3,4-dihydrochinazolin-1(2H)-yl}piperidin-1-carbaldehyd
Nr. 224: 4-{6-[2-Fluor-1-(fluormethyl)ethoxy]-3-[3-methoxy-4-(2-methylpropoxy)benzyl]-2,4-dioxo-3,4-dihydrochinazolin-1(2H)-yl}piperidin-1-carbaldehyd
Nr. 226: 4-{6-[2-Fluor-1-(fluormethyl)ethoxy]-3-[3-methoxy-4-(tetrahydrofuran-3-yloxy)benzyl]-2,4-dioxo-3,4-dihydrochinazolin-1(2H)-yl}piperidin-1-carbaldehyd
Nr. 228: 4-[3-{4-[(1-Benzylpyrrolidin-3-yl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 230: 4-[3-(1-Benzothiophen-5-ylmethyl)-6-[2-fluor-1-(fluormethyl)ethoxy]-2,4-dioxo-3,4-dihydrochinazolin-1(2H)-yl]piperidin-1-carbaldehyd
Nr. 232: 4-{6-[2-Fluor-1-(fluormethyl)ethoxy]-3-[3-methoxy-4-(1-methylethoxy)benzyl]-2,4-dioxo-3,4-dihydrochinazolin-1(2H)-yl}piperidin-1-carbaldehyd
Nr. 233: 4-[3-(3,4-Dimethoxybenzyl)-7-fluor-6-[2-fluor-1-(fluormethyl)ethoxy]-2,4-dioxo-3,4-dihydrochinazolin-1(2H)-yl]piperidin-1-carbaldehyd
Nr. 234: 4-[3-{4-[(1-Acetylpyrrolidin-3-yl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 239: 4-[3-{4-[(4-Fluorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 240: 4-[3-{4-[(4-Chlorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 242: 4-[3-{4-[(3-Chlorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 243: 4-{6-[2-Fluor-1-(fluormethyl)ethoxy]-2,4-dioxo-3-(3-(thiophen-3-yl)benzyl)-3,4-dihydrochinazolin-1(2H)-yl}piperidin-1-carbaldehyd
Nr. 245: 4-[3-(4-Ethoxy-3-methoxybenzyl)-6-(2-hydroxyethoxy)-2,4-dioxo-3,4-dihydrochinazolin-1(2H)-yl]piperidin-1-carbaldehyd
Nr. 246: 4-[3-{4-[2-(2,3-Dihydro-1H-indol-1-yl)-2-oxoethoxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 250: 4-[3-{4-[(3,4-Dichlorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 251: 4-{6-[2-Fluor-1-(fluormethyl)ethoxy]-3-[3-methoxy-4-(2-oxo-2-(piperidin-1-yl)ethoxy)benzyl]-2,4-dioxo-3,4-dihydrochinazolin-1(2H)-yl}piperidin-1-carbaldehyd
Nr. 254: 4-{3-[3-Ethoxy-4-(thiophen-2-ylmethoxy)benzyl]-6-[2-fluor-1-(fluormethyl)ethoxy]-2,4-dioxo-3,4-dihydrochinazolin-1(2H)-yl}piperidin-1-carbaldehyd
Nr. 258: 4-[3-(3,4-Dimethoxybenzyl)-6-[2-fluor-1-(hydroxymethyl)ethoxy]-2,4-dioxo-3,4-dihydrochinazolin-1(2H)-yl]piperidin-1-carbaldehyd
Nr. 263: (2R)-2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]propansäure
Nr. 264: 4-{6-[2-Fluor-1-(fluormethyl)ethoxy]-3-[(1-methyl-3-(thiophen-2-yl)-1H-pyrazol-5-yl)methyl]-2,4-dioxo-3,4-dihydrochinazolin-1(2H)-yl}piperidin-1-carbaldehyd
Nr. 270: 4-{6-[2-Fluor-1-(fluormethyl)ethoxy]-3-[4-(5-methyl-1,2,4-oxadiazol-3-yl)benzyl]-2,4-dioxo-3,4-dihydrochinazolin-1(2H)-yl}piperidin-1-carbaldehyd
Nr. 275: 4-{6-[2-Fluor-1-(fluormethyl)ethoxy]-2,4-dioxo-3-(4-(pyrimidin-5-yl)benzyl)-3,4-dihydrochinazolin-1(2H)-yl}piperidin-1-carbaldehyd
Nr. 276: 4-{6-[2-Fluor-1-(fluormethyl)ethoxy]-3-[(1-methyl-3-phenyl-1H-pyrazol-5-yl)methyl]-2,4-dioxo-3,4-dihydrochinazolin-1(2H)-yl}piperidin-1-carbaldehyd
Nr. 278: 4-{6-[2-Fluor-1-(fluormethyl)ethoxy]-2,4-dioxo-3-{[6-(1H-pyrazol-1-yl)pyridin-3-yl]methyl}-3,4-dihydrochinazolin-1(2H)-yl}piperidin-1-carbaldehyd
Nr. 279: 4-{6-[2-Fluor-1-(fluormethyl)ethoxy]-2,4-dioxo-3-[(2-(thiophen-2-yl)pyrimidin-5-yl)methyl]-3,4-dihydrochinazolin-1(2H)-yl}piperidin-1-carbaldehyd
Nr. 280: 4-{6-[2-Fluor-l-(fluormethyl)ethoxy]-3-[4-(1-methyl-1H-pyrazol-3-yl)benzyl]-2,4-dioxo-3,4-dihydrochinazolin-1(2H)-yl}piperidin-1-carbaldehyd
Nr. 282: 4-{6-[2-Fluor-l-(fluormethyl)ethoxy]-3-[4-(3-methyl-1,2,4-oxadiazol-5-yl)benzyl]-2,4-dioxo-3,4-dihydrochinazolin-1(2H)-yl}piperidin-1-carbaldehyd
Nr. 283: [2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]essigsäure
Nr. 285: 4-{6-[2-Fluor-1-(fluormethyl)ethoxy]-2,4-dioxo-3-(thieno[2,3-b]pyridin-2-ylmethyl)-3,4-dihydrochinazolin-1(2H)-yl}piperidin-1-carbaldehyd
Nr. 286: 4-{6-[2-Fluor-1-(fluormethyl)ethoxy]-2,4-dioxo-3-[(6-phenylpyridin-3-yl)methyl]-3,4-dihydrochinazolin-1(2H)-yl}piperidin-1-carbaldehyd
Nr. 287: 4-{6-[2-Fluor-1-(fluormethyl)ethoxy]-3-[(6-(morpholin-4-yl)pyridin-3-yl)methyl]-2,4-dioxo-3,4-dihydrochinazolin-1(2H)-yl}piperidin-1-carbaldehyd
Nr. 289: 4-{6-[2-Fluor-1-(fluormethyl)ethoxy]-2,4-dioxo-3-[(6-(thiophen-2-yl)pyridin-3-yl)methyl]-3,4-dihydrochinazolin-1(2H)-yl}piperidin-1-carbaldehyd
Nr. 292: 4-{6-[2-Fluor-1-(fluormethyl)ethoxy]-3-[(1-methyl-5-phenyl-1H-pyrazol-3-yl)methyl]-2,4-dioxo-3,4-dihydrochinazolin-1(2H)-yl}piperidin-1-carbaldehyd
Nr. 294: 4-({6-[2-Fluor-1-(fluormethyl)ethoxy]-1-(1-formylpiperidin-4-yl)-2,4-dioxo-1,4-dihydrochinazolin-3(2H)-yl}methyl)biphenyl-2-carbonitril
Nr. 295: (2R)-2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]-N-methylpropanamid
Nr. 297: 4-{7-Fluor-6-[2-fluor-1-(fluormethyl)ethoxy]-2,4-dioxo-3-(4-(thiophen-2-yl)benzyl)-3,4-dihydrochinazolin-1(2H)-yl}piperidin-1-carbaldehyd
Nr. 298: 4-{6-[2-Fluor-1-(fluormethyl)ethoxy]-3-[3-methoxy-4-(morpholin-4-ylmethyl)benzyl]-2,4-dioxo-3,4-dihydrochinazolin-1(2H)-yl}piperidin-1-carbaldehyd
Nr. 299: 4-{6-[2-Fluor-1-(fluormethyl)ethoxy]-3-[3-methoxy-4-(piperidin-1-ylmethyl)benzyl]-2,4-dioxo-3,4-dihydrochinazolin-1(2H)-yl}piperidin-1-carbaldehyd
Nr. 300: 4-[3-{4-[(3,4-Dichlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 301: 2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]-N-ethylacetamid
Nr. 302: (2S)-2-[2-(Cyclopentyloxy)-5-({6-[2-fluor-1-(fluormethyl)ethoxy]-1-(1-formylpiperidin-4-yl)-2,4-dioxo-1,4-dihydrochinazolin-3(2H)-yl}methyl)phenoxy]propansäure
Nr. 305: 4-{6-[2-Fluor-1-(fluormethyl)ethoxy]-3-(3-methoxy-4-{[(3R)-2-oxo-1-phenylpyrrolidin-3-yl]oxy}benzyl)-2,4-dioxo-3,4-dihydrochinazolin-1(2H)-yl}piperidin-1-carbaldehyd
Nr. 306: 4-{3-[4-(Cyclobutylmethoxy)-3-methoxybenzyl]-6-[2-fluor-1-(fluormethyl)ethoxy]-2,4-dioxo-3,4-dihydrochinazolin-1(2H)-yl}piperidin-1-carbaldehyd
Nr. 307: 4-{3-[4-(Benzyloxy)-3-methoxybenzyl]-7-fluor-6-[2-fluor-1-(fluormethyl)ethoxy]-2,4-dioxo-3,4-dihydrochinazolin-1(2H)-yl}piperidin-1-carbaldehyd
Nr. 308: 4-{7-Fluor-6-[2-fluor-1-(fluormethyl)ethoxy]-3-(4-hydroxy-3-methoxybenzyl)-2,4-dioxo-3,4-dihydrochinazolin-1(2H)-yl}piperidin-1-carbaldehyd
Nr. 309: 4-{3-[4-(Cyclopropylmethoxy)-3-methoxybenzyl]-7-fluor-6-[2-fluor-1-(fluormethyl)ethoxy]-2,4-dioxo-3,4-dihydrochinazolin-1(2H)-yl}piperidin-1-carbaldehyd
Nr. 310: 4-{7-Fluor-6-[2-fluor-1-(fluormethyl)ethoxy]-3-[3-methoxy-4-(2-methylpropoxy)benzyl]-2,4-dioxo-3,4-dihydrochinazolin-1(2H)-yl}piperidin-1-carbaldehyd
Nr. 311: 4-{7-Fluor-6-[2-fluor-1-(fluormethyl)ethoxy]-3-[3-methoxy-4-(1-methylethoxy)benzyl]-2,4-dioxo-3,4-dihydrochinazolin-1(2H)-yl}piperidin-1-carbaldehyd
Nr. 312: 4-[3-(4-Ethoxy-3-methoxybenzyl)-7-fluor-6-[2-fluor-1-(fluormethyl)ethoxy]-2,4-dioxo-3,4-dihydrochinazolin-1(2H)-yl]piperidin-1-carbaldehyd
Nr. 315: 4-{7-Fluor-6-[2-fluor-1-(fluormethyl)ethoxy]-3-{[6-(3-methoxyphenyl)pyridin-3-yl]methyl}-2,4-dioxo-3,4-dihydrochinazolin-1(2H)-yl}piperidin-1-carbaldehyd
Nr. 316: 4-{7-Fluor-6-[2-fluor-1-(fluormethyl)ethoxy]-3-{[6-(2-fluorphenyl)pyridin-3-yl]methyl}-2,4-dioxo-3,4-dihydrochinazolin-1(2H)-yl}piperidin-1-carbaldehyd
Nr. 317: 4-{7-Fluor-6-[2-fluor-1-(fluormethyl)ethoxy]-3-{[6-(4-fluorphenyl)pyridin-3-yl]methyl}-2,4-dioxo-3,4-dihydrochinazolin-1(2H)-yl}piperidin-1-carbaldehyd
Nr. 318: 4-{7-Fluor-6-[2-fluor-1-(fluormethyl)ethoxy]-3-{[6-(4-methoxyphenyl)pyridin-3-yl]methyl}-2,4-dioxo-3,4-dihydrochinazolin-1(2H)-yl}piperidin-1-carbaldehyd
Nr. 319: 4-{7-Fluor-6-[2-fluor-1-(fluormethyl)ethoxy]-2,4-dioxo-3-[(6-(thiophen-2-yl)pyridin-3-yl)methyl]-3,4-dihydrochinazolin-1(2H)-yl}piperidin-1-carbaldehyd
Nr. 320: 4-{3-[3-Ethoxy-4-(thiophen-2-ylmethoxy)benzyl]-7-fluor-6-[2-fluor-1-(fluormethyl)ethoxy]-2,4-dioxo-3,4-dihydrochinazolin-1(2H)-yl}piperidin-1-carbaldehyd
Nr. 321: 4-{7-Fluor-6-[2-fluor-1-(fluormethyl)ethoxy]-3-[4-(1-methyl-1H-pyrazol-3-yl)benzyl]-2,4-dioxo-3,4-dihydrochinazolin-1(2H)-yl}piperidin-1-carbaldehyd
Nr. 322: 4-{7-Fluor-6-[2-fluor-1-(fluormethyl)ethoxy]-2,4-dioxo-3-(4-(pyrimidin-5-yl)benzyl)-3,4-dihydrochinazolin-1(2H)-yl}piperidin-1-carbaldehyd
Nr. 323: 4-{7-Fluor-6-[2-fluor-1-(fluormethyl)ethoxy]-3-[(1-methyl-3-(thiophen-2-yl)-1H-pyrazol-5-yl)methyl]-2,4-dioxo-3,4-dihydrochinazolin-1(2H)-yl}piperidin-1-carbaldehyd
Nr. 324: 4-{7-Fluor-6-[2-fluor-1-(fluormethyl)ethoxy]-3-[3-methoxy-4-(2-oxo-2-(piperidin-1-yl)ethoxy)benzyl]-2,4-dioxo-3,4-dihydrochinazolin-1(2H)-yl}piperidin-1-carbaldehyd
Nr. 325: 4-[3-{4-[2-(2,3-Dihydro-1H-indol-1-yl)-2-oxoethoxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 326: 4-{7-Fluor-6-[2-fluor-1-(fluormethyl)ethoxy]-3-[4-(5-methyl-1,2,4-oxadiazol-3-yl)benzyl]-2,4-dioxo-3,4-dihydrochinazolin-1(2H)-yl}piperidin-1-carbaldehyd
Nr. 327: 4-[3-{4-[(3-Chlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 328: 4-[3-{[6-(3,5-Dichlorphenyl)pyridin-3-yl]methyl}-6-[2-fluor-1-(fluormethyl)ethoxy]-2,4-dioxo-3,4-dihydrochinazolin-1(2H)-yl]piperidin-1-carbaldehyd
Nr. 329: 4-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)biphenyl-2-carbonitril
Nr. 330: 4-{7-Fluor-6-[2-fluor-1-(fluormethyl)ethoxy]-2,4-dioxo-3-[4-(1H-pyrazol-1-yl)benzyl]-3,4-dihydrochinazolin-1(2H)-yl}piperidin-1-carbaldehyd
Nr. 331: 4-{7-Fluor-6-[2-fluor-1-(fluormethyl)ethoxy]-3-{[6-(3-fluorphenyl)pyridin-3-yl]methyl}-2,4-dioxo-3,4-dihydrochinazolin-1(2H)-yl}piperidin-1-carbaldehyd
Nr. 332: 3-[5-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)pyridin-2-yl]benzonitril
Nr. 333: 4-[3-(3,4-Diethoxybenzyl)-7-fluor-6-[2-fluor-1-(fluormethyl)ethoxy]-2,4-dioxo-3,4-dihydrochinazolin-1(2H)-yl]piperidin-1-carbaldehyd
Nr. 334: 4-[3-{4-[(4-Chlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 335: 4-{7-Fluor-6-[2-fluor-1-(fluormethyl)ethoxy]-3-[4-(morpholin-4-ylmethyl)benzyl]-2,4-dioxo-3,4-dihydrochinazolin-1(2H)-yl}piperidin-1-carbaldehyd
Nr. 336: 4-{7-Fluor-6-[2-fluor-1-(fluormethyl)ethoxy]-2,4-dioxo-3-{[6-(1H-pyrazol-1-yl)pyridin-3-yl]methyl}-3,4-dihydrochinazolin-1(2H)-yl}piperidin-1-carbaldehyd
Nr. 337: 4-{7-Fluor-6-[2-fluor-1-(fluormethyl)ethoxy]-3-(4-(morpholin-4-yl)benzyl)-2,4-dioxo-3,4-dihydrochinazolin-1(2H)-yl}piperidin-1-carbaldehyd
Nr. 338: 4-{7-Fluor-6-[2-fluor-1-(fluormethyl)ethoxy]-3-(3-methoxy-4-propoxybenzyl)-2,4-dioxo-3,4-dihydrochinazolin-1(2H)-yl}piperidin-1-carbaldehyd
Nr. 339: 4-{3-[4-(1H-Benzimidazol-1-yl)benzyl]-7-fluor-6-[2-fluor-1-(fluormethyl)ethoxy]-2,4-dioxo-3,4-dihydrochinazolin-1(2H)-yl}piperidin-1-carbaldehyd
Nr. 340: 5-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)-2-methoxybenzonitril
Nr. 341: 3-(3,4-Dimethoxybenzyl)-6-[2-fluor-1-(fluormethyl)ethoxy]-1-(1-formylpiperidin-4-yl)-2,4-dioxo-1,2,3,4-tetrahydrochinazolin-7-carbonitril
Nr. 342: 4-[3-(4-Brombenzyl)-7-fluor-6-[2-fluor-1-(fluormethyl)ethoxy]-2,4-dioxo-3,4-dihydrochinazolin-1(2H)-yl]piperidin-1-carbaldehyd
Nr. 343: 4-[3-{4-[(3,4-Dichlorbenzyl)oxy]-3-(2-methoxyethoxy)benzyl}-7-fluor-6-[2-fluor-1-(fluormethyl)ethoxy]-2,4-dioxo-3,4-dihydrochinazolin-1(2H)-yl]piperidin-1-carbaldehyd
Nr. 344: 4-{3-[4-(Benzyloxy)benzyl]-7-fluor-6-[2-fluor-1-(fluormethyl)ethoxy]-2,4-dioxo-3,4-dihydrochinazolin-1(2H)-yl}piperidin-1-carbaldehyd
Nr. 345: 4-[3-{4-[(3,4-Dichlorbenzyl)oxy]-3-ethoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 349: 4-[3-{4-[(3,4-Dichlorbenzyl)oxy]-3-(2-fluorethoxy)benzyl}-7-fluor-6-[2-fluor-1-(fluormethyl)ethoxy]-2,4-dioxo-3,4-dihydrochinazolin-1(2H)-yl]piperidin-1-carbaldehyd
Nr. 350: 4-[3-{4-[(2-Chlor-4-fluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 351: 4-[3-{4-[(2,4-Dichlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 352: 4-[3-{4-[(2-Chlor-6-fluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 353: 4-[3-{4-[(2,6-Dichlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 354: 4-[3-{4-[(2-Chlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 355: 4-[7-Fluor-3-{4-[(2-fluorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 357: 2-[(3,4-Dichlorbenzyl)oxy]-5-({7-fluor-6-[2-fluor-1-(fluormethyl)ethoxy]-1-(1-formylpiperidin-4-yl)-2,4-dioxo-1,4-dihydrochinazolin-3(2H)-yl}methyl)benzonitril
Nr. 358: 4-[3-{4-[(3,4-Dichlorphenoxy)methyl]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 360: 4-{7-Fluor-6-[2-fluor-1-(fluormethyl)ethoxy]-2,4-dioxo-3-[4-(2-phenylethyl)benzyl]-3,4-dihydrochinazolin-1(2H)-yl}piperidin-1-carbaldehyd
Nr. 362: 4-[3-{4-[(4,5-Dichlor-2-fluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 369: 4-[3-{4-[(4-Chlorphenoxy)methyl]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 371: 4-[3-{3-Chlor-4-[(4-chlorbenzyl)oxy]-5-ethoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 373: 4-[3-{3-Chlor-4-[(2,4-dichlorbenzyl)oxy]-5-ethoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 375: 4-[7-Fluor-3-{4-[(4-fluorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 376: 4-[3-{4-[(3,5-Dichlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 377: 4-[3-(4-{[4-Chlor-3-(trifluormethyl)benzyl]oxy}-3-methoxybenzyl)-7-fluor-6-[2-fluor-1-(fluormethyl)ethoxy]-2,4-dioxo-3,4-dihydrochinazolin-1(2H)-yl]piperidin-1-carbaldehyd
Nr. 379: 4-[3-{4-[(3-Chlorphenoxy)methyl]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 380: 4-[3-{4-[(3,5-Difluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 381: 4-{3-[4-(Benzyloxy)-3-methoxybenzyl]-7-fluor-6-[2-fluor-1-(fluormethyl)ethoxy]-2,4-dioxo-3,4-dihydrochinazolin-1(2H)-yl}piperidin-1-carbaldehyd
Nr. 382: 4-[3-{4-[(3-Chlor-5-fluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 383: 4-{7-Fluor-6-[2-fluor-1-(fluormethyl)ethoxy]-3-(3-methoxy-4-{[4-(trifluormethyl)benzyl]oxy}benzyl)-2,4-dioxo-3,4-dihydrochinazolin-1(2H)-yl}piperidin-1-carbaldehyd
Nr. 384: 4-[3-{4-[(2,5-Dichlorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 385: 4-{[4-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)-2-methoxyphenoxy]methyl}benzonitril
Nr. 386: 3-{[4-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)-2-methoxyphenoxy]methyl}benzonitril
Nr. 387: 4-[3-{4-[(4-Chlor-2-fluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 388: 4-[3-{4-[1-(3,4-Dichlorphenyl)ethoxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 389: 4-{7-Fluor-6-[2-fluor-1-(fluormethyl)ethoxy]-3-{4-[(3-hydroxybenzyl)oxy]-3-methoxybenzyl}-2,4-dioxo-3,4-dihydrochinazolin-1(2H)-yl}piperidin-1-carbaldehyd
Nr. 390: 4-[7-Fluor-3-{4-[(3-fluorbenzyl)oxy]-3-methoxybenzyl}-6-[2-fluor-1-(fluormethyl)ethoxy]-2,4-dioxo-3,4-dihydrochinazolin-1(2H)-yl]piperidin-1-carbaldehyd
Nr. 391: 4-[3-{4-[(3,4-Difluorbenzyl)oxy]-3-methoxybenzyl}-7-fluor-6-[2-fluor-1-(fluormethyl)ethoxy]-2,4-dioxo-3,4-dihydrochinazolin-1(2H)-yl]piperidin-1-carbaldehyd
Nr. 392: 4-{3-[4-(5,6-Dichlor-1H-benzimidazol-1-yl)-3-methoxybenzyl]-7-fluor-6-[2-fluor-1-(fluormethyl)ethoxy]-2,4-dioxo-3,4-dihydrochinazolin-1(2H)-yl}piperidin-1-carbaldehyd
Nr. 393: 4-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)phenyl-3,4-dichlorbenzolsulfonat
Nr. 394: 4-({7-Fluor-6-[2-fluor-1-(fluormethyl)ethoxy]-1-(1-formylpiperidin-4-yl)-2,4-dioxo-1,4-dihydrochinazolin-3(2H)-yl}methyl)-2-methoxyphenyl-3,4-dichlorbenzolsulfonat
Nr. 403: 3,4-Dichlor-N-[4-({7-fluor-6-[2-fluor-1-(fluormethyl)ethoxy]-1-(1-formylpiperidin-4-yl)-2,4-dioxo-1,4-dihydrochinazolin-3(2H)-yl}methyl)-2-methoxyphenyl]benzamid

12. Verfahren zur Herstellung einer Verbindung nach einem der Ansprüche 1 bis 11, **dadurch gekennzeichnet, dass** man eine Verbindung der Formel 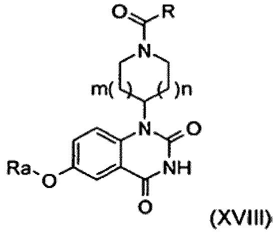 worin R, Ra, m und n die in Anspruch 1 angegebene Bedeutung besitzen und außerdem eine R₂-Gruppe gemäß der für die Verbindungen der Formel (I) angegebenen Definition in 7-Position der Chinazolindion-Struktur vorliegen kann, mit einer Verbindung der Formel 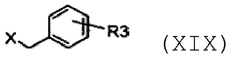 oder 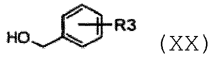 durch eine Alkylierungsreaktion bzw. eine Mitsunobu-Reaktion umsetzt.

13. Verbindung der allgemeinen Formel (XVIII) 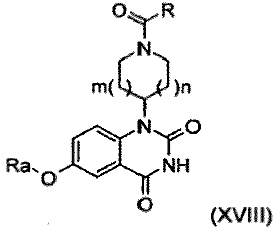 worin R, Ra, m und n die in Anspruch 1 angegebene Bedeutung besitzen und außerdem eine R₂-Gruppe gemäß der für die Verbindungen der Formel (I) angegebenen Definition in 7-Position der Chinazolindion-Struktur vorliegen kann.

14. Verbindung der allgemeinen Formel (XVIII) nach Anspruch 12, ausgewählt aus der Verbindung Nr. 32, 4-[6-(2,2-Difluorethoxy)-2,4-dioxo-3,4-dihydrochinazolin-1(2H)-yl]piperidin-1-carbaldehyd, und der Verbindung Nr. 55, 4-{6-[2-Fluor-1-(fluormethyl)ethoxy]-2,4-dioxo-3,4-dihydrochinazolin-1(2H)-yl}piperidin-1-carbaldehyd; in Basen-, Hydrat- oder Solvatform oder in Form von Mischungen davon.

15. Arzneimittel, **dadurch gekennzeichnet, dass** es mindestens eine Verbindung der Formel (I) nach einem der Ansprüche 1 bis 11 umfasst.

16. Pharmazeutische Zusammensetzung, **dadurch gekennzeichnet, dass** sie mindestens eine Verbindung der Formel (I) nach einem der Ansprüche 1 bis 11 sowie mindestens einen pharmazeutisch akzeptablen Hilfsstoff umfasst.

17. Verwendung einer Verbindung der Formel (I) nach einem der Ansprüche 1 bis 11 zur Herstellung eines Arzneimittels zur Behandlung und/oder Prävention von entzündlichen oder immunentzündlichen Erkrankungen einschließlich Asthma, chronisch obstruktiver Lungenerkrankung (COPD), allergischer Rhinitis, Allergien, Morbus Crohn, Colitis ulcerosa, Myasthenia gravis, atopischer Dermatitis, Psoriasis, systemischem Lupus erythematodes, rheumatoider Arthritis, Diabetes und multipler Sklerose, bei der Behandlung von Organtransplantationen, bei der Behandlung und/oder Prävention von Krebs, insbesondere Osteosarkom oder Adenokarzinom, bei der Behandlung und/oder Prävention von Knochenerkrankungen einschließlich Osteopenie und Osteoporose, bei der Behandlung und/oder Prävention von akuter Insuffizienz, bei der Behandlung und/oder Prävention von Schmerzen, neuropathischen Schmerzen und viszeralen Schmerzen.

18. Verwendung einer Verbindung der Formel (I) nach Anspruch 17 zur Herstellung eines Arzneimittels zur Behandlung und/oder Prävention von neuropathischen Schmerzen.

## Claims

1. Compound corresponding to the general formula (I) 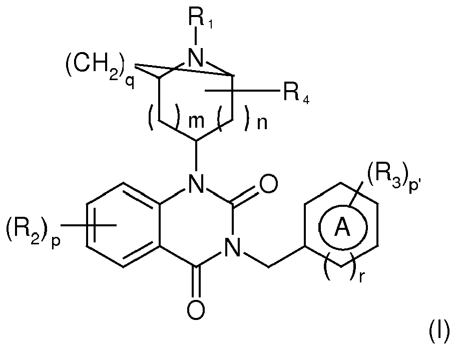 in which
- A represents an aryl group or a heteroaryl group;
- R₁ represents:
▪ a hydrogen atom,
▪ -C(O)R in which R is a hydrogen atom, a (C₁-C₆) alkoxy group, an aryl group, a (C₃-C₆) cycloalkyl group or a (C₁-C₆) alkyl group, the said alkyl optionally being substituted by:
. one or more hydroxyl group(s),
. a benzyloxy group,
. a (C₁-C₆) alkoxy group, optionally substituted by an aryl, or
. a (C₃-C₆) cycloalkyl group,
▪ an optionally substituted (C₁-C₆) alkyl group;
- R₂ represents:
▪ a hydrogen atom,
▪ a halogen atom,
▪ a cyano group,
▪ a nitro group,
▪ a (C₁-C₆) alkyl group optionally substituted by an -NH₂ or else by an -NHC(O)Rb group,
▪ an -ORa group in which Ra represents:
. a hydrogen atom,
. a (C₁-C₆) alkyl group optionally substituted by one or more halogen atom(s), by one or more hydroxyl group(s), by an aryl group and/or by one or more cyano group(s),
. a (C₂-C₆) alkynyl group,
. an aryl group;
- R₃ represents:
▪ a hydrogen atom,
▪ a halogen atom,
▪ a hydroxyl group,
▪ a cyano group,
▪ an -SCF₃ group,
▪ a nitro group,
▪ an oxo group,
▪ an -S (0) ₀₋₂-alkyl group, an -S (0)₀₋₂-heterocycloalkyl group, an -O-SO₂-aryl group optionally substituted by one or more halogen atom(s);
▪ an -alkylaminoalkyl or -cycloalkylaminoalkyl group, each optionally substituted on the end alkyl,
▪ an optionally substituted sulphonamide group,
▪ an aryl group or a heteroaryl group, the said group being monocyclic or polycyclic and in addition optionally being substituted by a (C₁-C₆) alkyl group, by one or more halogen atom(s) or by a (C₁-C₆) alkoxy group,
▪ a heterocycloalkyl group optionally substituted by a (C₁-C₆) alkyl group,
▪ a (C₁-C₆) alkyl group optionally substituted by:
- one or more halogen atom(s),
- an aryl group which can be substituted by one or more halogen atom(s) or by one or more hydroxyl group(s),
- a heteroaryl group,
- one or more hydroxyl group (s) which can be substituted by an aryl group itself optionally substituted by one or more halogen atom(s), or
- a heterocycloalkyl group optionally substituted by a CO(O)Ra group or by a (C₁-C₆) alkyl group,
▪ a -C(O)NRbRc group,
▪ a -C(O)ORc group or an -O-C(O)ORc group,
▪ a (C₁-C₆) alkoxy group, optionally substituted by
- an aminoalkyl group,
- an aminocycloalkyl group,
- a cycloalkyl group,
- a heterocycloalkyl group,
- a monocyclic or polycyclic heteroaryl group,
- one or more hydroxyl group(s),
- one or more halogen atom(s),
- a (C₁-C₆) alkoxy group,
- a -C(O)ORc group,
- a -C(O)NRbRc group,
- an oxo group, and/or
- an aryl group, itself optionally substituted by one or more halogen atom(s), a cyano group, a (C₁-C₆) alkoxy group, an -0-haloalkyl group and/or a haloalkyl group,
▪ an -0-cycaloalkyl group, an -0-aryl group or an -0-heterocycloalkyl group, each optionally substituted by
- an aryl group, itself optionally substituted by one or more halogen atom(s) or by a (C₁-C₆) alkyl group,
- an oxo group,
- one or more halogen atom(s), and/or
- a (C₁-C₆) alkyl group, which can itself be substituted by an aryl group and/or an oxo group,
▪ an -NH-CO-NH-aryl group, an -NH-CO-NH-heteroaryl group or an -NH-CO-NH-(C₁-C₆) alkyl group, each optionally being substituted by one or more halogen atom(s), by a cyano group, by a nitro group, by one or more hydroxyl group(s) or by a (C₁-C₆) alkoxy group,
▪ an -N-(C₁-C₆) alkyl group, it being possible for the (C₁-C₆) alkyl group to be substituted by
- one or more oxo group(s), and/or
- one or more aryl group (s) optionally substituted by one or more halogen atom(s) and/or by an SO₂ group,
▪ an -NH-CO-aryl group or an -NH-CO-heteroaryl group, each optionally being substituted by one or more halogen atom(s);
or else R₃ forms, with A, a polycyclic heteroaryl group optionally substituted by a (C₁-C₆) alkoxy group or a (C₁-C₆) alkyl group optionally substituted by an aryl group which can itself be substituted by one or more halogen atom(s);
- R₄ represents a hydrogen atom, an oxo group or a (C₁-C₆) alkyl group;
- Rb represents:
. a hydrogen atom,
. a (C₁-C₆) alkyl group optionally substituted by one or more halogen atom(s), by one or more hydroxyl, cyano, amino, heterocycloalkyl or (C₁-C₆) alkoxy group(s) or by an aryl group optionally substituted by one or more halogen atom(s),
. a (C₃-C₆) cycloalkyl group,
. a (C₂-C₆) alkynyl group,
. a (C₁-C₆) alkoxy group,
. an aryl group optionally substituted by one or more halogen atom(s);
- Rc represents a hydrogen atom or a (C₁-C₆) alkyl group optionally substituted by one or more halogen atom(s);
or then Rb and Rc form, together with the nitrogen atom to which they are attached, a polycyclic heteroaryl group or a heterocycloalkyl group;
- m and n represent, independently of one another, the value 0, 1 or 2, it being understood that m+n≤3;
- p and p' represent, independently of one another, the value 1, 2 or 3, it being understood that, when p is greater than or equal to 2, then the R₂ groups are on separate carbon atoms and can be different from one another and, when p' is greater than or equal to 2, then the R₃ groups are on separate carbon atoms and can be different from one another;
- q represents the value 0 or 2, it being understood that, when q = 0, then the nitrogenous heterocyclic group attached to the nitrogen situated in the 1 position of the 2,4-dioxo-1,2,3,4-tetrahydroquinazoline ring system is no longer bridged and is of the type: 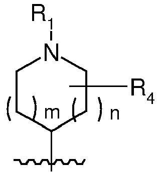
- r represents the value 0 or 1.

2. Compound of general formula (I) according to Claim 1, **characterized in that** A represents a phenyl group or a pyridyl group.

3. Compound of general formula (I) according to Claim 1 or 2, **characterized in that** q = 0, and m and n =1.

4. Compound of general formula (I) according to any one of Claims 1 to 3, **characterized in that** R₂ represents a (C₁-C₆) alkyl group, in particular a methyl substituted by an -NH-CO-Rb group, Rb being as defined in Claim 1.

5. Compound of general formula (I) according to any one of Claims 1 to 3, **characterized in that** R₂ represents an -ORa group, Ra being as defined in Claim 1.

6. Compound of general formula (I) according to any one of Claims 1 to 3, **characterized in that** R₂ is a halogen atom or a cyano or a hydrogen or a hydroxyl or a (C₁-C₆) alkyl optionally substituted by an -NH₂ or else by an-NHC(O)Rb group.

7. Compound of general formula (I) according to Claim 1, **characterized in that** A is a phenyl, R₁ is a-C(O)R group in which R represents a hydrogen atom, q is equal to 0, n and m have the value 1 and R₂ is -ORa.

8. Compound of general formula (I) according to Claim 1, **characterized in that** A is a phenyl, R₁ is a-C(O)R group in which R represents a hydrogen atom, q is equal to 0, n and m have the value 1 and R₂ is a methyl substituted by the -NH-CO-Rb group, Rb being as defined in Claim 1.

9. Compound of general formula (I) according to Claim 1, **characterized in that** A is a phenyl, R₁ is a-C(O)R group in which R represents a hydrogen atom, q is equal to 0, n and m have the value 1, p is equal to 2, one of the R₂ groups is -ORa, Ra being as defined in the general formula (I), and the other of the R₂ groups is a halogen atom.

10. Compound of general formula (I) according to any one of Claims 1 to 9, **characterized in that** the R₂ group is in the 6 position of the 2,4-dioxo-1,2,3,4-tetrahydroquinazoline ring system and **in that** there may additionally be an identical or different R₂ group in the 7 position of the 2,4-dioxo-1,2,3,4-tetrahydroquinazoline ring system ; in the base, hydrate or solvate form, in the form of isomers or in the form of their mixtures.

11. Compound of general formula (I) according to Claim 1, chosen from the following compounds:
No. 1: 2-{[3-(3,4-dimethoxybenzyl)-1-(1-formylpiperidin-4-yl)-2,4-dioxo-1,2,3,4-tetrahydroquinazolin-6-yl]oxy}propanenitrile
No. 2: 1-(1-acetylpiperidin-4-yl)-3-(3,4-dimethoxybenzyl)-6-hydroxyquinazoline-2,4(1H,3H)-dione
No. 3: {[1-(1-acetylpiperidin-4-yl)-3-(3,4-dimethoxybenzyl)-2,4-dioxo-1,2,3,4-tetrahydroquinazolin-6-yl]oxy}acetonitrile
No. 4: 2-{[1-(1-acetylpiperidin-4-yl)-3-(3,4-dimethoxybenzyl)-2,4-dioxo-1,2,3,4-tetrahydroquinazolin-6-yl]oxy}propanenitrile
No. 6: {[3-(3,4-dimethoxybenzyl)-1-(1-formylpiperidin-4-yl)-2,4-dioxo-1,2,3,4-tetrahydroquinazolin-6-yl]oxy}acetonitrile
No. 11: 4-[3-(3,4-dimethoxybenzyl)-6-[2-fluoro-1-(fluoromethyl)ethoxy]-2,4-dioxo-3,4-dihydroquinazolin-1(2H)-yl]piperidine-1-carbaldehyde
No. 12: 1-(1-acetylpiperidin-4-yl)-3-(3,4-dimethoxybenzyl)-6-[2-fluoro-1-(fluoromethyl)ethoxy]quinazoline-2,4(1H,3H)-dione
No. 13: 4-[3-(3,4-dimethoxybenzyl)-2,4-dioxo-6-(2,2,2-trifluoroethoxy)-3,4-dihydroquinazolin-1(2H)-yl]piperidine-1-carbaldehyde
No. 14: 1-(1-acetylpiperidin-4-yl)-6-(2,2-difluoroethoxy)-3-(3,4-dimethoxybenzyl)quinazoline-2,4(1H,3H)-dione
No. 16: 4-[6-(2,2-difluoroethoxy)-3-(3,4-dimethoxybenzyl)-2,4-dioxo-3,4-dihydroquinazolin-1(2H)-yl]piperidine-1-carbaldehyde
No. 20: N-{[3-(3,4-dimethoxybenzyl)-1-(1-formylpiperidin-4-yl)-2,4-dioxo-1,2,3,4-tetrahydroquinazolin-6-yl]methyl}acetamide
No. 22: 1-(1-acetylpiperidin-4-yl)-6-(aminomethyl)-3-(3,4-dimethoxybenzyl)quinazoline-2,4(1H,3H)-dione hydrochloride
No. 23: N-{[3-(3,4-dimethoxybenzyl)-1-(1-formylpiperidin-4-yl)-2,4-dioxo-1,2,3,4-tetrahydroquinazolin-6-yl]methyl}formamide
No. 24: N-{[1-(1-acetylpiperidin-4-yl)-3-(3,4-dimethoxybenzyl)-2,4-dioxo-1,2,3,4-tetrahydroquinazolin-6-yl]methyl}formamide
No. 25: N-{[1-(1-acetylpiperidin-4-yl)-3-(3,4-dimethoxybenzyl)-2,4-dioxo-1,2,3,4-tetrahydroquinazolin-6-yl]methyl}acetamide
No. 32: 4-[6-(2,2-difluoroethoxy)-2,4-dioxo-3,4-dihydroquinazolin-1(2H)-yl]piperidine-1-carbaldehyde
No. 33: 4-[3-(3,4-dichlorobenzyl)-6-(2,2-difluoroethoxy)-2,4-dioxo-3,4-dihydroquinazolin-1(2H)-yl]piperidine-1-carbaldehyde
No. 34: 4-[3-(4-chlorobenzyl)-6-(2,2-difluoroethoxy)-2,4-dioxo-3,4-dihydroquinazolin-1(2H)-yl]piperidine-1-carbaldehyde
No. 35: methyl 4-{[6-(2,2-difluoroethoxy)-1-(1-formylpiperidin-4-yl)-2,4-dioxo-1,4-dihydroquinazolin-3(2H)-yl]methyl}benzoate
No. 36: 4-{[6-(2,2-difluoroethoxy)-1-(1-formylpiperidin-4-yl)-2,4-dioxo-1,4-dihydroquinazolin-3(2H)-yl]methyl}benzoic acid
No. 37: 4-{[6-(2,2-difluoroethoxy)-1-(1-formylpiperidin-4-yl)-2,4-dioxo-1,4-dihydroquinazolin-3(2H)-yl]methyl}-N-(2-methoxyethyl)benzamide
No. 38: 4-[3-(3,4-dimethoxybenzyl)-6-methyl-2,4-dioxo-3,4-dihydroquinazolin-1(2H)-yl]piperidine-1-carbaldehyde
No. 39: 4-[6-(2,2-difluoroethoxy)-3-(3-hydroxy-4-methoxybenzyl)-2,4-dioxo-3,4-dihydroquinazolin-1(2H)-yl]piperidine-1-carbaldehyde
No. 40: 4-[6-(2,2-difluoroethoxy)-3-[3-(2-hydroxyethoxy)-4-methoxybenzyl]-2,4-dioxo-3,4-dihydroquinazolin-1(2H)-yl]piperidine-1-carbaldehyde
No. 41: 4-[6-(2,2-difluoroethoxy)-3-(3-ethoxy-4-methoxybenzyl)-2,4-dioxo-3,4-dihydroquinazolin-1(2H)-yl]piperidine-1-carbaldehyde
No. 42: 4-[6-(2,2-difluoroethoxy)-3-[4-methoxy-3-(2-methoxyethoxy)benzyl]-2,4-dioxo-3,4-dihydroquinazolin-1(2H)-yl]piperidine-1-carbaldehyde
No. 43: 4-[6-(2,2-difluoroethoxy)-3-(3,4-dimethoxybenzyl)-2,4-dioxo-3,4-dihydroquinazolin-1(2H)-yl]azepane-1-carbaldehyde
No. 47: 4-[6-(2,2-difluoroethoxy)-3-[3-(3-hydroxypropoxy)-4-methoxybenzyl]-2,4-dioxo-3,4-dihydroquinazolin-1(2H)-yl]piperidine-1-carbaldehyde
No. 48: 4-[5-chloro-3-(3,4-dimethoxybenzyl)-2,4-dioxo-3,4-dihydroquinazolin-1(2H)-yl]piperidine-1-carbaldehyde
No. 49: 4-{3-[3-(cyclopentyloxy)-4-methoxybenzyl]-6-(2,2-difluoroethoxy)-2,4-dioxo-3,4-dihydroquinazolin-1(2H)-yl}piperidine-1-carbaldehyde
No. 50: 2-(5-{[6-(2,2-difluoroethoxy)-1-(1-formylpiperidin-4-yl)-2,4-dioxo-1,4-dihydroquinazolin-3(2H)-yl]methyl}-2-methoxyphenoxy)acetamide
No. 51: 4-[6-(2,2-difluoroethoxy)-3-(3,4-dimethoxybenzyl)-2,4-dioxo-3,4-dihydroquinazolin-1(2H)-yl]-3-methylpiperidine-1-carbaldehyde
No. 52: 3-[6-(2,2-difluoroethoxy)-3-(3,4-dimethoxybenzyl)-2,4-dioxo-3,4-dihydroquinazolin-1(2H)-yl]-8-azabicyclo[3.2.1]octane-8-carbaldehyde
No. 56: 4-{3-[4-(cyclopentyloxy)-3-methoxybenzyl]-6-[2-fluoro-1-(fluoromethyl)ethoxy]-2,4-dioxo-3,4-dihydroquinazolin-1(2H)-yl}piperidine-1-carbaldehyde
No. 57: 4-[3-(3-chlorobenzyl)-6-[2-fluoro-1-(fluoromethyl)ethoxy]-2,4-dioxo-3,4-dihydroquinazolin-1(2H)-yl]piperidine-1-carbaldehyde
No. 58: 4-[3-(4-chlorobenzyl)-6-[2-fluoro-1-(fluoromethyl)ethoxy]-2,4-dioxo-3,4-dihydroquinazolin-1(2H)-yl]piperidine-1-carbaldehyde
No. 59: 4-{3-[3-(cyclopentyloxy)-4-methoxybenzyl]-6-[2-fluoro-1-(fluoromethyl)ethoxy]-2,4-dioxo-3,4-dihydroquinazolin-1(2H)-yl}piperidine-1-carbaldehyde
No. 72: 4-[3-(3,4-dimethoxybenzyl)-6-(2-hydroxyethoxy)-2,4-dioxo-3,4-dihydroquinazolin-1(2H)-yl]piperidine-1-carbaldehyde
No. 74: 4-[3-(3,4-dichlorobenzyl)-6-[2-fluoro-1-(fluoromethyl)ethoxy]-2,4-dioxo-3,4-dihydroquinazolin-1(2H)-yl]piperidine-1-carbaldehyde
No. 76: 4-{3-[(6-chloropyridin-3-yl)methyl]-6-[2-fluoro-1-(fluoromethyl)ethoxy]-2,4-dioxo-3,4-dihydroquinazolin-1(2H)-yl}piperidine-1-carbaldehyde
No. 78: 4-[3-(3-chloro-4-methoxybenzyl)-6-[2-fluoro-1-(fluoromethyl)ethoxy]-2,4-dioxo-3,4-dihydroquinazolin-1(2H)-yl]piperidine-1-carbaldehyde
No. 79: 4-[3-(3,4-dimethoxybenzyl)-6-(2-fluoroethoxy)-2,4-dioxo-3,4-dihydroquinazolin-1(2H)-yl]piperidine-1-carbaldehyde
No. 89: 2-[5-({6-[2-fluoro-1-(fluoromethyl)ethoxy]-1-(1-formylpiperidin-4-yl)-2,4-dioxo-1,4-dihydroquinazolin-3(2H)-yl}methyl)-2-methoxyphenoxy]acetamide
No. 90: 4-{6-[2-fluoro-1-(fluoromethyl)ethoxy]-3-(3-hydroxy-4-methoxybenzyl)-2,4-dioxo-3,4-dihydroquinazolin-1(2H)-yl}piperidine-1-carbaldehyde
No. 91: 4-[3-(3,4-dimethoxybenzyl)-6-ethoxy-2,4-dioxo-3,4-dihydroquinazolin-1(2H)-yl]piperidine-1-carbaldehyde
No. 97: 4-[5,7-dichloro-3-(3,4-dimethoxybenzyl)-2,4-dioxo-3,4-dihydroquinazolin-1(2H)-yl]piperidine-1-carbaldehyde
No. 102: 4-[7-chloro-3-(3,4-dimethoxybenzyl)-2,4-dioxo-3,4-dihydroquinazolin-1(2H)-yl]piperidine-1-carbaldehyde
No. 108: 4-{6-[2-fluoro-1-(fluoromethyl)ethoxy]-3-(3-fluoro-4-methoxybenzyl)-2,4-dioxo-3,4-dihydroquinazolin-1(2H)-yl}piperidine-1-carbaldehyde
No. 111: 4-[6-(difluoromethoxy)-3-(3,4-dimethoxybenzyl)-2,4-dioxo-3,4-dihydroquinazolin-1(2H)-yl]piperidine-1-carbaldehyde
No. 112: 4-[3-(3,4-dimethoxybenzyl)-6-(1-methylethoxy)-2,4-dioxo-3,4-dihydroquinazolin-1(2H)-yl]piperidine-1-carbaldehyde
No. 114: 4-{6-[2-fluoro-1-(fluoromethyl)ethoxy]-3-[4-methoxy-3-(1-methylethoxy)benzyl]-2,4-dioxo-3,4-dihydroquinazolin-1(2H)-yl}piperidine-1-carbaldehyde
No. 116: 4-{6-[2-fluoro-1-(fluoromethyl)ethoxy]-3-(3-methoxybenzyl)-2,4-dioxo-3,4-dihydroquinazolin-1(2H)-yl}piperidine-1-carbaldehyde
No. 117: 4-{3-[3,5-bis(trifluoromethyl)benzyl]-6-[2-fluoro-1-(fluoromethyl)ethoxy]-2,4-dioxo-3,4-dihydroquinazolin-1(2H)-yl}piperidine-1-carbaldehyde
No. 118: 4-[3-(3-ethoxybenzyl)-6-[2-fluoro-1-(fluoromethyl)ethoxy]-2,4-dioxo-3,4-dihydroquinazolin-1(2H)-yl]piperidine-1-carbaldehyde
No. 124: 4-{3-[3-chloro-4-(2-methoxyethoxy)benzyl]-6-[2-fluoro-1-(fluoromethyl)ethoxy]-2,4-dioxo-3,4-dihydroquinazolin-1(2H)-yl}piperidine-1-carbaldehyde
No. 130: 4-[3-(3,4-diethoxybenzyl)-6-[2-fluoro-1-(fluoromethyl)ethoxy]-2,4-dioxo-3,4-dihydroquinazolin-1(2H)-yl]piperidine-1-carbaldehyde
No. 131: 4-[3-(4-ethoxy-3-methoxybenzyl)-6-[2-fluoro-1-(fluoromethyl)ethoxy]-2,4-dioxo-3,4-dihydroquinazolin-1(2H)-yl]piperidine-1-carbaldehyde
No. 133: 4-{6-[2-fluoro-1-(fluoromethyl)ethoxy]-3-(4-methoxy-3-methylbenzyl)-2,4-dioxo-3,4-dihydroquinazolin-1(2H)-yl}piperidine-1-carbaldehyde
No. 134: 4-{6-[2-fluoro-1-(fluoromethyl)ethoxy]-2,4-dioxo-3-[4-(trifluoromethyl)benzyl]-3,4-dihydroquinazolin-1(2H)-yl}piperidine-1-carbaldehyde
No. 135: 4-{6-[2-fluoro-1-(fluoromethyl)ethoxy]-2,4-dioxo-3-[4-(trifluoromethyl)benzyl]-3,4-dihydroquinazolin-1(2H)-yl}piperidine-1-carbaldehyde
No. 143: 4-{3-[4-(benzyloxy)-3-methoxybenzyl]-6-[2-fluoro-1-(fluoromethyl)ethoxy]-2,4-dioxo-3,4-dihydroquinazolin-1(2H)-yl}piperidine-1-carbaldehyde
No. 145: 4-{6-[2-fluoro-1-(fluoromethyl)ethoxy]-3-(3-methoxy-4-nitrobenzyl)-2,4-dioxo-3,4-dihydroquinazolin-1(2H)-yl}piperidine-1-carbaldehyde
No. 155: 4-[3-(4-ethoxybenzyl)-6-[2-fluoro-1-(fluoromethyl)ethoxy]-2,4-dioxo-3,4-dihydroquinazolin-1(2H)-yl]piperidine-1-carbaldehyde
No. 158: 4-{6-[2-fluoro-1-(fluoromethyl)ethoxy]-3-[4-(morpholin-4-ylmethyl)benzyl]-2,4-dioxo-3,4-dihydroquinazolin-1(2H)-yl}piperidine-1-carbaldehyde
No. 160: 4-{6-[2-fluoro-1-(fluoromethyl)ethoxy]-3-(4-morpholin-4-ylbenzyl)-2,4-dioxo-3,4-dihydroquinazolin-1(2H)-yl}piperidine-1-carbaldehyde
No. 165: 4-[3-(biphenyl-4-ylmethyl)-6-[2-fluoro-1-(fluoromethyl)ethoxy]-2,4-dioxo-3,4-dihydroquinazolin-1(2H)-yl]piperidine-1-carbaldehyde
No. 166: 4-{6-[2-fluoro-1-(fluoromethyl)ethoxy]-3-[4-(methylsulphanyl)benzyl]-2,4-dioxo-3,4-dihydroquinazolin-1(2H)-yl}piperidine-1-carbaldehyde
No. 167: 4-{6-[2-fluoro-1-(fluoromethyl)ethoxy]-2,4-dioxo-3-(4-(pyridin-3-yl)benzyl)-3,4-dihydroquinazolin-1(2H)-yl}piperidine-1-carbaldehyde
No. 170: 4-{6-[2-fluoro-1-(fluoromethyl)ethoxy]-3-(3-methoxy-4-methylbenzyl)-2,4-dioxo-3,4-dihydroquinazolin-1(2H)-yl}piperidine-1-carbaldehyde
No. 175: 2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]acetamide
No. 178: 4-{6-[2-fluoro-1-(fluoromethyl)ethoxy]-3-(3-methoxy-4-propoxybenzyl)-2,4-dioxo-3,4-dihydroquinazolin-1(2H)-yl}piperidine-1-carbaldehyde
No. 183: 2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]-N-methylacetamide
No. 184: 2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]-N,N-dimethylacetamide
No. 185: 2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]-N-methoxy-N-methylacetamide
No. 186: 4-{3-[4-(cyclopentyloxy)-3-ethoxybenzyl]-6-[2-fluoro-1-(fluoromethyl)ethoxy]-2,4-dioxo-3,4-dihydroquinazolin-1(2H)-yl}piperidine-1-carbaldehyde
No. 188: 4-{3-[4-(cyclopentyloxy)-3-(1-methylethoxy)benzyl]-6-[2-fluoro-1-(fluoromethyl)ethoxy]-2,4-dioxo-3,4-dihydroquinazolin-1(2H)-yl}piperidine-1-carbaldehyde
No. 189: 4-{3-[4-(cyclopentyloxy)-3-propoxybenzyl]-6-[2-fluoro-1-(fluoromethyl)ethoxy]-2,4-dioxo-3,4-dihydroquinazolin-1(2H)-yl}piperidine-1-carbaldehyde
No. 190: 4-{3-[4-(cylopentyloxy)-3-hydroxybenzyl]-6-[2-fluoro-1-(fluoromethyl)ethoxy]-2,4-dioxo-3,4-dihydroquinazolin-1(2H)-yl}piperidine-1-carbaldehyde
No. 193: 4-{3-[4-(difluoromethoxy)-3-methoxybenzyl]-6-[2-fluoro-1-(fluoromethyl)ethoxy]-2,4-dioxo-3,4-dihydroquinazolin-1(2H)-yl}piperidine-1-carbaldehyde
No. 194: 4-{3-[4-(difluoromethoxy)-3-ethoxybenzyl]-6-[2-fluoro-1-(fluoromethyl)ethoxy]-2,4-dioxo-3,4-dihydroquinazolin-1(2H)-yl}piperidine-1-carbaldehyde
No. 200: 4-{6-[2-fluoro-1-(fluoromethyl)ethoxy]-2,4-dioxo-3-(4-(thiophen-3-yl)benzyl)-3,4-dihydroquinazolin-1(2H)-yl}piperidine-1-carbaldehyde
No. 201: 4-{6-[2-fluoro-1-(fluoromethyl)ethoxy]-2,4-dioxo-3-(4-(pyridin-4-yl)benzyl)-3,4-dihydroquinazolin-1(2H)-yl}piperidine-1-carbaldehyde
No. 203: 4-{6-[2-fluoro-1-(fluoromethyl)ethoxy]-3-[(1-methyl-1H-indol-6-yl)methyl]-2,4-dioxo-3,4-dihydroquinazolin-1(2H)-yl}piperidine-1-carbaldehyde
No. 206: 4-{3-[4-(cyclopropylmethoxy)-3-methoxybenzyl]-6-[2-fluoro-1-(fluoromethyl)ethoxy]-2,4-dioxo-3,4-dihydroquinazolin-1(2H)-yl}piperidine-1-carbaldehyde
No. 207: 2-[4-({6-[2-fluoro-1-(fluoromethyl)ethoxy]-1-(1-formylpiperidin-4-yl)-2,4-dioxo-1,4-dihydroquinazolin-3(2H)-yl}methyl)-2-methoxyphenoxy]-N-methylacetamide
No. 212: 4-{6-[2-fluoro-1-(fluoromethyl)ethoxy]-2,4-dioxo-3-[4-(1H-pyrazol-1-yl)benzyl]-3,4-dihydroquinazolin-1(2H)-yl}piperidine-1-carbaldehyde
No. 213: 4-{6-[2-fluoro-1-(fluoromethyl)ethoxy]-2,4-dioxo-3-(4-(pyridin-2-yl)benzyl)-3,4-dihydroquinazolin-1(2H)-yl}piperidine-1-carbaldehyde
No. 215: 4-{6-[2-fluoro-1-(fluoromethyl)ethoxy]-2,4-dioxo-3-(4-(thiophen-2-yl)benzyl)-3,4-dihydroquinazolin-1(2H)-yl}piperidine-1-carbaldehyde
No. 216: 4-{6-[2-fluoro-1-(fluoromethyl)ethoxy]-2,4-dioxo-3-(quinolin-7-ylmethyl)-3,4-dihydroquinazolin-1(2H)-yl}piperidine-1-carbaldehyde
No. 218: 4-{6-[2-fluoro-1-(fluoromethyl)ethoxy]-3-[(6-methoxynaphthalen-2-yl)methyl]-2,4-dioxo-3,4-dihydroquinazolin-1(2H)-yl}piperidine-1-carbaldehyde
No. 223: 4-{3-[4-(1H-benzimidazol-1-yl)benzyl]-6-[2-fluoro-1-(fluoromethyl)ethoxy]-2,4-dioxo-3,4-dihydroquinazolin-1(2H)-yl}piperidine-1-carbaldehyde
No. 224: 4-{6-[2-fluoro-1-(fluoromethyl)ethoxy]-3-[3-methoxy-4-(2-methylpropoxy)benzyl]-2,4-dioxo-3,4-dihydroquinazolin-1(2H)-yl}piperidine-1-carbaldehyde
No. 226: 4-{6-[2-fluoro-1-(fluoromethyl)ethoxy]-3-[3-methoxy-4-(tetrahydrofuran-3-yloxy)benzyl]-2,4-dioxo-3,4-dihydroquinazolin-1(2H)-yl}piperidine-1-carbaldehyde
No. 228: 4-[3-{4-[(1-benzylpyrrolidin-3-yl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 230: 4-[3-(1-benzothiophen-5-ylmethyl)-6-[2-fluoro-1-(fluoromethyl)ethoxy]-2,4-dioxo-3,4-dihydroquinazolin-1(2H)-yl]piperidine-1-carbaldehyde
No. 232: 4-{6-[2-fluoro-1-(fluoromethyl)ethoxy]-3-[3-methoxy-4-(1-methylethoxy)benzyl]-2,4-dioxo-3,4-dihydroquinazolin-1(2H)-yl}piperidine-1-carbaldehyde
No. 233: 4-[3-(3,4-dimethoxybenzyl)-7-fluoro-6-[2-fluoro-1-(fluoromethyl)ethoxy]-2,4-dioxo-3,4-dihydroquinazolin-1(2H)-yl]piperidine-1-carbaldehyde
No. 234: 4-[3-{4-[(1-acetylpyrrolidin-3-yl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 239: 4-[3-{4-[(4-fluorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 240: 4-[3-{4-[(4-chlorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 242: 4-[3-{4-[(3-chlorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 243: 4-{6-[2-fluoro-1-(fluoromethyl)ethoxy]-2,4-dioxo-3-(3-(thiophen-3-yl)benzyl)-3,4-dihydroquinazolin-1(2H)-yl}piperidine-1-carbaldehyde
No. 245: 4-[3-(4-ethoxy-3-methoxybenzyl)-6-(2-hydroxyethoxy)-2,4-dioxo-3,4-dihydroquinazolin-1(2H)-yl]piperidine-1-carbaldehyde
No. 246: 4-[3-{4-[2-(2,3-dihydro-1H-indol-1-yl)-2-oxoethoxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 250: 4-[3-{4-[(3,4-dichlorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 251: 4-{6-[2-fluoro-1-(fluoromethyl)ethoxy]-3-[3-methoxy-4-(2-oxo-2-(piperidin-1-yl)ethoxy)benzyl]-2,4-dioxo-3,4-dihydroquinazolin-1(2H)-yl}piperidine-1-carbaldehyde
No. 254: 4-{3-[3-ethoxy-4-(thiophen-2-ylmethoxy)benzyl]-6-[2-fluoro-1-(fluoromethyl)ethoxy]-2,4-dioxo-3,4-dihydroquinazolin-1(2H)-yl}piperidine-1-carbaldehyde
No. 258: 4-[3-(3,4-dimethoxybenzyl)-6-[2-fluoro-1-(hydroxymethyl)ethoxy]-2,4-dioxo-3,4-dihydroquinazolin-1(2H)-yl]piperidine-1-carbaldehyde
No. 263: (2R)-2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]propanoic acid
No. 264: 4-{6-[2-fluoro-1-(fluoromethyl)ethoxy]-3-[(1-methyl-3-(thiophen-2-yl)-1H-pyrazol-5-yl)methyl]-2,4-dioxo-3,4-dihydroquinazolin-1(2H)-yl}piperidine-1-carbaldehyde
No. 270: 4-{6-[2-fluoro-1-(fluoromethyl)ethoxy]-3-[4-(5-methyl-1,2,4-oxadiazol-3-yl)benzyl]-2,4-dioxo-3,4-dihydroquinazolin-1(2H)-yl}piperidine-1-carbaldehyde
No. 275: 4-{6-[2-fluoro-1-(fluoromethyl)ethoxy]-2,4-dioxo-3-(4-(pyrimidin-5-yl)benzyl)-3,4-dihydroquinazolin-1(2H)-yl}piperidine-1-carbaldehyde
No. 276: 4-{6-[2-fluoro-1-(fluoromethyl)ethoxy]-3-[(1-methyl-3-phenyl-1H-pyrazol-5-yl)methyl]-2,4-dioxo-3,4-dihydroquinazolin-1(2H)-yl}piperidine-1-carbaldehyde
No. 278: 4-{6-[2-fluoro-1-(fluoromethyl)ethoxy]-2,4-dioxo-3-{[6-(1H-pyrazol-1-yl)pyridin-3-yl]methyl}-3,4-dihydroquinazolin-1(2H)-yl}piperidine-1-carbaldehyde
No. 279: 4-{6-[2-fluoro-1-(fluoromethyl)ethoxy]-2,4-dioxo-3-[(2-(thiophen-2-yl)pyrimidin-5-yl)methyl]-3,4-dihydroquinazolin-1(2H)-yl}piperidine-1-carbaldehyde
No. 280: 4-{6-[2-fluoro-1-(fluoromethyl)ethoxy]-3-[4-(1-methyl-1H-pyrazol-3-yl)benzyl]-2,4-dioxo-3,4-dihydroquinazolin-1(2H)-yl}piperidine-1-carbaldehyde
No. 282: 4-{6-[2-fluoro-1-(fluoromethyl)ethoxy]-3-[4-(3-methyl-1,2,4-oxadiazol-5-yl)benzyl]-2,4-dioxo-3,4-dihydroquinazolin-1(2H)-yl}piperidine-1-carbaldehyde
No. 283: [2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]acetic acid
No. 285: 4-{6-[2-fluoro-1-(fluoromethyl)ethoxy]-2,4-dioxo-3-(thieno[2,3-b]pyridin-2-ylmethyl)-3,4-dihydroquinazolin-1(2H)-yl}piperidine-1-carbaldehyde
No. 286: 4-{6-[2-fluoro-1-(fluoromethyl)ethoxy]-2,4-dioxo-3-[(6-phenylpyridin-3-yl)methyl]-3,4-dihydroquinazolin-1(2H)-yl}piperidine-1-carbaldehyde
No. 287: 4-{6-[2-fluoro-1-(fluoromethyl)ethoxy]-3-[(6-(morpholin-4-yl)pyridin-3-yl)methyl]-2,4-dioxo-3,4-dihydroquinazolin-1(2H)-yl}piperidine-1-carbaldehyde
No. 289: 4-{6-[2-fluoro-1-(fluoromethyl)ethoxy]-2,4-dioxo-3-[(6-(thiophen-2-yl)pyridin-3-yl)methyl]-3,4-dihydroquinazolin-1(2H)-yl}piperidine-1-carbaldehyde
No. 292: 4-{6-[2-fluoro-1-(fluoromethyl)ethoxy]-3-[(1-methyl-5-phenyl-1H-pyrazol-3-yl)methyl]-2,4-dioxo-3,4-dihydroquinazolin-1(2H)-yl}piperidine-1-carbaldehyde
No. 294: 4-({6-[2-fluoro-1-(fluoromethyl)ethoxy]-1-(1-formylpiperidin-4-yl)-2,4-dioxo-1,4-dihydroquinazolin-3(2H)-yl}methyl)biphenyl-2-carbonitrile
No. 295: (2R)-2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]-N-methylpropanamide
No. 297: 4-{7-fluoro-6-[2-fluoro-1-(fluoromethyl)ethoxy]-2,4-dioxo-3-(4-(thiophen-2-yl)benzyl)-3,4-dihydroquinazolin-1(2H)-yl}piperidine-1-carbaldehyde
No. 298: 4-{6-[2-fluoro-1-(fluoromethyl)ethoxy]-3-[3-methoxy-4-(morpholin-4-ylmethyl)benzyl]-2,4-dioxo-3,4-dihydroquinazolin-1(2H)-yl}piperidine-1-carbaldehyde
No. 299: 4-{6-[2-fluoro-1-(fluoromethyl)ethoxy]-3-[3-methoxy-4-(piperidin-1-ylmethyl)benzyl]-2,4-dioxo-3,4-dihydroquinazolin-1(2H)-yl}piperidine-1-carbaldehyde
No. 300: 4-[3-{4-[(3,4-dichlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 301: 2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]-N-ethylacetamide
No. 302: (2S)-2-[2-(cyclopentyloxy)-5-({6-[2-fluoro-1-(fluoromethyl)ethoxy]-1-(1-formylpiperidin-4-yl)-2,4-dioxo-1,4-dihydroquinazolin-3(2H)-yl}methyl)phenoxy]propanoic acid
No. 305: 4-{6-[2-fluoro-1-(fluoromethyl)ethoxy]-3-(3-methoxy-4-{[(3R)-2-oxo-1-phenylpyrrolidin-3-yl]oxy}benzyl)-2,4-dioxo-3,4-dihydroquinazolin-1(2H)-yl}piperidine-1-carbaldehyde
No. 306: 4-{3-[4-(cyclobutylmethoxy)-3-methoxybenzyl]-6-[2-fluoro-1-(fluoromethyl)ethoxy]-2,4-dioxo-3,4-dihydroquinazolin-1(2H)-yl}piperidine-1-carbaldehyde
No. 307: 4-{3-[4-(benzyloxy)-3-methoxybenzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 308: 4-{7-fluoro-6-[2-fluoro-1-(fluoromethyl)ethoxy]-3-(4-hydroxy-3-methoxybenzyl)-2,4-dioxo-3,4-dihydroquinazolin-1(2H)-yl}piperidine-1-carbaldehyde
No. 309: 4-{3-[4-(cyclopropylmethoxy)-3-methoxybenzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 310: 4-{7-fluoro-6-[2-fluoro-1-(fluoromethyl)ethoxy]-3-[3-methoxy-4-(2-methylpropoxy)benzyl]-2,4-dioxo-3,4-dihydroquinazolin-1(2H)-yl}piperidine-1-carbaldehyde
No. 311: 4-{7-fluoro-6-[2-fluoro-1-(fluoromethyl)ethoxy]-3-[3-methoxy-4-(1-methylethoxy)benzyl]-2,4-dioxo-3,4-dihydroquinazolin-1(2H)-yl}piperidine-1-carbaldehyde
No. 312: 4-[3-(4-ethoxy-3-methoxybenzyl)-7-fluoro-6-[2-fluoro-1-(fluoromethyl)ethoxy]-2,4-dioxo-3,4-dihydroquinazolin-1(2H)-yl]piperidine-1-carbaldehyde
No. 315: 4-{7-fluoro-6-[2-fluoro-1-(fluoromethyl)ethoxy]-3-{[6-(3-methoxyphenyl)pyridin-3-yl]methyl}-2,4-dioxo-3,4-dihydroquinazolin-1(2H)-yl}piperidine-1-carbaldehyde
No. 316: 4-{7-fluoro-6-[2-fluoro-1-(fluoromethyl)ethoxy]-3-{[6-(2-fluorophenyl)pyridin-3-yl]methyl}-2,4-dioxo-3,4-dihydroquinazolin-1(2H)-yl}piperidine-1-carbaldehyde
No. 317: 4-{7-fluoro-6-[2-fluoro-1-(fluoromethyl)ethoxy]-3-{[6-(4-fluorophenyl)pyridin-3-yl]methyl}-2,4-dioxo-3,4-dihydroquinazolin-1(2H)-yl}piperidine-1-carbaldehyde
No. 318: 4-{7-fluoro-6-[2-fluoro-1-(fluoromethyl)ethoxy]-3-{[6-(4-methoxyphenyl)pyridin-3-yl]methyl}-2,4-dioxo-3,4-dihydroquinazolin-1(2H)-yl}piperidine-1-carbaldehyde
No. 319: 4-{7-fluoro-6-[2-fluoro-1-(fluoromethyl)ethoxy]-2,4-dioxo-3-[(6-(thiophen-2-yl)pyridin-3-yl)methyl]-3,4-dihydroquinazolin-1(2H)-yl}piperidine-1-carbaldehyde
No. 320: 4-{3-[3-ethoxy-4-(thiophen-2-ylmethoxy)benzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 321: 4-{7-fluoro-6-[2-fluoro-1-(fluoromethyl)ethoxy]-3-[4-(1-methyl-1H-pyrazol-3-yl)benzyl]-2,4-dioxo-3,4-dihydroquinazolin-1(2H)-yl}piperidine-1-carbaldehyde
No. 322: 4-{7-fluoro-6-[2-fluoro-1-(fluoromethyl)ethoxy]-2,4-dioxo-3-(4-(pyrimidin-5-yl)benzyl)-3,4-dihydroquinazolin-1(2H)-yl}piperidine-1-carbaldehyde
No. 323: 4-{7-fluoro-6-[2-fluoro-1-(fluoromethyl)ethoxy]-3-[(1-methyl-3-(thiophen-2-yl)-1H-pyrazol-5-yl)methyl]-2,4-dioxo-3,4-dihydroquinazolin-1(2H)-yl}piperidine-1-carbaldehyde
No. 324: 4-{7-fluoro-6-[2-fluoro-1-(fluoromethyl)ethoxy]-3-[3-methoxy-4-(2-oxo-2-(piperidin-1-yl)ethoxy)benzyl]-2,4-dioxo-3,4-dihydroquinazolin-1(2H)-yl}piperidine-1-carbaldehyde
No. 325: 4-[3-{4-[2-(2,3-dihydro-1H-indol-1-yl)-2-oxoethoxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 326: 4-{7-fluoro-6-[2-fluoro-1-(fluoromethyl)ethoxy]-3-[4-(5-methyl-1,2,4-oxadiazol-3-yl)benzyl]-2,4-dioxo-3,4-dihydroquinazolin-1(2H)-yl}piperidine-1-carbaldehyde
No. 327: 4-[3-{4-[(3-chlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 328: 4-[3-{[6-(3,5-dichlorophenyl)pyridin-3-yl]methyl}-6-[2-fluoro-1-(fluoromethyl)ethoxy]-2,4-dioxo-3,4-dihydroquinazolin-1(2H)-yl]piperidine-1-carbaldehyde
No. 329: 4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)biphenyl-2-carbonitrile
No. 330: 4-{7-fluoro-6-[2-fluoro-1-(fluoromethyl)ethoxy]-2,4-dioxo-3-[4-(1H-pyrazol-1-yl)benzyl]-3,4-dihydroquinazolin-1(2H)-yl}piperidine-1-carbaldehyde
No. 331: 4-{7-fluoro-6-[2-fluoro-1-(fluoromethyl)ethoxy]-3-{[6-(3-fluorophenyl)pyridin-3-yl]methyl}-2,4-dioxo-3,4-dihydroquinazolin-1(2H)-yl}piperidine-1-carbaldehyde
No. 332: 3-[5-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)pyridin-2-yl]benzonitrile
No. 333: 4-[3-(3,4-diethoxybenzyl)-7-fluoro-6-[2-fluoro-1-(fluoromethyl)ethoxy]-2,4-dioxo-3,4-dihydroquinazolin-1(2H)-yl]piperidine-1-carbaldehyde
No. 334: 4-[3-{4-[(4-chlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 335: 4-{7-fluoro-6-[2-fluoro-1-(fluoromethyl)ethoxy]-3-[4-(morpholin-4-ylmethyl)benzyl]-2,4-dioxo-3,4-dihydroquinazolin-1(2H)-yl}piperidine-1-carbaldehyde
No. 336: 4-{7-fluoro-6-[2-fluoro-1-(fluoromethyl)ethoxy]-2,4-dioxo-3-{[6-(1H-pyrazol-1-yl)pyridin-3-yl]methyl}-3,4-dihydroquinazolin-1(2H)-yl}piperidine-1-carbaldehyde
No. 337: 4-{7-fluoro-6-[2-fluoro-1-(fluoromethyl)ethoxy]-3-(4-(morpholin-4-yl)benzyl)-2,4-dioxo-3,4-dihydroquinazolin-1(2H)-yl}piperidine-1-carbaldehyde
No. 338: 4-{7-fluoro-6-[2-fluoro-1-(fluoromethyl)ethoxy]-3-(3-methoxy-4-propoxybenzyl)-2,4-dioxo-3,4-dihydroquinazolin-1(2H)-yl}piperidine-1-carbaldehyde
No. 339: 4-{3-[4-(1H-benzimidazol-1-yl)benzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 340: 5-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)-2-methoxybenzonitrile
No. 341: 3-(3,4-dimethoxybenzyl)-6-[2-fluoro-1-(fluoromethyl)ethoxy]-1-(1-formylpiperidin-4-yl)-2,4-dioxo-1,2,3,4-tetrahydroquinazoline-7-carbonitrile
No. 342: 4-[3-(4-bromobenzyl)-7-fluoro-6-[2-fluoro-1-(fluoromethyl)ethoxy]-2,4-dioxo-3,4-dihydroquinazolin-1(2H)-yl]piperidine-1-carbaldehyde
No. 343: 4-[3-{4-[(3,4-dichlorobenzyl)oxy]-3-(2-methoxyethoxy)benzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 344: 4-{3-[4-(benzyloxy)benzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 345: 4-[3-{4-[(3,4-dichlorobenzyl)oxy]-3-ethoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 349: 4-[3-{4-[(3,4-dichlorobenzyl)oxy]-3-(2-fluoroethoxy)benzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 350: 4-[3-{4-[(2-chloro-4-fluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 351: 4-[3-{4-[(2,4-dichlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 352: 4-[3-{4-[(2-chloro-6-fluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 353: 4-[3-{4-[(2,6-dichlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 354: 4-[3-{4-[(2-chlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 355: 4-[7-fluoro-3-{4-[(2-fluorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 357: 2-[(3,4-dichlorobenzyl)oxy]-5-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)benzonitrile
No. 358: 4-[3-{4-[(3,4-dichlorophenoxy)methyl]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 360: 4-{7-fluoro-6-[2-fluoro-1-(fluoromethyl)ethoxy]-2,4-dioxo-3-[4-(2-phenylethyl)benzyl]-3,4-dihydroquinazolin-1(2H)-yl}piperidine-1-carbaldehyde
No. 362: 4-[3-{4-[(4,5-dichloro-2-fluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 369: 4-[3-{4-[(4-chlorophenoxy)methyl]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 371: 4-[3-{3-chloro-4-[(4-chlorobenzyl)oxy]-5-ethoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 373: 4-[3-{3-chloro-4-[(2,4-dichlorobenzyl)oxy]-5-ethoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 375: 4-[7-fluoro-3-{4-[(4-fluorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 376: 4-[3-{4-[(3,5-dichlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 377: 4-[3-(4-{[4-chloro-3-(trifluoromethyl)benzyl]oxy}-3-methoxybenzyl)-7-fluoro-6-[2-fluoro-1-(fluoromethyl)ethoxy]-2,4-dioxo-3,4-dihydroquinazolin-1(2H)-yl]piperidine-1-carbaldehyde
No. 379: 4-[3-{4-[(3-chlorophenoxy)methyl]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 380: 4-[3-{4-[(3,5-difluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 381: 4-{3-[4-(benzyloxy)-3-methoxybenzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 382: 4-[3-{4-[(3-chloro-5-fluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 383: 4-{7-fluoro-6-[2-fluoro-1-(fluoromethyl)ethoxy]-3-(3-methoxy-4-{[4-(trifluoromethyl)benzyl]oxy}benzyl)-2,4-dioxo-3,4-dihydroquinazolin-1(2H)-yl}piperidine-1-carbaldehyde
No. 384: 4-[3-{4-[(2,5-dichlorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 385: 4-{[4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)-2-methoxyphenoxy]methyl}benzonitrile
No. 386: 3-{[4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)-2-methoxyphenoxy]methyl}benzonitrile
No. 387: 4-[3-{4-[(4-chloro-2-fluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 388: 4-[3-{4-[1-(3,4-dichlorophenyl)ethoxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 389: 4-{7-fluoro-6-[2-fluoro-1-(fluoromethyl)ethoxy]-3-{4-[(3-hydroxybenzyl)oxy]-3-methoxybenzyl}-2,4-dioxo-3,4-dihydroquinazolin-1(2H)-yl}piperidine-1-carbaldehyde
No. 390: 4-[7-fluoro-3-{4-[(3-fluorobenzyl)oxy]-3-methoxybenzyl}-6-[2-fluoro-1-(fluoromethyl)ethoxy]-2,4-dioxo-3,4-dihydroquinazolin-1(2H)-yl]piperidine-1-carbaldehyde
No. 391: 4-[3-{4-[(3,4-difluorobenzyl)oxy]-3-methoxybenzyl}-7-fluoro-6-[2-fluoro-1-(fluoromethyl)ethoxy]-2,4-dioxo-3,4-dihydroquinazolin-1(2H)-yl]piperidine-1-carbaldehyde
No. 392: 4-{3-[4-(5,6-dichloro-1H-benzimidazol-1-yl)-3-methoxybenzyl]-7-fluoro-6-[2-fluoro-1-(fluoromethyl)ethoxy]-2,4-dioxo-3,4-dihydroquinazolin-1(2H)-yl}piperidine-1-carbaldehyde
No. 393: 4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)phenyl 3,4-dichlorobenzenesulphonate
No. 394: 4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)-2-methoxyphenyl 3,4-dichlorobenzenesulphonate
No. 403: 3,4-dichloro-N-[4-({7-fluoro-6-[2-fluoro-1-(fluoromethyl)ethoxy]-1-(1-formylpiperidin-4-yl)-2,4-dioxo-1,4-dihydroquinazolin-3(2H)-yl}methyl)-2-methoxyphenyl]benzamide

12. Process for the preparation of a compound according to any one of Claims 1 to 11, **characterized in that** a compound of formula 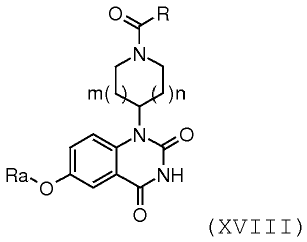 in which R, Ra, m and n are as defined in Claim 1 and in which there may additionally be an R₂ group, as defined for the compounds of formula (I), in the 7 position of the quinazolinedione structure, is reacted with a compound of formula 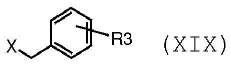 or 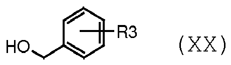 by an alkylation or Mitsunobu reaction respectively.

13. Compound of general formula (XVIII) 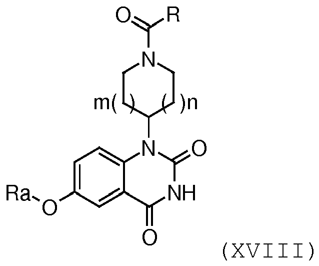 in which R, Ra, m and n are as defined in Claim 1 and in which there may additionally be an R₂ group, as defined for the compounds of formula (I), in the 7 position of the quinazolinedione structure.

14. Compound of general formula (XVIII) according to Claim 12 chosen from compound No. 32, 4-[6-(2,2-difluoroethoxy)-2,4-dioxo-3,4-dihydroquinazolin-1(2H)-yl]piperidine-1-carbaldehyde, and compound No. 55, 4-{6-[2-fluoro-1-(fluoromethyl)ethoxy]-2,4-dioxo-3,4-dihydroquinazolin-1(2H)-yl}piperidine-1-carbaldehyde ; in the base, hydrate or solvate form or in the form of their mixtures.

15. Medicament, **characterized in that** it comprises at least one compound of formula (I) according to any one of Claims 1 to 11.

16. Pharmaceutical composition, **characterized in that** it comprises at least one compound of formula (I) according to any one of Claims 1 to 11 and also at least one pharmaceutically acceptable excipient.

17. Use of a compound of formula (I) according to any one of Claims 1 to 11 in the preparation of a medicament intended for the treatment and/or prevention of inflammatory or immunoinflammatory diseases, including asthma, chronic obstructive pulmonary disease (COPD), allergic rhinitis, allergies, Crohn's disease, ulcerative colitis, myasthenia gravis, atopic dermatitis, psoriasis, lupus erythematosus disseminatus, rheumatoid arthritis, diabetes or multiple sclerosis, for the treatment of organ transplants, for the treatment and/or prevention of cancer, in particular osteosarcoma or adenocarcinoma, for the treatment and/or prevention of bone diseases, including osteopenia and osteoporosis, for the treatment and/or prevention of acute insufficiency, and for the treatment and/or prevention of pain, neuropathic pain and visceral pain.

18. Use of a compound of formula (I) according to Claim 17 in the preparation of a medicament intended for the treatment and/or prevention of neuropathic pain.
